(19) Europäisches Patentamt
European Patent Office
Office européen des brevets

(11) **EP 1 748 074 B1**

(12) **EUROPEAN PATENT SPECIFICATION**

(45) Date of publication and mention of the grant of the patent:
**16.11.2011 Bulletin 2011/46**

(21) Application number: **06014355.9**

(22) Date of filing: **15.01.2004**

(51) Int Cl.:
***C12N 15/54*** *(2006.01)* ***C12N 15/55*** *(2006.01)*
***C12N 9/10*** *(2006.01)* ***C12N 9/18*** *(2006.01)*
***A23L 1/03*** *(2006.01)* ***A23L 1/035*** *(2006.01)*

(54) **Method for the in situ production of an emulsifier in a foodstuff**

Verfahren zur in-situ-Herstellung eines Emulgators in einem Nahrungsmittel

Procédé pour la production in situ d'un émulsifiant dans un aliment

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority:
**17.01.2003 GB 0301121**
**17.01.2003 GB 0301122**
**17.01.2003 GB 0301117**
**17.01.2003 GB 0301120**
**17.01.2003 GB 0301119**
**17.01.2003 GB 0301118**
**23.07.2003 US 489441 P**
**24.12.2003 GB 0330016**

(43) Date of publication of application:
**31.01.2007 Bulletin 2007/05**

(83) **Declaration under Rule 32(1) EPC (expert solution)**

(62) Document number(s) of the earlier application(s) in accordance with Art. 76 EPC:
**04702393.2 / 1 619 961**

(73) Proprietor: **DANISCO A/S**
**1001 Copenhagen K. (DK)**

(72) Inventors:
- **de Kreij, Arno**
  **CH-1005 Lausanne (CH)**
- **Madrid, Susan Mampusta**
  **Trørød**
  **2950 Vedbaek (DK)**
- **Mikkelsen, Jørn Dalgaard**
  **2650 Hividovre (DK)**
- **Søe, Jørn Borch**
  **8381 Tilst (DK)**

(74) Representative: **Williams, Aylsa et al**
**D Young & Co LLP**
**120 Holborn**
**London EC1N 2DY (GB)**

(56) References cited:
**EP-A- 1 275 711** **WO-A1-00/05396**
**WO-A1-91/06661** **US-A- 6 066 482**

- **HILTON S ET AL: "PURIFICATION AND SPECTRAL STUDY OF A MICROBIAL FATTY ACYLTRANSFERASE ACTIVATION BY LIMITED PROTEOLYSIS" BIOCHEMISTRY, vol. 29, no. 38, 1990, pages 9072-9078, XP002337706 ISSN: 0006-2960 -& DATABASE UniProt [Online] 1 July 1989 (1989-07-01), "Phosphatidylcholine-sterol acyltransferase precursor (EC 2.3.1.43) (Glycerophospholipid-cholesterol acyltransferase) (GCAT)." XP002337710 retrieved from EBI accession no. UNIPROT: GCAT_AERHY Database accession no. P10480**
- **DATABASE UniProt [Online] 1 March 2001 (2001-03-01), "Glycerophospholipid-cholesterol acyltransferase." XP002337712 retrieved from EBI accession no. UNIPROT:Q9F7Y6 Database accession no. Q9F7Y6**
- **UPTON C ET AL: "A new family of lipolytic enzymes?" TIBS TRENDS IN BIOCHEMICAL SCIENCES, ELSEVIER PUBLICATION, CAMBRIDGE, EN, vol. 20, no. 5, May 1995 (1995-05), pages 178-179, XP004222260 ISSN: 0968-0004**
- **BRUMLIK MICHAEL J ET AL: "Identification of the catalytic triad of the lipase/acyltransferase from Aeromonas hydrophila" JOURNAL OF BACTERIOLOGY, vol. 178, no. 7, 1996, pages 2060-2064, XP002315734 ISSN: 0021-9193**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **NERLAND A H: "The nucleotide sequence of the gene encoding GCAT from Aeromonas salmonicida ssp. salmonicida" JOURNAL OF FISH DISEASES, vol. 19, no. 2, 1996, pages 145-150, XP008049669 ISSN: 0140-7775 -& DATABASE UniProt [Online] 1 November 1996 (1996-11-01), "Glycerophospholipid-cholesterol acyltransferase precursor." XP002337711 retrieved from EBI accession no. UNIPROT: Q44268 Database accession no. Q44268**
- **NEUGNOT VIRGINIE ET AL: "The lipase/ acyltransferase from Candida parapsilosis. Molecular cloning and characterization of purified recombinant enzymes" EUROPEAN JOURNAL OF BIOCHEMISTRY, vol. 269, no. 6, March 2002 (2002-03), pages 1734-1745, XP002320424 ISSN: 0014-2956**
- **"USE OF LIPOLYTIC ENZYME FROM AEROMONAS IN DETERGENTS" RESEARCH DISCLOSURE, KENNETH MASON PUBLICATIONS, WESTBOURNE, GB, no. 390, October 1996 (1996-10), pages 661-662, XP000639927 ISSN: 0374-4353**


Remarks:

The file contains technical information submitted after the application was filed and not included in this specification

## Description

FIELD OF INVENTION

The present invention relates to the use of a lipid acyltransferase to prepare from a water containing food material comprising 10-98% water an egg, egg-based or dairy product comprising an emulsifier.

The present invention further relates to a method of production of an egg, egg-based or dairy product comprising an emulsifier and a water containing foodstuff obtainable by said method.

TECHNICAL BACKGROUND

The beneficial use of phospholipases and lipases (referred to as lipolytic enzymes, (EC. 3.1.1.x) used in food and/or feed industrial applications has been known for many years.

For instance, in EP 0 585 988 it is claimed that lipase addition to dough resulted in an improvement in the antistaling effect. It is suggested that a lipase obtained from *Rhizopus arrhizus* when added to dough can improve the quality of the resultant bread when used in combination with shortening/fat. WO94/04035 teaches that an improved softness can be obtained by adding a lipase to dough without the addition of any additional fat/oil to the dough. Castello, P. ESEGP 89-10 Dec. 1999 Helsinki, shows that exogenous lipases can modify bread volume.

Lipolytic enzymes hydrolyse one or more of the fatty acids from lipids present in the food which can result in the formation of powerful emulsifier molecules within the foodstuff which provide commercially valuable functionality. The molecules which contribute the most significant emulsifier characteristics are the partial hydrolysis products, such as lyso-phospholipids, lyso-glycolipids, and mono-glyceride molecules. The polar lipid hydrolysis products, such as lyso-phospholipids and lyso-glycolipids are particularly advantageous. In bread making, such *in situ* derived emulsifiers can give equivalent functionality as emulsifiers, such as DATEM.

However, the activity of lipolytic enzymes also results in accumulation of free fatty acids, which can lead to detrimental functionality in the foodstuff. This inherent activity of lipolytic enzymes limits their functionality.

Numerous solutions to this problem have been attempted in the art. However, these result in a significant increase in free fatty acids in the foodstuff, particularly food stuffs with high water content, including, but not limited to bread doughs and egg yolk.

Phospholipases, particularly phospholipase A2 (E.C. 3.1.1.4), have been used for many years for the treatment of egg or egg-based products (see US 4,034,124 and Dutihl & Groger 1981 J. Sci. Food Agric. 32, 451-458 for example). The phospholipase activity during the treatment of egg or egg-based products results in the accumulation of polar lysolecithin, which can act as an emulsifier. Phospholipase treatment of egg or egg-based products can improve the stability, thermal stability under heat treatment such as pasteurisation and result in substantial thickening. Egg-based products may include, but are not limited to cake, mayonnaise, salad dressings, sauces, ice creams and the like. Use of phospholipases results in the accumulation of free fatty acids. The accumulation of free fatty acids can result in significant off-flavour. In addition, the accumulation of free fatty acids can result in enhanced susceptibility to oxidation, and hence result in poor shelf-life, product discoloration and alteration of other critical food characteristics such as flavour and texture. Recently, lipolytic enzymes with broader substrate specificity have been marketed for treatment of egg yolk and related food products. These have the advantage that, unlike most of the phospholipase A2s, they do not originate from a mammalian source. However, they result in significant accumulation of free fatty acids, not only due to the hydrolysis of phospholipids, but also triglycerides.

As mentioned above, another area where lipases have been extensively used is in the bakery industry. The use of phospholipases in baking dates bake to the early 1980s. The substrate for lipases in wheat flour is 1.5-3% endogenous wheat lipids, which are a complex mixture of polar and non-polar lipids. The polar lipids can be divided into glycolipids and phospholipids. These lipids are built up of glycerol esterified with two fatty acids and a polar group. The polar group contributes to surface activity of these lipids. Enzymatic cleavage of one of the fatty acids in these lipids leads to lipids with a much higher surface activity. It is well known that emulsifiers, such as DATEM, with high surface activity are very functional when added to dough.

However, the use of lipases (E.C. 3.1.1.X) in dough products may have a detrimental impact on yeast activity, and/or a negative effect on bread volume. The negative effect on bread volume is often explained by overdosing. Overdosing can lead to a decrease in gluten elasticity which results in a dough which is too stiff and thus results in reduced bread volumes. In addition, or alternatively, such lipases can degrade shortening, oil or milk fat added to the dough, resulting in off-flavour in the dough and baked product. Overdosing and off flavour have been attributed to the accumulation of free fatty acids in the dough.

In EP 1 193 314, EP 0 977 869 and also in WO 01/39602, the use of lipolytic enzymes active on glycolipids was reported to be particularly beneficial in application in bread making as the partial hydrolysis products the lyso-glycolipids were found to have very high emulsifier functionality, apparently resulting in a higher proportion of positive emulsifier functionality compared to the detrimental accumulation of free fatty acids. However, the enzymes were also found to have significant non selective activity on triglyceride which resulted in unnecessarily high free fatty acid.

The same finding was reported in WO 00/32758, which disclosed lipolytic enzyme variants with enhanced activity on phospholipids and/or glycolipids, in addition to variants which had a preference for long rather than short chain fatty acids. This latter feature, also disclosed in WO 01/39602, was deemed of particular importance to prevent the off-flavours associated with the accumulation of free short chain fatty acids. However, significant free fatty acids are produced.

The problem of high triglyceride activity was addressed in WO02/094123, where the use of lipolytic enzymes active on the polar lipids (i.e. glycolipids and phospholipids) in a dough, but substantially not active on triglycerides or 1-mono-glycerides is taught. However, significant free fatty acids are produced.

Some lipolytic enzymes have low or no activity on the lyso form of polar lipids (e.g. glycolipids/phospholipids). The use of such enzymes has been deemed preferable as they ensure the accumulation of the highly polar lyso-lipids, resulting in optimal functionality. Free fatty acids do however accumulate. This selective functionality is characteristic of phospholipase A2 enzymes, and the glycolipases disclosed in EP 0 977 869, EP 1 193 314, and WO01/39602. Variant enzymes of less selective lipolytic enzymes have been produced which have a lower activity on the lyso-polar lipids when compared to the parent enzyme (WO03/060112). However, significant free fatty acids are produced.

WO00/05396 teaches a process for preparing a foodstuff comprising an emulsifier, wherein food material is contacted with an enzyme such that an emulsifier is generated by the enzyme from a fatty acid ester and a second functional ingredient is generated from a second constituent. WO00/05396 teaches the use of in particular a lipase or esterase enzyme. Nowhere in WO00/05396 is the specific use of a lipid acyltransferase taught. In addition, in foodstuffs with high water content, the use of the esterases and lipases as taught in WO00/05396 would result in significant accumulation of free fatty acids.

A disadvantage associated with the use of lipases, including phospholipases and glycolipases, may be caused by the build-up of free fatty acids released from the lipids. Over the past couple of decades the use of lipolytic enzymes in foodstuffs has been limited due to the balance between the detrimental accumulation of free fatty acids and the production of the lyso-lipids which provide positive functionality. Although numerous strategies in the art have been attempted, some of which provided significant improvements in functionality, none have completely addressed and solved the fundamental problem in the art, i.e. the significant accumulation of free fatty acids in foodstuffs prepared using lipolytic enzymes *in situ.*

The presence of high levels of free fatty acids (FFA) in raw materials or food products is generally recognised as a quality defect and food processors and customers will usually include a maximum FFA level in the food specifications. The resulting effects of excess FFA levels can be in organoleptic and/or functional defects.

A result of lipolysis is hydrolytic rancidity, with the formation of characteristic "soapy" flavour. This "soapy" taste is especially acute with fatty acids of intermediate chain length (C8-C12) which, although not present in high concentrations, may be important constituents of, for example, dairy products or vegetable oils. A more common organoleptic defect is due to the combined effects of lipolytic enzymes and oxidation processes. Unsaturated fatty acids are more susceptible to enzymatic oxidation when unesterified than when esterified in acyl lipids.

Functional defects in food due to high FFA levels are recognised, but less readily explained. Without wishing to be bound by theory, the hydrolysis of unchanged lipids to carboxylic acids will increase [H+] and produce carbonyl groups that can combine with other compounds or metal ions. Free fatty acids also combine proteins by hydrophobic interactions and can complex with starch during cooking. FFA may also interfere with the action of surface-active agents, such as polar lipids and emulsifiers. (Lipid in Cereal Technology, P.J. Barnes, Academic Press 1983.)

WO03/100044 discloses a class of acyl transferases known as PDATs (or ATWAX). These enzymes use a monoglyceride or a diglyceride as the acceptor molecule, and phosphatidylcholine (PC) as the donor molecule to produce the following products: lyso phosphatidylcholine and triacylglycerol and/or diacylglycerol.

In one embodiment, the present invention relates to improvements in the incorporation of whey proteins into food products, providing for improved yields without impairing the qualities-such as the texture - of the food compositions and products.

Cheese compositions are typically prepared from dairy liquids by processes that include treating the liquid with a coagulating or clotting agent. The coagulating agent may be a curding enzyme, an acid or a suitable bacterial culture, or it may include such a culture. The curd that results generally incorporates transformed casein, fats including natural butter fat, and flavourings that arise especially when a bacterial culture is used. The curd may be separated from the liquid whey, which contains soluble proteins not affected by the coagulation and that therefore are not incorporate into the curd.

Whey is thus a by-product of manufacturing in commercial processes that produce food products - such as cheeses. Traditionally, whey is disposed of as unused waste or used as fertiliser or animal feed or processed into a food ingredient.

The inability of whey proteins to be substantially retained in the curd is an important factor contributing to a lack of efficiency in the conventional production of dairy products - such as cheese curds - and to a reduction in overall yield relating to the incorporation of all the protein solids that are present in the starting dairy liquids into resulting cheese curds.

There have been numerous attempts to include whey proteins in cheese e.g. by heat treatment of the milk, heat treatment of whey, or by filtration - such as ultrafiltration.

There are also several descriptions of the use of specific proteases to induce aggregation of whey proteins. A serine protease derived from *Bacillus licheniformis* has been shown to have the ability to induce aggregation of whey proteins (US 5,523,237).

However, there remains many difficulties associated with adding whey proteins in processes such as the manufacture of cheeses. For example, incorporation of whey protein into cheeses is associated with a deterioration in the taste and mouth-feel of the product, and furthermore tends to interfere with curding and subsequent processing of the product. Proteases that have been previously reported that can be added to cheese milk for hydrolysis of whey proteins result in significant hydrolysis of the caseins as described by Madsen,J.S. & Qvist,K.B. (1997) Hydrolysis of milk protein by a Bacillus licheniformis protease specific for acidic amino acid residues. J. Food Sci. 62, 579-582.

Thus, there is a need in the art for methods and compositions that provide for the improved incorporation of whey protein into food products while maintaining organoleptic and other desirable properties. Such optimisation would result in increased efficiency, higher yields of food products, and reduced overall material costs.

Lipase:cholesterol acyltransferases have been known for some time (see for example Buckley - Biochemistry 1983, 22, 5490-5493). In particular, glycerophospholipid:cholesterol acyl transferases (GCATs) have been found, which like the plant and/or mammalian lecithin:cholesterol acyltransferases (LCATs), will catalyse fatty acid transfer between phosphatidylcholine and cholesterol.

Upton and Buckley (TIBS 20, May 1995 p 178-179) and Brumlik and Buckley (J. of Bacteriology Apr. 1996 p 2060-2064) teach a lipase/acyltransferase from *Aeromonas hydrophila* which has the ability to carry out acyl transfer to alcohol acceptors in aqueous media.

Milton et al (Biochemistry, 1990, 29, 9072-9078) also teach purification of a fatty acetyltransferase from *Aeromonas salmonicida* containing the cloned *A. hydrophila* structural gene.

Furthermore, the *A. salmonicida* actyltransferase sequence was also entered in to the EBI UNIPROT database on 1 March 2001 under the accession no. Q9F7Y6.

## SUMMARY ASPECTS OF THE PRESENT INVENTION

According to a first aspect of the present invention there is provided use of a lipid acyltransferase to prepare from a water containing food material comprising 10-98% water a foodstuff selected from egg or an egg-based product or a dairy product comprising an emulsifier, wherein the emulsifier is generated from constituents of the food material by the lipid acyltransferase, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphatidylcholine mixture to a Wheaton glass, adding 15ml 50mM HEPES buffer pH 7 and dispersing the lipid in the buffer during agitation;
iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25μl 4M HCl to acidify the free fatty acid;
v) adding 3ml chloroform and shaking vigorously for 30 seconds, centrifuging and isolating 2ml of the chloroform phase, filtering through a 0.45μm filter into a 10ml tared Dram glass;
vii) evaporating the chloroform under a stream of nitrogen at 60°C, and scaling the samples;
viii) analysing the extracted lipid by GLC.

1. A method of production of egg or an egg-based product or a dairy product comprising an emulsifier, wherein the method comprises the step of adding a lipid acyltransferase to egg or an egg-based product or a dairy product containing 10-98% water, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphatidylcholine mixture to a Wheaton glass, adding 15ml 50mM HEPES buffer pH 7 and dispersing the lipid in the buffer during agitation;

iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25μl 4M HCl to acidify the free fatty acid;
v) adding 3ml chloroform and shaking vigorously for 30 seconds, centrifuging and isolating 2ml of the chloroform phase, filtering through a 0.45μm filter into a 10ml tared Dram glass;
vii) evaporating the chloroform under a stream of nitrogen at 60°C, and scaling the samples;
viii) analysing the extracted lipid by GLC.

According to a further aspect, the present invention provides a water containing foodstuff comprising 10-98% water obtainable by the method of the present invention, wherein the foodstuff comprises a lipid acyltransferase and an emulsifier, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphatidylcholine mixture to a Wheaton glass, adding 15ml 50mM HEPES buffer pH 7 and dispersing the lipid in the buffer during agitation;
iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25μl 4M HCl to acidify the free fatty acid;
v) adding 3ml chloroform and shaking vigorously for 30 seconds, centrifuging and isolating 2ml of the chloroform phase, filtering through a 0.45μm filter into a 10ml tared Dram glass;
vii) evaporating the chloroform under a stream of nitrogen at 60°C, and scaling the samples;
viii) analysing the extracted lipid by GLC.

The following methods, uses, foodstuffs and lipid acyltransferases are also disclosed herein.

A method of *in situ* production of an emulsifier in a foodstuff, wherein the method is such that the emulsifier is produced without increasing or without substantially increasing the free fatty acids in the foodstuff, and wherein the method comprises the step of adding a lipid acyltransferase to the foodstuff.

A method of *in situ* production of an emulsifier and either a sterol ester and/or a stanol ester in a foodstuff, wherein the method is such that the emulsifier is produced without increasing or without substantially increasing the free fatty acids in the foodstuff, and wherein the method comprises the step of adding a lipid acyltransferase to the foodstuff.

A method *of in situ* production of an emulsifier and either a sterol ester and/or a stanol ester in a foodstuff, wherein the method comprises the step of adding a lipid acyltransferase to the foodstuff.

A method for the *in situ* production of at least two emulsifiers in a foodstuff, wherein the method comprises the step of adding to the foodstuff a lipid acyltransferase.

A method of *in situ* production of at least two emulsifiers and either a sterol ester and/or a stanol ester in a foodstuff, wherein the method is such that the emulsifiers are produced without increasing or without substantially increasing the free fatty acids in the foodstuff, and wherein the method comprises the step of adding a lipid acyltransferase to the foodstuff.

A method *of in situ* production of at least two emulsifiers and either a sterol ester and/or a stanol ester in a foodstuff, wherein the method comprises the step of adding a lipid acyltransferase to the foodstuff.

A method for the *in situ* production of a carbohydrate ester in a foodstuff, wherein the method comprises the step of adding a lipid acyltransferase to the foodstuff.

A method for the *in situ* production of a carbohydrate ester together with an emulsifier in a foodstuff, wherein the method comprises the step of adding a lipid acyltransferase to the foodstuff.

A method of *in situ* production of an emulsifier, and one or more of a carbohydrate ester; a sterol ester; a stanol ester; a protein ester; a monoglyceride or a diglyceride in a foodstuff, and wherein the method comprises the step of adding a lipid acyltransferase to the foodstuff.

A method of production of a foodstuff comprising an emulsifier, wherein the method comprises the step of adding to the foodstuff a lipid acyltransferase as defined herein.

A method of production of a foodstuff comprising an emulsifier, wherein the method is such that the emulsifier is produced without increasing or without substantially increasing the free fatty acids in the foodstuff, and wherein the method comprises the step of adding a lipid acyltransferase to the foodstuff.

A method of the production of a foodstuff comprising an emulsifier and either a sterol ester and/or a stanol ester, wherein the method is such that the emulsifier is produced without increasing or without substantially increasing the free fatty acids in the foodstuff, and wherein the method comprises the step of adding a lipid acyltransferase to the foodstuff.

A method of the production of a foodstuff comprising an emulsifier and either a sterol ester and/or a stanol ester, wherein the method comprises the step of adding a lipid acyltransferase to the foodstuff.

A method for the production of a foodstuff comprising at least two emulsifiers, wherein the method comprises the step of adding to the foodstuff a lipid acyltransferase.

A method of the production of a foodstuff comprising at least two emulsifiers and either a sterol ester and/or a stanol ester, wherein the method is such that the emulsifiers are produced without increasing or without substantially increasing the free fatty acids in the foodstuff, and wherein the method comprises the step of adding a lipid acyltransferase to the foodstuff.

A method of the production of a foodstuff comprising at least two emulsifiers and either a sterol ester and/or a stanol ester, wherein the method comprises the step of adding a lipid acyltransferase to the foodstuff.

A method for the production of a foodstuff comprising a carbohydrate ester, wherein the method comprises the step of adding a lipid acyltransferase to the foodstuff.

A method for the production of a foodstuff comprising a carbohydrate ester and an emulsifier, wherein the method comprises the step of adding a lipid acyltransferase to the foodstuff.

A method of the production of a foodstuff comprising an emulsifier and one or more of a carbohydrate ester; a sterol ester; a stanol ester; a protein ester; a monoglyceride or a diglyceride, and wherein the method comprises the step of adding a lipid acyltransferase to the foodstuff.

Use of a lipid acyltransferase to prepare from a food material a foodstuff comprising an emulsifier, wherein the emulsifier is generated from constituents of the food material by the lipid acyltransferase.

Use of a lipid acyltransferase to prepare from a food material a foodstuff comprising an emulsifier, wherein the emulsifier is produced without increasing or without substantially increasing the free fatty acids in the foodstuff, and wherein the emulsifier is generated from constituents of the food material by the lipid acyltransferase.

Use of a lipid acyltransferase to prepare from a food material a foodstuff comprising an emulsifier and either a sterol ester and/or a stanol ester, wherein the emulsifier is produced without increasing or without substantially increasing the free fatty acids in the foodstuff, and wherein the emulsifier and/or sterol ester and/or stanol ester is/are generated from constituents of the food material by the lipid acyltransferase.

Use of a lipid acyltransferase to prepare from a food material a foodstuff comprising an emulsifier and either a sterol ester and/or a stanol ester, wherein the emulsifier and/or sterol ester and/or stanol ester is/are generated from constituents of the food material by the lipid acyltransferase.

Use of a lipid acyltransferase to prepare from a food material a foodstuff comprising at least two emulsifiers, wherein the two emulsifiers are generated from constituents of the food material by the lipid acyltransferase.

Use of a lipid acyltransferase to prepare from a food material a foodstuff comprising at least two emulsifiers and either a sterol ester and/or a stanol ester, wherein the emulsifiers are produced without increasing or without substantially increasing the free fatty acids in the foodstuff, and wherein one or both of the emulsifiers and/or the sterol ester and/or the stanol ester is/are generated from constituents of the food material by the lipid acyltransferase.

Use of a lipid acyltransferase to prepare from a food material a foodstuff comprising at least two emulsifiers and either a sterol ester and/or a stanol ester, wherein one or both of the emulsifiers and/or the sterol ester and/or the stanol ester is/are generated from constituents of the food material by the lipid acyltransferase.

Use of a lipid acyltransferase to prepare from a food material a foodstuff comprising a carbohydrate ester, wherein the carbohydrate ester is generated from constituents of the food material by the lipid acyltransferase.

Use of a lipid acyltransferase to prepare from a food material a foodstuff comprising at least a carbohydrate ester and a further emulsifier, wherein the carbohydrate ester and the emulsifier are generated from constituents of the food material by the lipid acyltransferase.

Use of a lipid acyltransferase to prepare from a food material a foodstuff comprising an emulsifier and one or more of a carbohydrate ester; a sterol ester; a stanol ester; a protein ester; a monoglyceride or a diglyceride, and wherein the emulsifier and/or the carbohydrate ester and/or the sterol ester and/or the stanol ester and/or the protein ester and/or the monoglyceride and/or the diglyceride is/are generated from constituents of the food material by the lipid acyltransferase.

A method of the *in situ* production of an emulsifier, preferably a lysolecithin and a sterol ester in a egg based foodstuff, wherein the method is such that the emulsifier is produced without increasing or without substantially increasing the free fatty acids in the foodstuff, and wherein the method comprises the step of adding a lipid acyltransferase to the foodstuff.

A method of the *in situ* production of an emulsifier, preferably a lysolecithin, and a sterol ester in an egg based foodstuff, wherein the method comprises the step of adding a lipid acyltransferase to the foodstuff.

A method of production of a egg based foodstuff comprising an emulsifier, preferably a lysolecithin, and a sterol ester in an egg based foodstuff, wherein the emulsifier is produced without increasing or without substantially increasing the free fatty acids in the foodstuff, and wherein the method comprises the step of adding a lipid acyltransferase to the foodstuff.

A method of production of an egg based foodstuff comprising an emulsifier, preferably a lysolecithin, and a sterol ester in an egg based foodstuff, wherein the method comprises the step of adding a lipid acyltransferase to the foodstuff.

A foodstuff obtainable by, preferably obtained by, a method according to the present invention.

A food enzyme composition and/or a feed enzyme composition, which contains a lipid acyltransferase, and the use of such a composition in the methods of the present invention.

A method of identifying a suitable lipid acyltransferase for use in accordance with the present invention, comprising the steps of testing an enzyme of interest using one or more of the "Transferase Assay in a Low Water environment", the "Transferase Assay in High Water Egg Yolk" or the "Transferase Assay in Buffered Substrate", and selecting a lipid acyltransferase if it is one which has one or more of the following characteristics: (a) when tested using the "Transferase Assay in a Low Water Environment", measured after a time period selected from 30, 20 or 120 minutes, has a relative transferase activity of at least 1 %; (b) when tested using the "Transferase Assay in High Water Egg Yolk" in an egg yolk with 54% water, has up to 100% relative transferase activity; or (c) when tested using the "Transferase Assay in Buffered Substrate" has at least 2% acyltransferase activity.

A lipid acyltransferase identified using a method as disclosed herein.

An immobilised lipid acyltransferase enzyme as defined herein.

## DETAILED ASPECTS OF THE PRESENT INVENTION

The term "lipid acyltransferase" as used herein means an enzyme which as well as having lipase activity (generally classified as E.C. 3.1.1.x in accordance with the Enzyme Nomenclature Recommendations (1992) of the Nomenclature Committee of the International Union of Biochemistry and Molecular Biology) also has acyltransferase activity (generally classified as E.C. 2.3.1.x), whereby the enzyme is capable of transferring an acyl group from a lipid to one or more acceptor substrates, such as one or more of the following: a sterol; a stanol; a carbohydrate; a protein; a protein subunit; glycerol.

Preferably, the lipid acyltransferase for use in the methods and/or uses of the present invention is capable of transferring an acyl group from a lipid (as defined herein) to one or more of the following acyl acceptor substrates: a sterol, a stanol, a carbohydrate, a protein or subunits thereof, or a glycerol.

For some aspects the "acyl acceptor" according to the present invention may be any compound comprising a hydroxy group (-OH), such as for example, polyvalent alcohols, including glycerol; sterol; stanols; carbohydrates; hydroxy acids including fruit acids, citric acid, tartaric acid, lactic acid and ascorbic acid; proteins or a sub-unit thereof, such as amino acids, protein hydrolysates and peptides (partly hydrolysed protein) for example; and mixtures and derivatives thereof. Preferably, the "acyl acceptor" according to the present invention is not water.

In one embodiment, the acyl acceptor is preferably not a monoglyceride and/or a diglyceride.

In one aspect, preferably the enzyme is capable of transferring an acyl group from a lipid to a sterol and/or a stanol.

In one aspect, preferably the enzyme is capable of transferring an acyl group from a lipid to a carbohydrate.

In one aspect, preferably the enzyme is capable of transferring an acyl group from a lipid to a protein or a subunit thereof. Suitably the protein subunit may be one or more of the following: an amino acid, a protein hydrolysate, a peptide, a dipeptide, an oligopeptide, a polypeptide.

Suitably in the protein or protein subunit the acyl acceptor may be one or more of the following constituents of the protein or protein subunit: a serine, a threonine, a tyrosine, or a cysteine.

When the protein subunit is an amino acid, suitably the amino acid may be any suitable amino acid. Suitably the amino acid may be one or more of a serine, a threonine, a tyrosine, or a.cysteine for example.

In one aspect, preferably the enzyme is capable of transferring an acyl group from a lipid to glycerol.

In one aspect, preferably the enzyme is capable of transferring an acyl group from a lipid to a hydroxy acid.

In one aspect, preferably the enzyme is capable of transferring an acyl group from a lipid to a polyvalent alcohol.

In one aspect, the lipid acyltransferase may, as well as being able to transfer an acyl group from a lipid to a sterol and/or a stanol, additionally be able to transfer the acyl group from a lipid to one or more of the following: a carbohydrate, a protein, a protein subunit, glycerol.

Preferably, the lipid substrate upon which the lipid acyltransferase according to the present invention acts is one or more of the following lipids: a phospholipid, such as a lecithin, e.g. phosphatidylcholine, a triacylglyceride, a cardiolipin; a diglyceride, or a glycolipid, such as digalactosyldiglyceride (DGDG) for example. This lipid substrate may be referred to herein as the "lipid acyl donor". The term lecithin as used herein encompasses phosphatidylcholine, phosphatidylethanolamine, phosphafidylinositol, phosphatidylserine and phosphatidylglycerol.

For some aspects, preferably the lipid substrate upon which the lipid acyltransferase acts is a phospholipid, such as lecithin, for example phosphatidylcholine.

For some aspects, preferably the lipid substrate is a glycolipid, such as DGDG for example.

Preferably the lipid substrate is a food lipid, that is to say a lipid component of a foodstuff.

For some aspects, preferably the lipid acyltransferase according to the present invention is incapable, or substantially incapable, of acting on a triglyceride and/or a 1-monoglyceride and/or 2-moiioglyceride.

Suitably, the lipid substrate or lipid acyl donor may be one or more lipids present in one or more of the following substrates: fats, including lard, tallow and butter fat; oils including oils extracted from or derived from palm oil, sunflower oil, soya bean oil, safflower oil, cotton seed oil, ground nut oil, com oil, olive oil, peanut oil, coconut oil, and rape seed oil. Lecithin from soya, rape seed or egg yolk is also a suitable lipid substrate. The lipid substrate may be an oat lipid or other plant based material containing galactolipids.

In one aspect the lipid acyl donor is preferably lecithin (such as phosphatidylcholine) in egg yolk.

For some aspects of the present invention, the lipid may be selected from lipids having a fatty acid chain length of from 8 to 22 carbons.

For some aspects of the present invention, the lipid may be selected from lipids having a fatty acid chain length of from 16 to 22 carbons, more preferably of from 16 to 20 carbons.

For some aspects of the present invention, the lipid may be selected from lipids having a fatty acid chain length of no greater than 14 carbons, suitably from lipids having a fatty acid chain length of from 4 to 14 carbons, suitably 4 to 10 carbons, suitably 4 to 8 carbons.

Suitably, the lipid acyltransferase according to the present invention may exhibit one or more of the following lipase activities: glycolipase activity (E.C. 3.1.1.26), triacylglycerol lipase activity (E.C. 3.1.1.3), phospholipase A2 activity (E.C. 3.1.1.4) or phospholipase A1 activity (E.C. 3.1.1.32). The term "glycolipase activity" as used herein encompasses "galactolipase activity".

Suitably, the lipid acyltransferase according to the present invention may have at least one or more of the following activities: glycolipase activity (E.C. 3.1.1.26) and/or phospholipase A1 activity (E.C. 3.1.1.32) and/or phospholipase A2 activity (E.C. 3.1.1.4).

For some aspects, the lipid acyltransferase according to the present invention may have at least glycolipase activity (E.C. 3.1.1.26).

Suitably, for some aspects the lipid acyltransferase according to the present invention is capable of transferring an acyl group from a glycolipid and/or a phospholipid to one or more of the following acceptor substrates: a sterol, a stanol, a carbohydrate, a protein, glycerol.

For some aspects, the lipid acyltransferase according to the present invention is capable of transferring an acyl group from a glycolipid and/or a phospholipid to a sterol and/or a stanol to form at least a sterol ester and/or a stanol ester.

For some aspects, preferably the lipid acyltransferase according to the present invention is capable of transferring an acyl group from a glycolipid and/or a phospholipid to a carbohydrate to form at least a carbohydrate ester.

For some aspects, preferably the lipid acyltransferase according to the present invention is capable of transferring an acyl group from a glycolipid and/or a phospholipid to a protein to form at least protein ester (or a protein fatty acid condensate).

For some aspects, preferably the lipid acyltransferase according to the present invention is capable of transferring an acyl group from a glycolipid and/or a phospholipid to glycerol to form at least a diglyceride and/or a monoglyceride.

For some aspects, preferably the lipid acyltransferase according to the present invention does not exhibit triacylglycerol lipase activity (E.C. 3.1.1.3).

In some aspects, the lipid acyltransferase is capable of transferring an acyl group from a lipid to a sterol and/or a stanol. Thus, in one embodiment the "acyl acceptor" according to the present invention may be either a sterol or a stanol or a combination of both a sterol and a stanol.

In one embodiment suitably the sterol and/or stanol may comprise one or more of the following structural features:

i) a 3-beta hydroxy group or a 3-alpha hydroxy group; and/or
ii) A:B rings in the *cis* position or A:B rings in the *trans* position or $C_5$-$C_6$ is unsaturated.

Suitable sterol acyl acceptors include cholesterol and phytosterols, for example alpha-sitosterol, beta-sitosterol, stigmasterol, ergosterol, campesterol, 5,6-dihydrosterol, brassicasterol, alpha-spinasterol, beta-spinasterol, gamma-spinasterol, deltaspinasterol, fucosterol, dimosterol, ascosterol, serebisterol, episterol, anasteerol, hyposterol, chondrillasterol, desmosterol, chalinosterol, poriferasterol, clionasterol, sterol glycosides, and other natural or synthetic isomeric forms and derivatives.

In one aspect of the present invention suitably more than one sterol and/or stanol may act as the acyl acceptor, suitably more than two sterols and/or stanols may act as the acyl acceptor. In other words, in one aspect of the present invention, suitably more than one sterol ester and/or stanol ester may be produced. Suitably, when cholesterol is the acyl acceptor one or more further sterols or one or more stanols may also act as the acyl acceptor. Thus, in one aspect, the present invention provides a method for the *in situ* production of both a cholesterol ester and at least one sterols or stanol ester in combination. In other words, the lipid acyltransferase for some aspects of the present invention may transfer an acyl group from a lipid to both cholesterol and at least one further sterol and/or at least one stanol.

In one aspect, preferably the sterol acyl acceptor is one or more of the following: alpha-sitosterol, beta-sitosterol, stigmasterol, ergosterol and campesterol.

In one aspect, preferably the sterol acyl acceptor is cholesterol. When it is the case that cholesterol is the acyl acceptor for the lipid acyltransferase, the amount of free cholesterol in the foodstuff is reduced as compared with the foodstuff prior to exposure to the lipid acyltransferase and/or as compared with an equivalent foodstuff which has not been treated with the lipid acyltransferase.

Suitable stanol acyl acceptors include phytostanols, for example beta-sitostanol or ss-sitostanol.

In one aspect, preferably the sterol and/or stanol acyl acceptor is a sterol and/or a stanol other than cholesterol.

In some aspects, the foodstuff prepared in accordance with the present invention may be used to reduce blood serum cholesterol and/or to reduce low density lipoproteins. Blood serum cholesterol and low density lipoproteins have both been associated with certain diseases in humans, such as atherosclerosis and/or heart disease for example. Thus, it is envisaged that the foodstuffs prepared in accordance with the present invention may be used to reduce the risk of such diseases.

Thus, in one aspect the present invention provides the use of a foodstuff according to the present invention for use in the treatment and/or prevention of atherosclerosis and/or heart disease.

In a further aspect, the present invention provides a medicament comprising a foodstuff according to the present invention.

In a further aspect, the present invention provides a method of treating and/or preventing a disease in a human or animal patient which method comprising administering to the patient an effective amount of a foodstuff according to the present invention.

Suitably, the sterol and/or the stanol "acyl acceptor" may be found naturally within the foodstuff. Alternatively, the sterol and/or the stanol may be added to the foodstuff. When it is the case that a sterol and/or a stanol is added to the foodstuff, the sterol and/or stanol may be added before, simultaneously with, and/or after the addition of the lipid acyltransferase according to the present invention. Suitably, the present invention may encompass the addition of exogenous sterols/stanols, particularly phytosterols/phytostanols, to the foodstuff prior to or simultaneously with the addition of the enzyme according to the present invention.

For some aspects, one or more sterols present in the foodstuff may be converted to one or more stanols prior to or at the same time as the lipid acyltransferase is added According to the present invention. Any suitable method for converting sterols to stanols may be employed. For example, the conversion may be carried out by chemical hydrogenation for example. The conversion may be conducted prior to the addition of the lipid acyltransferase in accordance with the present invention or simultaneously with the addition of the lipid acyltransferase in accordance with the present invention. Suitably enzymes for the conversion of sterol to stanols are taught in WO00/061771.

Suitably the present invention may be employed to produce phytostanol esters *in situ* in a foodstuff. Phytostanol esters have increased solubility through lipid membranes, bioavailability and enhanced health benefits (see for example WO92/99640).

In some embodiments of the present invention the stanol ester and/or the sterol ester may be a flavouring and/or a texturiser. In which instances, the present invention encompasses the *in situ* production of flavourings and/or texturisers.

For some aspects of the present invention, the lipid acyltransferase according to the present invention may utilise a carbohydrate as the acyl acceptor. The carbohydrate acyl acceptor may be one or more of the following: a monosaccharide, a disaccharide, an oligosaccharide or a polysaccharide. Preferably, the carbohydrate is one or more of the following: glucose, fructose, anhydrofructose, maltose, lactose, sucrose, galactose, xylose, xylooligosacharides, arabinose, maltooligosaccharides, tagatose, microthecin, ascopyrone P, ascopyrone T, cortalcerone.

Suitably, the carbohydrate "acyl acceptor" may be found naturally within the foodstuff. Alternatively, the carbohydrate may be added to the foodstuff. When it is the case that the carbohydrate is added to the foodstuff, the carbohydrate may be added before, simultaneously with, and/or after the addition of the lipid acyltransferase according to the present invention.

Carbohydrate esters can function as valuable emulsifiers in foodstuffs. Thus, when it is the case that the enzyme functions to transfer the acyl group to a sugar, the invention encompasses the production of a second *in situ* emulsifier in the foodstuff

In some embodiments, the lipid acyltransferase may utilise both a sterol and/or stanol and a carbohydrate as an acyl acceptor.

The utilisation of lipid acyltransferase which can transfer the acyl group to a carbohydrate as well as to a sterol and/or a stanol is particularly advantageous for foodstuffs comprising eggs. In particular, the presence of sugars, in particular glucose, in eggs and egg products is often seen as disadvantageous. Egg yolk may comprise up to 1% glucose. Typically, egg or egg based products may be treated with glucose oxidase to remove some or all of this glucose. However, in accordance with the present invention this unwanted sugar can be readily removed by "esterifying" the sugar to form a sugar ester.

For some aspects of the present invention, the lipid acyltransferase according to the present invention may utilise a protein as the acyl acceptor. Suitably, the protein may be one or more of the proteins found in a food product, for example in a dairy product and/or a meat product By way of example only, suitable proteins may be those found in curd or whey, such as lactoglobulin. Other suitable proteins include ovalbumin from egg, gliadin, glutenin, puroindoline, lipid transfer proteins from grains, and myosin from meat.

Thus in accordance with the present invention, one or more of the following advantageous properties can be achieved: *in situ* production of an emulsifier without an increase in free fatty acids; a reduction in the accumulation of free fatty acids in the foodstuff, a reduction in free cholesterol levels in the foodstuff an increase in sterol esters and/or stanol esters; a reduction in blood serum cholesterol and/or low density lipoproteins; an increase in carbohydrate esters; a reduction in unwanted free carbohydrates.

An advantage of the present invention is that the emulsifier(s) is/are prepared *in situ* in the foodstuff without an increase, or a substantial, increase, in the free fatty acid content of the foodstuff. The production of free fatty acids can be detrimental to foodstuffs. In particular, free fatty acids have been linked with off-odours and/or off-flavours in foodstuffs, as well other detrimental effects, including a soapy taste in cheese for instance. Preferably, the method according to the present invention results in the *in situ* preparation of an emulsifier(s) wherein the accumulation of free fatty acids is reduced and/or eliminated. Without wishing to be bound by theory, in accordance with the present invention the fatty acid which is removed from the lipid is transferred by the lipid acyltransferase to an acyl acceptor, for example a sterol and/or a stanol. Thus, the overall level of free fatty acids in the foodstuff does not increase or increases only to an insignificant degree. This is in sharp contradistinction to the situation when lipases (E.C. 3.1.1.x) are used to produce emulsifiers *in situ.* In particular, the use of lipases can result in an increased amount of free fatty acid in the foodstuff, which can be detrimental. In accordance with the present invention, the accumulation of free fatty acids is reduced and/or eliminated when compared with the amount of free fatty acids which would have been accumulated had a lipase enzyme, in particular a phospholipase A enzyme, been used in place of the lipid acyltransferases in accordance with the present invention.

The utilisation of a lipid acyltransferase which can transfer the acyl group to a sterol and/or stanol may be particularly advantageous for foodstuffs comprising eggs. In particular, it has been found that an egg-based product with significantly better properties can be obtained following treatment with a lipid acyltransferase as defined herein compared with egg-based products treated with conventional phospholipases, such as LipopanF® (Novozymes A/S, Denmark)), Lecitase Ultra® (Novozymes A/S, Denmark) or Lipomod 22 L from Biocatalysts, for instance.

Preferably, the lipid acyltransferase enzyme according to the present invention may be characterised using the following criteria:

(i) the enzyme possesses acyl transferase activity which may be defined as ester transfer activity whereby the acyl part of an original ester bond of a lipid acyl donor is transferred to an acyl acceptor to form a new ester; and
(ii) the enzyme comprises the amino acid sequence motif GDSX, wherein X is one or more of the following amino acid residues L, A, V, I, F, Y, H, Q, T, N, M or S.

Preferably, X of the GDSX motif is L. Thus, preferably the enzyme according to the present invention comprises the amino acid sequence motif GDSL.

The GDSX motif is comprised of four conserved amino acids. Preferably, the serine within the motif is a catalytic serine of the lipid acyltransferase enzyme. Suitably, the serine of the GDSX motif may be in a position corresponding to Ser-16 in *Aeromonas hydrophila* lipolytic enzyme taught in Brumlik & Buckley (Journal of Bacteriology Apr. 1996, Vol. 178, No. 7, p 2060-2064).

To determine if a protein has the GDSX motif according to the present invention, the sequence is preferably compared with the hidden markov model profiles (HMM profiles) of the pfam database.

Pfam is a database of protein domain families. Pfam contains curated multiple sequence alignments for each family as well as profile hidden Markov models (profile HMMs) for identifying these domains in new sequences. An introduction to Pfam can be found in Bateman A et al. (2002) Nucleic Acids Res. 30; 276-280. Hidden Markov models are used in a number of databases that aim at classifying proteins, for review see Bateman A and Haft DH (2002) Brief Bioinform 3; 236-245.
http://www.ncbi.nlm.nih.gov/entrez/query.fcgi?cmd=Retrieve&db=PubMed&list uids =12230032&dopt=Abstract
http://www.ncbi.nlm.nih.gov/entre/query.fchi?cmd=Retrieve&db=PubMed&list uids =11752314&dopt=Abstract

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Alternatively, the GDSX motif can be identified using the Hammer software package, the instructions are provided in Durbin R, Eddy S, and Krogh A (1998) Biological sequence analysis; probabilistic models of proteins and nucleic acids. Cambridge University Press, ISBN 0-521-62041-4 and the references therein, and the HMMER2 profile provided within this specification.

The PFAM database can be accessed, for example, through several servers which are currently located at the following websites.
http://www.sanger.ac.uk/Software/Pfam/index.shtml
http://pfam.wustl.edu/
http://pfam.jouy.inra.fr/
http://pffam.cgb.ki.se/

The database offers a search facility where one can enter a protein sequence. Using the default parameters of the database the protein sequence will then be analysed for the presence of Pfam domains. The GDSX domain is an established domain in the database and as such its presence in any query sequence will be recognised . The database will return the alignment of the Pfam00657 consensus sequence to the query sequence. A multiple alignment, including *Aeromonas salmonicida* or *Aeromonas hydrophile* can be obtained by:

a) manual
obtain an alignment of the protein of interest with the Pfam00657 consensus sequence and obtain an alignment of P10480 with the Pfam00657 consensus sequence following the procedure described above;
or

b) through the database
After identification of the Pfam00657 consensus sequence the database offers the option to show an alignment of the query sequence to the seed alignment of the Pfam00657 consensus sequence. P10486 is part of this seed alignment and is indicated by GCAT_AERHY. Both the query sequence and P10480 will be displayed in the same window.

The *Aeromonas hydrophila* reference sequence:
The residues of *Aeromonas hydrophila* GDSX lipase are numbered in the NCBI file P10480, the numbers in this text refer to the numbers given in that file which in the present invention is used to determine specific amino acids residues which, in a preferred embodiment are present in the lipid acyltransferase enzymes of the invention.

The Pfam alignment was performed (Figure 33 and 34):

The following conserved residues can be recognised and in a preferable embodiment may be present in the enzymes for use in the compositions and methods of the invention;

Block 1 - GDSX block

| | | | | | | | |
|---|---|---|---|---|---|---|---|
| hid | hid | hid | hid | Gly | Asp | Ser | hid |
| 28 | 29 | 30 | 31 | 32 | 33 | 34 | 35 |

Block 2 - GANDY block

| | | | | | |
|---|---|---|---|---|---|
| hid | Gly | hid | Asn | Asp | hid |
| 130 | 131 | 132 | 133 | 134 | 135 |

Block 3 - HPT block

His
309

Where 'hid' means a hydrophobic residue selected from Met, Ile, Leu, Val, Ala, Gly, Cys, His, Lys, Trp, Tyr, Phe.

Preferably the lipid acyltransferase enzyme for use in the compositions/methods of the invention can be aligned using the Pfam00657 consensus sequence.

Preferably, a positive match with the hidden markov model profile (HMM profile) of the pfam00657 domain family indicates the presence of the GDSL or GDSX domain according to the present invention.

Preferably when aligned with the Pfam00657 consensus sequence the lipid acyltransferase for use in the compositions/methods of the invention have at least one, preferably more than one, preferably more than two, of the following, a GDSx block, a GANDY block, a HPT block. Suitably, the lipid acyltransferase may have a GDSx block and a GANDY block. Alternatively, the enzyme may have a GDSx block and a HPT block. Preferably the enzyme comprises at least a GDSx block.

Preferably, when aligned with the Pfam00657 consensus sequence the enzyme for use in the compositions/ methods of the invention have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A. hydrophilia* polypeptide sequence, namely SEQ ID No. 32: 28hid, 29hid, 30hid, 31hid, 32gly, 33Asp, 34Ser, 35hid, 130hid, 131Gly, 132Hid, 133Asn, 134Asp, 135hid, 309His

The pfam00657 GDSX domain is a unique identifier which distinguishes proteins possessing this domain from other enzymes.

The pfam00657 consensus sequence is presented in Figure 1 as SEQ ID No.1. This is derived from the identification of the pfam family 00657, database version 6, which may also be referred to as pfam00657.6 herein.

The consensus sequence may be updated by using further releases of the pfam database.

For example, Figures 33 and 34 show the pfam alignment of family 00657, from database version 11, which may also be referred to as pfam00657.11 herein.

The presence of the GDSx, GANDY and HPT blocks are found in the pfam family 00657 from both releases of the database. Future releases of the pfam database can be used to identify the pfam family 00657.

Preferably, the lipid acyltransferase enzyme according to the present invention may be characterised using the following criteria:

(i) the enzyme possesses acyl transferase activity which may be defined as ester transfer activity whereby the acyl part of an original ester bond of a lipid acyl donor is transferred to acyl acceptor to form a new ester;

(ii) the enzyme comprises the amino acid sequence motif GDSX, wherein X is one or more of the following amino acid residues L, A, V, I, F, Y, H, Q, T, N, M or S.;

(iii) the enzyme comprises His-309 or comprises a histidine residue at a position corresponding to His-309 in the *Aeromonas hydrophile* lipolytic enzyme shown in Figure 2 (SEQ ID No. 2 or SEQ ID No. 32).

Preferably, the amino acid residue of the GDSX motif is L.

In SEQ ID No. 2 or SEQ ID No. 32 the first 18 amino acid residues form a signal sequence. His-309 of the full length sequence, that is the protein including the signal sequence, equates to His-291 of the mature part of the protein, i.e. the sequence without the signal sequence.

Preferably, the lipid acyltransferase enzyme according to the present invention comprises the following catalytic triad: Ser-34, Asp-134 and His-309 or comprises a serine residue, an aspartic acid residue and a histidine residue, respectively, at positions corresponding to Ser-34, Asp-134 and His-309 in the *Aeromonas hydrophila* lipolytic enzyme shown in Figure 2 (SEQ ID No. 2) or Figure 28 (SEQ ID No. 32). As stated above, in the sequence shown in SEQ ID No. 2 or SEQ ID No. 32 the first 18 amimo acid residues form a signal sequence. Ser-34, Asp-134 and His-309 of the full length sequence, that is the protein including the signal sequence, equate to Ser-16, Asp-116 and His-291 of the mature part of the protein, i.e. the sequence without the signal sequence. In the pfam00657 consensus sequence, as given in Figure 1 (SEQ ID No. 1) the active site residues correspond to Ser-7, Asp-157 and His-348.

Preferably, the lipid acyltransferase enzyme according to the present invention may be characterised using the following criteria:

(i) the enzyme possesses acyl transferase activity which may be defined as ester transfer activity whereby the acyl part of an original ester bond of a first lipid acyl donor is transferred to an acyl acceptor to form a new ester; and

(ii) the enzyme comprises at least Gly-32, Asp-33, Ser-34, Asp-134 and His-309 or comprises glycine, aspartic acid, serine, aspartic acid and histidine residues at positions corresponding to Gly-32, Asp-33, Ser-34, Asp-134 and His-309, respectively, in the *Aeromonas hydrophila* lipolytic enzyme shown in Figure 2 (SEQ ID No. 2) or Figure 28 (SEQ ID No. 32).

Suitably, the lipid acyltransferase enzyme according to the present invention may be obtainable, preferably obtained, from organisms from one or more of the following genera: *Aeromonas, Streptomyces, Saccharomyces, Lac-*

*tococcus, Mycobacterium, Streptococcus, Lactobacillus, Desulfitobacterium, Bacillus, Campylobacter, Vibrionaceae, Xylella Sulfolobus, Aspergillus, Schizosaccharomyces, Listeria, Neisseria, Mesorhuobium, Ralstonia, Xanthomonas* and *Candida.*

Suitably, the lipid acyltransferase enzymes according to the present invention may be obtainable, preferably obtained, from one or more of the following organisms: *Aeromonas hydrophila, Aeromonas salmonicida, Streptomyces coelicolor, Streptomyces rimosus, Mycobacterium, Streptococcus pyogenes, Lactococcus lactis, Streptococcus pyogenes, Streptococcus thermophilus, Lactobacillus helveticus, Desulfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schuosaccharomyces pombe, Listeria innocua, Listeria monocytogenes, Neisseria meningitidis, Mesorhiobium loti, Ralstonia solanacearum, Xanthomonas campestris, Xanthomonas axonopodis* and *Candida parapsilosis.*

In one aspect, preferably the lipid acyltransferase enzyme according to the present invention is obtainable, preferably obtained, from one or more of *Aeromonas hydrophila* or *Aeromonas salmonicida.*

Suitably, the lipid acyltransferase enzyme according to the present invention comprises one or more of the following amino acid sequences:

(i) the amino acid sequence shown as SEQ ID No. 2 (see Figure 2)
(ii) the amino acid sequence shown as SEQ ID No. 3 (see Figure 3)
(iii) the amino acid sequence shown as SEQ ID No. 4 (see Figure 4)
(iv) the amino acid sequence shown as SEQ ID No. 5 (see Figure 5)
(v) the amino acid sequence shown as SEQ ID No. 6 (see Figure 6)
(vi) the amino acid sequence shown as SEQ ID No. 12 (see Figure 14)
(vii) the amino acid sequence shown as SEQ ID No. 20 (Figure 16)
(viii) the amino acid sequence shown as SEQ ID No. 22 (Figure 18)
(ix) the amino acid sequence shown as SEQ ID No. 24 (Figure 20)
(x) the amino acid sequence shown as SEQ ID No. 26 (Figure 22)
(xi) the amino acid sequence shown as SEQ ID No. 28 (Figure 24)
(xii) the amino acid sequence shown as SEQ ID No. 30 (Figure 26)
(xiii) the amino acid sequence shown as SEQ ID No. 32 (Figure 28)
(xiv) the amino acid sequence shown as SEQ ID No. 34 (Figure 30) or
an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, or SEQ ID No. 34.

Suitably, the lipid acyltransferase enzyme according to the present invention comprises either the amino acid sequence shown as SEQ ID No. 2 or as SEQ ID No. 3 or SEQ ID No. 32 or SEQ ID No. 34 or comprises an amino acid sequence which has 75% or more, preferably 80% or more, preferably 85% or more, preferably 90% or more, preferably 95% or more, identity with the amino acid sequence shown as SEQ ID No. 2 or the amino acid sequence shown as SEQ ID No. 3 or the amino acid sequence shown as SEQ ID No. 32 or the amino acid sequence shown as SEQ ID No. 34.

For the purposes of the present invention, the degree of identity is based on the number of sequence elements which are the same. The degree of identity in accordance with the present invention may be suitably determined by means of computer programs known in the art, such as GAP provided in the GCG program package (Program Manual for the Wisconsin Package, Version 8, August 1994, Genetics Computer Group, 575 Science Drive, Madison, Wisconsin, US53711) (Needleman & Wunsch (1970), J. of Molecular Biology 48, 443-45) using the following settings for polypeptide sequence comparison: GAP creation penalty of 3.0 and GAP extension penalty of 0.1.

Suitably the lipid acyltransferase enzyme according to the present invention comprises an amino acid sequence which has 80% or more, preferably 85% or more, more preferably 90% or more and even more preferably 95% or more identity with any one of the sequences shown as SEQ ID No. 2, SEQ ID No. 3, SEQ ID No. 4, SEQ ID No: 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, or SEQ ID No. 34.

Suitably, the lipid acyltransferase enzyme according to the present invention comprises one or more of the following amino acid sequences:

(a) an amino acid sequence shown as amino acid residues 1-100 of SEQ ID No. 2 or SEQ ID No. 32;
(b) an amino acid sequence shown as amino acids residues 101-200 of SEQ ID No. 2 or SEQ ID No. 32;
(c) an amino acid sequence shown as amino acid residues 201-300 of SEQ ID No. 2 or SEQ ID No. 32; or
(d) an amino acid sequence which has 75% or more, preferably 85% or more, more preferably 90% or more, even more preferably 95% or more identity to any one of the amino acid sequences defined in (a)-(c) above.

Suitably, the lipid acyltransferase enzyme according to the present invention comprises one or more of the following amino acid sequences:

(a) an amino acid sequence shown as amino acid residues 28-39 of SEQ ID No. 2 or SEQ ID No. 32;
(b) an amino acid sequence shown as amino acids residues 77-88 of SEQ ID No. 2 or SEQ ID No. 32;
(c) an amino acid sequence shown as amino acid residues 126-136 of SEQ ID No. 2 or SEQ ID No. 32;
(d) an amino acid sequence shown as amino acid residues 163-175 of SEQ ID No. 2 or SEQ ID No. 32;
(e) an amino acid sequence shown as amino acid residues 304-311 of SEQ ID No. 2 or (SEQ ID No. 32; or
(f) an amino acid sequence which has 75% or more, preferably 85% or more, more preferably 90% or more, even more preferably 95% or more identity to any one of the amino acid sequences defined in (a)-(e) above.

Suitably, the lipid acyltransferase enzyme according to the present invention may comprise an amino acid sequence produced by the expression or one or more of the following nucleotide sequences:

(a) the nucleotide sequence shown as SEQ ID No. 7 (see Figure 9);
(b) the nucleotide sequence shown as SEQ ID No. 8 (see Figure 10);
(c) the nucleotide sequence shown as SEQ ID No. 9 (see Figure 11);
(d) the nucleotide sequence shown as SEQ ID No. 10 (see Figure 12);
(e) the nucleotide sequence shown as SEQ ID No. 11 (see Figure 13);
(f) the nucleotide sequence shown as SEQ ID No. 13 (see Figure 15);
(g) the nucleotide sequence shown as SEQ ID No. 21 (see Figure 17);
(h) the nucleotide sequence shown as SEQ ID No. 23 (see Figure 19);
(i) the nucleotide sequence shown as SEQ ID No. 25 (see Figure 21);
(j) the nucleotide sequence shown as SEQ ID No. 27 (see Figure 23);
(k) the nucleotide sequence shown as SEQ ID No. 29 (see Figure 25);
(l) the nucleotide sequence shown as SEQ ID No. 31 (see Figure 27);
(m)the nucleotide sequence shown as SEQ ID No. 33 (see Figure 29);
(n) the nucleotide sequence shown as SEQ ID No. 35 (see Figure 31);
(o) or
a nucleotide sequence which has 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35.

Suitably the nucleotide sequence may have 80% or more, preferably 85% or more, more preferably 90% or more and even more preferably 95% or more identity with any one of the sequences shown as SEQ ID No. 7, SEQ ID No. 8, SEQ ID No. 9, SEQ ID No. 10, SEQ ID No: 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35.

In one aspect, the lipid acyltransferase according to the present invention may be a lecithin:cholesterol acyltransferases (LCAT) or variant thereof (for example a variant made by molecular evolution)

Suitable LCATs are known in the art and may be obtainable from one or more of the following organisms for example: mammals, rat, mice, chickens, *Drosophila melanogaster,* plants, including *Arabidopsis* and *Oryza sativa,* nematodes, fungi and yeast.

In one embodiment the lipid acyltransferase enzyme according to the present invention may be the lipid acyltransferase obtainable, preferably obtained, from the E. *coli* strains TOP 10 harbouring pPet12aAhydro and pPet12aASalmo deposited by Danisco A/S of Langebrogade 1, DK-1001 Copenhagen K, Denmark under the Budapest Treaty on the International Recognition of the Deposit of Microorganisms for the purposes of Patent Procedure at the National Collection of Industrial, Marine and Food Bacteria (NCIMB) 23 St. Machar Street, Aberdeen Scotland, GB on 22 December 2003 under accession numbers NICMB 41204 and NCIMB 41205, respectively.

Preferably, when carrying out a method according to the present invention the product is produced without increasing or substantially increasing the free fatty acids in the foodstuff.

The term "transferase" as used herein is interchangeable with the term "lipid acyltransferase".

Suitably, the lipid acyltransferase as defined herein catalyses one or more of the following reactions: interesterification, transesterification, alcoholysis, hydrolysis.

The term "interesterification" refers to the enzymatic catalysed transfer of acyl groups between a lipid donor and lipid acceptor, wherein the lipid donor is not a free acyl group.

The term "transesterification" as used herein means the enzymatic catalysed transfer of an acyl group from a lipid donor (other than a free fatty acid) to an acyl acceptor (other than water).

As used herein, the term "alcoholysis" refers to the enzymatic cleavage of a covalent bond of an acid derivative by reaction with an alcohol ROH so that one of the products combines with the H of the alcohol and the other product combines with the OR group of the alcohol.

As used herein, the term "alcohol" refers to an alkyl compound containing a hydroxyl group.

As used herein, the term "hydrolysis" refers to the enzymatic catalysed transfer of an acyl group from a lipid to the OH group of a water molecule. Acyl transfer which results from hydrolysis requires the separation of the water molecule.

The term "without increasing or without substantially increasing the free fatty acids" as used herein means that preferably the lipid acyl transferase according to the present invention has 100% transferase activity (i.e. transfers 100% of the acyl groups from an acyl donor onto the acyl acceptor, with no hydrolytic activity); however, the enzyme may transfer less than 100% of the acyl groups present in the lipid acyl donor to the acyl acceptor. In which case, preferably the acyltransferase activity accounts for at least 5%, more preferably at least 10%, more preferably at least 20%, more preferably at least 30%, more preferably at least 40%, more preferably 50%, more preferably at least 60%, more preferably at least 70%, more preferably at least 80%, more preferably at least 90% and more preferably at least 98% of the total enzyme activity. The % transferase activity (i.e. the transferase activity as a percentage of the total enzymatic activity) may be determined by the following protocol:

*Protocol for the determination of % acyltransferase activity:*

A foodstuff to which a lipid acyltransferase according to the present invention has been added may be extracted following the enzymatic reaction with $CHCl_3$:$CH_3OH$ 2:1 and the organic phase containing the lipid material is isolated and analysed by GLC and HPLC according to the procedure detailed hereinbelow. From the GLC and HPLC analyses the amount of free fatty acids and one or more of sterol/stanol esters; carbohydrate esters, protein esters; diglycerides; or monoglycerides are determined. A control foodstuff to which no enzyme according to the present invention has been added, is analysed in the same way.

*Calculation:*

From the results of the GLC and HPLC analyses the increase in free fatty acids and sterol/stanol esters and/or carbohydrate esters and/or protein esters and/or diglycerides and/or monoglycerides can be calculated:
A % fatty acid = % Fatty acid(enzyme) - % fatty acid(control); Mv fatty acid = average molecular weight of the fatty acids;

> A = Δ % sterol ester/Mv sterol ester (where A % sterol ester = % sterol/stanol ester(enzyme) - % steml/stanol ester (control) and Mv sterol ester = average molecular weight of the sterol/stanol esters) applicable where the acyl acceptor is a sterol and/or stanol;
> B = Δ % carbohydrate ester/Mv carbohydrate ester (where Δ % carbohydrate ester = % carbohydrate ester(enzyme) - % carbohydrate ester(control) and Mv carbohydrate ester = average molecular weight of the carbohydrate ester) - applicable where the acyl acceptor is a carbohydrate;
> C = Δ % protein ester/Mv protein ester (where Δ % protein ester = % protein ester(enzyme) - % protein ester(control) and Mv protein ester = average molecular weight of the protein ester) - applicable where the acyl acceptor is a protein; and
> D = absolute value of diglyceride and/or monoglyceride/Mv di/monoglyceride (where Δ% diglyceride and/or monoglyceride = % diglyceride and/or monoglyceride (enzyme) - % diglyceride and/or monoglyceride (control) and Mv di/monoglyceride = average molecular weight of the diglyceride and/or monoglyceride) - applicable where the acyl acceptor is.glycerol.

The transferase activity is calculated as a percentage of the total enzymatic activity:

$$\%\ \text{transferase activity} = \frac{A^* + B^* + C^* + D^* \times 100}{A^* + B^* + C^* + D^* + \Delta\ \%\ \text{fatty acid}/(\text{Mv fatty acid})}.$$

* - delete as appropriate.

If the free fatty acids are increased in the foodstuff they are preferably not increased substantially, i.e. to a significant degree. By this we mean, that the increase in free fatty acid does not adversely affect the quality of the foodstuff

In some aspects of the present invention, the term "without substantially increasing free fatty acids" as used herein means that the amount of free fatty acid in a foodstuff or composition treated with an lipid acyltransferase according to the present invention is less than the amount of free fatty acid produced in the foodstuff or composition when an enzyme other than a lipid acyltransferase according to the present invention had been used, such as for example as compared with the amount of free fatty acid produced when a conventional phospholipase enzyme, e.g. LipopanF® (Novozymes A/S, Denmark), had been used.

The term *"in situ"* as used herein means that the emulsifier(s) and/or the sterol/stanol esters and/or the carbohydrate esters and/or the protein esters and/or the mono- or diglycerides are produced within the foodstuff or fraction of the foodstuff This contrasts the situation where the emulsifier(s) and/or the sterol/stanol esters and/or the carbohydrate esters and/or the protein esters and/or the mono- or diglycerides are produced separately of the foodstuff and are added as formed products to the foodstuff during preparation of the same. In other words, the term *"in situ"* as used herein means that by the addition of the lipid acyltransferase enzyme according to the present invention to a foodstuff, or to the food ingredients/materials constituting the foodstuff an emulsifier and/or a sterol ester and/or a stanol ester and/or a carbohydrate ester and/or a protein ester and/or a mono- or diglyceride may be produced from components of the foodstuff. Suitably, the components of the foodstuff may be the substrate(s) for the enzyme. If necessary, the components of the foodstuff may be supplemented by addition of one or more further components which further components may be the same as those present in the foodstuff or may be additional to those components already present in the foodstuff. For the avoidance of doubt, in one embodiment, the method according to the present invention may be a method for the production of an emulsifier and/or a sterol ester and/or a stanol ester and/or a carbohydrate ester and/or a protein ester and/or a mono- or diglyceride *in situ* in a foodstuff and is not a method for preparing an emulsifier and/or a sterol ester and/or a stanol ester (for example is an isolated and/or purified form) for subsequent addition to a foodstuff.

In another embodiment the lipase acyl-transferase may be used during the food processing , but not remain in the foodstuff For example, the lipase acyl transferase may be immobilised, allowing it to be reused.

Preferably, the lipid acyltransferase according to the present invention is capable of transferring an acyl group from a lipid to a sterol and/or stanol and/or a carbohydrate and/or a protein and/or glycerol when present in a polar environment, preferably in an aqueous environment, preferably a water containing foodstuff, Suitably, the aqueous environment may be an aqueous buffer or may be the aqueous phase in a foodstuff. The term "aqueous environment" as used herein preferably means an environment which is absent an organic solvent, preferably absent a polar organic solvent, more preferably absent an non-edible organic solvent. In particular, the term "aqueous environment" may refer to an environment to which no exogenous organic solvents, preferably no polar organic solvents, have been added. The term organic solvent as used herein does not encompass food oils, preferably does not encompass food oils that are high in non-polar lipids. In one embodiment the term organic solvent may exclude edible organic solvents, such as ethanol, propylene glycol and/or glycerol. Suitably, the aqueous environment according to the present invention may comprise less than 80% by volume organic solvents, less than 70% by volume organic solvents, less than 50% by volume organic solvents, less than 30% by volume organic solvents, more preferably less than 15% by volume organic solvents, more preferably less than 5%. Suitably the foodstuff may comprise between 1 and 5% organic solvent, for example ethanol. However, when the foodstuff comprises such an organic solvent, preferably it is produced endogenously within the foodstuff. That is to say, when the foodstuff comprises such an organic solvent, preferably the organic solvent is not an exogenous organic solvent.

The term "foodstuff" as used herein means a substance which is suitable for human and/or animal consumption.

Suitably, the term "foodstuff" as used herein may mean a foodstuff in a form which is ready for consumption. Alternatively or in addition, however, the term foodstuff as used herein may mean one or more food materials which are used in the preparation of a foodstuff By way of example only, the term foodstuff encompasses both baked goods produced from dough as well as the dough used in the preparation of said baked goods.

In a preferred aspect the present invention provides a foodstuff as defined above wherein the foodstuff is selected from one or more of the following: eggs, egg-based products, including but not limited to mayonnaise, salad dressings, sauces, ice creams, egg powder, modified egg yolk and products made therefrom; baked goods, including breads, cakes, sweet dough products, laminated doughs, liquid batters, muffins, doughnuts, biscuits, crackers and cookies; confectionery, including chocolate, candies, caramels, halawa, gums, including sugar free and sugar sweetened gums, bubble gum, soft bubble gum, chewing gum and puddings; frozen products including sorbets, preferably frozen dairy products, including ice cream and ice milk, dairy products, including cheese, butter, milk, coffee cream, whipped cream, custard cream, milk drinks and yoghurts; mousses, whipped vegetable creams, meat products, including processed meat products; edible oils and fats, aerated and non-aerated whipped products, oil-in-water emulsions, water-in-oil emulsions, margarine, shortening and spreads including low fat and very low fat spreads; dressings, mayonnaise, dips, cream based sauces, cream based soups, beverages, spice emulsions and sauces.

Suitably the foodstuff in accordance with the present invention may be a "fine foods", including cakes, pastry, confectionery, chocolates, fudge and the like.

In one aspect the foodstuff in accordance with the present invention may be a dough product or a baked product, such as a bread, a fried product, a snack, cakes, pies, brownies, cookies, noodles, snack items such as crackers, graham crackers, pretzels, and potato chips, and pasta.

In a further aspect, the foodstuff in accordance with the present invention may be a plant derived food product such as flours, pre-mixes, oils, fats, cocoa butter, coffee whitener, salad dressings, margarine, spreads, peanut butter, shortenings, ice cream, cooking oils.

In another aspect, the foodstuff in accordance with the present invention may be a dairy product, including butter, milk, cream, cheese such as natural, processed, and imitation cheeses in a variety of forms (including shredded, block, slices or grated), cream cheese, ice cream, frozen desserts, yoghurt, yoghurt drinks, butter fat, anhydrous milk fat, other dairy products. The enzyme according to the present invention may improve fat stability in dairy products.

It is particularly advantageous to utilise the present invention in cheese as the production of free fatty acids in cheese is associated with a "soapy" taste. Thus, the use of a lipid acyltransferase in accordance with the present invention advantageously produces cheese without a "soapy" taste.

In another aspect, the foodstuff in accordance with the present invention may be a food product containing animal derived ingredients, such as processed meat products, cooking oils, shortenings.

In a further aspect, the foodstuff in accordance with the present invention may be a beverage, a fruit, mixed fruit, a vegetable or wine. In some cases the beverage may contain up to 20 g/1 of added phytosterols.

In another aspect, the foodstuff in accordance with the present invention may be an animal feed. The animal feed may be enriched with phytosterol and/or phytostanols, preferably with beta-sitosterol/stanol. Suitably, the animal feed may be a poultry feed. When the foodstuff is poultry feed, the present invention may be used to lower the cholesterol content of eggs produced by poultry fed on the foodstuff according to the present invention.

In one aspect preferably the foodstuff is selected from one or more of the following:

eggs, egg-based products, including mayonnaise, salad dressings, sauces, ice cream,

egg powder, modified egg yolk and products made therefrom.

Preferably the foodstuff according to the present invention is a water containing foodstuff. Suitably the foodstuff may be comprised of 10-98% water, suitably 14-98%, suitably of 18-98% water, suitably of 20-98%, suitably of 40-98%, suitably of 50-98%, suitably of 70-98%, suitably of 75-98%.

For some aspects, preferably the foodstuff in accordance with the present invention is not a pure plant derived oil, such as olive oil, sunflower oil, peanut oil, rapeseed oil for instance. For the avoidance of doubt, in some aspects of the present invention the foodstuff according to the present invention may comprise an oil, but preferably the foodstuff is not primarily composed of oil or mixtures of oil. For some aspects, preferably, the foodstuff comprises less than 95% lipids, preferably less than 90% lipids, preferably less than 85%, preferably less than 80% lipids. Thus, for some aspects of the present invention oil may be a component of the foodstuff, but preferably the foodstuff is not an oil *per se.*

The claims of the present invention are to be construed to include each of the foodstuffs listed above.

When it is the case that a carbohydrate ester is produced in accordance with the present invention, the carbohydrate ester is preferably an oligosaccharide ester, a monosaccharide ester or a disaccharide ester.

Suitably, the carbohydrate ester when produced if accordance with the present invention may be one or more of the following: glucose ester, fructose ester, anhydrofructose ester, maltose ester, lactose ester, galactose ester, xylose ester, xylooligosaccharide ester, arabinose ester, maltooligosaccharide ester, tagatose ester, sucrose ester, microthecin ester, ascopyrone P ester, ascopyrone T ester or cortalcerone ester.

Preferably, the carbohydrate ester when produced in accordance with the present invention is one or more of the following: a carbohydrate mono-ester, a sugar mono-ester, an oligosaccharide mono-ester, a trisaccharide mono-ester, a disaccharide mono-ester, a monosaccharide mono-ester, a glucose mono-ester, a fructose mono-ester, anhydrofructose mono-ester, maltose mono-ester, lactose mono-ester, galactose mono--ester, xylose mono-ester, xylooligosacchride mono-ester, arabinose mono-ester, maltooligosaccharide mono-ester, tagatose mono-ester, sucrose mono-ester, microthecin ester, ascopymne P ester, ascopyrone T ester or cortalcerone ester.

In one embodiment, the microthecin ester, ascopyrone P ester, ascopyrone T ester and/or cortalcerone ester may function as an antimicrobial agent. Alternatively or in addition thereto, the microthecin ester, ascopyrone P ester, ascopyrone T ester and/or cortalcerone ester may function as one or both of an antioxidant and/or emulsifier.

Preferably, the formation of the carbohydrate ester (if any) in accordance with the present invention is independent of UDP-glucose.

Preferably, the foodstuff according to the present invention does not comprise UDP-glucose, or only comprises UDP-glucose in insignificant amounts. Suitably, the emulsifier in accordance with the present invention may be for example one or more of the following: a diglyceride, a monoglyceride, such as 1-monoglyceride or a lysolecithin, such as lysophosphatidylcholine for example, a digalactosyl monoglyceride (DGMG). The emulsifier is preferably produced from the lipid acyl donor following removal of one or more acyl groups from said lipid acyl donor. The term lysolecithin as used herein encompasses lysophosphatidylcholine, lysophosphatidylethanolamine, lysophosphatidylinositol, lysophosphatidylserine and lysophosphatidylglycerol

Where one of the emulsifiers is a carbohydrate ester, the second emulsifier may be for example one or more of the following: a diglyceride, a monoglyceride, such as 1-monoglyceride, lysophosphatidylcholine, or digalactosyl monoglyceride (DGMG). The second emulsifier is preferably produced from the lipid acyl donor following removal of one or more acyl groups from said lipid acyl donor. The term lysophosphatidylcholine as used herein is synonymous with the term lysolecithin and these terms may be used herein interchangeably.

Preferably the second emulsifier is DGMG. Suitably, the DGMG is produced *in situ* by the removal of an acyl group from DGDG with the transfer of the removed acyl group onto a carbohydrate to form a carbohydrate ester.

Where one of the emulsifiers is a protein ester and/or a diglyceride and/or a monoglyceride, the second emulsifier may be for example one or more of the following: a diglyceride, a monoglyceride, such as l-monoglyceride, lysophosphatidylcholine, or digalactosyl monoglyceride (DGMG). The second emulsifier is preferably produced from the lipid acyl donor following removal of one or more acyl groups from said lipid acyl donor. The term lysophosphatidylcholine as used herein is synonymous with the term lysolecithin and these terms may be used herein interchangeably.

In one embodiment the lipid acyl transferase can be used in a process for the preparation of a foodstuff such as a cooking oil, margarine or spread, whereby the foodstuff naturally contains, or has been supplemented with, glycerol, at least one phospholipid (for example lecithin) and/or glycolipid (for example digalactosyldiglyceride), and optionally a phytosterol or phytostanol.

When used as a cooking oil or margarine, the foodstuff may have enhanced anti-plattering properties. In addition or alternatively the foodstuff may have one or more beneficial technical properties, for example improved oxidative stability, improved emulsification properties, or health benefits.

The lipid acyl transferase for use in the invention can be in the preparation of low fat foodstuffs, such as low fat spreads, low fat salad dressings, low fat mayonnaise, low fat margarines etc. In such low fat food products, the fat content is typically reduced by the addition of emulsifiers and additional water compared to the higher fat equivalent.

The lipid acyl transferases used in the compositions and methods of the invention have been found to have unique properties when compared to lipolytic enzymes in that they have a marked preference for transfer of acyl groups from lipids to acceptors other than water, even in the presence of significant water. In a comparison with prior art enzymes, the lipid acyl transferase used in the invention were found to have a high relative transferase activity in the presence of 6% water, 54% water, 73% water, 89% water and approximately 95%. Lipolytic enzymes tested had virtually no significant relative transferase activity at these water concentrations.

The lipase and acyltransferase activity of an enzyme may be evaluated using the following assays. In this way, a lipid acyltransferase having the enzyme characteristics defined herein may be obtained/identified.

### Transferase Assay in Buffered Substrate (see Example 12)

Enzymes which function as lipid acyltransferases for use in the compositions and methods of the invention can be routinely identified using the assay taught herein in Example 12. This assay will be hereinafter referred to as the 'Transferase Assay in Buffered Substrate'. In Example 12 the lipid acyltransferase enzyme from *Aeromonas salmonicida* in accordance with the present invention was analysed and compared with a range of lipolytic enzymes not encompassed by the present invention. As can be seen, of the lipolytic enzymes only LIPOPAN® F (Novozymes, Denmark) was found to have any transferase activity and then only a very low level (1.3%).

Enzymes suitable for use in the compositions and methods of the invention can be routinely identified using the Transferase Assay in Buffered Substrate. Using this assay, in which there is a very high water content - approximately 95%, lipid acyltransferases in accordance with the present invention are those which have at least 2% acyltransferase activity (relative transferase activity), preferably at least 5% relative transferase activity, preferably at least 10% relative transferase activity, preferably at least 15%, 20%, 25% 26%, 28%, 30%, 40% 50%, 60% or 75% relative transferase activity. Suitably, the lipid acyltransferase in accordance with the present invention may have less than 28%, less than 30%, preferably less than 40%, 50%, 60%, 70%, 80%, 90% or 100% acyltransferase activity.

### Transferase Assay in high water egg yolk (see Example 11)

In addition to using the "Transferase Assay in Buffered Substrate" (see above), a lipid acyltransferase for use in accordance with the present invention may be identified using the "Transferase Assay in High Water Egg Yolk" taught in Example 11.

In one embodiment, the lipid acyltransferase suitable for use in the methods and/or compositions according to the present invention is one which when tested using the Transferase Assay in High Water Egg Yolk in an egg yolk with 54% water, has up to 100% relative transferase activity. Indeed, experiments in high water egg yolk have shown that at the start of the experiment the initial transferase rate was calculated to be 100% transferase activity, *i.e.* no hydrolytic activity was observed. In contrast, the lipolytic enzymes used as control, i.e. LIPOPAN® F and phospholipase A2, showed no detectable transferase activity in egg yolk with 54% water, or egg yolk with enriched water content (namely egg yolk with 73% water or 89% water). Preferably the increase in water content does not significantly decrease the percentage acyl transferase activity of a lipid acyltransferase for use in the methods or compositions according to the present invention.

In a preferable embodiment, with reference to the Transferase Assay in High Water Egg Yolk, with a water content of 54%, a lipid acyltransferase for use in the present invention will have an initial percentage acyltransferase activity (initial relative transferase activity) measured after 10% consumption of the donor molecule (i.e. phospholipid) of at least 0.1% relative transferase activity, preferably at least 1% relative transferase activity, preferably at least 5% relative transferase activity, preferable at least 10% relative transferase activity, preferably at least 20% relative transferase activity, preferably at least 30% relative transferase activity, preferably at least 40% relative transferase activity, preferably at least 50% relative transferase activity, preferably at least 60%, preferably at least 70%, preferably at least 80%, preferably at least 90%, preferably at least 95%, preferably at least 99%, preferably about 100% acyl transferase activity.

In a preferable embodiment, with reference to the Transferase Assay in High Water Egg Yolk, with a water content of 54%, and measured after 10% consumption of the donor molecule (i.e. phospholipid), the lipid acyltransferase for use in the compositions and methods of the invention has detectable transferase activity, *i.e.* relative transferase activity of between 0.1 and 100%, preferably at least 1% relative transferase activity, preferably at least 5% relative transferase activity, preferable at least 10% relative transferase activity, preferably at least 20% relative transferase activity, preferably at least 30% relative transferase activity, preferably at least 40% relative transferase activity preferably at least 45%, 50%, 60%, 70%, 80% or 90% relative transferase activity. Suitably, the lipid acyl transferase in accordance with the present invention may have, when using the Transferase Assay in High Water Egg Yolk with 54% water content and measured after 10% consumption of the donor molecule (i.e. phospholipid), a percentage acyl transferase activity (relative transferase activity) of less than 45%, 47%, 50%, 60%, 70%, 80%, 90% or 100%.

In a preferable embodiment, with reference to the Transferase Assay in High Water Egg Yolk, with a water content of 73%, measured after 10% consumption of the donor molecule (i.e. phospholipid), the lipid acyltransferase for use in the compositions and methods of the invention has detectable transferase activity, *i.e.* relative transferase activity of between 0.1 and 100%, preferably at least 1% relative transferase activity, preferably at least 5% relative transferase activity, preferable at least 10% relative transferase activity, preferably at least 20% relative transferase activity, preferably at least 30% relative transferase activity, preferably at least 40% relative transferase activity, preferably at least 45%, 50%, 58%, 60%, 70%, 80%, or 90% relative transferase activity. Suitably, the lipid acyl transferase in accordance with the present invention may have, when using the Transferase Assay in High Water Egg Yolk with 73% water content and measured after 10% consumption of the donor molecule (i.e. phospholipid), a percentage acyl transferase activity (relative transferase activity) of less than 45%, 47%, 50%, 58%, 60%, 70%, 80%, 90% or 100%.

In a preferable embodiment, with reference to the Transferase Assay in High Water Egg Yolk, with a water content of 89%, and measured after 10% consumption of the donor molecule (i.e. phospholipid), the lipid acyltransferase for use in the compositions and methods of the invention has detectable transferase activity, *i.e.* relative transferase activity of between 0.1 and 100%, preferably at least 1% relative transferase activity, preferably at least 5% relative transferase activity, preferable at least 10% relative transferase activity, preferably at least 20% relative transferase activity, preferably at least 30% relative transferase activity, preferably at least 40% relative transferase activity, preferably at least 45%, 50%, 60%, 70%, 80%, or 90% relative transferase activity. Suitably, the lipid acyl transferase in accordance with the present invention may have, when using the Transferase Assay in High Water Egg Yolk with 89% water content and measured after 10% consumption of the donor molecule (i.e. phospholipid), a percentage acyl transferase activity (relative transferase activity) of less than 45%, 47%, 50%, 60%, 70%, 80%, 90% or 100%.

In a preferable embodiment, with reference to the Transferase Assay in High Water Egg Yolk, a lipid acyltransferase for use in the compositions and methods of the invention has significant relative transferase activity (i.e. at least 0.1% at both water contents), and has an equivalent relative transferase activity in egg yolk with a water content of 54% as in an egg yolk with a water content of 73%, when measured after 10% consumption of the donor molecule (i.e. phospholipid).

In a preferable embodiment, with reference to the Transferase Assay in High Water Egg Yolk, a lipid acyltransferase for use in the compositions and methods of the invention has significant relative transferase activity (i.e. at least 0.1% at both water contents), and has an equivalent relative transferase activity in egg yolk with a water content of 54% as in an egg yolk with a water content of 89%, when measured after 10% consumption of the donor molecule (i.e. phospholipid).

In a preferable embodiment, with reference to the Transferase Assay in High Water Egg Yolk, a lipid acyltransferase for use in the compositions and methods of the invention has significant relative transferase activity (i.e. at least 0.1% at both water contents), and has an equivalent relative transferase activity in egg yolk with a water content of 73% as in an egg yolk with a water content of 89%, when measured after 10% consumption of the donor molecule (i.e. phospholipid).

The term "equivalent relative transferase activity" as referred to herein means that the enzyme has a relative transferase activity (% acyltransferase activity) which is at least 2% lower, preferably at least 5%, 10%, 20%, 30%, 40%, 50%, 60%, 70%, 80% or 90% lower, in the egg yolk with the higher water content compared with that in the egg yolk with the lower water content.

**Transferase Assay in a Low Water Environment**

In addition to using the "Transferase Assay in Buffered Substrate" and as an alternative to (or in addition to) using the "Transferase Assay in High Water Egg Yolk", lipid acyltransferases for use in accordance with the present invention may be identified using the "Transferase Assay in a Low Water Environment".

In order to determine if an enzyme is a lipid acyltransferase according to the present invention, one may carry out a "Transferase Assay in a Low Water Environment", namely in an oily environment with 6% water as taught in Example 22. This example illustrates that in an oily environment with 6% water content the lipid acyltransferase of the invention has a high relative transferase activity, where the prior art lipolytic enzymes have hydrolytic activity.

In one embodiment, the lipid acyltransferase suitable for use in the methods and/or compositions according to the present invention is one which when tested using the "Transferase Assay in a Low Water Environment", measured after a time period selected from 30, 20 or 120 minutes, has a relative transferase activity of at least 1%, preferably at least 2%, preferably at least 5%, preferably at least 10%, preferably at least 20%, preferably at least 30%, preferably at least 40%, preferably at least 50%, preferably at least 60%, preferably at least 70%, preferably at least 75%. Suitably, the lipid acyl transferase in accordance with the present invention may have less than 30%, 40%, 50%, 60%, 70%, or 80% activity when measured after a time period of 10, 20, 30 or 120 minutes using the "Transferase Assay in a Low Water Environment".

As described above, the lipase acyltransferase of the invention can be identified using the "Transferase Assay in Buffered Substrate" or, additionally, in the "Transferase Assay in Low Water Environment" using cholesterol as the acyl acceptor. Of course, the skilled person would be readily aware that, with obvious amendments to the analytical methods the 'Transferase Assay in Buffered Substrate' or the 'Transferase Assay in Low Water Environment" may be used to determine the lipid acyltransferase activity for any lipid acyl donor or any acyl acceptor combination. The skilled person would, if necessary, simply replace the acyl donor substrate (e.g. phospholipid) with an alternative acyl donor substrate (e.g. glycolipid, triacylglyceride) and/or replace the acyl acceptor (e.g. cholesterol) with an alternative acyl acceptor substrate (e.g. a carbohydrate, a protein, another sterol, a stanol or glycerol).

The term "high water" as used herein means any substrate or foodstuff with more than 2% water content, preferably more than 3%, 4%, 5%, 6%, 7%, 8%, 9%, 10%, 20%, 30%, 40%, 50%, 60%, 70%, 80% or 90%.

The term "low water" as used herein means any substrate or foodstuff with less than 6% water content, preferably less than 5%, 4%, 3%, 2%, 1% or 0.5%.

Preferably the method and/or use according to the present invention may be carried out, for example, in foodstuff at a temperature of 15-60°C, preferably at a temperature of 20-60°C, preferably 20-50°C, preferably 20-45°C, preferably 20-40°C. For some aspects, for example in dough, preferably the temperature of the food during which the acyltransferase reaction takes place is between 20 and 40°C. For other aspects, for example with regard to dairy products, such as cheese, the temperature of the food may suitably be between 30°C and 60°C. In yet other aspects, for example with regard to mayonnaise, the temperature of the food may suitably be between 20 and 40°C, more preferably between 25 and 30PC.

Preferably, the emulsifier produced according to the present invention comprises less than 5 wt % of the foodstuff.

Preferably, the emulsifier produced according to the present invention comprises from 0.01 to 4 wt % of the foodstuff.

Preferably, the emulsifier produced according to the present invention comprises from 0.01 to 2 wt % of the foodstuff.

Preferably, the emulsifier produced according to the present invention comprises from 0.01 to 1 wt % of the foodstuff.

Preferably, the emulsifier produced according to the present invention comprises from 0.01 to 0.5 wt % of the foodstuff.

Preferably, the emulsifier produced according to the present invention comprises from 0.01 to 0.3 wt % of the foodstuff.

Suitably, the method according to the present invention includes inactivating or denaturing the enzyme to provide a foodstuff comprising the enzyme in an inactive or denatured form. Suitably the enzyme may be denatured by either baking or by pasteurisation.

The present invention may further encompass the use of a lipid acyltransferase as defined herein in a water containing foodstuff comprising 10-98% water, and may encompass water containing foodstuff comprising 10-98% water comprising a lipid acyltransferase as defined herein. Such compositions may contain one or more further enzymes, such as those listed herein. Alternatively, the enzyme composition of the invention may be used in combination with other food ingredients/additives, such as those listed herein, including other enzyme compositions. By formulation of the lipid acyl transferase of the invention within a food and/or feed composition, the enzyme can be stabilised to allow for prolonged storage (under suitable conditions) prior to use in food and/or feed production. In addition the enzyme composition of the present invention provides the enzyme in a suitable form for safe use for the 'in situ' application in the preparation of foodstuffs and/or feedstuffs, or ingredients for use in food and/or feed preparation. Such compositions may be in either liquid, semi-liquid or solid/granular form.

The food enzyme composition may suitable be a dough improving composition. The dough improving composition may comprise other beneficial components such as an emulsifier and/or other enzymes as listed herein.

Food enzymes are sold as stabilised liquid concentrates or as particulate solids. Formulation into food enzyme composition minimises losses in enzymatic activity during transport, storage, and use. Enzymes are often exposed to humid, hot, or oxidative environments in food and beverage processing. Formulations enhance stability by counteracting the primary forces of deactivation: denaturation, catalyticsite deactivation, and proteololysis. Denaturation occurs by physical unfolding of an enzyme's tertiary protein structure under thermal or chemical stress. Once an enzyme begins to unfold it becomes dramatically more vulnerable to deactivation and proteolysis. To minimise unfolding, the formulator can alter the protein's environment so as to induce a compact protein structure; this is done most effectively by "preferential exclusion" of water from the protein surface by adding water-associating compounds such as sugars, polyhydric alcohols, and lyotropic salts. The best ways to combat active site inactivation are to ensure sufficient levels of any required cofactors, to add reversible inhibitors, and to exclude oxidising or reactive species from the formulation.

Besides enzymatic stability, a formulation should meet several key secondary requirements, including preservation against microbial contamination, avoidance of physical precipitation or haze formation, minimising the formation of sensitising dusts or aerosols, and the optimisation of aesthetic criteria such as colour and odour. Many of these problems are best addressed by focusing as far "upstream" as possible, including the choice of raw materials in the fermentation or enzyme recovery process. Downstream operations such as diafilttation, adsorption, chromatography, crystallization, and extraction can be used to remove impurities responsible for colour, odour, and precipitation The risk of physical precipitation is minimised by formulating near the isoelectric point of the enzyme with hydrophilic solvents such as glycerol or propylene glycol. One can effectively also add moderate levels of solvating salts to avoid either salting-out or "reverse salting-in". To prevent microbial contamination, one can use a combination of filtration, acidification, and the minimisation of free water; biocides can be effective, but the range of acceptable chemicals for controlling or killing microbes is increasingly circumscribed by health and safety regulations.

Two processes producing the most attrition-resistant granules to date are high-shear granulation and fluidised-bed spray coating, see for example T. Becker: "Separation and Purification Processes for Recovery of Industrial Enzymes" in R. K. Singh, S. S. H. Rizvi (eds.): Bioseparation Processes in Foods, Marcel Dekker, New York, pp. 427 - 445. These processes use various binders, coatings, and particle morphologies to produce nonfriable particles which still protect enzymes during storage but allow for their ready release in solution during use.

Food enzyme compositions containing the lipid acyl transferase for use in the invention may be made using standard formulation techniques, such as spray drying or liquid formulation.

The lipid acyl-transferase for use in the invention can be expressed in any suitable expression host. For example the lipid acyltransferase of the invention may be expressed in *Bacillus subtilis* and may be purified by ultrafiltration and/or by precipitation in ethanol and/or centrifugation, and may be subsequently spray dried using starch (maltodextrin) as carrier for the enzyme. The spray dried enzyme may be standardised to specified PLU activity by adding further carrier in powder form. The techniques involved are well established and routine in the art.

Alternatively, lipid acyltransferase for use in accordance with the present invention, for example the heterologously produced lipid acyl-transferase of the invention, once purified, may be stabilised in a suitable liquid formulation, such as those based on glycerol. Other methods of making stabilised enzyme formulations are described in EP 0 770 037 and EP 0 702 712.

The acyl transferase in powder form can also be used in combination with other enzymes as listed herein, for the production of enzyme compositions with defined activity according to the product specification.

Typically the dosage of the food enzyme formulation is between 10g and 1000g per 1000kg of foodstuff, preferably 50-200g per 1000kg of foodstuff, preferably, 75-125gm per 1000kg of foodstuff.

Preferably the enzyme according to the present invention is present in an inactive form or in a denatured form in the foodstuff.

In one embodiment, the enzyme according for use in the present invention is preferably not immobilised, in particular is not immobilised on a solid support.

In an alternative embodiment, the enzyme may be immobilised.

Immobilised lipid acyl transferase can be prepared using immobilisation techniques known in the art. There are numerous methods of preparing immobilised enzymes, which will be apparent to a person skilled in the art (for example the techniques referred to in EP 0 746 608; or Balcao VM, Paiva AL, Malcata FX., Enzyme Microb Technol. 1996 May 1;18(6):392-416; or Reetz MT, Jaeger KE.Chem Phys Lipids. 1998 Jun;93(1-2):3-14; or Bornscheuer UT, Bessler C, Srinivas R, Krishna SH.Trends Biotechnol. 2002 Oct; 20(10):433-7 .

In one embodiment, the foodstuff of the invention may contain food ingredients, which have been prepared using immobilised lipid acyltransferase, but do not contain the lipid acyltransferase in the food ingredient or foodstuff.

For example the foodstuff may contain one or more of the following: an emulsifier, more than one emulsifier, one or more flavouring agents, one or more textural enhancers and/or one or more sterol esters, such as phytosterol esters or phytostanol esters.

The enzyme according to the present invention may be used with one or more conventional emulsifiers, including for example monoglycerides, diacetyl tartaric acid esters of mono- and diglycerides of fatty acids, and lecithins e.g. obtained from soya. The enzyme according to the present invention may be used with one or more, other suitable food grade enzymes. Thus, it is within the scope of the present invention that, in addition to the enzyme of the invention, at least one further enzyme is added to the foodstuff. Such further enzymes include starch degrading enzymes such as endo- or exoamylases, pullulanases, debranching enzymes, hemicellulases including xylanases, cellulases, lipases, phospholipases, and proteases.

The enzyme according to the present invention may be used with one or more other suitable food grade enzymes. Thus, it is within the scope of the present invention that, in addition to the enzyme of the invention, at least one further enzyme is added to the foodstuff. Such further enzymes include starch degrading enzymes such as endo- or exoamylases, pullulanases, debranching enzymes, hemicellulases including xylanases, cellulases, oxidoreductases, e.g. glucose oxidase, pyranose oxidase, sulfhydryl oxidase or a carbohydrate oxidase such as one which oxidises maltose, for example hexose oxidase (HOX), lipases, phospholipases and hexose oxidase, and proteases.

In one preferred embodiment the lipid acyltransferase is used in combination with a lipase having one or more of the following lipase activities: glycolipase activity (E.C. 3.1.1.26, triacylglycerol lipase activity (E.C. 3.1.1.3), phospholipase A2 activity (E.C. 3.1.1.4) or phospholipase A1 activity (E.C. 3.1.1.32). Suitably, lipase enzymes are well know within the art and include by way of example the following lipases: LIPOPAN® F and/or LECTTASE® ULTRA (Novozymes A/S, Denmark), phospholipase A2 (e.g. phospholipase A2 from LIPOMOD™ 22L from Biocatalysts, LIPOMAX™ from Genecor), LIPOLASE® (Novozymes A/S, Denmark), the lipases taught in WO03/97835, EP 0 977 869 or EP 1 193 314. This combination of a lipid acyl transferase as defined herein and a lipase may be particularly preferred in dough or baked products or in fine food products such as cakes and confectionary.

The use of lipases in combination with the enzyme of the invention may be particularly advantageous in instances where some accumulation of free fatty acids maybe desirable, for example in cheese where the free fatty acids can impart a desirable flavour, or in the preparation of fine foods. The person skilled in the art will be able to combine proportions of lipolytic enzymes, for example LIPOPAN® F and/or LECTTASE® ULTRA (Novozymes A/S, Denmark), phospholipase A2 (e.g. phospholipase A2 from LIPOMOD™ 22L from Biocatalysts, LIPOMAX™ from Genecor), LIPOLASE® (Novozymes A/S, Denmark), the lipases taught in WO03/97835, EP 0 977 869 or EP 1 193, 314 and the lipid acyltransferase of the present invention to provide the desired ratio of hydrolytic to transferase activity which results in a preferred technical effect or combination of technical effects in the foodstuff (such as those listed herein under Technical Effects').

Traditionally the cake industry uses cake improvers for the production of cakes and to secure high quality cakes in terms of taste, structure, eating quality and appearance. These cake improvers are normally based on emulsifiers spray dried on a carrier like starch and malto dextrin. Some cake improvers are also in a gel form based on emulsifiers, sugars and water. These cake improvers are very important for the cake industry in order to produce cake of high quality. Cake improvers however contain emulsifiers and other "non-natural" ingredients with an E-number. Because of demand for the consumers to reduce the numbers of E-numbers, the cake industry has asked for alternative ways to produce cakes of high quality without using emulsifiers.

An alternative way to produce cake is to use an enzyme, i.e. the lipid acyltransferase defined herein or an enzyme composition according to the present invention.

The lipid acyltransferase as defined herein and/or the food enzyme composition of the present invention may be used in the preparations of a fine food, such as a cake. In such instances, the following constituents may be formed in the fine food:

i) sugar esters and lysolecithin (from the carbohydrate in the cake recipe and the lecithin in egg which also form part of the cake recipe); and/or

ii) acylated peptides and lysolecithin (by transferring a fatty acid from lecithin to a protein or peptide during formation of protein-fatty acid condensates, which are known to be highly efficient emulsifiers (Herstellung und Anvendungmöglichkeiten von Eiweiss-Fettsäurekondensaten. Andreas Sander, Eberhard Eilers, Andrea Heilemann, Edith von Kreis.Fett/lipid 99 (1997) Nr. 4, 115-120).

It is considered that in the production of some fine foods, particularly high fat fine foods, such as cakes, it may be desirable to have some accumulation of fatty acids. Therefore the combination of the use of lipolytic enzymes and the lipid acyl transferase as defined herein may be particularly beneficial for production of high fat fine foods. Alternatively, additional free fatty acids or fatty acid soap (E470a) may be selected and used in combination with the lipid acyl transferase.

The foodstuff according to the present invention may suitably comprise one or more of the following additives:

soy protein material; carotenoids, flavenoids, antioxidant and phytochemical (especially anthocyanonide, carotenoid, bioflavinoid, glutathione, catechin, isoflavone, lycopene, ginsenoside, pycnogenol, alkaloid, pygeum phytosterol, sulphoraphone, resveretol, grape seed extract or food containing stanol esters), vitamin (especially vitamin C, vitamin A, vitamin B3, vitamin D, vitamin E, thiamine, riboflavin, niacin, pyridoxine, cyanocobalamin, folic acid, biotin, pantothenic acid or vitamin K), minerals (especially calcium, iodine, magnesium, zinc, iron, selenium, manganese, chromium, copper, cobalt, molybdenum or phosphorus), fatty acid (especially gamma-linoleic acid, ucospentaenoic acid or decosahexaenoic acid), oil (especially borage oil, high carotenoid canola oil or flax seed oil), amino acid (especially tryptophan, lysine, methionine, phenylalanine, threonine, valine, leucine, isoleucine, alanine, arginine, aspartic acid, cystine, cysteine, glutamic acid, glutamine, glycine, histidine, proline, hydroxyproline, serine, taurine or tyrosine), enzyme (especially bromelain, papain, amylase, cellulase or coenzyme Q), lignin, stanol ester or friendly bacteria (especially Lactobacillus acidophilus, Lactobacillus bulgaricus, Lactobacillus bifidus, Lactobacillus plantarum or Streptococcus faecium), folic acid, and soluble fibre.

TECHNICAL EFFECT

Surprisingly lipid acyltransferases have significant acyltransferase activity in foodstuffs. This activity has surprising beneficial applications in methods of preparing foodstuffs.

The present invention is predicated upon the surprising finding that the lipid acyltransferases according to the present invention can perform carbohydrate-esterification via alcoholosis, i.e. acyl transfer from a lipid, in a foodstuff with a significant water content Prior art suggests that such enzymes if they would function at all in this manner would only function in a solvent environment (i.e. in environments with low or no water content).

The present invention may provide one or more of the following unexpected technical effects in egg products, particularly mayonnaise: an improved heat stability during pasteurisation; improved organoleptic properties, an improved consistency.

The present invention may provide one or more of the following unexpected technical effects in dough and/or baked products: an improved specific volume of either the dough or the baked products (for example of bread and/or of cake); an improved dough stability; an improved crust score (for example a thinner and/or crispier bread crust), an improved crumb score (for example a more homogenous crumb distribution and/or a finer crumb structure and/or a softer crumb); an improved appearance (for example a smooth surface without blisters or holes or substantially without blisters or holes); a reduced staling; an enhanced softness; an improved odour; an improved taste.

The present invention may provide a beneficial effect from formation of highly surface-active materials in a foodstuff without formation of substantial amount of free fatty acids, which reduce the ability of the foodstuff to oxidize upon storage, because free fatty acids are more prone to oxidation than the corresponding fatty acid esters. Suitably, the present invention may provide one or more of the following unexpected technical effects in a foodstuff: an improved appearance, an improved mouthfeel, an improved stability, in particular an improved thermal stability, an improved taste, an improved softness, an improved resilience, an improved emulsification.

Suitably, the present invention may provide one or more of the following unexpected technical effects in dairy products, such as ice cream for example: an improved motrthfeel (preferably a more creamy mouthfeel); an improved taste; an improved meltdown.

Suitably, the present invention may provide one or more of the following unexpected technical effects is egg or in egg products: improved stability of emulsion; thermal stability of emulsion; improved flavour; reduced mal-odour; improved thickening properties, improved consistency.

Specific technical effects associated with the use of a lipid acyltransferase as defined herein in the preparation of a foodstuff are listed in the table below:

| | Foodstuff | Effect |
|---|---|---|
| 1 | Bread, Muffins and Doughnuts | Strengthens dough and increases mechanical resistance and increases water absorption capacity. Increases volume of bakery products and maintains softness of crumb |
| 2 | Frozen dough | Prevents spoiling during refrigeration |
| 3 | Sponge cake | Makes good cake volume and a uniform soft texture |
| 4 | Biscuit, cracker and cookie | Makes stable emulsions of fat and prevents stickiness to the machine. Prevents blooming of high fat products |

(continued)

| | Foodstuff | Effect |
|---|---|---|
| 5 | Batter and breading | Improves texture of fried products. |
| 6 | Noodles | Prevents dough from sticking to the machine. Increases water content, and deceases cooking loss |
| 7 | Instant noodles | Prevent noodles form adhering to each other |
| 8 | Pasta | Dough conditioner prevents adhesion on cooking. |
| 9 | Custard cream | Makes starch paste with a smooth and creamy texture, and prevents dehydration. |
| 10 | Coffee whitener | Prevent oil and water separation |
| 11 | Whipping cream | Provides stable emulsion |
| 12 | Chocolate | Prevents or reduced blooming |
| 13 | Caramel, candy and nougat | Improves emulsification of molten sugar and oil. Prevents separation of oil. |
| 1 4 | Processed meat, sausages | Improves water holding capacity of sausages and pressed ham, and prevents separation of oil phase of pastes and pâté. |

Suitably, the present invention may provide one or more of the following unexpected technical effects in cheese: a decrease in the oiling-off effect in cheese; an increase in cheese yield; an improvement in flavour; a reduced malodour, a reduced "soapy" taste.

In food production, in particular cheese production, the use of the lipid acyltransferase in accordance with the present invention provides a significant advantage in the ability to recover soluble proteins from dairy products. For example, in cheese production nearly 20% of all milk protein is removed in the whey (i.e. the watery part of the milk that remains after the formation of curds). The whey comprises the soluble milk proteins, whereas the hydrophobic proteins are maintained in the curd. By use of the lipid acyltransferase in accordance with the present invention it is possible to transfer an acyl group from a lipid (preferably from a glycolipid or a phospholipid), to a protein (in particular to a whey protein such as lactoglobulin) to from a protein fatty acid condensate. Thus, producing a product which is more hydrophobic and which will stay in the curd rather than being eluted in the whey. In this way, more of the milk protein can be maintained in the final foodstuff, i.e. the final dairy product such as the cheese.

In one aspect, the present invention is based in part on the realisation that yields of foods - such as cheese - may be improved by the use of a lipid acyl transferase. In addition or alternatively, the flavour, texture, oxidative stability and/or shelf life of the food may be improved. In addition or alternatively, the food may have a reduced cholesterol level or enhanced content of phytosterol/stanol esters.

Without wishing to be bound to a particular theory it is considered that the increase in yield may be the result of the transesterification of whey proteins and peptides, resulting in significant increase in the hydrophobicity of the whey proteins and precipitation of the acylated whey proteins in the cheese curd.

In biological systems, for example, the deposition of membrane bound proteins and enzymes are achieved by two different mechanisms. The membrane bound proteins either possess a number of membrane-spanning or hydrophobic domains, or they have alternatively a fatty acid linked to the polypeptide chain. The fatty acids have normally a chain length of 14 or 16 carbon atoms. The fatty acids are covalently linked to the polypeptide chain at 3 different position, the N-terminal amino acid as an amide-bond, a cysteine residue as a thioester linkage, or a serine or threonine amino acid as an ester linkage. Only one fatty acid per polypeptide molecule is necessary to incorporate the protein into the cell membrane.

When a fatty acid is covalently linked to a non-membrane protein, the physical and functional properties will change drastically. WO97/14713 describes the transformed soy and gluten proteins into acyl derivatives by treatment with a lipase from *Mucor miehei* (Lipozyme™, Novozymes), and a fatty acid in organic solvent. A lipid acyl transferase may be used in the production of acylated proteins is a low or high water environment.

We note that acylated proteins form amphiphilic complexes that can be used for a number of cosmetic products. The acylated protein can form gels, bind water by retaining moisture, have emulsifying properties and is very active in the interphase between water and lipid.

Thus, a cosmetic composition comprising a lipid acyl transferase as defined herein is disclosed herein.

The present invention may provide the use of an acyltransferase as defined herein to produce a cosmetic composition is also disclosed herein.

A method of *in situ* production of a protein ester in a cosmetic composition, wherein the method comprises the step of adding to the cosmetic composition (or components thereof) a lipid acyltransferase as defined herein, is disclosed.

Many food proteins are soluble in aqueous solutions and are therefore suitable for *in situ* modification by the lipase acyl transferase. In the cheese production, β-lactoglobulin is lost to the whey fraction. After acylation with a lipase acyl transferase, or a lipase acyl transferase variant, initial results indicate that b-lactoglobulin may however, be deposited in the casein micelle surface during rennet coagulation. β-lactoglobulin has three potential acylation sites (serine residues) on three surface loops. Milk contains sufficient amounts of lecithin, a suitable substrate for a lipid acyl transferase enzyme to acylate the β-lactoglobulin. The lysolecithin formed may have an additional emulsifying effect.

The improvements observed with a lipid acyltransferase according are in comparison to when lipolytic enzymes without acyltransferase activity, such as triacylglycerol lipases and phospholipases, are used.

ADVANTAGES

The generation of an emulsifier and a sterol/stanol ester *in situ* from at least one constituent of the food material, means that the food material will contain at least one less additive material. This is advantageous because of the improvement in the ease of production. For example, no further processing or addition of ingredients or addition of emulsifiers may be required. Moreover, the foodstuff may contain less "additives". The reduction or elimination of "additives" is desirable to consumers and inclusion of additives often must be declared to the consumer in the ingredients listing on the foodstuff. Thus, the present invention is further advantageous.

An advantage of the present invention may be the production *in situ* of an emulsifier in a foodstuff without a detrimental increase in the free fatty acid content of the foodstuff.

The generation of two emulsifiers and/or a carbohydrate ester *in situ* from at least one constituent of the food material, means that the food material will contain at least one less additive material.

In addition, when the lipid acyltransferase acts on a glycolipid it is possible to advantageously produce the emulsifier DGMG *in situ* without a detrimental increase in the free fatty acid content of the foodstuff. Thus, reducing detrimental effects attributed to an increase in free fatty acids, including but not limited to a reduction in "soapy" taste in cheese, prevention of overdosing in dough and dough baked properties.

For some aspects, an advantage of the present invention is the reduction in free cholesterol levels in the foodstuff.

For other aspect, an advantage of the present invention is the increase in stanol and/or sterol esters in the foodstuff. Some sterol/stanol esters may be effective flavourants and/or texturisers. Thus, the present invention may not only results in the *in situ* production of an emulsifier in a foodstuff, but also the *in situ* production of a flavourant and/or a texturiser. Some sterol/stanol esters are known to reduce blood serum cholesterol and/or low density lipoproteins when consumed in a foodstuff. Thus, the present invention may be used to prepare a foodstuff with increased levels of sterol esters and/or stanol esters.

For some aspects, particularly when the enzyme according to the present invention is used in egg based products, an advantage is the removal of unwanted free carbohydrates.

Also advantageously the emulsification properties of the foodstuff are enhanced, leading to improved appearance and/or handling properties and/or structure and/or consistency and/or heat stability without a negative impact on taste.

In addition, for some embodiments advantageously the effect of "overdosing" observed when using lipases *per se,* is effectively overcome by the addition of an enzyme in accordance with the present invention. This is due at least in part to the fact that free fatty acids are not produced or only produced to an insignificant degree when using the enzyme according to the present invention.

ISOLATED

In one aspect, preferably the polypeptide or protein for use in the present invention is in an isolated form. The term "isolated" means that the sequence is at least substantially free from at least one other component with which the sequence is naturally associated in nature and as found in nature.

PURIFIED

In one aspect, preferably the polypeptide or protein for use in the present invention is in a purified form. The term "purified" means that the sequence is in a relatively pure state - e.g. at least about 51 % pure, or at least about 75%, or at least about 80%, or at least about 90% pure, or at least about 95% pure or at least about 98% pure.

CLONING A NUCLEOTIDE SEQUENCE ENCODING A POLYPEPTIDE ACCORDING TO THE PRESENT INVENTION

A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide. If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

NUCLEOTIDE SEQUENCES

The present invention also encompasses nucleotide sequences encoding polypeptides having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be doublestranded or single-stranded whether representing the sense or antisense strand.

The term "nucleotide sequence" in relation to the present invention includes genomic DNA, cDNA, synthetic DNA, and RNA. Preferably it means DNA, more preferably cDNA for the coding sequence.

In a preferred embodiment, the nucleotide sequence *per se* encoding a polypeptide having the specific properties as defined herein does not cover the native nucleotide sequence in its natural environment when it is linked to its naturally associated sequence(s) that is/are also in its/their natural environment. For ease of preference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment Thus, the polypeptide of the present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, in an alternative embodiment of the invention, the nucleotide sequence could be synthesised, in whole or in part, using chemical methods well known in the art (see Caruthers MH et al (1980) Nuc Acids Res Symp Ser 215-23 and Hom T et al (1980) Nuc Acids Res Symp Ser 225-232).

MOLECULAR EVOLUTION

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme in accordance with the present invention.

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characterisitics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shufifing and family shuffling technologies are suitable for the production of variants of lipid acyl transferases with preferred characteristics. Suitable methods for performing 'shuffling' can be found in EP0 752 008, EP1 138 763, EP1 103 606. Shuftling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The application of the above-mentioned and similar molecular evolution methods allows the identification and selection of variants of the enzymes of the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate

As will be apparent to a person skilled in the art, using molecular evolution tools an enzyme may be altered to improve the functionality of the enzyme.

Suitably, the lipid acyltransferase used in the invention may be a variant, i.e. may contain at least one amino acid substitution, deletion or addition, when compared to a parental enzyme. Variant enzymes retain at least 1%, 2%, 3%, 5%, 10%, 15%, 20%, 30%, 40%, 50 %, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

In a preferable embodiment a variant lipid acyltransferase enzyme retains or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods of the present invention.

Suitably, the lipid acyltransferase for use in the invention may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases for use in the invention may have decreased activity on triglycerides, and/or monoglycerides and/or diglycerides compared with the parent enzyme.

Suitably the variant enzyme may have no activity on triglycerides and/or monoglycerides and/or diglycerides.

Alternatively, the variant enzyme for use in the invention may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, and one or more of such variants may be suitable for use in the methods and uses according to the present invention and/or in the enzyme compositions according to the present invention. By way of example only, ' variants of lipid acyltransferases are described in the following references may be used in accordance with the present invention: Hilton & Buckley J Biol. Chem. 1991 Jan 15: 266 (2): 997-1000; Robertson et al J. Biol. Chem. 1994 Jan 21; 269(3):2146-50; Brumlik et al J. Bacteriol 1996 Apr, 178 (7): 2060-4; Peelman et al Protein Sci. 1998 Mar; 7(3):587-99.

AMINO ACID SEQUENCES

The present invention also encompasses amino acid sequences of polypeptides having the specific properties as defined herein.

As used herein, the term "amino acid sequence" is synonymous with the term "polypeptide" and/or the term "protein". In some instances, the term "amino acid sequence" is synonymous with the term "peptides".

The amino acid sequence may be prepared/isolated from a suitable source, or it may be made synthetically or it may be prepared by use of recombinant DNA techniques.

Suitably, the amino acid sequences may be obtained from the isolated polypeptides taught herein by standard techniques.

One suitable method for determining amino acid sequences from isolated polypeptides is as follows:

Purified polypeptide may be freeze-dried and 100 μg of the freeze-dried material may be dissolved in 50 μl of a mixture of 8 M urea and 0.4 M ammonium hydrogen carbonate, pH 8.4. The dissolved protein may be denatured and reduced for 15 minutes at 50°C following overlay with nitrogen and addition of 5 μl of 45 mM dithiothreitol. After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.

The resulting peptides may be separated by reverse phase HPLC on a VYDAC C18 column (0.46x15cm;10μm; The Separation Group, California, USA) using solvent A: 0.1 % TFA in water and solvent B: 0.1% TEA in acetonitrile. Selected peptides may be re-chromatographed on a Develosil C 18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and-4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software that can perform sequence comparisons include, but are not limited to, the BLAST package (see Ausubel et al 1999 Short Protocols in Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages. 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the pubic default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1),237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the a-carbon substituent group is on the residue's nitrogen atom rather than the a-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences for use in the present invention or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences.

The present invention also encompasses the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.

Polynucleotides which are not to homologous to the sequences of the preset invention but fall within the scope of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides.

Polynucleotides (nucleotide sequences) of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

HYBRIDISATION

The present invention also encompasses sequences that are complementary to the sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the subject sequences discussed herein, or any derivative, fragment or derivative thereof.

The present invention also encompasses sequences that are complementary to sequences that are capable of hybridising to the nucleotide sequences discussed herein.

Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

Preferably, the present invention encompasses sequences that are complementary to sequences that are capable of hybridising under high stringency conditions or intermediate stringency conditions to nucleotide sequences encoding polypeptides having the specific properties as defined herein.

More preferably, the present invention encompasses sequences that are complementary to sequences that are capable of hybridising under high stringent conditions (e.g. 65°C and 0.1xSSC {1xSSC = 0.15 M NaCl, 0.015 M Na-citrate pH 7.0}) to nucleotide sequences encoding polypeptides having the specific properties as defined herein.

The present invention also relates to nucleotide sequences that can hybridise to the nucleotide sequences discussed herein (including complementary sequences of those discussed herein).

The present invention also relates to nucleotide sequences that are complementary to sequences that can hybridise to the nucleotide sequences discussed herein (including complementary sequences of those discussed herein).

Also included within the scope of the present invention are polynucleotide sequences that are capable of hybridising to the nucleotide sequences discussed herein under conditions of intermediate to maximal stringency.

In a preferred aspect, the present invention covers nucleotide sequences that can hybridise to the nucleotide sequences discussed herein, or the complement thereof, under stringent conditions (e.g. 50°C and 0.2xSSC).

In a more preferred aspect, the present invention covers nucleotide sequences that can hybridise to the nucleotide sequences discussed herein, or the complement thereof, under high stringent conditions (e.g. 65°C and 0.1xSSC).

EXPRESSION OF POLYPEPTIDES

A nucleotide sequence for use in the present invention or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

## EXPRESSION VECTOR

The term "expression vector" means a construct capable of *in vivo* or *in vitro* expression.

Preferably, the expression vector is incorporated in the genome of the organism. The term "incorporated" preferably covers stable incorporation into the genome.

The nucleotide sequence of the present invention or coding for a polypeptide having the specific properties as defined herein may be present in a vector, in which the nucleotide sequence is operably linked to regulatory sequences such that the regulatory sequences are capable of providing the expression of the nucleotide sequence by a suitable host organism, i.e. the vector is an expression vector.

The vectors of the present invention may be transformed into a suitable host cell as described below to provide for expression of a polypeptide having the specific properties as defined herein.

The choice of vector, e.g. plasmid, cosmid, virus or phage vector, will often depend on the host cell into which it is to be introduced.

The vectors may contain one or more selectable marker genes - such as a gene which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect or transform a host cell.

Thus, in a further embodiment, the invention provides a method of making nucleotide sequences of the present invention or nucleotide sequences encoding polypeptides having the specific properties as defined herein by introducing a nucleotide sequence into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication ofplasmids pUC19, pACYC177, pUB110, pE194, pAMB1 and pIJ702.

## REGULATORY SEQUENCES

In some applications, a nucleotide sequence for use in the present invention or a nucleotide sequence encoding a polypeptide having the specific properties as defined herein may be operably linked to a regulatory sequence which is capable of providing for the expression of the nucleotide sequence, such as by the chosen host cell. By way of example, the present invention covers a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.

The term "regulatory sequences" includes promoters and enhancers and other expression regulation signals.

The term "promoter" is used in the normal sense of the art, e.g. an RNA polymerase binding site.

Enhanced Expression of the nucleotide sequence encoding the enzyme having the specific properties as defined herein may also be achieved by the selection of heterologous regulatory regions, e.g. promoter, secretion leader and terminator regions.

Preferably, the nucleotide sequence of the present invention may be operably linked to at least a promoter.

Examples of suitable promoters for directing the transcription of the nucleotide sequence in a bacterial, fungal or yeast host are well known in the are

## CONSTRUCTS

The term "construct" - which is synonymous with terms such as "conjugate", "cassette" and "hybrid" - includes a nucleotide sequence encoding a polypeptide having the specific properties as defined herein for use according to the present invention directly or indirectly attached to a promoter. An example of an indirect attachment is the provision of a suitable spacer group such as an intron sequence, such as the Shl-intron or the ADH intron, intermediate the promoter and the nucleotide sequence of the present invention. The same is true for the term "fused" in relation to the present invention which includes direct or indirect attachment In some cases, the terms do not cover the natural combination of the nucleotide sequence coding for the protein ordinarily associated with the wild type gene promoter and when they are both in their natural environment

The construct may even contain or express a marker which allows for the selection of the genetic construct

For some applications, preferably the construct comprises at least a nucleotide sequence of the present invention or a nucleotide sequence encoding a polypeptide having the specific properties as defined herein operably linked to a promoter.

HOST CELLOS

The term "host cell" - in relation to the present invention includes any cell that comprises either a nucleotide sequence encoding a polypeptide having the specific properties as defined herein or an expression vector as described above and which is used in the recombinant production of a polypeptide having the specific properties as defined herein.

Thus, a further embodiment of the present invention provides host cells transformed or transfected with a nucleotide sequence of the present invention or a nucleotide sequence that expresses a polypeptide having the specific properties as defined herein. The cells will be chosen to be compatible with the said vector and may for example be prokaryotic (for example bacterial), fungal, yeast or plant cells. Preferably, the host cells are not human cells.

Examples of suitable bacterial host organisms are gram negative bacterium or gram positive bacteria.

Depending on the nature of the nucleotide sequence encoding a polypeptide having the specific properties as defined herein, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells, such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain.

ORGANISM

The term "organism" in relation to the present invention includes any organism that could comprise a nucleotide sequence according to the present invention or a nucleotide sequence encoding for a polypeptide having the specific properties as defined herein and/or products obtained therefrom.

Suitable organisms may include a prokaryote, fungus, yeast or a plant.

The term "transgenic organism" in relation to the present invention includes any organism that comprises a nucleotide sequence coding for a polypeptide having the specific properties as defined herein and/or the products obtained therefrom, and/or wherein a promoter can allow expression of the nucleotide sequence coding for a polypeptide having the specific properties as defined herein within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, a nucleotide sequence coding for a polypeptide having the specific properties as defined herein, constructs as defined herein, vectors as defined herein, plasmids as defined herein, cells as defined herein, or the products thereof. For example the transgenic organism can also comprise a nucleotide sequence coding for a polypeptide having the specific properties as defined herein under the control of a heterologous promoter.

TRANSFORMATION OF HOST CELLS/ORGANISM

As indicated earlier, the host organism can be a prokaryotic or a eukaryotic organism. Examples of suitable prokaryotic hosts include *E. coli* and *Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

In another embodiment the transgenic organism can be a yeast.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

TRANSFORMED FUNGUS

A host organism may be a fungus - such as a filamentous fungus. Examples of suitable such hosts include any member belonging to the genera Thermomyces, Acremonium, Aspergillus, Penicillium, Mucor; Neurospora, Trichoderma and the like.

Teachings on transforming filamentous fungi are reviewed in US-A-5741665 which states that standard techniques for transformation of filamentous fungi and culturing the fungi are well know in the art An extensive review of techniques as applied to *N. crassa* is found, for example in Davis and de Serres, Methods Enzymol (1971) 17A: 79-143.

Further teachings on transforming filamentous fungi are reviewed in US-A-5674707.

In one aspect, the host organism can be of the genus *Aspergillus,* such as *Aspergillus niger.*

A transgenic *Aspergillus* according to the present invention can also be prepared by following, for example, the teachings of Turner G. 1994 (Vectors for genetic manipulation. In: Martinelli S.D., Kinghorn J.R.( Editors) Aspergillus: 50 years on. Progress in industrial microbiology vol 29. Elsevier Amsterdam 1994. pp. 641-666).

Gene expression in filamentous fungi has been reviewed in Punt et al. (2002) Trends Biotechnol 2002 May;20 (5):200-6, Archer & Peberdy Crit Rev Biotechnol (1997) 17(4):273-306.

TRANSFORMED YEAST

In another embodiment, the transgenic organism can be a yeast.

A review of the principles of heterologous gene expression in yeast are provided in, for example, Methods Mol Biol (1995), 49:341-54, and Curr Opin Biotechnol (1997) Oct;8(5):554-60

In this regard, yeast - such as the species *Saccharomyces cerevisi or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces according to the present invention can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers - such as auxotrophic markers dominant antibiotic resistance markers.

A suitable yeast host organism can be selected from the biotechnologically relevant yeasts species such as, but not limited to, yeast species selected from *Pichia* spp., *Hansenula* spp., *Kluyveromyces. Yarrowinia* spp., *Saccharomyces* spp., including *S. cerevisiae,* or *Schizosaccharomyce* spp. including *Schizosaccharomyce pombe.* A strain of the methylotmphic yeast species *Pichia pastoris* may be used as the host organism.

In one embodiment, the host organism may be a *Hansenula* species, such as *H. polymorpha* (as described in WO01/39544).

TRANSFORMED PLANTS/PLANT CELLS

A host organism suitable for the present invention may be a plant. A review of the general techniques may be found in articles by Potrykus (Annu Rev Plant Physiol Plant Mol Biol [1991] 42:205-225) and Christou (Agro-Food-Industry Hi-Tech March/April 1994 17-27), or in WOOI/16308. The transgenic plant may produce enhanced levels of phytosterol esters and phytostanol esters, for example.

Therefore the present invention also relates to a method for the production of a transgenic plant with enhanced levels of phytosterol esters and phytostanol esters, comprising the steps of transforming a plant cell with a lipid acyltransferase as defined herein (in particular with an expression vector or construct comprising a lipid acyltransferase as defined herein), and growing a plant from the transformed plant cell.

SECRETION

Often, it is desirable for the polypeptide to be secreted from the expression host into the culture medium from where the enzyme may be more easily recovered. According to the present invention, the secretion leader sequence may be selected on the basis of the desired expression host Hybrid signal sequences may also be used with the context of the present invention.

Typical examples of heterologous secretion leader sequences are those originating from the fungal amyloglucosidase (AG) gene (g/aA - both 18 and 24 amino acid versions e.g. from *Aspergillus*), the a-factor gene (yeasts e.g.

*Saccharomyces, Kluyveromyces* and *Hansenula*) or the α-amylase gene (*Bacillus*).

DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies (such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

FUSION PROTEINS

A polypeptide having the specific properties as defined herein may be produced as a fusion protein, for example to aid in extraction and purification thereof. Examples of fusion protein partners include glutathione-S-transferase (GSI), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the activity of the protein sequence.

Gene fusion expression systems in *E. coli* have been reviewed in Curr. Opin Biotechnol. (1995) 6(5):501-6.

In another embodiment of the invention, the amino acid sequence of a polypeptide having the specific properties as defined herein may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

The invention will now be described, by way of example only, with reference to the following Figures and Examples.

Figure 1 shows a pfam00657 consensus sequence from database version 6 (SEQ ID No. 1);

Figure 2 shows an amino acid sequence (SEQ ID No. 2) obtained from the organism *Aeromonas hydrophila* (P10480; GI:121051);

Figure 3 shows an amino acid sequence (SEQ ID No. 3) obtained from the organism *Aeromonas salmonicida* (AAG098404; GI:9964017);

Figure 4 shows an amino acid sequence (SEQ ID No. 4) obtained from the organism *Streptomyces coelicolor* A3 (2) (Genbank accession number NP_631558);

Figure 5 shows an amino acid sequence (SEQ ID No. 5) obtained from the organism *Streptomyces coelicolor* A3 (2) (Genbank accession number: CAC42140);

Figure 6 shows an amino acid sequence (SEQ ID No. 6) obtained from the organism *Saccharomyces cerevisiae* (Genbank accession number P41734);

Figure 7 shows an alignment of selected sequences to pfam00657 consensus sequence;

Figure 8 shows a pairwise alignment of SEQ ID No. 3 with SEQ ID No. 2 showing 93% amino acid sequence identity. The signal sequence is underlined. + denotes differences. The GDSX motif containing the active site serine 16, and the active sites aspartic acid 116 and histidine 291 are highlighted (see shaded regions). Numbers after the amino acid is minus the signal sequence;

Figure 9 shows a nucleotide sequence (SEQ ID No. 7) encoding a lipid acyl transferase according to the present invention obtained from the organism *Aeromonas hydophila;*

Figure 10 shows a nucleotide sequence (SEQ ID No. 8) encoding a lipid acyl transferase according to the present invention obtained from the organism *Aeromonas salmonicida;*

Figure 11 shows a nucleotide sequence (SEQ ID No. 9) encoding a lipid acyl transferase according to the present invention obtained from the organism *Streptomyces coelicolor* A3(2) (Genbank accession number NC_003888.1: 8327480..8328367);

Figure 12 shows a nucleotide sequence (SEQ ID No. 10) encoding a lipid acyl transferase according to the present invention obtained from the organism *Streptomyces coelicolor* A3(2) (Genbank accession number AL939131.1: 265480..266367);

Figure 13 shows a nucleotide sequence (SEQ ID No. 11) encoding a lipid acyl transferase according to the present invention obtained from the organism *Saccharomyces cerevisiae* (Genbank accession number Z75034);

Figure 14 shows an amino acid sequence (SEQ ID No. 12) obtained from the organism *Ralstonia* (Genbank accession number. AL646052);

Figure 15 shows a nucleotide sequence (SEQ ID No. 13) encoding a lipid acyl transferase according to the present invention obtained from the organism *Ralstonia;*

Figure 16 shows SEQ ID No. 20. Scoe1 NCBI protein accession code CAB39707.1 GI:4539178 conserved hypothetical protein [Streptomyces coelicolor A3(2)];

Figure 17 shows a nucleotide sequence shown as SEQ ID No. 21 encoding NCBI protein accession code CAB39707.1 GI:4539178 conserved hypothetical protein [Streptomyces coelicolor A3(2)];

Figure 18 shows an amino acid shown as SEQ ID No.22. Scoe2 NCBI protein accession code CAC01477.1 GI: 9716139 conserved hypothetical protein [Streptomyces coelicolor A3(2)];

Figure 19 shows a nucleotide sequence shown as SEQ ID No. 23 encoding Scoe2 NCBI protein accession code CAC01477.1 GI:9716139 conserved hypothetical protein [Streptomyces coelicolor A3(2)];

Figure 20 shows an amino acid sequence (SEQ DD No.24) Scoe3 NCBI protein accession code CAB88833.1 GI: 7635996 putative secreted protein. [Streptomyces coelicolor A3(2)];

Figure 21 shows a nucleotide sequence shown as SEQ ID No. 25 encoding Scoe3 NCBI protein accession code CAB88833.1 GI:7635996 putative secreted protein. [Streptomyces coelicolor A3(2)];

Figure 22 shows an amino acid sequence (SEQ ID No.26) Scoe4 NCBI protein accession code CAB89450.1 GI: 7672261 putative secreted protein. [Streptomyces coelicolor A3(2)];

Figure 23 shows an nucleotide sequence shown as SEQ ID No. 27 encoding Scoe4 NCBI protein accession code CAB89450.1 GI:7672261 putative secreted protein. [Streptomyces coelicolor A3(2)];

Figure 24 shows an amino acid sequence (SEQ ID No.28) Scoe5 NCBI protein accession code CAB62724.1 GI: 6562793 putative lipoprotein [Streptomyces coelicolor A3(2)];

Figure 25 shows a nucleotide sequence shown as SEQ ID No. 29, encoding Scoe5 NCBI protein accession code CAB62724.1 GI:6562793 putative lipoprotein [Streptomyces coelicolor A3(2)];

Figure 26 shows an amino acid sequence (SEQ ID No.30) Srim1 NCBI protein accession code AAK84028.1 GI: 15082088 ODSL-lipase [Streptomyces rimosus];

Figure 27 shows a nucleotide sequence shown as SEQ ID No. 31 encoding Srim1 NCBI protein accession code AAK84028.1 GI:15082088 GDSL-lipase [Streptomyces rimosus];

Figure 28 shows an amino acid sequence (SEQ ID No.32)A lipid acyl transferase from *Aeromonas hydrophila* (ATCC #7965);

Figure 29 shows a nucleotide sequence (SEQ ID No. 33) encoding a lipid acyltransferase from *Aeromonas hydrophila* (ATCC #7965);

Figure 30 shows an amino acid sequence (SEQ ID No.34) of a lipid acyltransferase from *Aeromonas salmonicida* subsp. *Salmonicida* (ATCC#14174);

Figure 31 shows a nucleotide sequence (SEQ ID No 35) encoding a lipid acyltransferase from *Aeromonas salmonicida* subsp. *Salmonicida* (ATCC#14174);

Figure 32 shows that homologues of the *Aeromonas* genes can be identified using the basic local alignment search tool service at the National Center for Biotechnology Information, NIH, MD, USA and the completed genome databases. The GDSX motif was used in the database search and a number of sequences/genes potentially encoding enzymes with lipolytic activity were identified. Genes were identified from the genus Streptomyces, Xanthomonas and Ralstonia. As an example below, the Ralstonia solanacearum was aligned to the Aeromonas salmonicida (satA) gene. Pairwise alignment showed 23% identity. The active site serine is present at the amino terminus and the catalytic residues histidine and aspartic acid can be identified;

Figure 33 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family member. The - symbol indicates a position where the hidden Markov model of the Pfam consensus expected to find a residue but did not, so a gap is inserted. The symbol indicates a residue without a corresponding residue in the Pfam consensus. The sequences are the amino acid sequences listed in Figures 16, 18; 20, 22, 24, 26, 28 and 30.

Figure 34 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a corresponding residue in the Pfam consensus. The sequences are the amino acid sequences listed in Figures 2, 16, 18, 20, 26, 28 and 30. All these proteins were found to be active against lipid substrates.

Figure 35 shows a expression vector pet12-AsalGCAT= pSM containing the C-terminal His-tagged *Aeromonas salmonicida* lipid acyltransferase gene;

Figure 36 shows the results of testing cell extracts in a NEFA Kit Assay, which depicts the activity of a recombinant, *A. salmonicida* lipid acyltransferase, towards lecithin. The wells from left to right indicate: a positive control, a negative control (i.e. extracts from empty plasmid) and samples collected after 0, 1, 2 and 3 hours cultivation after IPTG induction;

Figure 37 shows growth optimisation of BL21(DE3)pLysS harboring the expression vector pet12-AsalGCAT= pSM showing cultivation at 30 °C resulted in the production of enzyme with high activity towards lecithin. Cell extracts were tested for phospholipase activity using the NEFA kit assay. Wells from left to right: positive control; negative control; 20°C; 30°C;

Figure 38 shows crude cell extracts from BL21(DE3)pLysS expressing active lipid acyltransferase incubated with the substrate lecithin and reaction mixture was analyzed using thin layer chromatography showing the presence of degradation products. Lanes: 1. No enzyme; 2. + A.sal-10ul 37°C; 3. + A. sal -20ul 37°C; 4. + A.sal-10ul 24°C; 5. + A. sal -20u 24°C;

Figure 39 shows partial purification of the *Aeromonas salmonicida* Acyl Transferase showing the phospholipase activity associated with purified His-tag protein. SE = Sonicated extracts, His = Purified with Ni-NTA spin-kit from Qiagen;

Figure 40 shows the expression vector petl2-A.h. GCAT=pSMa containing the C-terminal His-tagged *Aeromonas*

*hydrophila* Glycerolipid Acyl Transferase (GCAT) gene was used to transform *E.coli* strain BL21(DE3)pLysS;

Figure 41 shows the activity of the crude extracts (5 & 10ul) containing the recombinant *Aeromonas hydrophila* GCAT enzyme was tested towards lecithin using Non-Esterified Fatty Acid (NEFA) kit (Roche, Switzerland), showing the presence of active enzyme towards the phospholipid, lecithin;

Figure 42 shows growth optimisation of BL21(DE3)pLysS harboring the expression vector pet12-AsaLGCAT= pSM showing cultivation at 30 °C resulted in the production of enzyme with high activity towards lecithin. Cell extracts were tested for phospholipase activity using the NEFA kit assay;

Figure 43 shows the partial purification of the *Aeromonas hydrophila & A. salmonicida* Acyl Transferases showing the phospholipase activity associated with purified His-tag protein. SE = Sonicated extracts,
His = Purified with Ni-NTA spin-kit from Qiagen);

Figure 44 shows the expression of the *Aeromonas* genes in *Bacillus subtilis* 163 showing the production of secreted enzyme with activity towards both lecithin and DGDG. pUB-AH= construct containing the *A. hydrophila* gene and pUB-AS, construct with the *A. salmonicida* gene, Culture filtrate was incubated with the substrates for 60 minutes.

Figure 45 and Fig 46 show a TLC plate in developing solvent IV (chloroform:methanol:water (65:25:4)); Lane 1: 40 mg sitosterol 30 min: Lane 2: Transferase+ 40 mg sitosterol 30 min; Lane 3: Transferase+ 80 mg sitosterol 30 min; Lane 4: Transferase+ 40 mg sitosterol 120 min; Lane 5: Transferase+ 80 mg sitosterol 120 min; Lane 6: Transferase+ 40 mg sitosterol 300 min; Lane 7: 40 mg sitosterol 300 min; Lane 8: Cholesterol; Lane 9: Sitosterol;

Figure 47 depicts the reaction between phosphatidylcholine and cholesterol which is catalysed by a lipid acyltransferase;

Figure 48 shows a TLC analysis of lipids extracted fiom enzyme treated or untreated egg yolk, 6) 0.31PLU/g Transferase #179, 7) 1.25PLU/g Transferase #178-9., 8) 23.25 PLU/g Phospholipase #3108., 9) Control.

Figure 49 shows mayonnaise test samples produced by enzyme treated or untreated egg yolk: 5) Transferase #179, 0.31 PLU/g. 6) Transferase #178-9, 1.25 PLU/g, 7) Phospholipase #3108, 23.3 PLU/g 8) Control, water

Figure 50 shows a TLC (in solvent I) of egg yolk lipid treated with a lipid acyl transferase from *A. hydrophila*;

Figure 51 shows a TLC (in solvent IV) of egg yolk lipid treated with a lipid acyl transferase from *A. hydrophila*;

Figure 52 shows a TLC analysis of transferase treated lipid from egg yolk over a time course;

Figure 53 shows the amount of fatty acid and cholesterol ester produced as a function of time when using a lipid acyltransferase (Tranf #178-9) compared with when using a control lipolytic enzyme, *Thermomyces lanuginosus*;

Figure 54 shows relative transferase activity as % of transferase and hydrolytic activity in enzymatic reactions in egg yolk with high water content, #1991 (phospholipase A2) and #2427 (phospholipase A1) are control phospholipases, #178 is a lipid acyltransferase;

Figure 55 shows the effect of water content in the assay on the transferase activity of the transferase #178 in transferase reactions in egg yolk with high water content;

Figure 56 shows the transferase activity for a lipid acyltransferase (#178) as a function of reaction time in transferase reactions in egg yolk with high water,

Figure 57 and Figure 58 show graphs depicting fatty acid and cholesterol ester as a function of time. The graphs depict results obtained for GLC analysis in the assay for measurement of acyltransferase activity using lecithin and cholesterol in buffer as substrate;

Figure 59 shows a TLC in solvent I. Egg yolk treated with lipid acyltransferase #138 from *Aeromonas salmonidica* (lane no. 1 and 2) or with a phospholipase #2938 (LIPOPAN®F) (lane no. 3) or Untreated egg yolk (lane no. 4);

Figure 60 shows a TLC in solvent IV. Egg yolk treated with lipid acyltransferase #138 (lane no. 1 and 2) or with Phospholipase #2938 (lane no. 3). Untreated egg yolk (lane no. 4);

Figure 61 shows egg yolk treated with lipid acyltransferase #138 (sample nos. 1 and 2) and with phospholipase #2938 (sample no. 3). Untreated egg yolk (sample no. 4);

Figure 62 shows a food emulsion after 2 hours at 100 °C. 0) Untreated egg yolk 1)Egg yolk treated with lipid acyl transferase #138 for 210 minutes. 3) Egg yolk treated with the control phospholipase #2938 for 210 minutes;

Figure 63 shows TLC plates showing the screening of transferase activity on plant sterol and glycerol. PC = phosphatidylcholine, LPC = lysophosphafdylcholine; PE = phosphatidylethanolamine; monogl = monoglyceride;

Figure 64 shows a TLC plate in solvent I, Samples 1 to 6 after 24 hours and samples 1 to 4 after 4 hours reaction time. The TLC analysis confirms the formation of sterol ester in samples 1, 2, 5 and 6;

Figure 65 shows a TLC plate in solvent I where the transferase activity of an immobilised acyltransferase from *Aeromonas salmonicida* was tested in an oil mixture - with samples taken at 0.5, 1, 3, 6 and 24 h;

Figures 66 and 67 show TLC plates in solvent I and IV. Lane 1 = lecithin; Lane 2 = control - 10mins; Lane 3 = 0.75 PLU, 10mins; Lane 4 = 0.75 PLU, 60mins; Lane 5 = 0.75 PLU, 220mins; Lane 6 = control, 20 h; Lane 7 = 0.75 PLU, 20h; and Lane 8 = cholesterol ester;

Figures 68 and 69 show TLC plates in solvent IV. Lane 1 = lecithin; Lane 2 = control - 10mins; Lane 3 = 1 PLU, 10mins; Lane 4 = 1 PLU, 60mins; Lane 5 = 1 PLU, 180mins; Lane 6 = 1PLU, 220mins; Lane 7 = 1PLU, 1200min; Lane 8 = control, 1200 min; Lane 9 = glucose ester; Lane 10 = cholesterol; and Lane 11 = glucose;

Figure 70 shows the reaction between DGDG and glucose when catalysed by a lipid acyltransferase;

Figure 71 shows an amino acid sequence (SEQ ID No. 36) of the fusion construct used for mutagenesis of the *Aeromonas hydrophila* lipid acyltransferase gene in Example 17. The underlined amino acids is a xylanase signal peptide;

Figure 72 shows a nucleotide sequence (SEQ ID No. 54) encoding an enzyme from *Aeromonas hydrophila* including a xylanase signal peptide; and

Figure 73 shows a TLC plate clearly showing the formation of plant sterol ester and monoglyceride. Lane 1 is after 1 hour reaction time, Lane 2 is after 4 hours reaction time, Lane 3 is after 24 hours reaction time and Lane 4 is a plant sterol.

## EXAMPLES

Except where stated TLC analysis was performed as described in Example 6 and GLC analysis was performed as described in Example 11.

### EXAMPLE 1: The cloning, sequencing and heterologous expression of a transferase from *Aeromonas salmonicida* subsp. *Salmonicida*

**Strains used:**

*Aeromonas salmonicida subsp. Salmonicida* (ATCC 14174) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat. 19131) and RNAse A (cat. 19101) were all obtained from Qiagen Ltd. (Boundary court Gatwick Court, West Sussex, RH10 2AX).

Host bacterial strain BL21(DE3)pLysS (Novagen) was used for production of the recombinant *Aeromonas* enzymes. Competent cells of BL21(DE3)pLysS were used as host for transformation with the expression vector **pet12-AsalGCAT=pSM.** Transformants containing the appropriate plasmid were grown at 37 °C in LB agar medium containing 100-ug ampicillin/ml.

**Construction of expression vector pet12-AsalGCAT- pSM:**

For all DNA amplifications of the transferase genes from *Aeromonas,* genomic DNA (0.2-1 ul) was used as template and *pfu* DNA polymerase (2.5 units) was used with 10ul of 10x pfu buffer, 1ul each primer (50pmol/ul), 200 uMdNTP in a total reaction volume of 100u1. PCR reactions were performed in a programmable thermal cycler using the following conditions: 95 °C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. salmonicida* was carried in 2 separate PCR reactions. PCR reaction 1 was performed using primer pairs, asIUSNEW(5'AGCATATGAAAA AATGGTTTGT TTGTTTATTG GGG 3' [SEQ ID No. 38]) and asls950new (5'GTG ATG GTG GGC GAG GAA CTC GTA CTG3' [SEQ ID No. 37]). A second PCR reaction was performed to incorporate a C-terminal Histidine tag using the PCR product from the first reaction and the primers: asIUSNEW(5'AGCATATGAAAA AATGGTTTGT TTGTTTATTG GGG 3' [SEQ ID No. 38]) and AHLS1001 (STTGGATCC GAATTCAT CAATG GTG ATG GTG ATG GTG GGC3' [SEQ ID No. 39]). The PCR product from the second reaction was purified and digested with restriction enzymes Ndel and BamHI. 2 ug of pET 12a vector DNA was also digested with restriction enzymes Ndel and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 100ug/ml ampicillin.

The T7 promoter primer (5TAATACGACTCACTATAG3' [SEQ ID No. 40]) and the T7 terminator primer (5'CTAGTTATTGCTCAGCGG3' [SEQ ID No. 41]) were used to verify the sequences and the orientation of the cloned transferase genes in pET12a vector. DNA sequencing was performed using ABI Prism® BigDye™. Terminators Cycle sequencing kit with 500ng plasmid DNA as template and 3.2pmol T7 promoter and terminator primers.

The construct shown in Figure 35 was used to transform competent bacterial host strain BL21(DE3)pLysS (Novagen) and ampicillin resistant transformants were picked and used for expression analysis.

Expression of the recombinant *Aeromonas salmonicida* lipid acyltransferase

Quantification of enzyme activity towards lecithin was determined on cell extracts using Non-Esterified Fatty Acid (NEFA) kit (Roche, Switzerland).

In Figure 36, BL21(DE3)pLysS harboring the expression vector pet12-AsalGCAT= pSM was grown in LB medium + 100ug/ml ampicillin and incubated with shaking at 37°C until $OD_{600}$ = 0.6 to1.0 is reached. The cultures are then induced using IPTG (0.4mM) and incubation was continued for the next 3 hours. Samples where taken at 0 hour, 1, 2, and 3 hours after IPTG induction. Enzyme Activity was tested using the NEFA kit and lecithin as substrate.

Growth Optimisation for the production of more active enzymes BL21(DE3)pLysS harboring the expression vector pet12-AsalGCAT= pSM was grown in LB medium + 100ug/ml ampicillin and incubated with shaking at different growth temperatures (37°C, 30°C, & 20 °C). The optimal condition for the production of active lipid acyltransferase enzyme was when cultures are grown at 30ºC as shown in Figure 37.

Partial purification of recombinant *Aeromonas salmonicida* transferase

Strain BL21 (DE3)pLysS harboring the expression vector pet12-AsalGCAT=pSM was grown at 37°C & crude cell extracts were prepared by sonication. The recombinant enzyme was further purified from the sonicated crude cell extracts using the Ni-NTA spin kit from Qiagen. Phospholipase activity using the NEFA kit & Lecithin as substrate. Crude cell extracts from BL21(DE3)pLysS expressing active transferase incubated with the substrate lecithin and reaction mixture was analysed using thin layer chromatography showing the presence of degradation products (see Figure 38).

Partial Purification of recombinant *Aeromonas salmonicidae* transferase. Strain BL21(DE3)pLysS harbouring the expression vector pet12-AsalGCAT=pSM was grown at 37°C and crude cell extracts were prepared by sonication. The recombinant enzyme ware further purified from the sonicated crude cell extract using the Ni-NTA spin kit from Qiagen. Phospholipase activity using the NEFA kit and lecithin as substrate was tested (see Figure 39).

**EXAMPLE 2 Cloning and Expression *of Aeromonas lrydroohila* transferase in *E. coli***

*Aeromonas hydrophila* (ATCC # 7965) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat 19131) and RNAse A (cat 19101) were all obtained from Qiagen Ltd. (Boundary court Gatwick Court, West Sussex, RH102AX).

Host bacterial strain BL21(DE3)pLysS (Novagen) was used for production of the recombinant *Aeromonas* enzymes. Competent cells of BL21(DE3)pLysS were used as host for transformation with the expression vector pet12a-A.h.GCAT=pSMa: Transformants containing the appropriate plasmid were grown at 37 °C in LB agar medium containing 100-ug ampicillin/ml.

**Construction of expression vector pet12a-A.h.GCAT- pSMa:**

For all DNA amplifications of the transferase gene from *Aeromonas,* genomic DNA (0.2-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95°C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. hydrophila* (ATCC # 7965) was carried out in 2 separate PCR reactions.

PCR reaction 1 was performed using primer pairs, AHUS1 (5'GTCATATGAAAAAATGGTTTGTGTGTTTATTGGGATTGGTC3', SEQ ID No. 42) and ahls950 (5'ATGGTGATGGTGGGCGAGGAACTCGTACTG3', SEQ ID No. 43).

A second PCR reaction was performed to incorporate a C-terminal Histidine tag using the PCR product from the first reaction and the primer pairs:

AHUS(5'GTCATATGAAAAAATGGTTTGTGTGTTTATTGGGATTGGTC3' SEQ ID No. 44, ) and AHLS1001(5'TTGGATCCGAATTCATCAATGGTGATGGTGATGGTGGGC3' SEQ ID No. 45).

The PCR product from the second reaction was purified and digested with restriction enzyme Nde1 and BamHI. 2 ug of pET 12a vector DNA was also digested with restriction enzymes NdeI and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 100ug/ml ampicillin.

The T7 promoter primer (5'TAATACGACTCACTATAG3') and the T7 terminator primer (5'CTAGTTATTGCTCAGCGG3') were used to verify the sequences and the orientation of the cloned GCAT genes in pET12a vector. DNA sequencing was performed using ABI Prism® BigDye™ Terminators Cycle sequencing kit with 500ng plasmid DNA as template and 3.2pmol T7 promoter and terminator primers..

The construct shown in Figure 40 was used to transform competent bacterial host strain BL21 (DE3)pLysS (Novagen) and ampicillin resistant transformants were picked and used for expression analysis.

**Expression of the *Aeromonas liydrophila* transferase in BL21(DE3)pLysS**

The *E. coli* strain BL21(DE3)pLysS harboring the expression vector pet12a-A.h.GCAT= pSMa was grown in LB medium + 100ug/ml ampicillin and incubated with shaking at 37°C until $OD_{600}$= 0.6 tol.0 is reached. The cultures are then induced using IPTG (0.4mM) and incubation was continued for the next 3 hours. Samples where taken at 0hour, 1, 2, and 3 hours after IPTG induction. Enzyme Activity was tested using the NEFA kit and lecithin as substrate (Figure 41).

**Growth Optimisation for the production of more active enzymes**

BL21(DE3)pLysS harboring the expression vector pet12a-A.h.GCAT=pSMa was grown in LB medium + 100ug/ml ampicillin and incubated with shaking at different growth temperatures (37°C, 30 °C, & 20 °C). The optimal condition for the production of active GCAT enzyme was when cultures are grown at 30°C as shown in Figure 42.

**Partial purification of recombinant *A.hydrophila* transferase (GCAT)**

Strain BL21 (DE3)pLysS harboring the expression vector pet12a-A.h.GCAT=pSMa was grown at 37°C & crude cell extracts were prepared by sonication. The recombinant enzyme was further purified from the sonicated crude cell extracts using the Ni-NTA spin kit from Qiagen. Phospholipase activity assay using the NEFA kit & Lecithin as substrate. (Figure 43).

**<u>EXAMPLE 3: Expression of *Aeromonas* transferases in *Bacillus subtilis* 163</u>**

**Plasmid Construction**

Two different *Bacillus subtilis* expression vectors (pUB 110 & pBE5) were used for the heterologous expression of the *Aeromonas* genes in *Bacillus subtilis.* The pUB 110 vector contains the alpha amylase promoter while the pBE vector has the P32 promoter as the regulatory region for the expression of the fused *Aeromonas* genes. In pUB110, the first amino acid of the mature GCAT genes of *Aeromonas* were fused in frame with the last amino acid of the xylanase signal peptide sequence from *Bacillus subtilis* via the restriction site NheI, creating an additional 2 amino acids in front of the mature proteins. pBE5 contains the cgtase signal sequence fusion at the Nco1 site for secretion of the recombinant proteins into the culture filtrate.

PCR reactions were carried out to obtain the *Aeromonas* genes fuse in frame to the signal sequences of the pUB 110 and the pBE5 vectors. PCRs were performed using the following primer pairs for *A. hydrophila* gene:

PCR reaction 1: usAHncoI (5'ATGCCATGGCCGACAGCCGTCCCGCC3', SEQ ID No. 46) and 1sAH (5'1TGGATCCGAATTCATCAATGGTGATG3', SEQ ID No. 47)

PCR reaction 2: US-Ahnhel (5'ITGCTAGCGCCGACAGCCGTCCCGCC3', SEQ ID No. 48.) and 1sAH (5'TTGGATCCGAATTCATCAATGGTGATG3, SEQ ID No. 49)

PCRs were performed using the following primer pairs for *A. salmonicida* gene:

PCR reaction 3: US-Asncol (5'TTGCCATGGCCGACACTCGCCCCGCC3', SEQ ID No. 50) and 1sAH (5'TTGGATCCGAATTCATCAATGGTGATG3', SEQ ID No. 51)

PCR reaction 4: US-ASnhel (5'TTGCTAGCGCCGACACTCGCCCCGCC3', SEQ ID No. 52) and 1sAH (STTGGATCCGAATTCATCAATGGTGATG3', SEQ ID No. 53)

All the PCR products were cloned into PCR blunt II (TOPO vector) and sequenced with reverse & forward sequencing primers:

Clones from PCR reactions 1 & 3 were cut with NcoI & Bam HI and used as inserts for ligation to the pBE5 vector cut with Nco1/BamH1/phosphatase. Clones from PCR reactions 2 & 4 were cut with NheI & Bam H1 and used as inserts for ligation to the pUB vector that was cut with Nhe1/BamH1/phosphatase.

**Expression of the *Aeromonas* transferase genes in *Bacillus subtilis* and characterization of the enzyme activity.**

The acyl transferases from the two *Aeromonas* species have been successfully expressed in *E. coli* (results above). The *Bacillus* pUB110 & pBE5 gene fusion constructs were used to transform *Bacillus subtilis* and transformants were selected by plating on kanamycin plates. The kanamycin resistant transformants isolated and grown in 2xYT are capable of heterologous expression of the *Aeromonas* genes in *Bacillus.* The culture filtrates have digalactosyldiacylglycerol (DGDG) galactolipase activity, in addition to having both acyl transferase and phospholipase activities. The activity towards digalactosyldiacylglycerol (DGDG) was measured after 60 minutes of incubation of culture supernatant with the substrate, DGDG from wheat flour (obtainable form Sigma) as well as the activity towards lecithin as shown in Figure 44. *Bacillus* produced the enzyme after overnight (20-24 hours) to 48 hours of cultivation in the culture medium as a secreted protein. In some instances, the expression of the *Aeromonas* genes has been shown to interfere with cell viability and growth in *Bacillus & E. coli,* it is therefore necessary to carefully select expression strain and optimise the growth conditions to ensure expression. For example, several *Bacillus* host strains (B.s 163, DB104 and OS 21) were transformed with the expression vectors for growth comparison. B.s163 is transformable with the 2 *Aeromonas* genes and is capable of expressing active protein. DB104 is transformable with all the constructs but is only able to express *A. salmonicida* transferase.

## EXAMPLE 4: Ferznentation and Purification of *Aeromonas* livid acyltransferases produced in *E.coli*

### *E.coli* Fermentations:

### Microorganisms

Two strains of *Eschericia coli, one* containing an *Aeromonas hydrophila* (Example 2) lipid acyltransferase and two containing *Aeromonas salmonicida* lipid acyltransferases, (Example 1) were used in this study.

The *E. coli* strain containing the *A. hydrophila* gene was named DIDK0124 , and the *E. coli* strain containing the *A. salmonicida* gene was named DIDK0125. The fermentation with DIDK0124 was named HYDRO0303 and the fermentation with DIDK0125 was named SAL0302. The purified protein from HYDRO025 was named REF#138. The purified protein from HYDRO0303 was named REF#135.

## Growth media and culture conditions

### LB-agar

The LB agar plates used for maintaining the strains contained: 10 g/L tryptone, 5 g/L yeast extract, 5 g/L NaCl, 15 g/L agar, 100 mg/L ampicillin and 35 mg/L chloramphenicol. The agar plates were incubated at 30°C.

### LB shake flask

The LB medium (50 mL pr shake flask) used for production of inoculum material for the bioreactor cultivations contained: 10 g/L tryptone, 5 g/L yeast extract, 5 g/L NaCl, 100 mg/L ampicillin and 35 mg/L chloramphenicol. The shake flasks were inoculated from the LB agar plates, and incubated at 30°C and 200 rpm.

### Bioreactor cultivation

The bioreactor cultivations were carried out in 6 L in-house built bioreactors filled with 4 L medium containing: 10 g/L tryptone, 5 g/L yeast extract, 5 g/L NaCl, 8 g/L $KH_2PO_4$, 0.9 g/L $MgSO_4$, $7H_2O$, 40 g/L glucose monohydrate, 0.4 mL/ ADD APT® Foamstop Sin 260 (ADD APT Chemicals AG, Helmond, The Netherlands), 10 mg/L $(NH_4)_2Fe(SO_4)_2 \cdot 6H_2O$, 0.7 mg/L $CuSO_4 \cdot 5H_2O$, 3 mg/L $ZnSO_4 \cdot 7H_2O$, 3 mg/L $MnSO_4 \cdot H_2O$, 10 mg/L EDTA, 0.1 mg/L $NiSO_4 \cdot 6H_2O$, 0.1 mg/L $CoCl_2$, 0.1 mg/L $H_3BO_4$, 0.1 mg/L KI, 0.1 mg/L $Na_2MoO_4 \cdot 2H_2O$, 1 g/L ampicillin and 35 mg/L chloramphenicol.

The bioreactors were inoculated with an amount of LB culture ensuring end of growth after approximately 20 hours of cultivation (calculated from the maximum specific growth rate of 0.6 $h^{-1}$, the $OD_{600}$ of the LB shake flask and the final $OD_{600}$ in the bioreactor of approximately 20).

SAL0302 was inoculated with 10 mL of LB culture, and HYDR00303 was inoculated with 4 mL of LB culture.

The bioreactors were operated at the following conditions: temperature 30°C, stirring 800-1000 rpm (depending on experiment), aeration 5 L/min, pH 6.9, pH control 8.75% (w/v) $NH_3$-water and 2 M $H_2SO_4$. Induction was achieved by addition of isopropyl β-D-thiogalactoside to a final concentration of 0.6 mM, when 0.4 moles (HYDRO0303) and 0.7 moles $CO_2$ was produced respectively.

### Harvest

The following procedure was used for harvest and homogenisation of the biomass:

1) The fermentation broth from the fermentations was centrifuged at 5000 x g and 4°C for 10 minutes, and the supernatant was discharged. The biomass was stored at -20°C until use. The biomass was thawed and resuspended in 500 mL of 20 mM $NaH_2PO_4$, pH 7.4, 500 mM NaCl, 10 mM Imidazole and Complete (EDTA-free) protease inhibitor (Roche, Germany).
2) The suspended biomass was homogenized at 2 kbar and 4°C in a cell disrupter from Constant Systems Ltd (Warwick, UK).
3) The cell debris was removed by centrifugation at 10.000 x g and 4°C for 30 minutes followed by collection of the supernatant.
4) The supernatant was clarified further by centrifugation at 13.700x g and 4°C for 60 minutes, followed by collection of the supernatant.
5) The supernatant was filtered through 0.2 μm Vacu Cap filters (Pall Life Sciences, UK) and the filtrate was collected for immediate chromatographic purification.

### Chromatographic purification of the Transferases

A column (2.5 x 10 cm) was packed with 50 ml of Chelating Sepharose ff. gel and charged with Ni-sulphate (according to the method described by manufacturer, Amersham Biosciences). The column was equilibrated with 200 ml of 20 mM $NaH_2PO_4$, pH 7.4, 500 mM NaCl, 10 mM Imidazole. 400 ml of crude was applied to the column at a flow rate of 5 ml/min. The column was then washed with 20 mM $NaH_2PO_4$, pH 7.4, 500 mM NaCl, 10 mM Imidazole until the $UV_{280}$ reached the base line. The GCAT was then eluted with 40 ml of 20 mM $NaH_2PO_4$, pH 7.4, 500 mM NaCl and 500 mM Imidazole.

**EXAMPLE 5: Fermentation and Purification of *Aeromonas* lipid acyltrabsferases produced in *Bacillus subtilis.***

**Fermentadons**

BAC0318-19, BAC0323-24

**Microorganism**

The microorganisms used in this study originate from transformation of a *Bacillus subtilis* host strain, #163 with a plasmid containing the gene encoding the *Aeromonas salmonicida* transferase inserted in the vector pUB110OIS. The expression of the gene is controlled by an alpha-amylase promoter, and the secretion of the transferase is mediated by the *B. subtilis* xylanase signal sequence (Example 3). The strains were named DIDK0138 (fermentation BAC0318-19) and DIDK0153 (fermentation BAC0323-24).

**Growth media and culture conditions**

**Pre culture medium**

A shake flask (500 mL total volume, with baffles) was added 100 mL of a medium containing:

| | |
|---|---|
| NaCl | 5 g/L |
| $K_2BPO_4$ | 10 g/L |
| Soy flour | 20 g/L |
| Yeast extract, BioSpringer 106 | 20 g/L |
| Antifoam, SIN260 | 5 mL/L |

pH was adjusted to 7.0 before autoclaving

After autoclaving 6 mL 50% (w/w) Nutriose were added pr flask. Kanamycin was added at a concentration of 50 mg/L after autoclaving.

**Inoculation**

A pre culture shake flask was inoculated with frozen culture directly from a 25% (w/v) glycerol stock. The shake flask was incubated at 33°C and 175 rpm for approximately 16 hours, whereupon 50 mL was used to inoculate the fermentor.

***Fermentations***

The fermentations were carried out in 6 L in house built fermentors.
The batch medium (3 L) contained:

| | |
|---|---|
| Com steep liquor (50% dw) | 40 g/L |
| Yeast extract BioSpringer 153 (50% dw) | 10 g/L |
| NaCl | 5 g/L |
| $CaCl_2$, $2H_2O$ | 0.25 g/L |
| $Mn(NO_3)_2$, $H_2O$ | 0.2 g/L |
| Antifoam SIN260 | 1 mL/L |
| Kanamycin (filter sterilised to the fermentor after autoclaving | 50 mg/L |

The feed contained:

| | |
|---|---|
| Glucose monohydrate | 540 g/kg |
| $MgSO_4$, $7H_2O$ | 4.8 g/kg |
| Antofoam SIN260 | 4 mL/kg |
| Yeast extract, BioSpringer 153 (50% dw) (autoclaved separately) | 150 g/kg |

The feed in fermentation BAC0318 and BAC0323 was started based on the accumulated $CO_2$, according to the equations below:

$$\text{Feed} - \text{flow}[g/h] = 0, \text{AcCO}_2 < 0.15$$

$$\text{Feed} - \text{flow}[g/h] = 2.85 + t \cdot 1.54, \text{AcCO}_2 \geq 0.15 \text{ and } t < 12$$

$$\text{Feed} - \text{flow}[g/h] = 21.3, t > 12$$

t: time (hours) from the point when the accumulated $CO_2$ ($AcCO_2$) reached 0.15 moles.

The feed in fermentation BAC0319 and BAC0324 was started based on the accumulated $CO_2$ according to the equations below:

$$\text{Feed} - \text{flow}[g/h] = 0, \text{AcCO}_2 < 0.15$$

$$\text{Feed} - \text{flow}[g/h] = 2.0 + t \cdot 1.08, \text{AcCO}_2 \geq 0.15 \text{ and } t < 12$$

$$\text{Feed} - \text{flow}[g/h] = 15, t > 12$$

t: time (hours) from the point when the accumulated $CO_2$ ($AcCO_2$) reached 0.15 moles.

The pH was controlled at 7.0 by adding 12.5% (w/v) $NH_3$-water or 2M phosphoric acid.
The aeration was 3 L/min corresponding to 1 vvm.
The temperature was 33°C.
The fermentor was equipped with two 8 cm ø Rushton impellers placed with a distance of 10 cm.

Harvest

The biomass was removed by centrifugation at 16,000x g for 10 minutes at room temperature. The supernatant was filter sterilized, and the filtrate was used for purification and application tests.

**EXAMPLE 6. Application tests in egg yolk.**

In the following experiments the isolated transferase from *Aeromonas salmonicida* expressed in *E-coli* was tested in egg yolk alone and in egg yolk where .a plant sterol had been added

Material

Transferase from *Aeromonas salmonicida* REF#138
Egg yolk: from fresh egg (hens eggs)
Plant sterol: β-sitosterol, Sigma S 5753
TLC plates: Silica plates Merck nr. 1.05715.0001
TLC analysis.
TLC-plate was activated in a heat cupboard (110°C) for ½h.
100 ml developing solvent was poured into a chromatography camber with lid. The walls of the chamber were covered with filter paper (Whatman 2) in order to saturate the chamber with the solvent vapor.
The TLC-plate was placed in a frame and the sample was applied onto the TLC plate 2 cm from the bottom. The TLC plate was then placed in the TLC chamber with the developing solvent. When the developing solvent reached 14 cm from the bottom of the plate. The TLC plate was taken out and dried in fume board, and then placed in the heat cupboard at 110 °C for 10 minutes.

The TLC-plate was then immersed in the developing reagent, and dried in the heat cupboard at 110 °C for 15 minutes

Developing solvent:

Nr. IV: Chloroform : Methanol : $H_2O$ ( 65:25:4 )
Nr. I : P-ether : MTBE : Acetic acid (60:40:1)

Developing Buffer (Vanadate-buffer):

32 g $Na_2CO_3$ ad 300 ml $H_2O$ (1M)
18.2 g vanadate pentoxide ($V_2O_5$) is added and dissolved during gentle heating.

The solution is cooled to ambient
Carefully 460 ml 2.5 M $H_2SO_4$. (460 ml $H_2O$ +61 ml $H_2SO_4$) is added
Water is added to 1000 ml.

Phospholipase activity.

Substrate:

0.6% L-α Phosphatidylcholine 95% Plant (Avanti #441601) + 0.4% Triton-X 100( Sigma X-100) + 5 mM $CaCl_2$ is dissolved in 0.05M HEPES buffer pH 7.

Procedure.

400 µl substrate was added to an 1.5 ml Eppendorf tube and placed in a Eppendorf thermomixer at 30°C for 5 minutes.

To the time T= 0 50µl enzyme solution was added. Also a blank with water instead of enzyme was analysed.

The sample was mixed at 1000 rpm on Eppendorf Termomixer at 30°C for 10 minutes. To the time T=10 min. The Eppendorf tube was placed in another termomixer at 99°C for 10 minutes to stops the reaction.

Free fatty acid in the samples were analyzed by using the NEFA kit from WAKO GmbH.

Enzyme activity PLU-7 pH 7 was calculated as micromole fatty acid produced per minute under assay conditions.

Lipid extraction. 1 g egg yolk and 7.5 ml Chloroform:Medianol 2:1 was mixed on a Whirley and centrifuged at 750 x g form 10 minutes.

3ml of the chloroform phase was isolated and used for further lipid analysis.

Results:

The transferase (REF#138), from *Aeromonas salmonicida* expressed in *E-coli* was analysed for phospholipase activity as described above, and was also tested in egg yolk with and without β-sitosterol. The sample was stirred with a magnetic stirrer during the reaction. The experimental design is shown in Table 1

Table 1

| Test | Reaction time at 37°C | Egg yolk | Sitosterol | Transferase #138 |
|---|---|---|---|---|
| Nr | Minutes | gram | mg | Units |
| 1 | 30 | 1 | 40 | |
| 2 | 30 | 1 | 40 | 0,75 PLU |
| 3 | 30 | 1 | 80 | 0,75 PLU |
| 4 | 120 | 1 | 40 | 0,75 PLU |
| 5 | 120 | 1 | 80 | 0,75 PLU |
| 6 | 300 | 1 | 40 | 0,75 PLU |
| 8 | 300 | 1 | 40 | |

The reaction was stopped by adding 7.5 ml Chloroform:Methanol (2:1) and mixed on a Whirley mixer for 30 seconds. The chloroform phase was isolated by centrifugation and 2 μl of the chloroform phase was transferred to a pre-activated silica TLC plate and eluted with developing solvent nr. I, and another TLC-plate in developing solvent IV.
The results from the TLC analysis are shown in Figures 45 and 46.
Transferase reaction with a transferase from *Aeromonas salmonicida* in egg yolk where plant sterol was added has shown that the enzyme transfers fatty acid from lecithin in the egg yolk to the cholesterol during formation of cholesterolester. The TLC chromatogram also indicated that part of the sterol added to egg yolk was transferred to sterol ester.
The amount of sterol ester relative to the amount of cholesterol ester formed during the reaction can be analysed by HPLC or GLC.
It is known that plant sterol esters reduce the absorption of cholesterol in the intestine. It is also indicated in the literature that cholesterolesters are absorbed less than free cholesterol in the intestine. When a transferase and plant sterol is added to egg yolk a product with causes reduced cholesterol absorption is obtained, and at the same time lysolecithin is produced which improves the emulsification properties of the egg yolk. A further advantage of adding transferase and plant sterol to egg yolk is that plant sterol ester is ingested together with the natural available cholesterol, which is expected to have the highest effect on the reduction of cholesterol absorption.

**EXAMPLE 7: Modification of egg yolk by lipid acyl transferase from *Aeromonas salmonicida***

In accordance with the present invention it has now been shown that it is possible to produce lysolecithin from egg yolk without substantial free fatty acid formation by use of a transferase.
The lecithin content of egg yolk is an important emulsifier for the production of mayonnaise with the limitation that the mayonnaise is not heat stable. It has therefore been known for several years to use a phospholipase from pancreas to modify lecithin in egg yolk to lysolecithin, which is a more efficient emulsifier. The use of enzyme modified egg yolk in mayonnaise production contributes to better heat stability of the mayonnaise during pasteurisation. A limitation of using pancreas phospholipase in egg yolk is that the amount of free fatty acid also increases, which contributes to reduced oxidative stability because free fatty acids are more prone to oxidation than the corresponding ester. Free fatty acid may also contribute to a soapy off taste.
The transferase from *Aeromonas salmonicida* was successfully expressed in *B. subtilis* and fermented in lab scale as descried in Example 5, purified by liquid chromatography and used to modify egg yolk lipids. The enzyme modified egg yolk was used to produce heat stable mayonnaise.
The transferase from *A. salmonicida* can be used to produce lysolecithin and cholesterolester in egg yolk without production of significant amounts of free fatty acids. That is to say without increasing or substantially increasing the free fatty acids in the foodstuff.
The enzyme modified egg yolk produced by transferase showed improved emulsification properties and can be used for heat stable mayonnaise.
This enzyme was highly functional in modification of egg yolk by catalysing the lipid transfer reaction between lecithin and cholesterol Figure 47.
This study further investigated the use of transferase for modification of egg yolk and the use of modified egg yolk in the production of heat stable mayonnaise.
This example describes the fermentation, isolation, and application of the transferase in egg yolks as well as the application of the enzyme modified egg yolk in mayonnaise. The example is divided into two parts:

**A. Application of transferase in egg yolk**

**B. Testing of enzyme modified egg yolk in mayonnaise**

**EXPERIMENTAL**

**A. Application**

***Enzyme and substrate***

Transferase #178-9 from *A. salmonicida,* purification 2554-100 C73,15 PLU-7/ml.
Transferase #179 from *A. salmonicida,* 18.5 PLU-7/ml.
Phospholipase A1 LECITASE™ Ultra (Novozymes A/S, Denamrk)
Egg yolk: Liquid egg yolk with 8% salt, SANOVA FOODS, DK
TLC analysis was performed as described previously (see above Example 6).

Phospholipase activity: See previous examples.

***Lipid extraction***

1 g egg yolk and 7.5 ml Chloroform:Methanol 2:1 was mixed on a Whirley for 30 sec. and centrifuged at 750 x g for 10 minutes.
4 ml of the chloroform phase was isolated and used for further lipid analysis.

**Oxidation stability test**

Oxidation stability of mayonnaise was measured in an ML OXIPRESS equipment where the sample is oxidative stressed by means of heat under pressure in an oxygen atmosphere.
After a certain- time, called the induction period (IP), the oxidation of the sample causes a certain consumption of oxygen which is registered as pressure change of a pressure transducer. Higher induction period indicates better oxidation stability. Procedure.
5-gram mayonnaise is placed in a glass container and the glass container is closed with the pressure transducer. The container is filled with oxygen to 5 bars. The valve is opened to empty the container. This procedure is repeated twice and the sample with 5 bar oxygen atmosphere is placed at 80 °C. The oxygen pressure as a function of time is measured and the induction period (IP) calculated in hours.

***Results***

Purified transferase from Aeromonas salmonicide sample no. #179 and #178-9 were used to treat egg yolk as outlined in Table 2. The initial test has shown that GCAT transferase should be added with much lower phospholipase (PLU) activity, than a commercial Phospholipase. This is explained by the fact that GCAT is a transferase and therefore has much lower by hydrolytic activity than a normal phospholipase.

**Table 2**

| | Sanofo egg yolk 8%salt | 2344-44 C89 18,5 PLU-7/ml | Transferase #178-9 | # 3108, Lecitase Ultra | Water | |
|---|---|---|---|---|---|---|
| nr | Egg yolk | Transferase #179 | 18.5 PLU-7/ml | 1500 PLU-7/ml 7/ml | | |
| | gram | gram | gram | ml | gram | PLU-7/ml |
| 6 | 120 | 2.00 | | | 8.00 | 0.31 |
| 7 | 120 | | 10 | | 0 | 1.25 |
| 8 | 120 | | | 1.86 | 8.14 | 23.25 |
| 9 | 120 | | | | 10 | 0 |

The enzymatic reactions were conducted by scaling the egg yolk and the enzyme in a beaker. The samples were placed in a heating cabinet at 37 °C during slow agitation. After 1, 2 and 4 hours reaction time a sample was taken out for TLC analysis. After 4 hours reaction time the product was stored at 5 °C and used for mayonnaise experiments.
The TLC analyses of lipids extracted from enzyme treated egg yolk is shown in Figure 48.
The TLC analysis in Figure 48 shows a clear hydrolytic effect of Phospholipase #3108 on triglyceride during-formation of free fatty acids, as well as some mono- and diglyceride. Phospholipase #3108 seem to have no effect on cholesterol. Both transferase samples clearly contribute to the formation of cholesterol ester concomitant with the reduction of the cholesterol content

**D. Enzyme modified egg yolk in Mayonnaise**

In order to investigate the effect of the modification of the egg yolk samples mentioned in Table 2, application trials were performed on mayonnaise with a fat content of 50%. A mayonnaise containing untreated egg yolk was also produced.
The aim of the investigation was to determine the impact of enzymatscally modified egg yolks' emulsification properties and the impact on heat stability. All mayonnaise samples contained the same oil level and were emulsified with only egg yolk.
The mayonnaise samples were all produced using a Konuna mixer (Disho V60/10) and heated during processing to 95 °C for 5 minutes.

Samples of the mayonnaises (Figure 49) produced by enzyme treated egg yolk were nice and homogenous with no oil separation. The control sample separated in an oil and a water phase.

The particle size of oil droplet in the mayonnaise samples with enzyme treated egg yolk was measured on a Malvem Mastersizer. The sample was mixed with 0.1 %SDS in 0.1 M phosphate buffer pH 7 prior to measurement Reading was mean size of all particles as shown in Table 3. **Table 3.**

| Experiment | Enzyme | Mean particle size, μm |
|---|---|---|
| 6 | Transferase #179, 0.31 PLU-7/g | 12.9 |
| 7 | Transferase #178-9, 1.25 PLU-7/g | 7.2 |
| 8 | #3108, Lecitase Ultra, 23 PLU-7/g | 5.2 |

The results from the particle size measurement clearly show the effect of increased dosage of transferase from *A. salmonicida.* With the high dosage of transferase the particle size is close to the mayonnaise produced by Lecitase Ultra. It should however be kept in mind that Lecitase Ultra produces a lot of fatty acids, which might contribute to a finer particle distribution.

The oil droplet size of the mayonnaise prepared with the enzyme is significantly smaller than the oil droplet size of the mayonnaise prepared without the enzyme (i.e. the control mayonnaise).

Oxidation stability

The oxidation stability of the mayonnaise samples 7 and 8 were analyzed on a ML OXIPRES with results shown in Table 4.

**Table 4**

| Sample | Induction period | Induction period |
|---|---|---|
| | 1. determination hours | 2. determination hours |
| 7 | 37.44 | 38.08 |
| 8 | 35.68 | 35.52 |

Measurement of oxidation stability gave a clear significant difference in oxidation stability. The mayonnaise with transferase 179-8 treated egg yolk had a significant better oxidation stability than the mayonnaise with Lecitase Ultra treated egg yolk. This might be explained by the fact that Lecitase Ultra produces more free fatty acids which are more prone to oxidation that the corresponding fatty acid esters.

A sample of the egg yolks used for mayonnaise production were extracted with chloroform, and the lipids from the egg yolk were analysed by GLC with results shown in Table 5.

**Table 5**

| Experiment | Enzyme | Fatty acid | Cholesterol | Cholesterol ester | Triglyceride |
|---|---|---|---|---|---|
| 6 | Transferase #179 | 0.96 | 0.94 | 0.49 | 23.95 |
| 7 | Transferase #178-9 | 1.84 | 0.60 | 1.06 | 24.54 |
| 8 | #3108, Lecitase Ultra | 14.05 | 1.16 | 0.12 | 2.45 |
| 9 | Control | 0.48 | 1.16 | 0.13 | 22.87 |

The GLC results in Table 5 confirm the results form the TLC analysis that Lecitase Ultra produces a very high amount of free fatty acids and a large part of the triglyceride is hydrolysed. On the other hand the transferase produces only modest amount of free fatty acids and no triglycerides are hydrolysed. It is also clearly shown that transferase produce cholesterol ester from cholesterol.

The results indicate that the amount of PC in the "enzyme treated" mayonnaise is reduced as compared with the control mayonnaise, whilst the amount of LPC is increase in the enzyme treated mayonnaise as compared with the control mayonnaise. The increase in the amount of LPC may well explain the improved emulsification properties of the enzyme treated mayonnaise as compared with the control mayonnaise. The HPLC and GLC analyses also indicate a lower level of free cholesterol in the enzymes treated mayonnaise as compared with the control mayonnaise, probably due to the cholesterol being used as an acceptor molecule in the transferase reaction resulting in an increase in the amount of cholesterol esters in the enzyme treated mayonnaise as compared with the control mayonnaise. In addition, the results indicate that the amount of free fatty acids do not increases significantly when egg yolk is treated with the transferase. The results further indicate that the amount of free fatty acids produced in the foodstuff treated with the lipid acyltransferase is significantly lower than in the foodstuff treated with the control phospholipase, this is true even if the amount of lysolecithin formed in the foodstuffs is the same.

**EXAMPLE 8: Effect of *Aeromonas salmonicida* transferase in cakes.**

The effect of GCAT acyl-transferase form *Aeromonas salmonicida* is tested in a cake recipe. The enzyme is tested alone and in combination with other lipolytic enzymes. The enzymes are added to some of the cake ingredients or added together with the other cake ingredients before mixing the cake.

Preliminary results show that acyl-transferase combined with a triglyceride-hydrolysing enzyme improves the cake volume and crumb structure compared with a control.

In the following experiments a transferase from *A. salmonicida* and variants are tested alone and in combination with triglyceride hydrolysing enzymes. These enzymes are active on the lipid components in the egg and the shortening as well as on the carbohydrates, protein, glycerol and cholesterol (in egg), which forms part of the cake recipe.

**Materials and method**

Enzyme

#179, Acyl-transferase from *Aeromonas salmonicida*
Grindamyl EXEL 16, Lipase from *Thermomyces lanuginisus*

Cake recipe:

| Ingredients | % | g |
|---|---|---|
| Sugar 35/20 | 20,37 | 204 |
| Cake flour, Albatros | 18,11 | 181 |
| Wheat starch | 5,21 | 52 |
| Baking powder | 0,36 | 4 |
| Pasteurised liquid whole egg | 22,63 | 226 |
| Shortening Vegao (Aarhus United) | 18,11 | 181 |
| Whey powder | 0,68 | 7 |
| Glucose sirup ,75% 42 DE | 4,53 | 45 |
| Glycerol | 1,36 | 14 |
| Salt | 0,32 | 3 |
| Rape seed oil | 6,34 | 63 |
| Potassium sorbate | 0,18 | 1,8 |

**Equipment:**

Mixer: Hobart N50 with a spatula
Oven: Simon cake oven

**Procedure:**

All ingredients must be tempered to room temperature.

1. Cream up sugar and shortening for 3 minutes - start at $2^{nd}$ speed and move to $3^{rd}$ speed within 30 sec
2. Add remaining ingredients - start at $1^{st}$ speed and move to $2^{nd}$ speed within 30 sec - mix total 5 min
3. Measure the volume of the batter in 1 dl cup
4. The pound cake tins are sprayed with "Babette" oil spread, and covered with paper
5. Scale 2 x 350 g into the pound cake tins
6. Spread out the mass evenly with a spatula
7. Before put in the oven - add a string of oil on top of the cake (lengthwise in the middle - to make the cake break in the middle
8. Bake for 50 min. at 180°C
9. After baking take the tins out of the oven, and "drop" it on the table, before taking the cakes out of the tins
10. Take paper off the cakes and turn the right side up
11. The cakes are cooled on a grating for 60 min. before weighing and measuring of the volume

Remarks:

The enzyme(s) used is/are added at the beginning of mixing or is/are added to some of the cake ingredients before added to the other cake ingredients.

The enzymes are only active during the mixing or reaction of cake components, and the enzymes are inactivated during baking of the cake.

Results.

The following experiments are conducted as shown in the following table:

| | | 1 | 2 | 3 | 4 |
|---|---|---|---|---|---|
| Whole egg | G | 250 | 250 | 250 | 250 |
| Glucose syrup, 75% DE 42 | G | 10 | 10 | 10 | 10 |
| #179 acyl-transferase, 26 PLU/ml | Ml | 25 | | 25 | |
| Grindamyl EXEL 16, | Mg | | 37,5 | 37,5 | |
| Water | | | | | 25 |

Egg, Glucose syrup and enzyme are reacted for 30 minutes at 37 °C and shortly after the eggs are use to produce cake according to the recipe mentioned above.

Preliminary results show that a combination of acyltransferase and a triglyceride hydrolysing lipase from *Thermomyces lanoginosus* improves the cake volume, and also the crumb structure, eating quality and appearance is improved compared with a water control. Preliminary results indicate in cake it may be preferably to use a combination of lipid acyltransferase and a lipase.

**EXAMPLE 9: The purpose of these experiments was to test a transferase from A. *hvdrophila* expressed in *E. coli.***

The transferase reaction *of A. hydrophila* #135 (0.5 NEFA-PLU/ml) was tested in egg yolk. The experimental set-up is shown in Table 6.

**Table 6**

| | Reaction time | Egg yolk | #135 conc. |
|---|---|---|---|
| Nr | Minutes | Gram | units, PLU-NEFA |
| 1 | 30 | 1 | 0,000 |
| 2 | 30 | 2 | 0,100 |

(continued)

| | Reaction time | Egg yolk | #135 conc. |
|---|---|---|---|
| Nr | Minutes | Gram | units, PLU-NEFA |
| 3 | 60 | 2 | 0,100 |
| 4 | 150 | 2 | 0,100 |
| 5 | 240 | 2 | 0,100 |
| 6 | 1560 | 2 | 0,100 |
| 7 | 1560 | 1 | 0,000 |

The egg yolk was heated to 37°C and the enzyme added. After reaction time 7 ml $CHCl_3$:Methanol 2:1 was added and mixed on a Whirley for 30 sec.

The sample was centrifuged 800 x g for 10 minutes and the lower solvent phase isolated. 2 μl of this sample was applied onto a TLC Silica plate and eluted in elution solvent IV. The results from the TLC analysis is shown in Figures 50 and 51.

The methods and materials mentioned in this Example are those detailed in Examples above.

Samples from this experiment was also analysed by GLC as TMS derivatives. The results from the GLC analysis are shown in Table 7.

**Table 7.** GLC analysis of lipid from egg yolk

| No. | Reaction | Transferase #135 conc. | | | |
|---|---|---|---|---|---|
| | time | Units/g egg yolk | Free fatty acid | Cholesterol | Cholesterolester |
| | min | | % | % | % |
| 7 | control | 0 | 0,25 | 2,88 | 0,34 |
| 3 | 60 | 0,025 | 0,25 | 2,68 | 0,56 |
| 4 | 150 | 0,025 | 0,29 | 1,85 | 1,72 |
| 5 | 240 | 0,025 | 0,53 | 1,42 | 3,54 |
| 6 | 1560 | 0,025 | 0,95 | 0,3 | 4,43 |

From the GLC analysis of free fatty acid, cholesterol and cholesterolester it is possible to calculate the molar concentration of each component and calculate % transferase activity as shown in Table 7.

Calculation of % transferase activity

From the results the increase in free fatty acid, sterol esters are calculated

$$\Delta \text{ \% fatty acid} = \text{\% Fatty acid(enzyme)} - \text{\% fatty acid(control)}$$

$$\Delta \text{ \% sterol ester} = \text{\% sterol/stanol ester(enzyme)} - \text{\% sterol/stanol ester(control)}$$

The transferase activity is calculated as % of the total enzymatic activity:

$$\% \text{ transferase activity} = \frac{\Delta \% \text{ sterol ester}/(\text{Mv sterol ester}) \times 100}{\Delta \% \text{ sterol ester}/(\text{Mv sterol ester}) + \Delta \% \text{ fatty acid}/(\text{Mv fatty acid})}$$

where:

Mv sterol ester = average molecular weight of the sterol esters
Mv fatty acid = average molecular weight of the fatty acids

Table 8 Transferase activity in egg yolk of *A.hydrophila* #135

| No. | Reaction. | Transferase #135 conc. | | | | |
|---|---|---|---|---|---|---|
| | Time | Units/g egg yolk | Free fatty acid | Cholesterol | Cholesterol-ester | Transferase activity |
| | min | | mM | mM | mM | % |
| 7 | Control | 0 | 8,9 | 74,5 | 5,3 | - |
| 3 | 60 | 0,05 | 8,9 | 69,3 | 8,7 | 100 |
| 4 | 150 | 0,05 | 10,4 | 47,8 | 26,5 | 93 |
| 5 | 240 | 0,05 | 18,9 | 36,7 | 54,6 | 77 |
| 6 | 1560 | 0,05 | 33,9 | 7,8 | 68,4 | 48 |

Both TLC and GLC analysis confirm that initially the transferase reaction of A. *hydrophila* #135 is the dominating reaction. After 150 minutes reaction time some hydrolytic activity occurs. After 1560 minutes the transferase reaction and the hydrolytic reaction has almost reached the same level. The results also indicate that as long as the acceptor molecule cholesterol is available the transferase reaction is the dominating reaction. When the concentration of cholesterol decreases the hydrolytic activity becomes more dominant.

**EXAMPLE 10: Assay for measurement of transferase activity using egg yolk as substrate - hereinafter referred to as the "Egg Yolk Assay"**

A lipid acyltransferase was isolated from *Aeromonas salmonicida* and expressed in *Bacillus subtilis.* The purpose of this work is to develop an analytical method, which is able to measure both transferase and hydrolytic activity of enzymes and from these analyses it is possible to define both transferase and hydrolytic activity of enzymes using a Substrate which contain lecithin and cholesterol.

In this work egg yolk was used as substrate for the enzyme assay because egg yolk contain both lecithin and cholesterol and it is known that transferases and phospholipases works very well in this substrate.

The drawback by using egg yolk is that this substrate is a complex mixture of water, lipids, and proteins. Lipid components include glycerides, 66.2%; phospholipids, 29.6%; and cholesterol, 4.2%. The phospholipids consist of 73% lecithin, 15% cephalin, and 12% other phospholipids. Of the fatty acids, 33% are saturated and 67% unsaturated, including 42% oleic acid and 7% linoleic acid (ref. Kirk-Othmer Encyclopedia of Chemical Technology, John Wiley & Sons, Inc.)

Some variations in the egg yolk composition might be expected. In the literature (Biochimica et Biophysica Acta, 1124 (1992) 205-222) it is however mentioned that "The mature egg yolk of the domestic hen possesses remarkably constant lipid and lipoprotein composition despite much variation in dietary and environmental conditions", and further it is quoted "As a result the egg yolk continues to provide a food product of nearly constant composition, which serves to maintain its chemical and physical-chemical properties for reliable utilization in the baking, cosmetic and pharmaceutical industries"

This reference indicates that egg yolk composition is very constant and it was therefore decided to use hens egg yolk as substrate for the Egg Yolk Assay.

Quantification of lipid reaction products from enzymatic treatment of egg yolk was made by extraction of lipids from the substrate followed by GLC analysis of the lipid components.

Procedure

Materials.

Egg yolk: Pasteurised liquid egg yolk from Danæg Products A/S, DK- 4000 Roskilde.
HEPES buffer Sigma cat. no. H 3375
Chloroform, Analytical grade

Enzymes.

Purified lipid acyltransferase from *A. salmonicida* #178-9
*Thermomyces lanuginosus* lipase. GRINDAMYL EXEL 16, item nr. 147060 (Control)

<u>Enzyme assay with egg yolk substrate.</u>

5 gram liquid egg yolk was scaled in a 20ml Wheaton glass and heated to 35 °C 0.25ml enzyme solution was added and a stopwatch is started.
At regular intervals 0.5 g samples were transferred to a 10ml Dram glass.
20 µl 4M HCl was added in order to stop the enzyme reaction and acidify the fatty acid soap.
3 ml Chloroform was added. And the sample was mixed on a Whirley mixer for 30 see.

The sample was centrifuged at 3000 g for 10 min and 0.8 ml of the chloroform phase was transferred to a tarred Dram glass. Chloroform was evaporated at 60 °C under a steam of nitrogen. The dram glass was scaled again. The isolated lipids were analysed by GLC and TLC.

TLC analysis - as described herein.

GLC analysis- as described herein.

**<u>Results</u>**

For the Egg Yolk Assay using egg yolk as substrate the experiment shown in Table 9 was conducted.

**Table 9**

| | | **1** | **2** | **3** |
|---|---|---|---|---|
| Egg yolk, liquid. | gram | 5 | 5 | 5 |
| Transferase# 178-9,32 PLU-7/ml* | ml | | | 0.25 |
| T.lanuginosus lipase, 200 LIPU/ml | ml | | 0.25 | |
| Water | ml | 0.25 | | |

0.5g samples were taken out after 15, 30, 60 120 and 1080 minutes, and the lipid isolated by solvent extraction. The lipids were analysed by TLC using solvent I and IV respectively. Picture of the TLC plate is shown in Figure 52.

The TLC analysis clearly indicates the activity of transferase #178-9 from A. *salmonicida* (sample 3). This can be seen from the decrease in the phospholipids PC and PE. The results also indicate that the amount of lysolecithin LPC is not as high as expected. This might indicate hydrolytic activity on lysolecithin or it might also be caused by insufficient extraction because lysolecithin is very polar and therefore could be partly distributed in the water phase.

The lipids isolated by solvent extraction was also analysed by GLC in order to quantify the amount of free fatty acid, cholesterol and cholesterol ester. The GLC results are shown in Table 10.

Table 10. GLC analysis of lipid from enzyme treated egg yolk. Results are in % based on lipid content.

| | | | 15 | 30 | 60 | 120 | 1080 |
|---|---|---|---|---|---|---|---|
| | | | Minutes | Minutes | Minutes | Minutes | Minutes |
| Free fatty acids | Control | 1 | 0.328 | 0.304 | 0.332 | 0.333 | 0.369 |
| | T.lanuginosus | 2 | 0.391 | 0.376 | 0.459 | 0.627 | 22.909 |
| | A. salmonicida#178-9 | 3 | 1.007 | 1.668 | 4.013 | 6.761 | 15.098 |
| | | | | | | | |
| Cholesterol | Control | 1 | 3.075 | 2.968 | 3.103 | 3.056 | 3.099 |
| | T.lanuginosus | 2 | 3.130 | 3.032 | 3.045 | 3.026 | 3.225 |
| | A. salmonicida #178-9 | 3 | 2.835 | 1.912 | 0.356 | 0.220 | 0.206 |
| | | | | | | | |
| Cholesterol | | | | | | | |
| ester | Control . | 1 | 0.416 | 0.397 | 0.422 | 0.408 | 0.437 |
| | T.lanuginosus | 2 | 0.436 | 0.400 | 0.425 | 0.419 | 0.416 |
| | A.salmonicida #178-9 | 3 | 1.414 | 2.988 | 6.107 | 6.694 | 5.760 |
| | | | | | | | |
| Triglyceride | Control | 1. | 76.153 | 73.505 | 75.565 | 79.344 | 77.382 |
| | T.lanuginosus | 2 | 74.099 | 74.413 | 77.079 | 74.284 | 21.781 |
| | A.salmonicida #178-9 | 3 | 73.781 | 73.342 | 77.857 | 82.040 | 72.117 |

From the results it was observed that almost all the cholesterol was esterified after 60 minutes in sample 3. It was concluded that for the first 30 minutes there was surplus substrate for the reaction. The results form samples taken out after 15 and 30 minutes were therefore used to calculate the activities of the enzymes.

Based on the information in table 10 and the fact that egg yolk contain 27% lipid the amount of micromole fatty acid and cholesterol ester produced per ml enzyme was calculated with results shown in Table 11

The results in Table 11 were obtained be the following calculations of the results from fatty acids in sample no.3 (*A. salmonicida,* 15 min.)

Lipid in 5 g egg yolk = 5*0.27 = 1.35 gram

1.35 gram lipid contain 1.007% fatty acids = 1.35*1.007/100 = 0.01359 gram

Average molecular weight of fatty acids is 272

0.01359gram = 0.01359* 1000000/272 μmol = 49.9798 μmol

0.25 ml enzyme is added

μmol Fatty acid/ml enzyme = 49.9798/0.25 = 199.9

**Table 11**

| Micromole/ml enzyme | | | | |
|---|---|---|---|---|
| | | 0 min | 15 min | 30 min |
| Free fatty acid | Control | | 65.01 | 60.37 |
| | T. lanuginosa | | 77.61 | 74.71 |
| | Transferase # 178-9 | | 199.86 | 331.06 |
| | | | | |
| Cholesterol ester | Control | | 35.09 | 33.50 |
| | T. lanuginosa | | 36.77 | 33.73 |
| | Transf. #178-9 | | 119.29 | 252.15 |

From the results in Table 11 it is possible to calculate the change in amount of fatty acid and cholesterol ester caused by the enzyme relative to control as shown in Table 12.

**Table 12.**

| Δ Micromole/ml enzyme | | 0 min | 15 min | 30 min |
|---|---|---|---|---|
| Free fatty acid | T. lanuginosus | 0 | 12.593 | 14.340 |
| | Transf. #1.78-9 | 0 | 134.843 | 270.691 |
| | | | | |
| Cholesterol ester | T. lanuginosus | 0 | 1.677 | 0.235 |
| | Transf. #178-9 | 0 | 84.196 | 218.652 |

The amount of fatty acid or cholesterol ester produced as a function of time is shown in Figure 53.

From the slope of the curve the hydrolytic activity (FFA formation) and the lipid acyltransferase activity (cholesterol ester formation) as a function of time was calculated. The relative transferase activity (% acyltransferase activity) and the relative hydrolytic activity were then calculated as shown in Table 13. The relative transferase activity was determined using the protocol for the determination of % acyltransferase activity as described hereinbefore. For example, calculation of relative activity for #178-9:Total activity is FFA activity + transferase activity = 9,023+7,2884= 16,311 μmol/min/ml, Relative transferase activity= 7,28884*100/16,311=44,7, Relative hydrolytic activity= 9,023*100/16,311= 55,3

**Table 13.**

| | | | |
|---|---|---|---|
| T. lanuginosus | FFA activity | 0.4780 | μmol/min/ml |
| A. salmonicida #178-9 | FFA activity | 9.0230 | μmol/min/ml |
| | | | |
| T. lanuginosus | Cholesterol ester. Activity | 0.0078 | μmol/min/ml |
| A. salmonicida#178-9 | Cholesterol ester. Activity | 7.2884 | μmol/min/ml |
| | | | |
| T .lanuginosus | Relative transferase activity | 1.6 | |
| A. salmonicida #178-9 | | 44.7 | |
| | | | |
| T. lanuginosus | Relative hydrolytic activity | 98.4 | |
| A. salmonicida #178-9 | | 55.3 | |

The results in Table 13 confirmed that the transferase enzyme from *A. salmonicida* has a significant transferase activity, but the results also confirmed that this enzyme has a significant hydrolytic activity.

The lipase from T. lanuginosus mainly has hydrolytic activity, and the relative transferase activity 1.6 was not a proof of any transferase activity but was explained by the uncertainty of the analysis.

Conclusion.

Egg yolk was used as substrate for the measurement of transferase and hydrolase activity of lipid acyltransferase from *Aeromonas salmonicida* and a lipase from *Thermomyces lanuginosus.* Under assay conditions there was initially a linear relation between cholestererol ester and free fatty acid formation and time for the lipid acyltransferase enzyme. Based on this linear relationship it was possible to calculate the hydrolytic activity (FFA formation) and the transferase activity (cholesterol ester formation). The relative hydrolytic and transferase activity was also calculated. The lipid acyltransferase (in this case a GCAT) from *Aeromonas salmonicida* showed almost equal hydrolytic and transferase activity under these assay conditions. Lipase from *Thermomyces lanuginosus* showed very low hydrolytic activity and the transferase activity was not significant.

## EXAMPLE 11: Transferase Assay in High Water Egg Yolk.

### Introduction

A lipid acyltransferase in accordance with the present invention was isolated from *Aeromonas salmonicida* and expressed in *Bacillus subtilis.* Initial experiments have shown that this enzyme is very efficient in transferring fatty acid from lecithin to cholesterol using egg yolk as a substrate.
In the following experiments the transferase reaction was studied in further detail using egg yolk as a substrate with special focus on the water concentration in the substrate.

Procedure

Materials.

Egg yolk: Pasteurised liquid egg yolk from Danæg Products A/S, DK- 4000 Roskilde.
HEPES buffer Sigma cat. no. H 3375
Chloroform, Analytical grade
Squalane, analytival grade

Enzymes.

#178-9 Lipid acyl transferase in accordance with present invention from A. *salmonicida*
#2427 Phospholipase A1 from *Fusarium oxysporum.* LIPOPAN® F from Novozymes, DK (comparative lipolytic enzyme)
#1991 Phospholipase A2 from Pancreas, LIPOMOD 22L from Biocatalysts, UK (comparative lipolytic enzyme)

Enzvme assay with egg yolk substrate.

5 gram liquid egg yolk substrate was scaled in a 20ml Wheaton glass and heated to 35 °C
Water and enzyme solution was added and a stopwatch is started.
At regular intervals 0.5g samples was transferred to a 10ml Dram glass.
20 µl 4M HCl was added in order to stop the enzyme reaction and acidify the fatty acid soap.
3 ml Chloroform was added. And the sample was mixed on a Whirley mixer for 30 see.
The sample was centrifuged at 3000 g for 10 min and 0.8 ml of the chloroform phase was transferred to a tarred Dram glass. Chloroform was evaporated at 60 °C under a steam of nitrogen. The dram glass is scaled again.
The isolated lipids are analysed by GLC

GLC analysis

Perkin Elmer Autosystem 9000 Capillary Gas Chromatograph equipped with WCOT fused silica column 12.5 m x 0.25 mm ID x 0.1 µ film thickness 5% phenyl-methylsilicone (CP Sil 8 CB from Chrompack).
Carrier gas; Helium.
Injector. PSSI cold split injection (initial temp 50°C heated to 385°C), volume 1.0µl Detector FID: 395°C

| Oven program: | 1 | 2 | 3 |
|---|---|---|---|
| Oven temperature, °C. | 90 | 280 | 350 |
| Isothermal, time,min. | 1 | 0 | 10 |
| Temperature rate, °C/min. | 15 | 4 | |

Sample preparation: 30 mg of sample was dissolved in 9 ml Heptane:Pyridin, 2:1 containing internal standard heptadecane, 0.5 mg/ml. 300µl sample solution was transferred to a crimp vial, 300 µl MSTFA (N-Methyl-N-trimethylsilyl-trifluoraceamid) was added and reacted for 20 minutes at 60°C.
Calculation: Response factors for mono-di-triglycerides and free fatty acid were determined from Standard 2 (mono-di-triglyceride), for Cholesterol, Cholesteryl palmitate and Cholesteryl stearate the response factors were determined from pure reference material (weighing for pure material 10mg).

**Results**

Egg yolk containing 2% squalane was used as substrate for the reactions. Squalane was added as an internal standard for the GLC analysis, in order to quantify the lipid components in egg yolk.
The experiment was set up as shown in Table 14.

**Table 14.**

| | | 1 | 2 | 3 | 4 | 5 | 6 | 7 | 8 |
|---|---|---|---|---|---|---|---|---|---|
| Substrate, egg yolk with 2%squalane | g | 5 | 5 | 5 | 5 | 5 | 5 | 2.5 | 2.5 |
| Transferase # 178-9, 14 PLU-7/ml | ml | | 0.25 | | | 025 | | 0.13 | |
| LIPOPAN® Fsolution, 200 PLU-7/ml | ml | | | 0.25 | | | | | 0.13 |
| #1991 Phospholipase A2, 6300 PLU/ml | ml | | | | 0.25 | | 0.25 | | |
| | | | | | | | | | |
| Water | ml | 0.25 | | | | 3.8 | 3.8 | 8.75 | 8.75 |

Samples were taken out after 30, 60 and 120 minutes and analysed according the method described above (0.5 ml (exp 1-4) 0.86 ml (exp. 5-6) and 2.2 ml(exp.7-8) samples were taken).

The results from the GLC analysis are shown in Table 15. The GLC results were expressed in percent of the substrate (egg yolk). The table also indicate the reaction time and the total amount of water in the reaction mixture.

**Table 15.**

| Enzyme | Reaction time | Water % | GLC | GLC | GLC |
|---|---|---|---|---|---|
| | minutes | in reaction | % Fatty acid | % cholesterol% | cholesterol ester |
| Control | 120 | 54 | 0,247 | 0,863 | 0,083 |
| # 178 | 30 | 54 | 0,4°22 | 0,669 | 0,445 |
| #178 | 60 | 54 | 0,515 | 0,549 | 0,672 |
| #178 | 120 | 54 | 0,711 | 0,364 | 1,029 |
| #2427 | 30 | 54 | 2,366 | 0,848 | 0,090 |
| #2427 | 60 | 54 | 3,175 | 0,837 | 0,088 |
| #2427 | 120 | 54 | 3,926 | 0,833 | 0,082 |
| #1991 | 30 | 54 | 1,606 | 0,911 | 0,083 |
| #1991 | 60 | 54 | 1,701 | 0,838 | 0,080 |
| #1991 | 120 | 54 | 1,781 | 0,763 | 0,053 |
| # 178 | 30 | 73 | 0,377 | 0,764 | 0,495 |
| #178 | 60 | 73 | 0,488 | 0,665 | 0,719 |
| #178 | 120 | 73 | 0,626 | 0,426 | 0,931 |
| #2427 | 30 | 73 | 2,471 | 0,853 | 0,092 |
| #2427 | 60 | 73 | 3,284 | 0,858 | 0,087 |
| #2427 | 120 | 73 | 4,176 | 0,837 | 0,081 |
| #178 | 30 | 89 | 0,344 | 0,720 | 0,308 |
| #178 | 60 | 89 | 0,443 | 0,725 | 0,446 |
| #178 | 120 | 89 | 0,610 | 0,597 | 0,607 |

(continued)

| Enzyme | Reaction time | Water % | GLC | GLC | GLC |
|---|---|---|---|---|---|
| | minutes | in reaction | % Fatty acid | % cholesterol% | cholesterol ester |
| #2427 | 30 | 89 | 0,510 | 0,167 | 0,010 |
| #2427 | 60 | 89 | 0,602 | 0,133 | 0,010 |
| #2427 | 120 | 89 | 0,867 | 0,147 | 0,009 |

Based on the analyses of fatty acid, cholesterol and cholesterol ester it was possible to calculate the amount of free fatty acid, and cholesterol ester produced as a function of reaction time and water content. Based on these results it was then possible to calculate the total enzymatic activity as the sum of the fatty acid formation and the cholesterol ester formation. The relative hydrolytic activity and the relative transferase activity (i.e. % acyltransferase activity) were then calculated with the results shown in Table 16.

The results in Table 16. were also analysed statistically using a Statgraphic Multifactor ANOVA . The statistical results in Figure 54 confirm that Phospholipase A1, #2427 and phospholipase A2, #1991 have no transferase activity whereas the transferase #178-9 showed almost 50% transferase activity under these assay conditions.

The effect of water content in the assay on the transferase activity of the transferase #178 was also analysed statistically as shown in Figure 55. These results indicate that in the range from 54 to 89% water in the assay there was no strong effect of the water content on the relative transferase activity.

The impact of reaction time on transferase activity for transferase #178 was evaluated with results shown in Table 16 and Figure 56. The results in Figure 56 indicate that the relative transferase activity decreases as a function of reaction time. This might be explained by the fact that most of the acceptor molecule cholesterol is consumed and therefore the relative hydrolytic activity increases. The negative values for transferase reaction for #2427 only indicate no transferase activity within the variation for the analytical method.

**Table 16.**

| Enzyme | Reaction time minutes | Water % in reaction mixture | Fatty acid Produced | Cholesterol Consumed | Cholesterol ester produced | Hydrolytic activity % | Transferase activity % |
|---|---|---|---|---|---|---|---|
| # 178 | 30 | 54 | 0,175 | 0,194 | 0,362 | 53 | 47 |
| # 178 | 60 | 54 | 0,268 | 0,314 | 0,589 | 52 | 48 |
| # 178 | 120 | 54 | 0,464 | 0,499 | 0,946 | 53 | 47 |
| #2427 | 30 | 54 | 2,119 | 0,015 | 0,007 | 100 | 0 |
| #2427 | 120 | 54 | 2,928 | 0,026 | 0,005 | 100 | 0 |
| #2427 | 60 | 54 | 3,679 | 0,030 | -0,001 | 100 | 0 |
| #1991 | 30 | 54 | 1,359 | -0,048 | 0,000 | 100 | 0 |
| #1991 | 60 | 54 | 1,454 | 0,025 | -0,003 | 100 | 0 |
| #1991 | 120 | 54 | 1,534 | 0,100 | -0,030 | 101 | -1 |
| # 178 | 30 | 73 | 0,130 | 0,099 | 0,412 | 42 | 58 |
| # 178 | 60 | 73 | 0,241 | 0,198 | 0,636 | 47 | 53 |
| # 178 | 120 | 73 | 0,379 | 0,437 | 0,848 | 51 | 49 |
| #2427 | 30 | 73 | 2,224 | 0,010 | 0,009 | 100 | 0 |
| #2427 | 60 | 73 | 3,037 | 0,005 | 0,004 | 100 | 0 |
| #2427 | 120 | 73 | 3,929 | 0,026 | -0,002 | 100 | 0 |
| # 178 | 30 | 89 | 0,097 | 0,143 | 0,225 | 50 | 50 |
| # 178 | 60 | 89 | 0,196 | 0,138 | 0,363 | 56 | 44 |
| # 178 | 120 | 89 | 0,363 | 0,266 | 0,524 | 62 | 38 |

(continued)

| Enzyme | Reaction time minutes | Water % in reaction mixture | Fatty acid Produced | Cholesterol Consumed | Cholesterol ester produced | Hydrolytic activity % | Transferase activity % |
|---|---|---|---|---|---|---|---|
| #2427 | 30 | 89 | 0,263 | 0,696 | -0,073 | 113 | -13 |
| #2427 | 60 | 89 | 0,355 | 0,730 | -0,073 | 110 | -10 |
| #2427 | 120 | 89 | 0,620 | 0,716 | -0,074 | 105 | -5 |

Conclusion.

The lipid acyltransferase from *Aeromonas salmonicida* was tested in egg yolk as substrate and with different levels of water content. This enzyme was compared with control lipolytic enzymes, namely Phospholipase A1 from *Fusarium oxysporum* and a *Phospholipase* A2 from pancreas.

The results have proved that only the transferase catalysed the transferase reaction between lecithin and cholesterol during formation of cholesterol ester. The results showed that in the range from 54% to 89 % water in the substrate the relative transferase activity was almost the same for transferase from *Aeromonas salmonicida.*

**EXAMPLE 12 : The "Transferase Assay in Buffered Substrate" for measurement of acyltransferase activity (e.g. for use in a foodstuff using lecithin and cholesterol).**

The lipid acyltransferase was isolated from *Aeromonas salmonicida* and expressed in *Bacillus subtilis.* This enzyme is very efficient in transferring fatty acid from lecithin to cholesterol during formation of cholesterol esters. It has also been shown that the enzyme has some hydrolytic activity, which is observed by the formation of free fatty acid. Traditional phospholipases (EC3.1.1.4 and EC3.1.1.32) have the ability to hydrolyse lecithin during formation of free fatty acids and lysolecithin, and no transferase reactions has been reported for these enzymes.

We detail herein an assay that is able to measure both transferase and hydrolytic activity of enzymes and thus to identify lipid acyltransferases in accordance with the present invention, the assay uses a substrate which contains lecithin and cholesterol. In this work a substrate based on phosphatidylcholine and cholesterol dispersed in a buffer was used. Quantification of reaction products was made by extraction of lipids from the substrate followed by GLC analysis of the lipid components.

Procedure

Materials

L-alpha-Phosphatidylcholine 95% (Plant) Avanti no. 441601
Cholesterol: Sigma cat. C 8503
Cholesteryl Palmitate, Sigma C 6072
Cholesteryl Stearate, Sigma C 3549
HEPES buffer Sigma cat. No. H 3375
Chloroform, Analytical grade.

Enzymes

Purified GCAT from *A. salmonicida* #178-9
TLC analysis was carried out as described in Example 6.
GLC analysis was carried out as described in Example 11.

Results: Transferase assay based on phosphatidylcholine and cholesterol as substrate.

In the following the transferase activity of the transferase was tested in a substrate based on phosphatidylcholine and cholesterol according to the following procedure.

450 mg phosphatidylcholine (>95% PC Avanti item no. 441601) and 50 mg cholesterol was dissolved in chloroform and evaporated to dryness under vacuum. 300 mg cholesterol/phosphatidylcholine mixtures was transferred to a Wheaton glass and 15 ml 50mM HEPES buffer pH 7 was added. The lipid was dispersed in the buffer during agitation. The substrate was heated to 35 °C during mixing with a magnetic stirrer and 0.25 ml enzyme solution was added. This is a very high water environment of approximately 95% water.

Samples of 2 ml were taken out after 0, 5, 10, 15, 25, 40 and 60 minutes reaction time. Immediately 25 µl 4M HCl was added to acidify the free fatty acid and stop the enzymes reaction. 3.00 ml chloroform was added, and the sample was shaken vigorously on a Whirley for 30 seconds. The sample was centrifuged and 2 ml of the chloroform phase was isolated and filtered through 0.45-µm filters into a 10 ml tared Dram glass. The chloroform was evaporated under a stream of nitrogen at 60°C, and the samples were scaled again. The extracted lipid was analysed by GLC.

The results from the GLC analysis are shown in Table 17. The results are expressed in % calculated on extracted lipid. The amount of fatty acid and cholesterol ester formed as a function of time is illustrated in. Figure 57 It can be concluded from Figure 57 that the enzyme reaction is not linear as a function of time, because an initially strong both hydrolytic and transferase activity is observed. After approximately 10 minutes and until approximately 60 minutes the reaction shows an almost linear response of fatty acid and cholesterol ester formation as a function of time. It was therefore decided to look at the enzymatic reaction in this time interval.

**Table 17**

| Minutes | 0 | 5 | 10 | 15 | 25 | 40 | 60 |
|---|---|---|---|---|---|---|---|
| | | | | | | | |
| Cholesterol, % | 10.064 | 8.943 | 8.577 | 8.656 | 8.102 | 7.856 | 7.809 |
| Cholesterol ester, % | 0.000 | 1.571 | 2.030 | 2.058 | 2.282 | 2.659 | 3.081 |
| FFA total, % | 0.260 | 1.197 | 1.239 | 1.466 | 2.445 | 2.943 | 3.940 |

From the knowledge about the amount of lipid in the reaction mixture and the amount of enzyme added it was possible to calculate the formation of fatty acid and cholesterol ester expressed in µmol/ml enzyme (Table 18 and Figure 58)

**Table 18**

| Minutes | 10 | 15 | 25 | 40 | 60 |
|---|---|---|---|---|---|
| | µmol/ml | µmol/ml | µmol/ml | µmol/ml | µmol/ml |
| FFA total | 58.1 | 68.7 | 114.6 | 138.0 | 184.7 |
| Cholesterol ester | 88.8 | 90.0 | 99.3 | 115.6 | 133.8 |

From the results in Table 18 and the slope of the curves in Figure 58 it was possible to calculate the amount of fatty acid and cholesterol ester as a function of time expressed in µmol/min per ml enzyme.

The calculation of the hydrolytic activity and the transferase activity is shown in Table 19. The relative transferase activity was determined using the protocol for the determination of % acyltransferase activity as described hereinbefore.

**Table 19**

| | | |
|---|---|---|
| Hydrolytic activity (fatty acid) | 2.52 | µmol/min per ml enzyme |
| Transferase activity(cholesterol ester) | 0.94 | µmol/min per ml enzyme |
| Total activity | 3.45 | µmol/min per ml enzyme |
| | | |
| Relative Transferase activity | 27.1 | % |
| Relative hydrolytic activity | 72.9 | % |

**Screening of other enzymes for transferase activity.**

The method mentioned above was used to screen different lipolytic enzymes for transferase and hydrolytic activity. The enzymes were tested as shown in Table 20

**Table 20**

| | | 1 | 2 | 3 | 4 | 5 |
|---|---|---|---|---|---|---|
| Substrate | ml | 15 | 15 | 15 | 15 | 15 |
| #178-9Tmnsfense A. salmonicida 32 PLU-7/ml | ml | 0.25 | | | | |
| 5% #3016, LIPOPAN® F (F. oxysporum) | ml | | 0.25 | | | |
| 5%, Thermomyces lanuginosus | ml | | | 0.25 | | |
| 5% Candida rugosa #2983 | ml | | | | 0.25 | |
| 5% Candida cylindracea #3076 | ml | | | | | 0.25 |

The substrate containing 300mg phosphatidylcholine/cholesterol dispersed in 50 mM HEPES buffer pH 7.0 was heated to 35°C with agitation. Enzyme solution was added and the sample was kept at 35°C with agitation. Samples were taken out with regular interval and extracted with Chloroform. The isolated lipids were analysed by GLC with results shown in Table 21.

**Table 21**

| Sample | | | | | | | | |
|---|---|---|---|---|---|---|---|---|
| **1** | **Transferase 178-9** | | | | | | | |
| | Minutes | 0 | 5 | 10 | 15 | 25 | 40 | 60 |
| | FFA | 1.216 | 2.516 | 2.983 | 2.62 | 2.894 | 3.448 | 3.911 |
| | Cholesterol | 7.547 | 6.438 | 6.365 | 6.15 | 6.136 | 5.936 | 5.662 |
| | Chl. Ester | 0 | 1.835 | 2.177 | 2.44 | 2.58 | 2.851 | 3.331 |
| | | | | | | | | |
| **2** | **Fusarium oxysporum (LIPOPAN® F)** | 0 | 5 | 10 | 15 | 25 | 40 | 60 |
| | FFA | 1.216 | 1.345 | 1.796 | 1.95 | 2.487 | 2.424 | 2.977 |
| | Cholesterol | 7.547 | 7.309 | 7.366 | 7.33 | 7.429 | 7.341 | 7.326 |
| | Chl. Ester | 0 | 0.26 | 0.386 | 0.35 | 0.267 | 0.36 | 0.394 |
| | | | | | | | | |
| **3** | **Thermomyces lanuginosus** | 0 | 5 | 10 | 15 | 25 | 40 | 60 |
| | FFA | 1.216 | 0.853 | 0.875 | 1 | 0.896 | 1.105 | 1.009 |
| | Cholesterol | 7.547 | 7.384 | 7.639 | 7.63 | 7.675 | 7.603 | 7.529 |
| | Chl. Ester | 0 | 0 | 0 | 0 | 0 | 0 | 0 |
| | | | | | | | | |
| **4** | **Candida rugosa (#2938)** | 0 | 5 | 10 | 15 | 25 | 40 | 60 |
| | FFA | 1.216 | 0.982 | 0.987 | 1.02 | 1.135 | 1.131 | 1.15 |
| | Cholesterol | 7.547 | 7.438 | 7.656 | 7.66 | 7.638 | 7.575 | 7.585 |
| | Chl. Ester | 0 | 0 | 0 | 0 | 0 | 0 | 0 |
| | | | | | | | | |

(continued)

| 5 | **Candida cylandracea (#3076)** | 0 | 5 | 10 | 15 | 25 | 40 | 60 |
|---|---|---|---|---|---|---|---|---|
| | FFA | 1.216 | 1.032 | 1.097 | 1.07 | 1.203 | 1.131 | 1.43 |
| | Cholesterol | 7.547 | 7.502 | 7.425 | 7.65 | 7.619 | 7.502 | 7.411 |
| | Chl. Ester | 0 | 0 | 0 | 0 | 0 | 0 | 0 |

From the GLC analysis it was observed that only the lipid acyltransferase (178-9) produced significant amount of cholesterol ester and fatty acids. Phospholipase from *Fusarium oxysporum* also gave a steady increase in free fatty acid but only an initial small amount formation of cholesterol ester was formed but no increase in cholesterol ester as a function of time was observed.

Based on the knowledge about the amount of lipid substrate and the GLC analyses it was possible to calculate the relative transferase activity and the relative hydrolytic activity based on the results from 10 to 60 minutes reaction time. The results from Transferase 178-9 and *Fusarium oxysporum* lipase are shown in Table 21. The other enzymes tested showed no activity.

**Table 21**

| | Transferase 178-9 | Fusarium oxysporum |
|---|---|---|
| Hydrolytic activity, micromole/min per ml enzyme | 1.03 | 0.96 |
| Transferase activity, micromole/min per ml enzyme | 0.40 | 0.01 |
| Total activity, micromole/min per ml enzyme | 1.43 | 0.98 |
| | | |
| Relative hydrolytic activity | 71.8 | 98.7 |
| Relative transferase activity | 28.2 | 1.3 |

The result shown in Table 21 confirm a significant transferase activity from the lipid acyltransferase (sample 178-9). It is also observed that the relative transferase activity is in good agreement with the experiment mentioned in Table 19

A very low transferase activity form Fusarium oxysporum phospholipase is however observed. This transferase level is so low that it falls within the uncertainty of the analysis. As expected *Fusarium oxysporum* phospholipase has a significant hydrolytic activity.

Conclusion.

Instead of egg yolk (shown in Example 11) an artificial substrate based on purified phosphatidylcholine and cholesterol was used as a substrate to measure the activity of transferase from *Aeromonas salmonicida.* Between 10 minutes and 60 minutes reaction time the assay gave an almost linear formation of free fatty acids and cholesterol ester as a function of time. Based on the activity between 10 and 60 minutes reaction time the hydrolytic activity and the transferase activity was calculated.
The concentration of substrates in this assay was relatively lower than in egg yolk, and the amount of water in the assay was relatively higher.

Based on the results from the assay of the lipid acyltransferase (in this instance a GCAT) from *Aeromonas salmonicida* in a artificial substrate of phosphatidylcholine/cholesterol in buffer it is concluded that this enzyme has very good transferase activity also in a system with a very high water content.
Both assays based on egg yolk (see Example 11) and phosphatidylcholine/cholesterol in buffer (Example 12), can be used to measure the transferase and hydrolytic activity of enzymes. The egg yolk is preferred from the point of view that the hydrolytic and the transferase activity is linear as a function of time, but the phosphatidylcholine/cholesterol in buffer is only linear within a certain time limit.

## EXAMPLE 13: Food Emulsions

The effect of enzyme modified liquid egg yolk was tested in a standard Food emulsion recipe with 60 % oil.

Standard methods and materials are as per those detailed in the Examples above.
The egg yolk was treated with a lipid acyl transferase from *Aeromonas salmonicida(* #138) or phospholipase, namely a commercially available enzyme LipopanF® (Novozymes A/S, Denmark) (#2938) as shown in Table 22.

**Table 22.** Enzyme treatment of egg yolk.

| | | 1 | 2 | 3 | 4 |
|---|---|---|---|---|---|
| Egg Yolk, Sanofo product no 1123P2 | Gram | 10 | 10 | 10 | 10 |
| #138, 10 PLU/ml | Ml | 1 | 1 | | |
| #2938, 200 PLU/ml | Ml | | | 1 | |
| Water | Ml | | | | 1 |
| Reaction time | Minutes | 210 | 360 | 210 | 210 |

TLC analysis of the egg yolk lipids from enzyme treated egg yolk (Table 9) is shown in Figures 59 and 60.
In this experiment the dosage of #2938 was increased by a factor of 10 and this gave a very clear activity on egg yolk. The amount of free fatty acid increased significantly and lecithin (PC) was hydrolysed to lysolecithin (LPC). The transferase #138 gave a clear transferase reaction because free cholesterol was converted to cholesterol ester and part of the lecithin was converted to lysolecithin.
Another interesting aspect of the enzyme modification was the consistency of the product. The sample treated with Phospholipase #2938 became very solid, whereas the samples treated with the lipid acyltransferase #138 kept the same liquid consistency as the control sample (see Figure 61).
These modified egg yolks were tested in a Food Emulsion recipe shown in Table 23.

**Table 23.** Mayonnaise with enzyme modified egg yolk.

| | 0 | 1a | 2a | 3a | 4a |
|---|---|---|---|---|---|
| | % | % | % | % | % |
| Rapsolie | 60 | 60 | 60 | 60 | 60 |
| Egg yolk, Sanofo product no.1123P2 | 2,8 | | | | |
| Enz. Modified egg yolk no. 1 | | 2,8 | | | |
| Enz. Modified egg yolk no. 2 | | | 2,8 | | |
| Enz. Modified egg yolk no. 3 | | | | 2,8 | |
| Control (untreated) egg yolk no. 4 | | | | | 2,8 |
| Water | 39 | 36,2 | 36,2 | 36,2 | 36,2 |
| Vinegar, 10% acetic acid | 1 | 1 | 1 | 1 | 1 |

Modified egg yolks 1 and 2 were treated with the lipid acyl transferase; and modified egg yolk 3 was treated with the commercially available phospholipase.
The food emulsion was produced as an oil in water emulsion according to the following procedure: Egg yolk and water was scaled in a beaker. The oil was scaled separately.
A Turrax mixer(20000 rpm) was immersed in the water phase. Oil was pumped to the water phase at a constant speed over 2 minutes. The mixing continued for further 1 minute. The vinegar was then added and mixed for 5 seconds.
The stability of the emulsion was tested in a heating cabinet at 100 °C. After 2 hours at 100 °C the emulsion was evaluated (see Figure 62).
The emulsion stability of untreated egg yolk was quite good in this experiment. Treatment of egg yolk with the lipid acyltransferase #138 however improved the stability because the amount of water separation was reduced. Egg yolk treated with phospholipase #2938 gave a very unstable emulsion with almost complete separation of the oil-and the water phase at 100 °C.
It is considered that in some applications the use of the compositions and methods of the invention can provide enhanced thermal stability of emulsions, such as oil in water salad dressings and the like. This is particularly inportant in food emulsions which are pasturised to ensure long shelf life and/or are heated prior to serving, e.g. in pre-prepared meals for re-heating prior to serving (e.g. microwave meals). Although not wishing to be bound by any particular theory, it is considered that in some applications the accumulation of free fatty acid may be determental to the thermal stability of such emulsions. It should be recognised that the enhanced thermal stability of the food emulsions produced using the methods of the invention, may not be found, or even desirable, in all food applications. It will be apparent to the person skilled in the art in which applications such characteristics are desirable, and the stability of the emuslions can be easily determiend using a simple heat tests, equivalent to, for example pasturisation and or microwave reheating. The inventors have discovered that in a preferable embodiment the food emulsions obtained using the enzymes of the invention have enhanced thermal stability.

**EXAMPLE 14: Transferase reaction in plant sterol enriched egg yolk:**

Transferase form *Aeromonas salmonicida* was able to catalyse to formation of lysolecithin, monoglyceride and plant sterol esters in egg yolk enriched with plant sterol and glycerol. The same enzyme was also tested in a low water system containing palm oil, lecithin, plant sterol and glycerol By TLC and GLC analyses it was shown that monoglyceride, and plant sterol esters were produced under these reaction conditions.

Introduction:

The transferase from *Aeromonas salmonicida* was tested for transferase activity in almost water free system of lecithin, fat, plant sterol and glycerol.

Materials:

Egg yolk: Pasteurised liquid egg yolk from Danæg Products A/S, DK- 4000 Roskilde
GCAT transferase purifiation 178-9, 32 PLU-7/ml(Journal 2254-100)
Soya lecithin. Yolkin from Aarhus United, Denmark.
Palm oil 43, from Aarhus United, Denmark.
L-α Phosphatidylcholine 95% Plant (Avanti #441601)
Sitosterol, Sigma no S5753
Plant Sterol: Generol N122 from Cognis, Germany
Glycerol Item no.085915

Results

Initial screening of transferase activity on plant sterol and glycerol was conducted in egg yolk as shown in Table 24.

**Table 24**

| | | 1 | 2 | 3 | 4 |
|---|---|---|---|---|---|
| Egg yolk | Gram | 1 | 1 | 1 | 1 |
| Glycerol | Gram | 0.1 | 0.1 | | |
| Sitosterol:olie 3:7 | Gram | | | 0.13 | 0.13 |
| Transferase # 178-9 | Units | 1 | | 1 | |
| Water | | | * | | * |
| *Water corresponding to the amount of water in the enzyme solution= 83 μl | | | | | |

The ingredients were mixed and heated to 37°C and kept at this temperature during agitation with a magnetic stirrer.
0.1 gram samples were taken out after 3 and 23 hours and analysed by TLC.
The results from the TLC analysis is shown in Figure 63.
The result in Figure 63 indicated that both cholesterol and plant sterols were esterified by the transferase reaction, concomitant with the formation of lysolecithin (sample 3 and 4), because almost all free sterol and cholesterol was converted to the corresponding ester in sample 3.
The results also indicated that the sample with only glycerol and egg yolk produced monoglyceride. The amount of monoglyceride needs to be confirmed by GLC analysis. When sterol was added together with glycerol (sample 3) the amount of monoglyceride was very low and not detectable by TLC. This indicated that as long as there were surplus of sterol or cholesterol the transferase reaction using glycerol was modest.

In another experiment the transferase enzyme 178-9 was added to a mixture soybean lecithin, glycerol and plant sterol, in order to study the catalytic activity of the enzyme in this reaction mixture.

The composition of the reaction mixtures in these experiments are shown in Table 25

**Table 25**

| | | 1 | 2 | 3 | 4 | 5 | 6 |
|---|---|---|---|---|---|---|---|
| Soya lecithin | gram | 1.875 | 2.25 | 1.875 | 2.5 | 3:5 | 3.5 |
| Plantesterol; Generol N 122 | gram | 0.225 | 0.225 | 0 | 0 | 0.225 | 0.5 |
| Palm oil 43 | gram | 2.675 | 225 | 2.8 | 2.125 | 1.062 | 0.831 |
| Glycerol | gram | 0.225 | 0.275 | 0.325 | 0.375 | 0.248 | 0.238 |
| | | | | | | | |
| Transferase #178 -9, 32 PLU/ml | ml | 0.2 | 0.2 | 0.2 | 0.2 | 0.2 | 0.2 |

The experiment was conducted by mixing the lipid components during agitation at 46 °C. The enzyme was added and samples were taken out after 4 and 24 hours.

The samples were analysed by TLC as shown in Figure 64.

Sample from experiment 2, 4 and 5 after 24 hours reaction time were also analysed by GLC with results shown in Table 26

**Table 26.**

| | | 2 | 4 | 5 |
|---|---|---|---|---|
| Glycerol | % | 3.16 | 5.71 | 4.17 |
| Fatty acids | % | 4.23 | 5.36 | 6.67 |
| Mono | % | 2.24 | 3.87 | 3.92 |
| Sterol | % | 2.13 | | 2.62 |
| Sterolester | % | 2.89 | | 2.14 |

The results confirmed that transferase 178-9 was able to catalyse to formation plant sterol esters and monoglyceride from a reaction mixture containing soybean lecithin, glycerol and plant sterol. Such reaction mixture could be of interest for use in margarine production where monoglyceride is wanted for their emulsification properties and plant sterol esters for their cholesterol lowering effect.

Conclusion

CGAT transferase from Aeromonas salmonicida was able to catalyse the formation of plant sterol esters and monoglyceride in egg yolk where plant sterol and glycerol was added. The same enzyme also catalysed the formation of plant sterol esters and monoglyceride in a mixture of palm oil, lecithin, plant sterol and glycerol. This enzyme therefore is of interest for use in margarine and other oil containing food products where monoglyceride and lysolecithin are needed for improved emulsification and the plant sterol ester for their cholesterol lowering effects.

**EXAMPLE 15 : Immobilisation of a lipid acyltransferase from *Aeromonas sabnoiticida* and the use in the synthesis of Sterol esters.**

A lipid acyltransferase (in this instance a GCAT) from *A. salmonicida* was immobilised on Celite by acetone precipitation. 10 ml enzyme solution in 20 mM TEA buffer pH 7 was agitated slowly with 0,1 gram Celite 535 535 (from Fluka) for 2 hours at room temperature.
50ml cool acetone was added during continued agitation.
The precipitate was isolated by centrifugation 5000 g for 1 minute.
The precipitate was washed 2 times with 20 ml cold acetone.
The Celite was tried at ambient temperature for about 1 hour

The immobilised transferase was tested in a oil mixture containing 13 % Phosphatidylcholin and 7 % plant sterol.(Table 27)

**Table 27**

| | % |
|---|---|
| Avanti lecithin | 12.0 |
| Plant sterol, Generol 122N | 6.6 |
| Palm 43 | 71.4 |
| Glycerol | 5.0 |
| Immobilised Transferase #178, 45 U/g | 2.0 |
| Water | 3.0 |

Lecithin, plant sterol and soybean oil was heated to 46 °C and the plant sterol was dissolved. The immobilised transferase was added.
The transferase reaction continued at 46 °C during gentle agitation with a magnetic stirrer. Samples were taken out for analyses after ½, 1 3 6 and 24 hours and analysed by TLC. The reaction was stopped after 24 hours reaction time and the immobilised enzyme was filtered off.

The samples were analysed by TLC as shown in Figure 65.

The TLC analysis clearly shows the effect of immobilised transferase from A. *salmonicida* in the transformation of cholesterol into cholesterol ester. It is also observed that small amount of monoglyceride is formed. The enzyme has also been shown to have a high activity in environments with high water content (6- 89 % )water environments, the use of the transferase, and other transferases for use in the invention can therefore also be used in immobilised enzyme applications with a significant water content. This allows the replacement of the solvents used by the current immobilised lipases in the bioconvertion of lipids using transferases.

**EXAMPLE 16 The Aeromonas hydrophilia transferase can transfer from a phospholipid to a sterol to form a sterol ester, and/or a sugar molecule to form a sugar ester.**

A lipid acyltransferase from Aeromonas hydrophila expressed in E. coli (Hydro 0303 HVP ), labelled #139 was purified on a Chelating Sepharose FF, HR 2.5/10colunm and analysed for Phospholipase activity. The transferase activity was evaluated in egg yolk for enzyme activity and functionality in egg yolk. The enzyme was also tested in egg yolk containing glucose.

Phospholipase activity.

Transferase #139 isolated from a Chelating Sepharose FF, HR 2.5/10column was assayed by NEFA-PLU(pH7) The activity was 1,15 Units NEFA-PLU/ml.

Egg yolk

In an initial application test transferase #139 was tested in egg yolk according to the following procedure.
1-gram fresh egg yolk was scaled in a 10 ml flask with screw lid. The enzyme preparation was added and mixed on a Vortex mixer. The sample was placed at 37 °C and agitated with a magnetic stirrer.
The reaction was stopped by adding 7.5 ml Chloroform:Methanol (2:1) and mixed on a Whirley mixer for 30 seconds. The chloroform phase was isolated by centrifugation and 2 µl of the chloroform phase was transferred to a pre-activated silica TLC plate and eluted with running buffer nr. I and another TLC-plate in running buffer IV,

The experimental set up is shown in table 28

**Table 28**

| Test | Reaction time | Egg yolk | Transferase #139 |
|---|---|---|---|
| no. | min. | gram | units |
| 1 | 10 | 1 | |

(continued)

| Test | Reaction time | Egg yolk | Transferase #139 |
|---|---|---|---|
| no. | min. | gram | units |
| 2 | 10 | 1 | 0.75 NEFA-PLU |
| 3 | 60 | 1 | 0.75 NEFA-PLU |
| 4 | 300 | 1 | 0.75 NEFA-PLU |
| 5 | 1200 | 1 | |
| 6 | 1200 | 1 | 0.75 NEFA-PLU |

TLC analysis are shown in Figure 66 and Figure 67. The TLC analysis clearly demonstrates the transferase reaction of transferase #139. The cholesterol is converted to cholesterol ester and the amount of lecithin is reduced. The results however also indicate that lysolecithin are only accumulated in very small amount because transferase #139 also is active on lysolecithin. This observation is supported by the formation of free fatty acids(FFA).

Egg yolk and glucose

It was earlier shown that a transferase from *Aeromonas salmonicida* (#138) was able to use glucose as acceptor molecule in a transferase reaction. It has also been tested if transferase #139 can use glucose as acceptor molecule. The experimental set up is seen in Table 29.

**Table 29**

| Test | Reaction time | Egg yolk | Glucose, 70% | Transferase #139 |
|---|---|---|---|---|
| no. | Minutes | gram | mg | units |
| 1 | 10 | 1 | 500 | |
| 2 | 10 | 1 | 500 | 1 NEFA-PLU |
| 3 | 60 | 1 | 500 | 1 NEFA-PLU |
| 4 | 180 | 1 | 500 | 1 NEFA-PLU |
| 5 | 300 | 1 | 500 | 1 NEFA-PLU |
| 6 | 1200 | 1 | 500 | 1 NEFA-PLU |
| 7 | 1200 | 1 | 500 | |

The reaction products were analysed by TLC (Figures 68 and Figure 69).
The TLC analysis indicates formation of glucose ester after 220 min. reaction time (Figure 69 lane 6) but after 1200 min reaction time no glucose ester is seen.
It must therefore be concluded that transferase #139 has both transferase and hydrolytic activity. This is also supported by the fact that the amount of free fatty acids steadily increases as a function of reaction time.

Resume:

Transferase from *Aeromonas hydrophila* was tested in egg yolk. The results confirm that this enzyme catalyses the formation of cholesterol ester concomitant with the formation of lysolecithin. After extended reaction time when most of the cholesterol is consumed free fatty acid are also formed. It can therefore be concluded that the enzyme has primary transferase activity but also Hydrolytic activity was observed when only water was available as donor molecule.
In an experiment with egg yolk and glucose it has been observed that transferase from *Aeromonas hydrophila* is able to catalyse the formation of glucose ester *in situ* in a high water food environment (Figure 70).

**EXAMPLE 17: Variants of a lipid acyltransferase from *Aeromonas hydrophila* (Ahyd2) (SEQ ID No. 36 (see Figure 71))**

Mutations were introduced using the QuikChange® Multi-Site Directed Mutagenesis kit from Stratagene, La Jolla, CA 92037, USA following the instructions provided by Stratagene.

Variants at Tyr256 showed an increased activity towards phospholipids.

Variants at Tyr256 and Tyr260 showed an increased activity towards galactolipids.

Variants at Tyr265 show an increased transferase activity with galactolipids as the acyl donor.

The numbers indicate positions on the following sequence: An enzyme from *Aeromonas hydrophila* the amino acid sequence of which is shown as SEQ ID No: 36 in Figure 71 (the underlined amino acids show a xylanase signal peptide). The nucleotide sequence is as shown as SEQ ID No 54 in FIGURE 72.

**EXAMPLE 18: Use of Acyl-transferase reaction for the production of plant sterol ester and monoglyceride for margarine production.**

An acyltransferase from *Aeromonas salmonicida* expressed in *Bacillus subtilis* was tested in a palm oil mixture containing plant lecithin, plant sterol and glycerol. The acyl-transferase showed the ability to utilise both plant sterol and glycerol as acceptor molecules during production of plant sterol ester and monoglyceride. The reaction mixture was used to produce table margarine of good quality based on the monoglyceride in the reaction mixture and at the same time the margarine was enriched with plant sterol ester, which has been shown to have a cholesterol lowering effect.

The aim of this work was to study to possibility to produce monoglyceride and plant sterol ester by enzymatic reaction of lecithin, plant sterol and glycerol dissolved in vegetable fat

Initial experiments has shown that it was possible to use acyl-transferase from *Aeromonas salmonicida* to produce monoglyceride and plant sterol ester from lecithin, glycerol and plant sterol.

In this experiment such reaction mixture was used to produce table margarine.

Materials:

Lipid acyltransferase from *Aeromonas salmonicida,* # 196 C101, 18.6 PLU/g (Journal 2254-104)
Palm Oil 43, from Aarhus United, DK
L-α Phosphatidylcholine 95% Plant (Avanti #441601)
Plant Sterol: Generol N122 from Cognis, Germany
Glycerol Item no.085915
Distilled Monoglyceride, Dimodan HP from Danisco.

Margarine production.

1. Blend the water phase ingredients. (If required, pasteurise the water phase by heating to approx. 80°C). Adjust pH 5.5.

2. Melt the fat phase, and temper to approx. 40-45°C..

3. Heat the emulsifier with some of the oil in a ratio of
1 part emulsifier to 5 parts oil to a temperature (75-80°), which is 5-10°C higher than the melting point of the emulsifier. When this blend is fully melted and well stirred, add it to the remaining heated oil, stirring continuously.

4. Add the flavouring.

5. Add the water phase to the fat phase, stirring continuously.

6. Cool in a tube chiller (normal capacity, normal cooling) to an outlet temperature of 8-10°C.

Results

Acyltransferase from *A. salmonicida* was tested in an palm oil mixture as shown in

Table 30. Lecithin, plant sterol, glycerol and palm oil was heated to 60°C during agitation in order to solubilize plant sterol and lecithin.

Table 30

| Substrate: | % |
|---|---|
| Avanti lecithin | 12 |
| Plant sterol, Generol 122N | 6.6 |
| Palm oil, melting point 43 | 76.4 |
| Glycerol | 5 |

The substrate was cooled to 48 °C and acyl-transferase #196 was added in the amount shown in Table 31. The reaction mixture was kept at 48 °C for 24 hours during slow agitation.

Table 31

| | gram |
|---|---|
| Substrate | 220 |
| Transferase # 196 C101, 18.6 PLU/g | 15 |

Samples from the reaction mixture were taken out after 1, 4 and 24 hours reaction time, and analysed by TLC in solvent I (Figure 73). The TLC results clearly show the formation of plant sterol ester and monoglyceride. In Figure 73, the first lane is after 1 hour reaction time, Lane 2 is 4 hours reaction time, Lane 3 is 24 hours reaction time and Lane 4 is a plant sterol.

The reaction was stopped after 24 hours reaction time and residues of undissolved plant sterol was removed, and the clear solution was used to produce margarine.

Margarine.

The reaction mixture containing monoglyceride and plant sterol ester was used to produce table margarine according to the recipe shown in Table 32.

Table 32

| Jour. No 3734 | 1 | 2 |
|---|---|---|
| **Water phase** | | |
| Water phase | 16 | 16 |
| Salt | 0.5 | 0.5 |
| Skim milk powder | 1 | 1 |
| Potassium sorbate | 0.1 1 | 0.1 |
| EDTA | 0.015 | 0.015 |
| PH | 5.5 | 5.5 |
| Water phase total | 16.6 | 16.6 |
| **Fat phase** | | |
| Palm 43 | 25 | 25 |
| Rapeseed Oil | 75 | 75 |
| Fat phase total | 83.2 | 78.4 |
| Dimodan HP | 0.2 | |
| Reaction mixture | | 5 |

The margarine produced from the reaction mixture was evaluated of good quality with good spreadability, and good mouth feel and without any off flavour. The margarine was compared to be on quality level with the reference margarine produced by using distilled monoglyceride Dimodan HP.

The only difference observed was that the margarine jour. 3734 no 2 with the reaction mixture was slightly more firm, which was explained by the fact that this recipe contained more Palm 43 than the reference margarine.

**EXAMPLE 19: Use of a lipid acyltransferase during bread production.**

One of the limitations of using lipases in bread making is that free fatty acid is formed during the lipase reaction. It is well known that formation of too much free fatty acid will have a negative impact on the baking performance of flour, because the gluten gets too stiff and a bucky (i.e. less elastic) dough is formed which can not expand during fermentation and baking.

Formation of free fatty acid should also be avoided from the point of oxidative stability, because free fatty acids are more prone to lipid oxidation than the corresponding triglyceride.

In the present invention the problems with free fatty acid formation when adding a lipolytic enzyme to a dough has been overcome by using a lipid acyltransferase which, instead of producing free fatty acids, transfers one or more fatty acids from the lipid acyl donor to a non water acceptor molecule present in the dough, such as a carbohydrate, a protein or peptide, or if used in bread with milk fat, a sterol, alternatively or in combinantion other acceptors listed above mat be added to a dough, for example phytosterols or phytostanols. Preferably, the acceptor molecule in a dough may be one or more of glucose, sucrose or maltose and/or other carbohydrates normally available in a dough.

In the following experiments acyl transferase is tested in mini scale baking experiments. The formation of reaction products, and the lipid components in fully proved dough is extracted by water saturated butanol and analysed by HPLC and GLC analysis.

Materials and methods

Enzymes:

Acyl Transferase, 550 PLU-7/ml
Lipopan™ F BG, a commercial lipase from Novozymes. 12000 LIPU/g or Grindamyl Expel 16.12000 LIPU/g
Lecithin powder, 95% phospholipid (available from Danisco A/S Denmark) Digalactosyldiglyceride from whole wheat flour (from Sigma D4651)
Flour : Solvmel nr. 2001084 (Danish wheat flour, obtained from Havnemclleme, Odense, Denmark)

Mini baking test.

Flow, 50 gram, Dry yeast 10 gram, glucose 0.8. gram, salt 0.8 gram, 70 ppm ascorbic acid and, water 400 Brabender units was kneaded in a 50 g Brabender mixing bowl for 5 min at 30 °C.
Resting time was 10 min. at 34 °C . The dough was scaled 15 gram per dough. Then moulded on a special device where the dough is rolled between a wooden plate and a plexiglas frame. The doughs were proofed in tins for 45 min. at 34 °C, and baked in a Voss household oven 8 min. 225 °C.
After baking the breads are cooled to ambient temperature and after 20 min. the breads are scaled and the volume is determined by rape seed displacement method. The breads are also cut and crumb and crust evaluated.

Results and conclusion:

Preliminary results indicate that the lipid acyltransferase clearly demonstrates a positive effect on both bread volume and bread appearance. In particular, preliminary results indicate that the use of the lipid acyltransferase results in increased specific bread volume as compared with that obtained with the control (no enzyme) and that obtained with the use of a commercially available lipolytic enzyme, namely Grindamyl Exel 16 or LipopanF™.

**EXAMPLE 20: Standard ice cream witch dairy fat**

The function of emulsifiers used in ice cream is to bring about controlled fat crystallisation and mild destabilization due to protein desorbtion during ageing of the ice cream. This change improves the ice cream quality. Mono-diglycerides are normally used for the production of ice cream, but is also known to use polar emulsifiers like polysorbate and sugar esters in ice cream production in combination with mono-diglyceride to facilitate controlled fat destabilization and produce ice cream with very good creamy and smooth eating texture.

Emulsifiers used for ice cream are normally added the ice cream mix as a powder. Recently it has however been shown that mono-diglyceride can bee produced by enzymatic reaction of the fat in the ice cream recipe using lipases. The problem by using lipases is however that lipases also catalyse the formation of free fatty acids, when water is available in the reaction mixture.

It has however surprisingly been shown that lipid acyl-transferase overcomes the limitation by lipase because acyl-transferase is able to transfer fatty acid from lecithin and other lipids to acceptor molecules like sterol, cholesterol, glucose, glycerol and proteins/peptides without formation of significant amount of free fatty acids.

One of the main ingredients in ice cream is dairy cream containing 38 % milk fat. Dairy cream also contains smaller amount of lecithin, which is a donor molecule for acyl-transferase. ("Complex milk lipids account for about 1 % of the total milk fat and are mainly composed of phospholipids". Ref. Ullmann's Encyclopedia of Industrial ChemistryCopyright © 2003 by Wiley-VCH Verlag GmbH & Co. KGaA.). Dairy cream also contains small amount of cholesterol, which is an acceptor molecule for acyl-transferase.

From the constituents of ice cream it is thus possible to produce both monoglyceride and polar emulsifiers like lyso-lecithin and sugar ester, which are known for the beneficial effects in ice cream production.

A further beneficial effect form the reaction of acyl-transferase in dairy cream is the formation of cholesterol ester, which might slow down the absorption of cholesterol in the intestine.

Ice cream Recipe

| | With emulsifier | With enzyme |
|---|---|---|
| Dairy cream, 38% | 23,65 | 23,65 |
| Skimmed milk | 53,30 | 53,30 |
| Skimmed milk powder | 4,90 | 11,30 |
| Sugar | 12,00 | 12,00 |
| Glucose sirup, DE 42, 75% TS | 4,25 | 4,25 |
| Glycerol | 1,0 | 1,0 |
| Stabilizer blend | 0,2 | 0,2 |
| Cremodan SE 30 | 0,6 | |
| Lipid acyl transferase, 500 PLU/g | | 0,1 |
| Grindsted Flavouring 2976 | 0,1 | 0,1 |
| Colour | + | + |

Ice Cream production proces.

**1.** Heat dairy cream, glucose syrup and glycerol to approx. 40 °C Add the lipid acyl transferase and let the mixture react for 30 minutes. A sample is taken out for analysis **2.** Heat all the other liquid ingredients to approx. 40 ° **3.** Add the other dry ingredients. (stabiliser blend is mixed with sugar before addition) **4.** When the dry ingredients are dissolved add the dairy cream-glucose mixture.

**5.** Pasteurise at 80 -85 °C/20-40 seconds **6.** Homogenise at 80 °C (190 bar for recipe 1 and 175 bar for recipe 2) **7.** Cool to ageing temperature, 4 °C **8.** Freeze in continuous freezer to desired overrun (100% recommended) **9.** Harden in tunnel at -40 °C **10.** Store below -25 °C

results:

Uses of Acyl-transferase in the production of ice cream contribute to the production of ice cream with very good taste and excellent creamy mouth feel comparable the ice cream produced by using a commercial emulsifier Cremodan SE 30. The melt down of the ice cream produced by the lipid acyl transferase is also improved.

**Example 21: Acyl transferase in Cheese.**

Cheese is the fresh or matured solid or semisolid product obtained by coagulating milk, skimmed milk, partly skimmed milk, cream, whey cream, or buttermilk, or any combination of these materials, through the action of rennet or other suitable coagulating agents, and partially draining the whey that results from such coagulation.

The cheese yield depends primarily on the fat and protein contents of the milk. The salt (particularly calcium salts) and protein concentrations, as well as the acidity, are very important for coagulation. (ref. Ullmann's Encyclopedia of Industrial Chemistry Copyright © 2003 by Wiley-VCH Verlag GmbH & Co).

Such effort has been made in order to optimise and increase the cheese yield by optimisation of the cheese making procedure (USP 4,959,229) or by using improved clotting method (USP 4,581,240), which increase the amount of whey protein in the curd.

In the present invention the amount of whey protein in the curd is increased by enzymatic modification of the whey protein by treatment of the milk during cheese making with a lipid acyl transferase.

When a fatty acid is covalently linked to a non-membrane protein like β-lactoglobulin, the physical and functional properties will change drastically.

For cheese production of the present invention acyl transferase is added to the milk before or at the same time as rennet is added to the milk.

During casein precipitation acyl transferase is able to use lecithin and other lipids in the milk as donor and peptides or protein as acceptor molecule during formation of acylated protein or acylated peptides.

The change in hydrophobic properties of milk protein contributes to increased protein precipitation in the curd during cheese production.

Since the increase in cheese yield obtained by the present invention originates from increased retention in the cheese coagulum of proteins that are normally lost in the whey, a suitable method, directly related to the mechanism of the invention, is based on determination of the amount of protein that ends up in the whey. Less protein in the whey necessarily means more protein in the curd, and higher cheese yield.

The test for the amount of protein in the whey can be performed in the following way. Skim or whole milk is warmed to a temperature suitable for rennet coagulation, typically 30-35oC in a 100 ml beaker. Optionally 1% of a bulk lactic acid bacteria starter is added, and standard rennet is added in an amount corresponding to e.g. 0.03-0.05%. When the milk has turned into a coagulum solid enough to allow it to be cut into cubes with a side length of about 0.5 em, such cutting is performed with a sharp knife. Syneresis is thereby initiated, and after 30 min holding period, that allows the curd to settle, a whey sample is withdrawn, and centrifuged in a laboratory centrifuge for 10 min. This sample is analyzed for protein content, using e.g. the Kjeldahl method. Alternatively, and/or as a supplement, the sample may be analyzed with methods that allow the type and quantity of the individual protein components to be established.

## EXAMPLE 22 "Assay in Low Water Environment"

Transferase reactions of lipolytic enzymes in low water environment .

Procedure

Materials.

Cholesterol Sigma cat C 8503
L-alpha-Phosphatidylcholine 95% (Plant) Avanti #441601
Soybean oil, Aarhus United, DK.
Chloroform, Analytical grade

Enzymes.

#179, GCAT from *A. salmonicida*
#2427, Phospholipase A1 from *Fusarium oxysporum.* LIPOPAN® F from Novozymes, Denmark
#1991, Phospholipase A2 from Pancreas, LIPOMOD 22L from Biocatalysts, UK #2373, *Candida Antarctica* lipase, Novozyme 525 L from Novozymes Denmark..

Enzyme assay

13.1 % Lecithin and 6.6% cholesterol was dissolved in soybean oil by heating to 60 °C during agitation
The substrate was scaled in a 20ml Wheaton glass and heated to 46 °C
Water and enzyme solution was added and a stopwatch is started.
At regular intervals 50 mg samples ware transferred to a 10ml Dram glass and frozen.
The isolated lipids were analysed by GLC

GLC analysis

GLC analysis was carried out as described in Example 11

**Results**

The experiment was set up as shown in Table 33
The substrate based on soybean oil containing 13.1 % lecithin and 6.6% cholesterol was heated to 46°C. The enzyme solution was added and a stopwatch started.
After 30, 60 and 120 minutes reaction time samples were taken out for GLC analysis.

Table 33

| | | 1 | 2 | 3 | 4 | 5 |
|---|---|---|---|---|---|---|
| Substrate | gram | 5 | 5 | 5 | 5 | 5 |
| Transferase #179-C72, 56 PLU-7/ml | ml | | 0.3 | | | |
| #2427, 200 PLU-7/ml | ml | | | 0.3 | | |
| Pancreas PLA 2 #1991 6300 PLU/ml | ml | | | | 0.3 | |
| Novozyme 525 L, #2373, 200 LIPU/rnlml | | | | | | 0.3 |
| Water | ml | 0.3 | | | | |
| % water | | 6 | 6 | 6 | 6 | 6 |

The results from the GLC analysis is shown in Table 34 . The results are expressed in percent based total sample composition. Based on the GLC results it was possible to calculate the amount of fatty acid and cholesterol ester produced by enzymatic reaction relative to the control sample without enzyme added. Under these experimental conditions the total enzymatic activity was estimated as the hydrolytic activity measured as free fatty acid formation and the transferase activity estimated as cholesterol ester formation. From these results and the information about molecular weight of fatty acid and cholesterol ester it was possible to calculate to relative molar hydrolytic activity and the relative molar transferase activity as shown in Table 35.

Table 34

| Enzyme | Reaction time. minutes. | Fatty acid % | cholesterol % | Cholesterol ester % |
|---|---|---|---|---|
| Control | 120 | 0.533 | 7.094 | 0.000 |
| #179 | 30 | 0.770 | 5.761 | 2.229 |
| #179 | 60 | 0.852 | 5.369 | 2.883 |
| #179 | 120 | 0.876 | 4.900 | 3.667 |
| #2427 | 30 | 3.269 | 7.094 | 0.000 |
| #2427 | 60 | 3.420 | 7.094 | 0.000 |
| #2427 | 120 | 3.710 | 7.094 | 0.000 |
| #1991 | 30 | 2.871 | 7.094 | 0.000 |
| #1991 | 60 | 3.578 | 7.094 | 0.000 |
| #1991 | 120 | 3.928 | 7.094 | 0.000 |
| #2373 | 30 | 1.418 | 7.094 | 0.000 |
| #2373 | 60 | 1.421 | 7.094 | 0.000 |
| #2373 | 120 | 1.915 | 7.094 | 0.000 |

Table 35

| Enzyme | Reaction time minutes | Fatty acid produced | Cholesterol Used | Cholesterol ester produced | Hydrolytic activity % | Transferase activity % |
|---|---|---|---|---|---|---|
| #179 | 30 | 0.238 | 1.334 | 2.229 | 20 | 80 |
| #179 | 60 | 0.319 | 1.725 | 2.883 | 21 | 79 |
| #179 | 120 | 0.343 | 2.195 | 3.667 | 18 | 82 |

(continued)

| Enzyme | Reaction time minutes | Fatty acid produced | Cholesterol Used | Cholesterol ester produced | Hydrolytic activity % | Transferase activity % |
|---|---|---|---|---|---|---|
| #2427 | 30 | 2.737 | 0.000 | 0.000 | 100 | 0 |
| #2427 | 60 | 2.887 | 0.000 | 0.000 | 100 | 0 |
| #2427 | 120 | 3.177 | 0.000 | 0.000 | 100 | 0 |
| #1991 | 30 | 2.338 | 0.000 | 0.000 | 100 | 0 |
| #1991 | 60 | 3.046 | 0.000 | 0.000 | 100 | 0 |
| #1991 | 120 | 3.395 | 0.000 | 0.000 | 100 | 0 |
| #2373 | 30 | 0.885 | 0.000 | 0.000 | 100 | 0 |
| #2373 | 60 | 0.888 | 0.000 | 0.000 | 100 | 0 |
| #2373 | 120 | 1.383 | 0.000 | 0.000 | 100 | 0 |

Conclusion

In these experiments it was observed that all the tested enzymes showed hydrolytic activity because the amount of fatty acid increased. However the only enzyme which showed transferase activity was GCAT from *A. salmonicida.* It is therefore concluded that in an oily system with lecithin and cholesterol containing 6% water phospholipase A1 from *Fusarium oxysporum ,* phospholipase A2 from *pancreas* and a lipase from *Candida antarctica* only showed hydrolytic activity.

Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

## Claims

**1.** Use of a lipid acyltransferase to prepare from a water containing food material comprising 10-98% water a foodstuff selteted from egg or an egg-based product or a dairy product comprising an emulsifier, wherein the emulsifier is generated from constituents of the food material by the lipid acyltransferase, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphaddylcholine mixture to a Wheaton glass, adding 15ml some HEPES buffer pH 7 and dispersing the lipid in the buffer during agitation;
iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25μl 4M HCl to acidify the free fatty acid;
v) adding 3ml chloroform and shaking vigorously for 30 seconds, centrifuging and isolating 2ml of the chloroform phase, filtering through a 0.45μm filter into a 10ml tared Dram glass;
vii) evaporating the chloroform under a stream of nitrogen at 60°C, and scaling the samples;
viii) analysing the extracted lipid by GLC.

**2.** Use according to claim 1 wherein the egg-based product is mayonnaise, salad dressing, sauce, ice-cream, egg powder, modified egg yolk and products made therefrom.

**3.** Use according to claim 1 wherein the dairy product is butter, milk, cream, cheese, cream cheese, ice cream, frozen desserts, yoghurt, yoghurt drinks, butter fat or anhydrous milk fat.

**4.** Use according to any one of claims 1 to 3 wherein at least two emulsifiers are produced.

**5.** Use according to any one of the preceding claims wherein the lipid acyltransferase is one which is capable of transferring an acyl group from a lipid to one or more of the following acyl acceptors: a sterol, a stanol, a carbohydrate, a protein or a subunit thereof, glycerol

**6.** Use according to any one of the preceding claims wherein at least one of the emulsifiers is a sterol ester and/or a stanol ester.

**7.** Use according to claim 6 wherein the sterol ester is one or more of alpha-sitosterol ester, beta-sitosterol ester, stigmasterol ester, ergosterol ester, campesterol ester or cholesterol ester.

**8.** Use according to any one of the preceding claims wherein both a cholesterol ester and at least one sterol or stanol ester in combination is produced *in situ.*

**9.** Use according to claim 7 or 8 wherein the amount of free cholesterol in the foodstuff is reduced.

**10.** Use according to claim 6 wherein the stanol ester is one or more beta-sitostanol or ss-sitostanol.

**11.** Use according to any one of the preceding claims wherein the lipid acyltransferase is one which when tested using the Transferase Assay in High Water Egg Yolk in an egg yolk with 54% water, has up to 100% relative transferase activity; said transferase assay comprising the steps of:

i) scaling 5 gram liquid egg yolk substrate in a 20ml Wheaton glass and heating to 35°C;
ii) adding water and enzyme solution;
iii) at regular intervals transferring 0.5g samples to a 10ml Dram glass;
iv) adding 20μl 4M HCl to stop the enzyme reaction and acidify the fatty acid soap;
v) adding 3ml Chloroform, mixing the sample for 30 seconds and centrifuging at 3000g for 10 min;
vi) transferring 0.8ml of the chloroform phase to a tarred Dram glass, evaporating the Chloroform at 60°C under a steam of nitrogen; scaling the dram glass;
vii) analysing isolated lipids by GLC.

**12.** Use according to claim 11 wherein the lipid acyltransferase is one which has an initial percentage acyltransferase activity measured after 10% consumption of the donor molecule of at least 1% relative transferase activity.

**13.** Use according to claim 12 wherein the initial percentage acyltransferase activity measured after 10% consumption of the donor molecule is at least 5%.

**14.** Use according to claim 1 wherein the acyl acceptor substrate is a carbohydrate, a protein, a sterol, a stanol or glycerol.

**15.** Use according to claim 1 wherein the acyl acceptor substrate is a sterol.

**16.** Use according to any one of the preceding claims wherein the lipid acyltransferase is one which when tested using the Transferase Assay in a Low Water Environment has a relative transferase activity of at least 1%; said transferase assay comprising the steps of:

i) dissolving 13.1 % Lecithin and 6.6% cholesterol in soybean oil by heating to 60°C during agitation;
ii) scaling the substrate in a 20ml Wheaton glass and heating to 46°C;
iii) adding water and enzyme solution;
iv) at regular intervals transferring 50mg samples to a 10ml Dram glass and freezing;
v) analysing the isolated lipids by GLC.

**17.** Use according to claim 16 wherein the acyl acceptor substrate in the assay is a carbohydrate, a protein, a sterol, a stanol or glycerol.

**18.** Use according to claim 17 wherein the acyl acceptor substrate in the assay is a sterol.

**19.** Use according to any one of the preceding claims wherein the lipid acyltransferase is **characterised** as an enzyme which possesses acyl transferase activity and which comprises the amino acid sequence motif GDSX, wherein X is one or more of the following amino acid residues L, A, V, I, F, Y, H, Q, T, N, M or S.

**20.** Use according to any one of the preceding claims wherein the lipid acyltransferase enzyme comprises H-309 or comprises a histidine residue at a position corresponding to His-309 in the amino acid sequence of the *Aeromonas hydrophila* lipolytic enzyme shown as SEQ ID No. 2 or SEQ ID No. 32.

**21.** Use according to any one of the preceding claims wherein the lipid acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

**22.** Use according to any one of the preceding claims wherein the lipid acyltransferase comprises one or more of the following amino acid sequences:

(i) the amino acid sequence shown as SEQ ID No. 2; (ii) the amino acid sequence shown as SEQ ID No. 3; (iii) the amino acid sequence shown as SEQ ID No. 4; (iv) the amino acid sequence shown as SED ID No. 5; (v) the amino acid sequence shown as SEQ ID No. 6; (vi) the amino acid sequence shown as SEQ ID No. 12, (vii) the amino acid sequence shown as SEQ ID No. 20, (viii) the amino acid sequence shown as SEQ ID No. 22, (ix) the amino acid sequence shown as SEQ ID No. 24, (x) the amino acid sequence shown as SEQ ID No. 26, (xi) the amino acid sequence shown as SEQ ID No. 28, (xii) the amino acid sequence shown as SEQ ID No. 30, (xiii) the amino acid sequence shown as SEQ ID No. 32, (xiv) the amino acid sequence shown as SEQ ID No. 34, or an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 or SEQ ID No. 34.

**23.** Use according to any one of the preceding claims, wherein the emulsifier is one or more of the following: a monoglyceride, a lysophosphatidylcholine, digalactosyl monoglyceride.

**24.** Use according to any one of the preceding claims, wherein the lipid acyltransferase is used in combination with a lipase having one or more of the following lipase activities: glycolipase activity (E.C.3.1.1.26); triacylglycerol lipase activity (E.C.3.1.1.3); phospholipase A2 activity (E.C.3.1.1.4) or phospholipase A1 activity (E.C.3.1.1.32).

**25.** A method of production of egg or an egg-based product or a dairy product comprising an emulsifier, wherein the method comprises the step of adding a lipid acyltransferase to egg or an egg-based product or a dairy product contaning 10 - 98% water, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphatidylcholine mixture to a Wheaton glass, adding 15ml 50mM HEPES buffer pH 7 and dispersing the lipid in the buffer during agitation;
iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25μl 4M HCl to acidify the free fatty acid;
v) adding 3ml chloroform and shaking vigorously for 30 seconds, centrifuging and isolating 2ml of the chloroform phase, filtering through a 0.45μm filter into a 10ml tared Dram glass;
vii) evaporating the chloroform under a stream of nitrogen at 60°C, and scaling the samples;
viii) analysing the extracted lipid by GLC.

**26.** A method according to claim 25 wherein the egg or egg based product is mayonnaise, salad dressing, sauce, ice-cream, egg powder, modified egg yolk and products made therefrom.

**27.** A method according to claim 25 wherein the dairy product is butter, milk, cream, cheese, cream cheese, ice cream, frozen desserts, yoghurt, yoghurt drinks, butter fat or anhydrous milk fat.

**28.** A method according to any one of claims 25-27 wherein at least 2 emulsifiers are produced.

**29.** A method according to any one claims 25-28 wherein the lipid acyltransferase is one which is capable of transferring an acyl group from a lipid to one or more of the following acyl acceptors: a sterol, a stanol, a carbohydrate, a protein or a subunit thereof, glycerol

**30.** A method according to any one of claims 25-28 wherein at least one of the emulsifiers is a sterol ester and/or a stanol ester.

**31.** A method according to claim 29 wherein the sterol ester is one or more of alpha-sitosterol ester, beta-sitosterol ester, stigmasterol ester, ergosterol ester, campesterol ester or cholesterol ester.

**32.** A method according to any one of claims 25-31 wherein both a cholesterol ester and at least one sterol or stanol ester in combination is produced *in situ.*

**33.** A method according to claim 30 or 31 wherein the amount of free cholesterol in the foodstuff is reduced.

**34.** A method according to claim 30 wherein the stanol ester is one or more beta-sitostanol or ss-sitostanol.

**35.** A method according to any one of claims 25-34 wherein the lipid acyltransferase is one which when tested using the Transferase Assay in High Water Egg Yolk in an egg yolk with 54% water, has up to 100% relative transferase activity; said transferase assay comprising the steps of:

i) scaling 5 gram liquid egg yolk substrate in a 20ml Wheaton glass and heating to 35°C;
ii) adding water and enzyme solution;
iii) at regular intervals transferring 0.5g samples to a 10ml Dram glass;
iv) adding 20μl 4M HCl to stop the enzyme reaction and acidify the fatty acid soap;
v) adding 3ml Chloroform, mixing the sample for 30 seconds and centrifuging at 3000g for 10 min;
vi) transferring 0.8ml of the chloroform phase to a tarred Dram glass, evaporating the Chloroform at 60°C under a steam of nitrogen; scaling the dram glass;
vii) analysing isolated lipids by GLC.

**36.** A method according to claim 35 wherein the lipid acyltransferase is one which has an initial percentage acyltransferase activity measured after 10% consumption of the donor molecule of at least 1% relative transferase activity.

**37.** A method according to claim 36 wherein the initial percentage acyltransferase activity is at least 5%.

**38.** A method according to claim 36 wherein the acyl acceptor substrate is a carbohydrate, a protein, a sterol, a stanol or glycerol.

**39.** A method according to claim 38 wherein the acyl acceptor substrate is a sterol.

**40.** A method according to any one of claims 25-39 wherein the lipid acyltransferase is one which when tested using the Transferase Assay in a Low Water Environment has a relative transferase activity of at least 1%; said transferase assay comprising the steps of:

i) dissolving 13.1 % Lecithin and 6.6% cholesterol in soybean oil by heating to 60°C during agitation;
ii) scaling the substrate in a 20ml Wheaton glass and heating to 46°C;
iv) adding water and enzyme solution;
iv) at regular intervals transferring 50mg samples to a 10ml Dram glass and freezing;
v) analysing the isolated lipids by GLC.

**41.** A method according to any one of claims 25-40 wherein the lipid acyltransferase is **characterised** as an enzyme which possesses acyl transferase activity and which comprises the amino acid sequence motif GDSX, wherein X is one or more of the following amino acid residues L, A, V, I, F, Y, H, Q, T, N, M or S.

**42.** A method according to any one of claims 25-41 wherein the lipid acyltransferase enzyme comprises H-309 or comprises a histidine residue at a position corresponding to His-309 in the amino acid sequence of the *Aeromonas hydrophila* lipolytic enzyme shown as SEQ ID No. 2 or SEQ ID No. 32.

**43.** A method according to any one of claims 25-42 wherein the lipid acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas and Candida.*

**44.** A method according to any one of claims 25-43 wherein the lipid acyltransferase comprises one or more of the following amino acid sequences:

(i) the amino acid sequence shown as SEQ ID No. 2; (ii) the amino acid sequence shown as SEQ ID No. 3; (iii) the amino acid sequence shown as SEQ ID No. 4; (iv) the amino acid sequence shown as SED ID No. 5; (v) the amino acid sequence shown as SEQ ID No. 6; (vi) the amino acid sequence shown as SEQ ID No. 12, (vii) the amino acid sequence shown as SEQ ID No. 20, (viii) the amino acid sequence shown as SEQ ID No. 22, (ix) the amino acid sequence shown as SEQ ID No. 24, (x) the amino acid sequence shown as SEQ ID No. 26, (xi) the amino acid sequence shown as SEQ ID No. 28, (xii) the amino acid sequence shown as SEQ ID No. 30, (xiii) the amino acid sequence shown as SEQ ID No. 32, (xiv) the amino acid sequence shown as SEQ ID No. 34, or an amino acid sequence which has 75% or more identity with any one of the sequences shown as SEQ ID No. 2, SEQ ID No. 3, SEQ ID No. 4, SEQ ID Neo. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 or SEQ ID No. 34.

**45.** A method according to any one of claims 25-44, wherein the emulsifier is one or more - of the following: a monoglyceride, a lysophosphatidylcholine, digalactosyl monoglyceride.

**46.** A method according to any one of claims 25-45, wherein the lipid acyltransferase is used in combination with a lipase having one or more of the following lipase activities: glycolipase activity (E.C.3.1.1.26); triacylglycerol lipase activity (E.C.3.1.1.3); phospholipase A2 activity (E.C.3.1.1.4) or phospholipase A1 activity (E.C.3.1.1.32).

**47.** A water containing foodstuff comprising 10-98% water obtainable by the method according to any one of claims 25-46, wherein the foodstuff comprises a lipid acyltransferase as detailed in claim 25 and an emulsifier.

## Patentansprüche

**1.** Verwendung einer Lipidacy1tlransferase zur Verstellung eines Nahrungsmittels aus einem wasserenthaltenden Nahrungsmaterial, welches 10 bis 98 % Wasser umfasst, wobei das Nahrungsmittel ausgewählt ist unter Ei oder einem Produkt auf Eibasis oder einem Milchprodukt, welches einen Emulgator umfasst, wobei der Emulgator aus Bestandteiles des Nahrungsmaterials durch die Lipidacyltransferase erzeugt wird, wobei die Lipidacyltransferase wenigstens 2 % Acyltransferaseaktivität aufweist, wenn sie mithilfe des Transferasetests in gepuffertem Substrat getestet wird, wobei der Transferasetest die folgenden Stufen umfasst:

i) Auflösen von 450 mg Phosphatidylcholin und 50 mg Cholesterol in Chloroform, Verdampfen unter Vakuum zur Trockne,
ii) überführen von 300 mg Chofesterol/Phosphatidylcholin-Gemisch in ein Wheaton-Glas, wobei 15 ml 50mM HEPES-Puffer mit einem pH-Wert von 7 zugegeben werden, und Auflösen des Lipids in dem Puffer unter Belegung,
iii) Erhitzen des Substrats auf 35°C während dem Mischen mit einem Magnetrührer und Zugeben von 0,25 ml Enzymlösung,
iv) Nehmer von 2 ml-Proben bei einer Reaktionszeit von 0, 5, 10, 15, 25, 40 und 60 Minuten und sofortiges Stoppeln der Enzymreaktion durch Zugabe von 25 $\mu$l 4M HCl, um die freie Fettsäure zu azidifizieren,
v) Zugeben von 3 ml Chloroform und kräftiges Schütteln für 30 Sekunden, Zentrifugieren und Isolieren von 2 ml der Chloroform phase, Filtert durch einen 0,45 pm-Filter in ein tariertes 10 ml-Drarn-Glas,
vii) Verdampfen des Chloroforms unter einem Stickstoffstrom bei 60°C und Messern der Proben,
viii) Analysieren des extrahierten Lipids durch GLC.

**2.** Verwendung gemäß Anspruch 1, wobei das Produkt auf Eibasis Mayonnaise, Salatdressing, Soße, Eiskrem, Eipulver, modifiziertes Eigelb und daraus hergestellte Produkte sind.

**3.** Verwendung gemäß Anspruch 1, wobei das Milchprodukt Butter, Milch, Sahne, Fräse; Frischkäse, Eiskrem, gefrorenes Dessert, Joghurt, Trinkjoghurt, Butterfett oder wasserfreies Milchfett ist.

**4.** Verwendung gemäß einem der Ansprüche 1 bis 3, wobei wenigstens zwei Emulgatoren erzeugt werden.

**5.** Verwendung gemäß einem der vorhergehenden Ansprüche, wobei die Lipidacyltransferase eine solche ist, die eine Arylgruppe von einem Lipid auf einen oder mehrere der folgenden Acylakzeptoren übertragen kant: ein Sterol, ein Stanol, ein Kohlenhydrat, ein Protein oder eine Untereinheit davor, Glycerol.

**6.** Verwendung gemäß einem der vorhergehenden Ansprüche, wobei wenigstens einer der Emulgatoren ein Sterolester und/oder ein Stanolester ist.

**7.** Verwendung gemäß Anspruch 6, wobei der Sterolester einer oder mehrere von Alpha-Sitosterolester, Beta-Sitosterolester, Stigmasterolester, Ergosterolester, Campesterolester oder Cholesterolester ist.

**8.** Verwendung gemäß einem der vorhergehenden Ansprüche, wobei sowohl ein Chotesterolester als auch wenigstens ein Sterol- oder Stanolester in Kombination *in situ* erzeugt werden.

**9.** Verwendung gemäß Anspruch 7 oder Anspruch 8, wobei die Menge an freiem Cholesterol in dem Nahrungsmittel reduziert ist.

**10.** Verwendung gemäß Anspruch 6, wobei der Stanolester einer oder mehrere von Beta-Sitostanol oder ss-Sitostanol ist.

**11.** Verwendung gemäß einem der vorhergehenden Ansprüche, wobei die Lipidacyltransferase eine solche ist, die bis zu 100 % relative Transferaseaktivität aufweiset, wenn sie mithilfe des Transferasetests in Eiggelb mit hohem Wassergehalt in einem Eigelb mit einem Wassergehalt von 54 % getestet wird, wobei der Transferasetest die folgenden Stufen umfasst:

i) Einwiegen von 5 Gramm flüssigem Eigelbsubstrat vorn 5 Gramm in einem 20 ml Wheaton-Glas und Erhitzen auf 35°C,
ii) Zugeben von Wasser und einer Enzynilösung,
iii) Überführen von 0,5 g-Proben in ein 10 ml Dram-Glas in regelmäßigen Intervallen,
iv) Zugeben von 20 µl 4M HCl, um die Enzymreaktion zu stoppen und die Fettsäureseife zu azidifizieren,
v) Zugeben von 3 ml Chloroform, Mischen der Probe für 30 Sekunden und Zentrifugieren bei 3.000 g für 10 Minuten,
vi) Überführen von 0,8 ml der Chloroformphase in ein tariertes Dram-Glas, Verdampfen des Chloroforms bei 60 °C unter einem Stickstoffstrom, Messen des Dram-Glases,
vii) Analysieren des isolierten Lipids durch GLC.

**12.** Verwendung gemäß Anspruch 11, wobei die Lipidacyltransferase eine solche ist, die eine anfängliche prozentuale Acyitransferaseaktivität von wenigstens 1 % relativer Transferaseaktivität aufweist, gemessen nachdem 10 % des Donormoleküls verbraucht waren.

**13.** Verwendung gemäß Anspruch 12, wobei die anfängliche prozentuale Acyltransferaseaktivität, gemessen nachdem 10 % des Donormoteküls verbraucht waren, wenigstens 5 % beträgt.

**14.** Verwendung gemäß Anspruch 1, wobei das Acylakzeptorsubstrat ein Kohlenhydrat, ein Protein, ein Sterol, ein Stanol oder Glycerol ist.

**15.** Verwendung gemäß Anspruch 1, wobei das Acylakzeptorsubstrat ein Sterol ist.

**16.** Verwendung gemäß einem der vorhergehenden Ansprüche, wobei die Lpidacyltransferase eine solche ist, die, wenn sie mithilfe des Transferastests in einer Umgehung mit niedrigem Wassergehalt getestet wird, eine relative Transferaseaktivität von wenigstens 1 % aufweist, wobei der Transferasetest die folgenden Stufen umfasst:

i) Auflösen von 13,1 % Lecithin und 6,6 % Cholesterol in Sojabohnenöl durch Erhitzen auf 60 °C unter Belegung.
ii) Einliegen des Substrats in ein 20 ml Wheaton-Glas und Erhitzen auf 46 °C,
iii) Zugeben von Wasser und einer Enzymlösung,
iv) Übertragen von 50 mg-Proben in ein 10 ml-Dram-Glas in regelmäßigen Intervallen und Einfrieren,
v) Analysieren der isolierten Lipide durch GLC.

**17.** Verwendung gemäß Anspruch 16, wobei das Acylakzeptorsubstrat in dem Test ein Kohlenhydrat, ein Protein, ein Sterol, ein Stanol oder Glycerol ist,

**18.** Verwendung gemäß Anspruch 17, wobei das Acylakzeptorsubstrat in dem Test ein Sterol ist.

**19.** Verwendung gemäß einem der vorhergehenden Ansprüche, wobei die Lipidacyltransferase als Enzym charakterisiert ist, welches Acyltransferaseaktivität besitzt und welches das Aminosäuresequenzmotiv GDSX, umfasst, wobei X einer oder mehrere der folgenden Aminosäurereste L, A, V, I, F, Y, H, Q, T, N, M oder S ist.

**20.** Verwendung gemäß einem der vorhergehenden Ansprüche, wobei das Lipidacyitransferase-Enzym H-309 oder einen Histidinrest an einer Postition entsprechend der von His-309 in der Aminosäuresequenz des lipolytischen Enzymes *Aeromonas hydrophil* umfasst, wie als SEQ ID No. 2 oder SEQ ID No. 32 gezeigt ist.

**21.** Verwendung gemäß einem der vorhergehenden Ansprüche, wobei die Lipidacyltransferase erhältlich ist aus einem Organismus einer oder mehrerer der folgenden Gattungen: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**22.** Verwendung gemäß einem der vorhergehenden Ansprüche, wobei die Lipidacyltransferase eine oder mehrere der folgenden Aminosäuresequenzen umfasst: (i) die als SEQ ID No. 2 gezeigte Aminosäuresequenz, (ii) die als SEQ ID No. 3 gezeigte Aminosäuresequenz, (iii) die als SEQ ID No. 4 gezeigte Aminosäuresequenz, (iv) die als SEQ ID No. 5 gezeigte Aminosäuresequenz, (v) die als SEQ ID No. 6 gezeigte Aminosäuresequenz, (vi) die als SEQ ID No. 12 gezeigte Aminosäuresequenz, (vii) die als SEQ ID No. 20 gezeigte Aminosäuresequenz, (viii) die als SEQ ID No. 22 gezeigte Aminosäuresequenz, (ix) die als SEQ ID No. 24 gezeigte Aminosäure-sequenz, (x) die als SEQ ID No. 26 gezeigte Aminosäuresequenz, (xi) die als SEQ ID No. 28 gezeigte Aminosäuresequenz, (xii) die als SEQ ID No. 30 gezeigte Aminosäuresequenz, (xiii) die als SEQ ID No. 32 gezeigte Aminosäuresequenz, (xiv) die als SEQ ID No, 34 gezeigte Aminosäuresequenz, oder eine Aminosäuresequenz, die 75 % oder mehr Identität mit einer der Sequenzen aufweist, die gezeigt sind als SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 oder SEQ ID No. 34.

**23.** Verwendung gemäß einem der vorhergehenden Ansprüche, wobei der Emulgator einer oder mehrere der folgenden ist: ein Monoglycerid, ein Lysophosphatidylcholin, Digalactosylmonoglycerid.

**24.** Verwendung gemäß einem der vorhergehenden Ansprüche, wobei die Lipidacyitransferase in Kombination mit einer Lipase verwendet wird, die eine oder mehrere der folgenden Lipaseaktivitäten aufweist: Glycolipaseaktivität (E.C. 3.1.1.26), Triacylglycerol-Lipaseaktivität (E.C.3.1.1.3), Phospholipaso-A2-Aktivität (E.C.3.1.1.4) oder Phospholipase-A1-Aktivität (E.C.3.1.1.32).

**25.** Verfahren zur Herstellung von Ei oder einem Produkt auf Eibasis oder einem Milchprodukt, das einen Emulgator umfasst, wobei das Verfahren die Stufe des Zugebers einer Lipidacyltransferase zu einem Ei oder einem Produkt auf Eibasis oder einem Milchprodukt, das 10-98 % Wasser enthält, umfasst, wobei die Lipidacyltransferase eine solche ist, die, wenn sie mithilfe des Transferasetests in gepuffertem Substrat getestet wird, wenigstens 2 % Acyltransferaseaktivität aufweist, wobei der Transferasetest die Stufen umfasst:

i) Auflösen von 450 mg Phosphatidylcholin und 50 mg Cholesterol in Chloroform, Verdampfen unter Vakuum zur Trockne,
ii) Überführen von 300 mg des Cholesterol/Phosphatidylcholin-Gemischs in ein Wheaton-Glas, Zugeben von 15 ml 50 mM HEPES-Puffer mit einem pH-Wert von 7 und Auflösen des Lipids in dem Puffer unter Belegung,
iii) Erhitzen des Substrats auf 35°C während dem Mischen mit einem Magnetrührer und Zugeben einer 0,25 ml Enzymlösung,
iv) Nehmer von 2 mi-Proben bei einer Reaktionszeit von 0, 5, 10, 15, 25, 40 und 60 Minuten und sofortiges Stoppen der Enzymreaktion durch Zugabe von 25 μl 4M HCl, um die freie Fettsäure zu azidifizieren,
v) Zugeben von 3 ml Chloroform und kräftiges Schütten für 30 Sekunden, Zentrifugieren und Isolieren von 2 ml der Chloroformphase, Filtern durch einen 0,45 μm-Filter in ein tariertes 10 ml-Dram-Glas.
vii) Verdampfen des Chloroforms unter einem Stickstoffstrom bei 60°C und Messen der Probe,
viii) Analysieren des extrahierten Lipids durch GLC.

**26.** Verfahren gemäß Anspruch 25, wobei das Ei oder das Produkt auf Eibasis Mayonnaise, Salatdressing, Soßen, Eiskrem, Eispulver, modifiziertes Eigelb und daraus hergestellte Produkte sind.

**27.** Verfahren gemäß Anspruch 25, wobei das Milchprodukt Butter, Milch, Sahne, Käse, Frischkäse, Eiskrem, gefrorenes Dessert, Joghurt, Trinkjoghurt, Butterfett oder wasserfreies Milchfett ist.

**28.** Verfahren nach einem der Ansprüche 25 bis 27, wobei wenigstens 2 Emulgatoren erzeugt werden.

**29.** Verfahren gemäß einem der Ansprüche 25 bis 28, wobei die Lipidacyltransferase eine solche ist, die eine Arylgruppe von einem Lipid auf einen oder mehrere der folgenden Acylakzeptoren übertragen kann: ein Sterol, ein Stanol, ein Kohlenhydrat, ein Protein oder eine Untereinheit davon, Glycerol.

**30.** Verfahren gemäß einem der Ansprüche 25 bis 28, wobei wenigstens einer der Emulgatoren ein Sterolester und/ oder ein Stanolester ist.

**31.** Verfahren gemäß Anspruch 29, wobei der Sterolester einer oder mehrere aus Alpha-Sitosterolester, Beta-Sitosterotester, Stigmasterolester, Ergosterolester, Campesterolester oder Cholesterolester ist.

**32.** Verfahren gemäß einem der Ansprüche 25-31, wobei sowohl ein Cholesterolester als auch wenigstens ein Sterol- oder Stanolester in Kombination *in situ* erzeugt werden.

**33.** Verwendung gemäß Anspruch 30 oder Anspruch 31, wobei die Menge an freiem Cholesterol in dem Nahrungsmittol reduziert ist.

**34.** Verwendung gemäß Anspruch 30, wobei der Stanolester einer oder mehrere aus Beta-Sitostanol oder ss-Sitostanol ist.

**35.** Verfahren gemäß einem der Ansprüche 25 bis 34, wobei die Lipidacyltransferase eine solche ist, die bis zu 100 % relative Transferaseaktivität aufweist, wenn sie mithilfe des Transferasetests in Eigelb mit hohem Wassergehalt in einem Eigelb mit einem Wassergehalt von 54 % getestet wird, wobei der Transferasrwtest die folgenden Stufen umfasst:

i) Einwiegen von 5 Gramm eines flüssigen Eigelbsubstrats in einem 20 ml Wheaton-Glas und Erhitzen auf 35°C,
ii) Zugeben von Wasser und einer Enzymlösung,
iii) Überführen von 0,5 g-Proben in ein 10 ml Dram-Glas in regelmäßigen Intervallen,
iv) Zugeben von 20 µl 4M HCl, um die Enzymreaktion zu stoppen und die Fettsäureseife zu azidifizieren,
v) Zugeben von 3 ml Chloroform, Mischen der Probe für 30 Sekunden und Zentrifugieren für 10 Minuten bei 3.000 g,
vi) Überführen von 0,8 ml der Chloroformphase in ein tariertes Dram-Glas, Verdampfen des Chloroform bei 60 °C unter einem Stickstoffstrom, Messen des Dram-Giases,
vii) Analysieren des isolierten Lipids durch GLC.

**36.** Verfahren gemäß Anspruch 35, wobei die Lipidacyltransferase eine solche ist, die eine anfängliche prozentuale Acyltransferaseaktivität von wenigstens 1 % relativer Trensferaseaktivität aufweist, gemessen nachdem 10 % des Donormoleküls verbraucht waren.

**37.** Verwendung gemäß Anspruch 36, wobei die anfängliche prozentuale Acyltransferaseaktivität wenigstens 5 % beträgt.

**38.** Verwendung gemäß Anspruch 36, wobei das Acylakzeptorsubstrat ein Kohlenhydrat, ein Protein, ein Sterol, ein Stanol oder Glycerol ist.

**39.** Verwendung gemäß Anspruch 38, wobei das Acylakzeptorsubstrat ein Sterol ist.

**40.** Verfahren gemäß einem der Ansprüche 25 bis 39, wobei die Lipidacyltransferase eine solch ist, die eine relative Transferaseaktivität von wenigstens 1 % aufweist, wenn sie mit dem Transferasetest in einer Umgebung mit geringem Wassergehalt getestet wird, wobei der Transferasetest die folgenden Stufen umfasst:

i) Auflösen von 13,1 % Lecithin und 6,6 % Cholasterel in Sojabohnenöl durch Erhitzen auf 60 °C unter Bewectung,
ii) Einliegen des Substrats in ein 20 ml-Wheaton-Glas und Erhitzen auf 46 °C,
iii) Zugeben von Wasser und einer Enzymlösung,

iv) Überführen von 50 mg-Proben in ein 10 ml-Dram-Glas in regelmäßigen Intervallen und Einfrieren,
v) Analysieren der isolierten Lipide durch GLC.

**41.** Verfahren gemäß einem der Ansprüche 25 bis 40, wobei die Lipidacyltransferase als Enzym charakterisiert ist, welches Acyltransferaseaktivität besitzt und welches das Aminosäurensequenzmotiv GDSX umfasst, wobei X einer oder mehrere der folgenden Aminosäurereste L, A, V, I, F, Y, H, Q, T, N, M oder S ist.

**42.** Verwendung gemäß einem der Ansprüche 25 bis 41, wobei das Lipidacyitransferase-Enzym H-309 oder einen Histidinrest an einer Position entsprechend der von His-309 in der Aminosäuresequenz des lipolytischen Enzymes *Aeromonas hydrophila* umfasst. wie als SEQ ID No. 2 oder SED ID No. 32 gezeigt ist.

**43.** Verfahren gemäß einem der Ansprüche 25 bis 42, wobei die Lipidacyltransferase erhältlich ist aus einem Organismus einer oder mehrerer der folgenden Gattungen: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**44.** Verfahren gemäß einem der Ansprüche 25 bis 43, wobei die Lipidacyltransferase eine oder mehrere der folgenden Aminosäuresequenzen umfasst: (i) die als SEQ ID No. 2 gezeigte Aminosäuresequenz, (ii) die als SEQ fD No. 3 gezeigte Aminosäuresequenz, (iii) die als SEQ ID No. 4 gezeigte Aminosäuresequenz, (iv) die als SEQ ID No. 5 gezeigte Aminosäuresequenz, (v) die als SEQ ID No. 6 gezeigte Aminosäuresequenz, (vi) die als SEQ ID No. 12 gezeigte Aminesäuresequenz, (vii) die als SEQ ID No. 20 gezeigte Aminosäuresequenz, (viii) die als SEQ ID No. 22 gezeigte Aminosäuresequenz, (ix) die als SEQ ID No. 24 gezeigte Aminosäuresequenz, (x) die als SEQ ID No. 26 gezeigte Aminosäuresequenz, (xi) die als SEQ ID No. 28 gezeigte Aminosäuresequenz, (xii) die als SEQ ID No. 30 gezeigte Aminosäuresequenz, (xiii) die als SEQ ID No. 32 gezeigte Aminosäuresequenz, (xiv) die als SEQ ID No. 34 gezeigte Aminosäuresequenz, oder eine Aminosäuresequenz, die 75 % oder mehr Identität mit einer der Sequenzen aufweist, die gezeigt sind als SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, (SEQ ID No. 32 oder SEQ ID No. 34.

**45.** Verfahren gemäß einem der Ansprüche 25 bis 44, wobei der Emulgator einer oder mehrere der folgenden ist: ein Monoglycerid, ein Lysophosphatidylcholin, Digalactosylmonoglycerid.

**46.** Verfahren gemäß einem der Ansprüche 25 bis 45, wobei die Lipidacyltransferase in Kombination mit einer Lipase verwendet wird, die eine oder mehrere der folgenden Lipaseaktivitäten aufweist: Glycolipaseaktivität (E.C.3.1.1.26), Triacylglycerol-Lipaseaktivität (E.C.3.1.1.3), Phospholipase-A2-Aktivität (E.C.3.1.1.4) oder Phospholipase-A1-Aktivität (E.C.3.1.1.32).

**47.** Ein wasserenthaltendes Nahrungsmittel, welches 10 bis 98 % Wasser umfasst und durch das Verfahren gemäß einem der Ansprüche 25 bis 46 erhältlich ist, wobei das Nahrungsmittel eine Lipidacyltransferase, wie nähe in Anspruch 25 beschrieben, und einen Emulgator umfasst.

## Revendications

**1.** Utilisation d'une lipide acyltransférase pour préparer un matériau alimentaire contenant de l'eau, comprenant 10 à 98 % d'eau, un produit alimentaire choisi parmi un produit d'oeuf ou à base d'oeuf ou un produit laitier comprenant un émulsifiant, où l'émulsifiant est généré à partir de constituants du matériau alimentaire par la lipide acyltransférase, la lipide acyltransférase étant une acétyl transférase qui lorsqu'elle est testée à l'aide de l'essai de transférase dans un substrat tamponné, a une activité d'au moins 2 % d'acyltransférase ; ledit essai de transférase comprenant les étapes consistant à :

i) dissoudre 450 mg de phosphatidylcholine et 50 mg de cholestérol dans du chloroforme, évaporer à dessiccation sous vide;
ii) transférer 300 mg de mélange de cholestérol/phosphatidylcholine dans un verre Wheaton, ajouter 15 ml de tampon HEPES 50 mM, pH 7 et disperser le lipide dans le tampon en agitant ;
iii) chauffer le substrat à 35° C pendant le mélange avec un agitateur magnétique et ajouter 0,25 ml de solution enzymatique ;

iv) prélever des échantillons de 2 ml à 0, 5, 10, 15, 25, 40 et 60 minutes de réaction et arrêter immédiatement la réaction enzymatique par l'addition de 25 μl de HCl 4 M pour acidifier l'acide gras libre ;
v) ajouter 3 ml de chloroforme et secouer vigoureusement pendant 30 secondes, centrifuger et isoler 2 ml de phase de chloroforme, filtrer dans un filtre de 0,45 μm dans un verre Dram de 10 ml taré ;
vii) évaporer le chloroforme sous un courant d'azote à 60°C et jauger les échantillons ;
viii) analyser le lipide extrait par GLC.

**2.** Utilisation selon la revendication 1, dans laquelle le produit à base d'oeuf est une mayonnaise, un assaisonnement de salade, une sauce, une crème glacée, de la poudre d'oeuf, du jaune d'oeuf modifié et des produits fabriqués à partir de ceux-ci.

**3.** Utilisation selon la revendication 1, dans laquelle le produit laitier est du beurre, du lait, de la crème, du fromage, du fromage à tartiner, une crème glacée, des desserts congelés, un yaourt, un yaourt à boire, de la graisse de beurre ou de la graisse de lait anhydre.

**4.** Utilisation selon l'une quelconque des revendications 1 à 3, dans laquelle au moins deux émulsifiants sont produits.

**5.** Utilisation selon l'une quelconque des revendications précédentes, dans laquelle la lipide acyltransférase est telle qu'elle peut transférer un groupe acyle d'un lipide à un ou plusieurs des récepteurs d'acyle suivantes : un stérol, un stanol, un glucide, une protéine ou une sous-unité de ceux-ci, un glycérol.

**6.** Utilisation selon l'une quelconque des revendications précédentes, dans laquelle au moins l'un des émulsifiants est un ester de stérol et/ou un ester de stanol.

**7.** Utilisation selon la revendication 6, dans laquelle l'ester de stérol est un ou plusieurs des esters : ester d'alpha-sitostérol, ester de bêta-sitostérol, ester de stigmastérol, ester d'ergostérol, ester de campestérol ou ester de cholestérol.

**8.** Utilisation selon l'une quelconque des revendications précédentes, dans laquelle à la fois un ester de cholestérol et au moins un ester de stérol ou de stanol en combinaison, est produit *in situ*.

**9.** Utilisation selon la revendication 7 ou 8, dans laquelle la quantité de cholestérol libre dans le produit alimentaire est réduite.

**10.** Utilisation selon la revendication 6, dans laquelle l'ester de stanol est un ou plusieurs des bêta-sitostanol ou ss-sitostanol.

**11.** Utilisation selon l'une quelconque des revendications précédentes, dans laquelle la lipide acyltransférase est telle que lorsqu'elle est testée à l'aide de l'essai de transférase dans du jaune d'oeuf à teneur élevée en eau dans un jaune d'oeuf comportant 54 % d'eau, elle a une activité relative de transférase allant jusqu'à 100 % ; ledit essai de transférase comprenant les étapes consistant à :

i) placer 5 grammes de substrat de jaune d'oeuf liquide dans un verre Wheaton de 20 ml et chauffer à 35° C ;
ii) ajouter de l'eau et la solution enzymatique ;
iii) à intervalles réguliers, transférer 0,5 g d'échantillons dans un verre Dram de 10 ml ;
iv) ajouter 20 μl de HCl 4 M pour arrêter la réaction enzymatique et acidifier le savon d'acide gras ;
v) ajouter 3 ml de chloroforme, mélanger l'échantillon pendant 30 secondes et centrifuger à 3000 g pendant 10 main ;
vi) transférer 0,8 ml de la phase de chloroforme dans un verre Dram taré, évaporer le chloroforme à 60° C sous un courant d'azote ; jauger le verre Dram ;
vii) analyser les lipides isolés par GLC.

**12.** Utilisation selon la revendication 11, dans laquelle la lipide acyltransférase est telle qu'elle présente un pourcentage initial d'activité d'acyltransférase mesurée après 10 % de consommation de la molécule donneuse d'au moins 1 % d'activité de transférase relative.

**13.** Utilisation selon la revendication 12, dans laquelle le pourcentage initial d'activité d'acyltransférase mesurée après 10 % de consommation de la molécule donneuse est d'au moins 5 %

**14.** Utilisation selon la revendication 1, dans laquelle le substrat accepteur d'acyle est un glucide, une protéine, un stérol, un stanol ou un glycérol.

**15.** Utilisation selon la revendication 1, dans laquelle le substrat accepteur d'acyle est un stérol.

**16.** Utilisation l'une quelconque des revendications précédentes, dans laquelle la lipide acyltransférase est telle que lorsqu'elle est testée à l'aide de l'essai de transférase dans un environnement à faible teneur en eau, il a une activité relative de transférase d'au moins 1 % ; ledit essai de transférase comprenant les étapes consistant à :

i) dissoudre 13,1 % de lécithine et 6,6 % de cholestérol dans de l'huile de soja en chauffant à 60° C en agitant ;
ii) placer le substrat dans un verre Wheaton de 20 ml et chauffer à 46° C ;
iii) ajouter de l'eau et la solution enzymatique ;
iv) à intervalles réguliers, transférer des échantillons de 50 mg dans un verre Dram de 10 ml et congeler ;
v) analyser les lipides isolés par GLC.

**17.** Utilisation selon la revendication 16, dans laquelle le substrat accepteur d'acyle dans l'essai est un glucide, une protéine, un stérol, un stanol ou un glycérol.

**18.** Utilisation selon la revendication 17, dans laquelle le substrat accepteur d'acyle dans l'essai est un stérol.

**19.** Utilisation selon l'une quelconque des revendications précédentes, dans laquelle la lipide acyltransférase est **caractérisée** comme une enzyme qui possède une activité d'acyltransférase et qui comprend le motif de séquence d'acides aminés GDSX, où X est un ou plusieurs des résidus d'acides aminés suivants : L, A, V, I, F, Y, H, Q, T, N, M ou S.

**20.** Utilisation selon l'une quelconque des revendications précédentes, dans laquelle l'enzyme lipide acyltransférase comprend H-309 ou comprend un résidu d'histidine sur une position correspondant à His-309 dans la séquence d'acides aminés de l'enzyme lipolytique *Aeromonas hydrophila* présentée dans SEQ ID N° : 2 ou SEQ ID N° : 32.

**21.** Utilisation selon l'une quelconque des revendications précédentes, dans laquelle la lipide acyltransférase peut être obtenue à partir d'un organisme d'un ou plusieurs des genres suivants : *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergilles, Schizosaccharomyces, Listeria, Neisseria, Mesorhizobium, Ralstonia, Xanthomonas* et *Candida.*

**22.** Utilisation selon l'une quelconque des revendications précédentes, dans laquelle la lipide acyltransférase comprend une ou plusieurs des séquences d'acides aminés suivantes :

(i) la séquence d'acides aminés présentée dans SEQ ID N°: 2 ; (ii) la séquence d'acides aminés présentée dans SEQ ID N° : 3 ; (iii) la séquence d'acides aminés présentée dans SEQ ID N° : 4 ; (iv) la séquence d'acides aminés présentée dans SEQ ID N° : 5 ; (v) la séquence d'acides aminés présentée dans SEQ ID N° : 6; (vi) la séquence d'acides aminés présentée dans SEQ ID N° : 12 ; (vii) la séquence d'acides aminés présentée dans SEQ ID N° : 20 ; (viii) la séquence d'acides aminés présentée dans SEQ ID N° : 22 ; (ix) la séquence d'acides aminés présentée dans SEQ ID N° : 24 ; (x) la séquence d'acides aminés présentée dans SEQ ID N° : 26 ; (xi) la séquence d'acides aminés présentée dans SEQ ID N° : 28 ; (xii) la séquence d'acides aminés présentée dans SEQ ID N° : 30 : (xiii) la séquence d'acides aminés présentée dans SED ID N° : 32 ; (xiv) la séquence d'acides aminés présentée dans SEQ ID N° : 34 ; ou une séquence d'acides aminés qui à une identité de 75 % ou plus avec l'une quelconque des séquences présentées dans SED ID N° : 2, SEQ ID N° : 3, SEQ ID N° : 4, SEQ ID N° : 5, SEQ ID N° : 6, SEQ ID N° : 12, SEQ ID N° : 20, SED ID N° : 22, SEQ ID N° : 24, SEQ ID N° : 26, SEQ ID N° : 28, SEQ ID N° : 30, SEQ ID N° : 32 ou SEQ ID N° : 34.

**23.** Utilisation selon l'une quelconque des revendications précédentes, dans laquelle l'émulsifiant est un ou plusieurs des suivants: un monoglycéride, une lysophosphatidylcholine. un monoglycéride de digalactosyle.

**24.** Utilisation selon l'une quelconque des revendications précédentes, dans laquelle la lipide acyltransférase est utilisée en combinaison avec une lipase ayant une ou plusieurs des activités de lipase suivantes : activité de glycolipase (E.C.3.1.1.26) ; activité de triacylglycérol-lipase (E.C.3.1.1.3) ; activité de phospholipase A2 (E.C.3.1.1.4) ; ou activité de phospholipase A1 (E.C.3.1.1.32).

**25.** Procédé de production d'un produit d'oeuf ou à base d'oeuf ou d'un produit laitier comprenant un émulsifiant, le procédé comprenant l'étape d'addition d'une lipide acyltransférase à un produit d'oeuf ou à base d'oeuf ou à un produit initier contenant 10 à 98 % d'eau, la lipide acyltransférase étant telle que lorsqu'elle est testée à l'aide de l'essai de transférase dans un substrat tamponné, elle a une activité d'acyltransférase d'au moins 2 % ; ledit essai de transférase comprenant les étapes consistant à :

i) dissoudre 450 mg de phosphatidylcholine et 50 mg de cholestérol dans du chloroforme, évaporer à dessiccation sous vide ;
ii) transférer 300 mg de mélange de cholestérol/phosphatidylcholine dans un verre Wheaton, ajouter 15 ml de tampon HEPES 50 mM, pH 7 et disperser le lipide dans le tampon en agitant ;
iii) chauffer le substrat à 35° C en mélangeant avec un agitateur magnétique et ajouter 0,25 ml de solution enzymatique ;
iv) prélever des échantillons de 2 ml à 0, 5, 10, 15, 25, 40 et 60 minutes de réaction et arrêter immédiatement la réaction enzymatique par l'addition de 25 μl de HCl 4 M pour acidifier l'acide gras libre;
v) ajouter 3 ml de chloroforme et secouer vigoureusement pendant 30 secondes, centrifuger et isoler 2 ml de phase de chloroforme, filtrer dans un filtre de 0,45 μm dans un verre Dram de 10 ml taré ;
vii) évaporer le chloroforme sous un courant d'azote à 60° C et jauger les échantillons ;
viii) analyser le lipide extrait par GLC.

**26.** Procédé selon la revendication 25, dans lequel le produit d'oeuf ou à base d'oeuf est une mayonnaise, un assaisonnement de salade, une sauce, une crème glacée, de la poudre d'oeuf, du jaune d'oeuf modifié et des produits fabriqués à partir de ceux-ci.

**27.** Procédé selon la revendication 25, dans lequel le produit laitier est du beurre, du lait, de la crème, du fromage, du fromage à tartiner, une crème glacée, des desserts congelés, un yaourt, un yaourt à boire, de la graisse de beurre ou de la graisse de lait anhydre.

**28.** Procédé selon l'une quelconque des revendications 25 à 27, dans lequel au moins deux émulsifiants sont produits.

**29.** Procédé selon l'une quelconque des revendications 25 à 28, dans lequel la lipide acyltransférase est telle qu'elle peut transférer un groupe acyle d'un lipide à un ou plusieurs des récepteurs d'acyle suivants : un stérol, un stanol, un glucide, une protéine ou une sous-unité de ceux-ci, un glycérol.

**30.** Procédé selon l'une quelconque des revendications 25 à 28, dans lequel au moins l'un des émulsifiants est un ester de stérol et/ou un ester de stanol.

**31.** Procédé selon la revendication 29, dans lequel l'ester de stérol est un ou plusieurs des esters : ester d'alphasitostérol, ester de bêia-sitostérol, ester de stigmastérol, ester d'ergostérol, ester de campestérol ou ester de cholestérol.

**32.** Procédé selon l'une quelconque des revendications 25 à 31, dans lequel à la fois un ester de cholestérol et au moins un ester de stérol ou de stanol en combinaison, est produit *in situ.*

**33.** Procédé selon la revendication 30 ou 31, dans lequel la quantité de cholestérol libre dans le produit alimentaire est réduite.

**34.** Procédé selon la revendication 30, dans lequel l'ester de stanol est un ou plusieurs des bèta-sitostanol ou ss-sitostanol.

**35.** Procédé selon l'une quelconque des revendications 25 à 34, dans lequel la lipide acyltransférase est telle que lorsqu'elle est testée à l'aide de l'essai de transférase dans du jaune d'oeuf à teneur élevée en eau dans un jaune d'oeuf comportant 54 % d'eau, elle a une activité relative de transférase allant jusqu'à 100 % ; ledit essai de transférase comprenant les étapes consistant à :

i) placer 5 grammes de substrat de jaune d'oeuf liquide dans un verre Wheaton de 20 ml et chauffer à 35° C ;
ii) ajouter de l'eau et la solution enzymatique ;
iii) à intervalles réguliers, transférer 0,5 g d'échantillons dans un verre Dram de 10 ml ;
iv) ajouter 20 μl de HCl 4 M pour arrêter la réaction enzymatique et acidifier le savon d'acide gras ;

v) ajouter 3 ml de chloroforme, mélanger l'échantillon pendant 30 secondes et centrifuger à 3000 g pendant 10 min ;
vi) transférer 0,8 ml de la phase de chloroforme dans un verre Dram taré, évaporer le chloroforme à 60° C sous un courant d'azote ; jauger le verre Dram ;
vii) analyser les lipides isolés par GLC.

**36.** Procédé selon la revendication 35, dans lequel la lipide acyltransférase est telle qu'elle présente un pourcentage initial d'activité d'acyltransférase mesurée après 10 % de consommation de la molécule donneuse d'au moins 1 % d'activité de transférase relative.

**37.** Procédé selon la revendication 36, dans lequel le pourcentage initial d'activité d'acyltransférase est d'au moins 5 %.

**38.** Procédé selon la revendication 36, dans lequel le substrat accepteur d'acyle est un glucide, une protéine, un stérol, un stanol ou un glycérol

**39.** Procédé selon la revendication 38, dans lequel le substrat accepteur d'acyle est un stérol.

**40.** Procédé selon l'une quelconque des revendications 25 à 39, dans lequel la lipide acyltransférase est telle que lorsqu'elle est testée à l'aide de l'essai de transférase dans un environnement à faible teneur en eau, elle a une activité relative de transférase d'au moins 1 % ; ledit essai de transférase comprenant les étapes consistant à :

i) dissoudre 13,1 % de lécithine et 6,6 % de cholestérol dans de l'huile de soja en chauffant à 60° C en agitait ;
ii) placer le substrat dans un verre Wheaton de 20 ml et chauffer à 46° C ;
iii) ajouter de l'eau et la solution enzymatique ;
iv) à intervalles réguliers, transférer des échantillons de 50 mg dans un verre Dram de 10 ml et congeler ;
v) analyser les lipides isolés par GLC.

**41.** Procédé selon l'une quelconque des revendications 25 à 40, dans lequel la lipide acyltransférase est **caractérisée** comme une enzyme qui possède une activité d'acyltransférase et qui comprend le motif de séquence d'acides aminés GDSX, où X est un ou plusieurs des résidus d'acides aminés suivants : L, A, V, I, F, Y, H, Q, T, N, M ou S.

**42.** Procédé selon l'une quelconque des revendications 25 à 41, dans lequel l'enzyme lipide acyltransférase comprend H-309 ou comprend un résidu d'histidine sur une position correspondant à His-309 dans la séquence d'acides aminés de l'enzyme lipolytique *Aeromonas hydrophila* présentée dans SEQ ID N° : 2 ou SEQ ID N° : 32.

**43.** Procédé selon l'une quelconque des revendications 25 à 42, dans lequel la lipide acyltransférase peut être obtenue à partir d'un organisme d'un ou plusieurs des genres suivantes ; *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces. Listeria, Neisseria Mesorhizobium, Ralstonia, Xanthomonas* et *Candida.*

**44.** Procédé selon l'une quelconque des revendications 25 à 43, dans lequel la lipide acyltransférase comprend une ou plusieurs des séquences d'acides aminés suivantes :

(i) la séquence d'acides aminés présentée dans SEQ ID N° : 2; (ii) la séquence d'acides aminés présentée dans SEQ ID N° : 3 ; (iii) la séquence d'acides aminés présentée dans SEQ ID N° : 4 ; (iv) la séquence d'acides aminés présentée dans SEQ ID N° : 5 ; (v) la séquence d'acides aminés présentée dans SEQ ID N° : 6 ; (vi) la séquence d'acides aminés présentée dans SEQ ID N° : 12 ; (vii) la séquence d'acides aminés présentée dans SEQ ID N° : 20 ; (viii) la séquence d'acides aminés présentée dans SEQ ID N° : 22; (ix) la séquence d'acides aminés présentée dans SEQ ID N° : 24 ; (x) la séquence d'acides aminés présentée dans SEQ ID N° : 26 ; (xi) la séquence d'acides aminés présentée dans SEQ ID N° : 28 ; (xii) la séquence d'acides aminés présentée dans SEQ ID N° : 30 ; (xiii) la séquence d'acides aminés présentée dans SEQ ID N° : 32 ; (xiv) la séquence d'acides aminés présentée dans SEQ ID N° : 34 : ou une séquence d'acides aminés qui a une identité de 75 % ou plus avec l'une quelconque des séquences présentées dans SEQ ID N° : 2, SEQ ID N° : 3, SEQ ID N° : 4, SEQ ID N° : 5, SEQ ID N° : 6, SEQ ID N° : 12, SEQ ID N° : 20, SEQ ID N° : 22, SEQ ID N° : 24, SEQ ID N° : 26, SEQ ID N° : 28, SEQ ID N° : 30, SEQ ID N° : 32 ou SED ID N° : 34.

**45.** Procédé selon l'une quelconque des revendications 25 à 44, dans lequel l'émulsifiant est un ou plusieurs des suivants : un monoglycéride, une lysophosphatidylelholine, un monoglycéride de digalactosyle.

**46.** Procédé selon l'une quelconque des revendications 25 à 45, dans lequel la lipide acyltransférase est utilisée en combinaison avec une lipase ayant une ou plusieurs des activités de lipase suivantes : activité de glycolipase (E.C. 3.1.1.26) ; activité de triacylglycérol-lipase (E.C.3,1.1.3) ; activité de phospholipase A2 (E,C.3.1.1.4) ; ou activité de phospholipase Al (E.C.3.1.1.32).

**47.** Produit alimentaire contenant de l'eau, comprenant 10 à 98 % d'eau, pouvant être obtenu par le procédé selon l'une quelconque des revendications 25 à 46, le produit alimentaire comprenant une lipide acyltransférase telle que définie dans la revendication 25 et un émulsifiant.

Figure 1

SEQ ID No. 1

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

Figure 2

SEQ ID No. 2

```
  1 mkkwfvcllg lvaltvqaad srpafsrivm fgdslsdtgk myskmrgylp ssppyyegrf
 61 sngpvwleql tnefpgltia neaeggptav aynkiswnpk yqvinnldye vtqflqkdsf
121 kpddlvilwv gandylaygw nteqdakrvr daisdaanrm vlngakeill fnlpdlgqnp
181 sarsqkvvea ashvsayhnq lllnlarqla ptgmvklfei dkqfaemlrd pqnfglsdqr
241 nacyggsyvw kpfasrsast dsqlsafnpq erlaiagnpl laqavaspma arsastlnce
301 gkmfwdqvhp ttvvhaalse paatfiesqy eflah
```

Figure 3

SEQ ID No. 3

```
  1 mkkwfvcllg lialtvqaad trpafsrivm fgdslsdtgk myskmrgylp ssppyyegrf
 61 sngpvwleql tkqfpgltia neaeggatav aynkiswnpk yqvynnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

Figure 4

SEQ ID No. 4

```
  1 mpkpalrrvm tatvaavgtl algltdatah aapaqatptl dyvalgdsys agsgvlpvdp
 61 anllclrsta nyphviadtt garltdvtcg aagtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

## Figure 5

### SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

## Figure 6

### SEQ ID No. 6

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgsnims
```

# Figure 7

```
Alignment of pfam00657.6 consensus sequence with P10480
                   *->ivafGDSlTdg.................eayygdsdgggwgagladrL
                      iv+fGDSl+d+++  ++ ++  +++++++ +++s+g  w ++l + +
     P10480     28    IVMFGDSLSDTgkmyskmrgylpssppYYEGRFSNGPVWLEQLTNEF 74

                   tall..rlrarprgvdvfnrgisGrtsdGrlivDalvallFlaqslglpn
                   + l    + ++++++++ +n+  +
     P10480     75 PGLTiaNEAEGGPTAVAYNKISWNPK------------------------ 100

                   LpPYLsgdflrGANFAsagAtIlptsgpfliQvqFkdfksqvlelrqalg
                                                                 ++  ++
     P10480    101 ------------------------------------------YQVINN 106

                   llqellrllpvldakspdlvtimiGtNDlitsaffgpkstesdrnvsvpe
                   l++e+ ++l +++ k+ dlv++++G+ND+          ++ ++ ++++++
     P10480    107 LDYEVTQFLQKDSFKPDDLVILWVGANDY--------LAYGWNTEQDAKR 148

                   fkdnlrqlikrLrsnngariivlitlvilnlgplGClPlklalalasskn
                   ++d ++++++r+    nga+       ++++nl+ lG+ P+
     P10480    149 VRDAISDAANRMV-LNGAK-----EILLFNLPDLGQNPS----------- 181

                   vdasgclerlneavadfnealrelaiskledqlrkdglpdvkgadvpyvD
                    ++++ +e +  ++a++n++l +la     +gl+++g+++++++d ++++
     P10480    182 ARSQKVVEAASHVSAYHNQLLLNLA-----RQLAPTGMVKLFEIDKQFAE 226

                   lysifqdldgiqnpsayv.y....GFe.ttkaCCGyGgr.yNyn.rv.CG
                   +    +q+++ + + +a+++++    +++ +++a++++++++ +N+++r+ ++
     P10480    227 MLRDPQNFGLSDQRNACYgGsyvwKPFaSRSASTDSQLSaFNPQeRLaIA 276

                   nag.l.c.nvtakaC.npssyll.sflfwDgfHpsekGykavAeal<-*
                   +++ l +  ++++a++ +s+ ++++++fwD++Hp+    ++a+ e
     P10480    277 GNPlLaQaVASPMAArSASTLNCeGKMFWDQVHPTTVVHAALSEPA    322

Alignment of pfam00657.6 consensus sequence with AAG09804
                   *->ivafGDSlTdg.................eayygdsdgggwgagladrL
                      iv+fGDSl+d+++  ++ ++  +++++++ +++s+g  w ++l + +
   AAG09804     28    IVMFGDSLSDTgkmyskmrgylpssppYYEGRFSNGPVWLEQLTKQF 74

                   tallrlrarprgvdvfnrgisGrtsdGrlivDalvallFlaqslglpnLp
                             +g+++ n + +G+t
   AAG09804     75 ----------PGLTIANEAEGGAT-------------------------- 88

                   PYLsgdflrGANFAsagAtIlptsgpfliQvqFkdfksqvlelrqa....
                                                       ++++ + ++++ +
   AAG09804     89 -----------------------------------AVAYNKISWNpkyq 102

                   ..lgllqellrllpvldakspdlvtimiGtNDlitsaffgpkstesdrnv
                     ++l++e+ ++l +++ k+ dlv++++G+ND+          ++ ++ ++
   AAG09804    103 vyNNLDYEVTQFLQKDSFKPDDLVILWVGANDY--------LAYGWNTEQ 144

                   svpefkdnlrqlikrLrsnngariivlitlvilnlgplGClPlklalala
                   ++++++d ++++++r+    nga+      +++++nl+ lG+ P+
   AAG09804    145 DAKRVRDAISDAANRMV-LNGAK-----QILLFNLPDLGQNPS------- 181

                   ssknvdasgclerlneavadfnealrelaiskledqlrkdglpdvkgadv
                       ++++ +e +  ++a++n++l +la     +gl+++g+++++++d
   AAG09804    182 ----ARSQKVVEAVSHVSAYHNKLLLNLA-----RQLAPTGMVKLFEIDK 222

                   pyvDlysifqdldgiqnpsayv.y....GFe.ttkaCCGyGgr.yNyn.r
                   +++++    +q+++ +  ++ +++++    +++ t++ +++ +++ + +++r
   AAG09804    223 QFAEMLRDPQNFGLSDVENPCYdGgyvwKPFaTRSVSTDRQLSaFSPQeR 272

                   v.CGnag.l.c.nvtakaC.npssyll.sflfwDgfHpsekGykavAeal
                   + +++++ l +  ++++a++ +s  ++++++fwD++Hp+    ++a+ e+
   AAG09804    273 LaIAGNPlLaQaVASPMARrSASPLNCeGKMFWDQVHPTTVVHAALSERA 322

                   <-*
```

```
AAG09804      -     -

Alignment of pfam00657.6 consensus sequence with NP_631558
                    *->ivafGDSlTdgeayygdsdgggwgagladrLtallrlrarprgvdvf
                       +va+GDS ++g          +g  + +++L     + + + ++  +
   NP_631558     42    YVALGDSYSAG---------SGVLPVDPANL----LCLRSTANYPHV 75

                    nrgisGrtsdGrlivD.a.l.vallFlaqslglpnLpPYLsgdflrGANP
                     + ++G++       D + + +
   NP_631558     76 IADTTGAR-----LTDvTcGaAQ------------------------- 93

                    AsagAtIlptsgpfliQvqFkdfksqvlelrqalgllqellrllpvldak
                                                +++    ++ +  ++ +++
   NP_631558     94 ----------------------------TADFTRAQYPGVAPQLDALGT 114

                    spdlvtimiGtNDl................itsaffgpkstesdrnvsvp
                    + dlvt+ iG+ND ++   +   +  ++ +   ++  + +k   ++ + +++
   NP_631558    115 GTDLVTLTIGGNDNstfinaitacgtagvlSGGKGSPCKDRHGTSFDDEI 164

                    efkdn..lrqlikrLrs.nngariivlitlvilnlg...........plG
                    e  +++ l++++  +r+++ +ar+ +l  ++i+++  +++    + +   G
   NP_631558    165 EANTYpaLKEALLGVRArAPHARVAALGYPWITPATadpscflklplAAG 214

                    ClPlklalalassknvdasgclerlneavadfnealrelaiskledqlrk
                      P+                      l+ ++a  n a+r  a
   NP_631558    215 DVPY-------------------LRAIQAHLNDAVRRAA---------- 234

                    dglpdvkgadvpyvDlysifqdldgiqnpsayvyGPettkaCCGyGgryN
                          ++ + +yvD+ ++
   NP_631558    235 ------EETGATYVDFSGVSDG-------------------------- 250

                    ynrvCGnaglcnvtakaC.npssyll.sflfwDgf...HpsekGykavAe
                                    ++aC+ p +++ +  lf + + + Hp++ G +++Ae
   NP_631558    251 --------------HDACeAPGTRWIePLLFGHSLvpvHPNALGERRMAE 286

                    al<-*
                    +
   NP_631558    287 HT    288

Alignment of pfam00657.6 consensus sequence with CAC42140
                    *->ivafGDSlTdgeayygdsdgggwgagladrLtallrlrarprgvdvf
                       +va+GDS ++g          +g  + +++L     + + + ++  +
   CAC42140      42    YVALGDSYSAG---------SGVLPVDPANL----LCLRSTANYPHV 75

                    nrgisGrtsdGrlivD.a.l.vallFlaqslglpnLpPYLsgdflrGANP
                     + ++G++       D + + +
   CAC42140      76 IADTTGAR-----LTDvTcGaAQ------------------------- 93

                    AsagAtIlptsgpfliQvqFkdfksqvlelrqalgllqellrllpvldak
                                                +++    ++ +  ++ +++
   CAC42140      94 ----------------------------TADFTRAQYPGVAPQLDALGT 114

                    spdlvtimiGtNDl................itsaffgpkstesdrnvsvp
                    + dlvt+ iG+ND ++   +   +  ++ +   ++  + +k   ++ + +++
   CAC42140     115 GTDLVTLTIGGNDNstfinaitacgtagvlSGGKGSPCKDRHGTSFDDEI 164

                    efkdn..lrqlikrLrs.nngariivlitlvilnlg...........plG
                    e  +++ l++++  +r+++ +ar+ +l  ++i+++  +++    + +   G
   CAC42140     165 EANTYpaLKEALLGVRArAPHARVAALGYPWITPATadpscflklplAAG 214

                    ClPlklalalassknvdasgclerlneavadfnealrelaiskledqlrk
                      P+                      l+ ++a  n a+r  a
   CAC42140     215 DVPY-------------------LRAIQAHLNDAVRRAA---------- 234

                    dglpdvkgadvpyvDlysifqdldgiqnpsayvyGPettkaCCGyGgryN
                          ++ + +yvD+ ++
   CAC42140     235 ------EETGATYVDFSGVSDG-------------------------- 250

                    ynrvCGnaglcnvtakaC.npssyll.sflfwDgf...HpsekGykavAe
                                    ++aC+ p +++ +  lf + + + Hp++ G +++Ae
   CAC42140     251 --------------HDACeAPGTRWIePLLFGHSLvpvHPNALGERRMAE 286
```

```
                 al<-*
                 +
    CAC42140  287 HT    288

Alignment of pfam00657.6 consensus sequence with P41734
              *->ivafGDSlTdg....eayygdsdgggwgagladrLtallrlrarprg
                  ++fGDS+T+     +++ + +d+   ga+l + +         +r+
    P41734      6 FLLFGDSITEPafntRPIEDGKDQYALGAALVNEY---------TRK 43

                 vdvfnrgisGrtsdGrlivDalvallFlaqslglpnLpPYLsgdflrGAN
                 +d+  rg++G+t
    P41734     44 MDILQRGFKGYT-------------------------------------- 55

                 FAsagAtIlptsgpfliQvqFkdfksqvlelrqalgllqellrllpvlda
                                               +r+al++l+e+l+      +
    P41734     56 ------------------------------SRWALKILPEILKH-----E 70

                 kspdlvtimiGtNDlitsaffgpkstesdrnvsvpefkdnlrqlikrLrs
                  +  + ti++G+ND+         ++ +++ v++pef+dn+rq++++++s
    P41734     71 SNIVMATIFLGANDA---------CSAGPQSVPLPEFIDNIRQMVSLMKS 111

                 nngariivlitlvilnlgplGClPlklalalassknvdasgclerlneav
                  ++++ii++++lv   ++             ++ k ++ +  + r+ne +
    P41734    112 YHIRPIIIGPGLVDREKW-------------EKEKSEEIALGYFRTNENF 148

                 adfnealrelaiskledqlrkdglpdvkgadvpyvDlysifqdldgiqnp
                 a +  al +la                ++ +vp+v l+++fq+ +g++++
    P41734    149 AIYSDALAKLA----------------NEEKVPFVALNKAPQQEGGDAWQ 182

                 sayvyGPettkaCCGyGgryNynrvCGnaglcnvtakaCnpssyllsflf
                 +                                               l+
    P41734    183 Q-----------------------------------------------LL 185

                 wDgfHpsekGykavAeal<-*
                  Dg+H+s kGyk+++++l
    P41734    186 TDGLHFSGKGYKIFHDEL    203
```

## Figure 8

```
A.sal   1   MKKWFVCLLGLIALTVQAADTRPAFSRIVMFGDSLSDTGKMYSKMRGYLPSSPPYYEGRP 60
                      .+         +
A.hyd   1   MKKWFVCLLGLVALTVQAADSRPAFSRIVMFGDSLSDTGKMYSKMRGYLPSSPPYYEGRP 60
A. sal 61   SNGPVWLEQLTKQPPGLTIANEAEGGATAVAYNKISWNPKYQVINNLDYEVTQFLQKDSP 120
                           ++            +
A. hyd 61   SNGPVWLEQLTNEPPGLTIANEAEGGPTAVAYNKISWNPKYQVINNLDYEVTQFLQKDSP 120
A. sal 121  KPDDLVILWVGANDYLAYGWNTEQDAKRVRDAISDAANRMVLNGAKQILLFNLPDLGQNP 180
                                             .                          +
A. hyd 121  KPDDLVILWVGANDYLAYGWNTEQDAKRVRDAISDAANRMVLNGAKEILLFNLPDLGQNP 180
A. sal 181  SARSQKVVEAVSHVSAYHNKLLLNLARQLAPTGMVKLFEIDKQFAEMLRDPQNFGLSDVE 240
                           +        +       .                                 ++
A.hyd  181  SARSQKVVEAASHVSAYHNQLLLNLARQLAPTGMVKLFEIDKQFAEMLRDPQNFGLSDQR 240
A. sal 241  NPCYDGGYVWKPFATRSVSTDRQLSAPSPQERLAIAGNPLLAQAVASPMARRSASPLNCE 300
             + ++ +        +  +   +    +                      +    +
A. hyd 241  NACYGGSYVWKPFASRSASTDSQLSAFNPQERLAIAGNPLLAQAVASPMAARSASTLNCE 300
A. sal 301  GKMFWDQVHPTTVVHAALSERAATFIETQYEFLAH 335
                                 +     +          .
A. hyd 301  GKMFWDQVHPTTVVHAALSEPAATFIESQYEFLAH 335
```

Figure 9

```
  1  ATGAAAAAAT GGTTTGTGTG TTTATTGGGA TTGGTCGCGC TGACAGTTCA GGCAGCCGAC
 61  AGCCGTCCCG CCTTCTCCCG GATCGTGATG TTTGGCGACA GCCTCTCCGA TACCGGCAAG
121  ATGTACAGCA AGATGCGCGG TTACCTCCCC TCCAGCCCCC CCTACTATGA GGGCCGCTTC
181  TCCAACGGGC CCGTCTGGCT GGAGCAGCTG ACCAACGAGT TCCCGGGCCT GACCATAGCC
241  AACGAGGCGG AAGGCGGACC GACCGCCGTG GCTTACAACA AGATCTCCTG GAATCCCAAG
301  TATCAGGTCA TCAACAACCT GGACTACGAG GTCACCCAGT TCCTGCAAAA AGACAGCTTC
361  AAGCCGGACG ATCTGGTGAT CCTCTGGGTC GGCGCCAACG ACTATCTGGC CTATGGCTGG
421  AACACAGAGC AGGATGCCAA GCGGGTGCGC GACGCCATCA GCGATGCGGC CAACCGCATG
481  GTGCTGAACG GCGCCAAGGA GATACTGCTG TTCAACCTGC CGGATCTGGG CCAGAACCCC
541  TCGGCCCGCA GCCAGAAGGT GGTCGAGGCG GCCAGCCATG TCTCCGCCTA CCACAACCAG
601  CTGCTGCTGA ACCTGGCACG CCAGCTGGCT CCCACCGGCA TGGTGAAGCT GTTCGAGATC
661  GACAAGCAGT TTGCCGAGAT GCTGCGTGAT CCGCAGAACT TCGGCCTGAG CGACCAGAGG
721  AACGCCTGCT ACGGTGGCAG CTATGTATGG AAGCCGTTTG CCTCCCGCAG CGCCAGCACC
781  GACAGCCAGC TCTCCGCCTT CAACCCGCAG GAGCGCCTCG CCATCGCCGG CAACCCGCTG
841  CTGGCCCAGG CCGTCGCCAG CCCCATGGCT GCCCGCAGCG CCAGCACCCT CAACTGTGAG
901  GGCAAGATGT TCTGGGATCA GGTCCACCCC ACCACTGTCG TGCACGCCGC CCTGAGCGAG
961  CCCGCCGCCA CCTTCATCGA GAGCCAGTAC GAGTTCCTCG CCCAC
```

## Figure 10

```
  1 ATGAAAAAAT GGTTTGTTTG TTTATTGGGG TTGATCGCGC TGACAGTTCA GGCAGCCGAC
 61 ACTCGCCCCG CCTTCTCCCG GATCGTGATG TTCGGCGACA GCCTCTCCGA TACCGGCAAA
121 ATGTACAGCA AGATGCGCGG TTACCTCCCC TCCAGCCCGC CCTACTATGA GGGCCGTTTC
181 TCCAACGGAC CCGTCTGGCT GGAGCAGCTG ACCAAGCAGT TCCCGGGTCT GACCATCGCC
241 AACGAAGCGG AAGGCGGTGC CACTGCCGTG GCTTACAACA AGATCTCCTG GAATCCCAAG
301 TATCAGGTCT ACAACAACCT GGACTACGAG GTCACCCAGT TCTTGCAGAA AGACAGCTTC
361 AAGCCGGACG ATCTGGTGAT CCTCTGGGTC GGTGCCAATG ACTATCTGGC ATATGGCTGG
421 AATACGGAGC AGGATGCCAA GCGAGTTCGC GATGCCATCA GCGATGCGGC CAACCGCATG
481 GTACTGAACG GTGCCAAGCA GATACTGCTG TTCAACCTGC CGGATCTGGG CCAGAACCCG
541 TCAGCCCGCA GTCAGAAGGT GGTCGAGGCG GTCAGCCATG TCTCCGCCTA TCACAACAAG
601 CTGCTGCTGA ACCTGGCACG CCAGCTGGCC CCCACCGGCA TGGTAAAGCT GTTCGAGATC
661 GACAAGCAAT TTGCCGAGAT GCTGCGTGAT CCGCAGAACT TCGGCCTGAG CGACGTCGAG
721 AACCCCTGCT ACGACGGCGG CTATGTGTGG AAGCCGTTTG CCACCCGCAG CGTCAGCACC
781 GACCGCCAGC TCTCCGCCTT CAGTCCGCAG GAACGCCTCG CCATCGCCGG CAACCCGCTG
841 CTGGCACAGG CCGTTGCCAG TCCTATGGCC CGCCGCAGCG CCAGCCCCCT CAACTGTGAG
901 GGCAAGATGT TCTGGGATCA GGTACACCCG ACCACTGTCG TGCACGCAGC CCTGAGCGAG
961 CGCGCCGCCA CCTTCATCGA GACCCAGTAC GAGTTCCTCG CCCACGGATG A
```

Figure 11

```
  1 ATGCCGAAGC CTGCCCTTCG CCGTGTCATG ACCGCGACAG TCGCCGCCGT CGGCACGCTC
 61 GCCCTCGGCC TCACCGACGC CACCGCCCAC GCCGCGCCCG CCCAGGCCAC TCCGACCCTG
121 GACTACGTCG CCCTCGGCGA CAGCTACAGC GCCGGCTCCG GCGTCCTGCC CGTCGACCCC
181 GCCAACCTGC TCTGTCTGCG CTCGACGGCC AACTACCCCC ACGTCATCGC GGACACGACG
241 GGCGCCCGCC TCACGGACGT CACCTGCGGC GCCGCGCAGA CCGCCGACTT CACGCGGGCC
301 CAGTACCCGG GCGTCGCACC CCAGTTGGAC GCGCTCGGCA CCGGCACGGA CCTGGTCACG
361 CTCACCATCG GCGGCAACGA CAACAGCACC TTCATCAACG CCATCACGGC CTGCGGCACG
421 GCGGGTGTCC TCAGCGGCGG CAAGGGCAGC CCCTGCAAGG ACAGGCACGG CACCTCCTTC
481 GACGACGAGA TCGAGGCCAA CACGTACCCC GCGCTCAAGG AGGCGCTGCT CGGCGTCCGC
541 GCCAGGGCTC CCCACGCCAG GGTGGCGGCT CTCGGCTACC CGTGGATCAC CCCGGCCACC
601 GCCGACCCGT CCTGCTTCCT GAAGCTCCCC CTCGCCGCCG GTGACGTGCC CTACCTGCGG
661 GCCATCCAGG CACACCTCAA CGACGCGGTC CGGCGGGCCG CCGAGGAGAC CGGAGCCACC
721 TACGTGGACT TCTCCGGGGT GTCCGACGGC CACGACGCCT GCGAGGCCCC CGGCACCCGC
781 TGGATCGAAC CGCTGCTCTT CGGGCACAGC CTCGTTCCCG TCCACCCCAA CGCCCTGGGC
841 GAGCGGCGCA TGGCCGAGCA CACGATGGAC GTCCTCGGCC TGGACTGA
```

Figure 12

```
  1  TCAGTCCAGG CCGAGGACGT CCATCGTGTG CTCGGCCATG CGCCGCTCGC CCAGGGCGTT
 61  GGGGTGGACG GGAACGAGGC TGTGCCCGAA GAGCAGCGGT TCGATCCAGC GGGTGCCGGG
121  GGCCTCGCAG GCGTCGTGGC CGTCGGACAC CCCGGAGAAG TCCACGTAGG TGGCTCCGGT
181  CTCCTCGGCG GCCCGCCGGA CCGCGTCGTT GAGGTGTGCC TGGATGGCCC GCAGGTAGGG
241  CACGTCACCG GCGGCGAGGG GGAGCTTCAG GAAGCAGGAC GGGTCGGCGG TGGCCGGGGT
301  GATCCACGGG TAGCCGAGAG CCGCCACCCT GGCGTGGGGA GCCCTGGCGC GGACGCCGAG
361  CAGCGCCTCC TTGAGCGCGG GGTACGTGTT GGCCTCGATC TCGTCGTCGA AGGAGGTGCC
421  GTGCCTGTCC TTGCAGGGGC TGCCCTTGCC GCCGCTGAGG ACACCCGCCG TGCCGCAGGC
481  CGTGATGGCG TTGATGAAGG TGCTGTTGTC GTTGCCGCCG ATGGTGAGCG TGACCAGGTC
541  CGTGCCGGTG CCGAGCGCGT CCAACTGGGG TGCGACGCCC GGGTACTGGG CCCGCGTGAA
601  GTCGGCGGTC TGCGCGGCGC CGCAGGTGAC GTCCGTGAGG CGGGCGCCCG TCGTGTCCGC
661  GATGACGTGG GGGTAGTTGG CCGTCGAGCG CAGACAGAGC AGGTTGGCGG GGTCGACGGG
721  CAGGACGCCG GAGCCGGCGC TGTAGCTGTC GCCGAGGGCG ACGTAGTCCA GGGTCGGAGT
781  GGCCTGGGCG GGCGCGGCGT GGGCGGTGGC GTCGGTGAGG CCGAGGGCGA GCGTGCCGAC
841  GGCGGCGACT GTCGCGGTCA TGACACGGCG AAGGGCAGGC TTCGGCAT
```

Figure 13

```
  1 ATGGATTACG AGAAGTTTCT GTTATTTGGG GATTCCATTA CTGAATTTGC TTTTAATACT
 61 AGGCCCATTG AAGATGGCAA AGATCAGTAT GCTCTTGGAG CCGCATTAGT CAACGAATAT
121 ACGAGAAAAA TGGATATTCT TCAAAGAGGG TTCAAAGGGT ACACTTCTAG ATGGGCGTTG
181 AAAATACTTC CTGAGATTTT AAAGCATGAA TCCAATATTG TCATGGCCAC AATATTTTTG
241 GGTGCCAACG ATGCATGCTC AGCAGGTCCC CAAAGTGTCC CCCTCCCCGA ATTTATCGAT
301 AATATTCGTC AAATGGTATC TTTGATGAAG TCTTACCATA TCCGTCCTAT TATAATAGGA
361 CCGGGGCTAG TAGATAGAGA GAAGTGGGAA AAAGAAAAAT CTGAAGAAAT AGCTCTCGGA
421 TACTTCCGTA CCAACGAGAA CTTTGCCATT TATTCCGATG CCTTAGCAAA ACTAGCCAAT
481 GAGGAAAAAG TTCCCTTCGT GGCTTTGAAT AAGGCGTTTC AACAGGAAGG TGGTGATGCT
541 TGGCAACAAC TGCTAACAGA TGGACTGCAC TTTTCCGGAA AAGGGTACAA AATTTTTCAT
601 GACGAATTAT TGAAGGTCAT TGAGACATTC TACCCCCAAT ATCATCCCAA AAACATGCAG
661 TACAAACTGA AAGATTGGAG AGATGTGCTA GATGATGGAT CTAACATAAT GTCTTGA
```

Figure 14

(SEQ ID No. 12)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

Figure 15

(SEQ ID No. 13)

```
atgaacctgc gtcaatggat gggcgccgcc acggctgccc ttgccttggg cttggccgcg      60
tgcgggggcg gtgggaccga ccagagcggc aatcccaatg tcgccaaggt gcagcgcatg     120
gtggtgttcg gcgacagcct gagcgatatc ggcacctaca cccccgtcgc gcaggcggtg     180
ggcggcggca agttcaccac caacccgggc ccgatctggg ccgagaccgt ggccgcgcaa     240
ctgggcgtga cgctcacgcc ggcggtgatg ggctacgcca cctccgtgca gaattgcccc     300
aaggccggct gcttcgacta tgcgcagggc ggctcgcgcg tgaccgatcc gaacggcatc     360
ggccacaacg gcggcgcggg ggcgctgacc tacccggttc agcagcagct cgccaacttc     420
tacgcggcca gcaacaacac attcaacggc aataacgatg tcgtcttcgt gctggccggc     480
agcaacgaca ttttcttctg gaccactgcg gcggccacca gcggctccgg cgtgacgccc     540
gccattgcca cggcccaggt gcagcaggcc gcgacggacc tggtcggcta tgtcaaggac     600
atgatcgcca agggtgcgac gcaggtctac gtgttcaacc tgcccgacag cagcctgacg     660
ccggacggcg tggcaagcgg cacgaccggc caggcgctgc tgcacgcgct ggtgggcacg     720
ttcaacacga cgctgcaaag cgggctggcc ggcacctcgg cgcgcatcat cgacttcaac     780
gcacaactga ccgcggcgat ccagaatggc gcctcgttcg gcttcgccaa caccagcgcc     840
cgggcctgcg acgccaccaa gatcaatgcc ctggtgccga gcgccggcgg cagctcgctg     900
ttctgctcgg ccaacacgct ggtggcttcc ggtgcggacc agagctacct gttcgccgac     960
ggcgtgcacc cgaccacggc cggccatcgc ctgatcgcca gcaacgtgct ggcgcgcctg    1020
ctggcggata acgtcgcgca ctga                                           1044
```

Figure 16 (SEQ ID No. 20)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalraggal glrvpadpdq pwpplpprgt ldvrrddvhw areylvpwig rrlrgessgd
241 hvtakgtlsp daiktriaav a
```

Figure 17 (SEQ ID No. 21)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

Figure 18
(SEQ ID No. 22)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltgaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrfarqhllp wigrrltgrs
241 sgdglpakrp dllpyedpar
```

Figure 19 (SEQ ID No. 23)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cgggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggaccccgag
601 tggcacgcgc cgatcccggc gacgccgccg ccggggtggg tgacgcgcag gaccgcggac
661 gtccggttcg cccggcagca cctgctgccc tggataggcc gcaggctgac cgggcgctcg
721 tccggggacg gcctgccggc caagcgcccg gacctgctgc cctacgagga ccccgcacgg
781 tga
```

Figure 20 (SEQ ID No. 24)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprrmrsd
421 ydsgdhlhpg dkgyarmgav idlaalkgaa pvka
```

Figure 21 (SEQ ID No. 25)

```
   1 atgacccggg gtcgtgacgg gggtgcgggg gcgcccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gcccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgccccgc
 841 tacagcgtcg tcaacgaggg catcagcggc aaccggctcc tgaccagcag gccgggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcaccccggc gacaaggggt acgcgcgcat gggcgcggtc
1321 atcgacctgg ccgcgctgaa gggcgcggcg ccggtcaagg cgtag
```

## Figure 22 (SEQ ID No. 26)

```
  1 mtsmsrarva rriaagaayg gggiglagaa avglvvaevq larrrvgvgt ptrvpnaggl
 61 yggtlptagd pplrlmmlgd staagggvhr agqtpgalla sglaavaerp vrlgsvaqpg
121 acsddldrqv alvlaepdrv pdicvimvga ndvthrmpat rsvrhlssav rrlrtagaev
181 vvgtcpdlgt iervrqplrw larrasrqla aaqtigaveq ggrtvslgdl lgpefagnpr
241 elfgpdnyhp saegyataam avlpsvcaal glwpadeehp dalrregflp varaaaeaas
301 eagtevaaam ptgprgpwal lkrrrrrrvs eaepsspsgv
```

## Figure 23 (SEQ ID No. 27)

```
   1 atgacgagca tgtcgagggc gagggtggcg cggcggatcg cggccggcgc ggcgtacggc
  61 ggcggcggca tcggcctggc gggagcggcg gcggtcggtc tggtggtggc cgaggtgcag
 121 ctggccagac gcagggtggg ggtgggcacg ccgacccggg tgccgaacgc gcagggactg
 181 tacggcggca ccctgcccac ggccggcgac ccgccgctgc ggctgatgat gctgggcgac
 241 tccacggccg ccgggcaggg cgtgcaccgg gccgggcaga cgccgggcgc gctgctggcg
 301 tccgggctcg cggcggtggc ggagcggccg gtgcggctgg ggtcggtcgc ccagccgggg
 361 gcgtgctcgg acgacctgga ccggcaggtg gcgctggtgc tcgccgagcc ggaccgggtg
 421 cccgacatct gcgtgatcat ggtcggcgcc aacgacgtca cccaccggat gccggcgacc
 481 cgctcggtgc ggcacctgtc ctcggcggta cggcggctgc gcacggccgg tgcggaggtg
 541 gtggtcggca cctgtccgga cctgggcacg atcgagcggg tgcggcagcc gctgcgctgg
 601 ctggcccggc gggcctcacg gcagctcgcg gcggcacaga ccatcggcgc cgtcgagcag
 661 ggcgggcgca cggtgtcgct gggcgacctg ctgggtccgg agttcgcgca gaacccgcgg
 721 gagctcttcg gccccgacaa ctaccacccc tccgccgagg ggtacgccac ggccgcgatg
 781 gcggtactgc cctcggtgtg cgccgcgctc ggcctgtggc cggccgacga ggagcacccg
 841 gacgcgctgc gccgcgaggg cttcctgccg gtggcgcgcg cggcggcgga ggcggcgtcc
 901 gaggcgggta cggaggtcgc cgccgccatg cctacggggc ctcgggggcc ctgggcgctg
 961 ctgaagcgcc ggagacggcg tcgggtgtcg gaggcggaac cgtccagccc gtccggcgtt
1021 tga
```

# Figure 24 (SEQ ID No. 28)

```
  1 mgrgtdgrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

# Figure 25 (SEQ ID No. 29)

```
  1 atgggtcgag ggacggacca gcggacgcgg tacggccgtc gccgggcgcg tgtcgcgctc
 61 gccgccctga ccgccgccgt cctgggcgtg ggcgtggcgg gctgcgactc cgtgggcggc
121 gactcacccg ctccttccgg cagcccgtcg aagcggacga ggacggcgcc cgcctgggac
181 accagcccgg cgtccgtcgc cgccgtgggc gactccatca cgcgcggctt cgacgcctgt
241 gcggtgctgt cggactgccc ggaggtgtcg tgggcgaccg gcagcagcgc gaaggtcgac
301 tcgctggccg tacggctgct ggggaaggcg gacgcggccg agcacagctg gaactacgcg
361 gtcaccgggg cccggatggc ggacctgacc gctcaggtga cgcgggcggc gcagcgcgag
421 ccggagctgg tggcggtgat ggccggggcg aacgacgcgt gccggtccac gacctcggcg
481 atgacgccgg tggcggactt ccgggcgcag ttcgaggagg cgatggccac cctgcgcaag
541 aagctcccca aggcgcaggt gtacgtgtcg agcatcccgg acctcaagcg gctctggtcc
601 cagggccgca ccaacccgct gggcaagcag gtgtggaagc tcggcctgtg cccgtcgatg
661 ctgggcgacg cggactccct ggactcggcg gcgaccctgc ggcgcaacac ggtgcgcgac
721 cgggtggcgg actacaacga ggtgctgcgg gaggtctgcg cgaaggaccg gcggtgccgc
781 agcgacgacg gcgcggtgca cgagttccgg ttcggcacgg accagttgag ccactgggac
841 tggttccacc cgagtgtgga cggccaggcc cggctggcgg agatcgccta ccgcgcggtc
901 accgcgaaga atccctga
```

## Figure 26 (SEQ ID No. 30)

```
  1 mrlsrraata salltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

## Figure 27 (SEQ ID No. 31)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc gggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgcccccctg
 841 gctgcacagc gtcacccttc ccgtggagaa ctcctaccac cccacggcca acggacagtc
 901 caagggctac ctgcccgtcc tgaactccgc cacctgatct cgcggctact ccgcccctga
 961 cgaagtcccg cccccgggcg gggcttcgcc gtaggtgcgc gtaccgccgt cgcccgtcgc
1021 gccggtggcc ccgccgtacg tgccgccgcc cccggacgcg gtcggttc
```

Figure 28 (SEQ ID No. 32)

```
1    MKKWFVCLLG LVALTVQAAD SRPAFSRIVM FGDSLSDTGK MYSKMRGYLP
51   SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNQ
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIANQY EFLAH*
```

# Figure 29 (SEQ ID No. 33)

```
   1  ATGAAAAAAT GGTTTGTGTG TTTATTGGGA TTGGTCGCGC TGACAGTTCA
      TACTTTTTTA CCAAACACAC AAATAACCCT AACCAGCGCG ACTGTCAAGT

  51  GGCAGCCGAC AGTCGCCCCG CCTTTTCCCG GATCGTGATG TTCGGCGACA
      CCGTCGGCTG TCAGCGGGGC GGAAAAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAACAGT TCCCGGGTCT GACCATCGCC AACGAAGCGG
      CCTCGTCGAC TGGTTTGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC CTATGGCTGG AACACGGAGC AGGATGCCAA GCGGGTTCGC
      TGATAGACCG GATACCGACC TTGTGCCTCG TCCTACGGTT CGCCCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCTCGCA
      CTATGACGAC AAGTTGGACG GCCTAGACCC GGTCTTGGGC AGTCGAGCGT

 551  GTCAGAAGGT GGTCGAGGCG GTCAGCCATG TCTCCGCCTA TCACAACCAG
      CAGTCTTCCA CCAGCTCCGC CAGTCGGTAC AGAGGCGGAT AGTGTTGGTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGC GAACCAGTAC GAGTTCCTCG
      GGACTCGCTC GCGCGGCGGT GGAAGTAGCG CTTGGTCATG CTCAAGGAGC

1001  CCCAC TGA
      GGGTG ACT
```

Figure 30 (SEQ ID No. 34)

```
  1  MKKWFVCLLG LIALTVQAAD TRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNK
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIETQY EFLAHG*
```

# Figure 31 (SEQ ID No. 35)

```
   1  ATGAAAAAAT GGTTTGTTTG TTTATTGGGG TTGATCGCGC TGACAGTTCA
      TACTTTTTTA CCAAACAAAC AAATAACCCC AACTAGCGCG ACTGTCAAGT

  51  GGCAGCCGAC ACTCGCCCCG CCTTCTCCCG GATCGTGATG TTCGGCGACA
      CCGTCGGCTG TGAGCGGGGC GGAAGAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAGCAGT TCCCGGGTCT GACCATCGCC AACGAAGCGG
      CCTCGTCGAC TGGTTCGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC ATATGGCTGG AATACGGAGC AGGATGCCAA GCGAGTTCGC
      TGATAGACCG TATACCGACC TTATGCCTCG TCCTACGGTT CGCTCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCCCGCA
      CTATGACGAC AAGTTGGACG GCCTAGACCC GGTCTTGGGC AGTCGGGCGT

 551  GTCAGAAGGT GGTCGAGGCG GTCAGCCATG TCTCCGCCTA TCACAACAAG
      CAGTCTTCCA CCAGCTCCGC CAGTCGGTAC AGAGGCGGAT AGTGTTGTTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGA GACCCAGTAC GAGTTCCTCG
      GGACTCGCTC GCGCGGCGGT GGAAGTAGCT CTGGGTCATG CTCAAGGAGC

1001  CCCACGGATG A
      GGGTGCCTAC T
```

Figure 32

```
           1        10        20        30        40        50
           |--------+---------+---------+---------+---------|
satA          ADTRPAFSRIVMFGDSLSDTGKMYSKMRGYLPSSPPYYEGRFSN--G
R.sol      QSGNPNVAKVQRMVVFGDSLSDIGT-------YTPVAQAVGGGKFTTNPG
Consensus  ...adnraafqRiVmFGDSLSDiGk.......YlPsaqaygeGrFsn..G

           51       60        70        80        90        100
           |--------+---------+---------+---------+---------|
satA       PVWLEQLTKQFPGLTIANEAEGGATAVAYNKISWNPKYQVINNLDYEVTQ
R.sol      PIWAETVAAQL-GVTLTPAVMGYATSVQNCPKAGCFDYAQGGSRVTDPNG
Consensus  P!WaEqlaaQl.GlTianaaeGgATaVannkiagnfdYaqgnnrdt#pnq

           101      110       120       130       140       150
           |--------+---------+---------+---------+---------|
satA       FLQKDSFKPDDLVILWVGANDYLAYG--WNTEQDAKRVRDAISDAANRMV
R.sol      IGHNGGAGALTYPVQQQLANFYAASNNTFNGNNDVVFVLAGSNDIFFWTT
Consensus  igqndgagaddlp!qqqgANdYaAsn..fNg##DakrVraainDaanrmt

           151      160       170       180       190       200
           |--------+---------+---------+---------+---------|
satA       LNGAKQILLFNLPDLGQNPSARSQKVVEAVSHVSAYHNKL-LLNLARQLA
R.sol      AAATSGSGVTPAIATAQVQQAATDLVGYVKDMIAKGATQVYVFNLPDSSL
Consensus  aaaakqiglfnaialaQnqqAas#lVgeakdh!aaganql.llNLarqla

           201      210       220       230       240       250
           |--------+---------+---------+---------+---------|
satA       PTGMVKLFEIDKQFAEMLRDPQNFGLSDVENPCYDGGYVWKPFATRSVST
R.sol      TPDGVASGTTGQALLHALVGTFNTTLQSGLAGTSARIIDFNAQLTAAIQN
Consensus  ppdgValgeidqalaeaLrdpqNfgLqdgeagcsargidfnaqaTaa!qn

           251      260       270       280       290       300
           |--------+---------+---------+---------+---------|
satA       DRQLSAFSPQERLAIAG--NPLLAQAVASPM---ARRSASPLNCEGKMFW
R.sol      GASFGFANTSARACDATKINALVPSAGGSSLFCSANTLVASGADQSYLFA
Consensus  daqlgaanpqaRaadAg..NaLlaqAgaSp$...Arrlaapgad#gk$Fa

           301      310       320       330
           |--------+---------+---------|
satA       DQVHPTTVVHAALSERAATFIETQYEFLAH
R.sol      DGVHPTTAGHRLIASNVLARLLA--DNVAH
Consensus  DqVHPTTagHaaiaeraaariea..#nlAH
```

Figure 33

```
                                  ▼
Pfam       *->ivafGDSltdggg...............ayygdsdgggwgagladrltsla..rlrargrgvdv
Sriml  38     YVALGDSYSSGVG.............agSYDSSSGSCKRSTKSYPALWAAS..-----HTGTRF 81
Scoel   5     YVAVGDSFTEG--...............--VGDPGPDGAFVGWADRLAVLL..ADRRPEGDFTY 47
Scoe2  10     LVAVGDSFTEG--...............--MSDLLPDGSYRGWADLLATRM..--AARSPGFRY 50
Scoe3 239     VVAFGDSITDG--.................ARSQSDANHRWTDVLAARLHEAA..GDGRDTPRYSV 283
Scoe4  75     LMMLGDSTAAG--...............------QGVHRAGQTPGALLASG..LAAVAERPVRL 113
Scoe5  66     VAAVGDSITRGFD.............acAVLSDCPEVSWATGSSAKVDSLAvrLLGKADAAEHS 116
Ahyd1  28     IVMFGDSLSDTGKmyskmrgylpssppyYEGRFSNGPVWLEQLTNEFPGLTiaNEAEGGPTAVA 91
Asal1  28     IVMFGDSLSDTGKmyskmrgylpssppyYEGRFSNGPVWLEQLTKQF----.------PGLTI 79
Ahyd2  40     IVMFGDSLSDTGKmyskmrgylpssppyYEGRFSNGPVWLEQLTKQFPGLTiaNEAEGGATAVA 103

Pfam       fnrgisGrtsdGrlvvDarlvatllFlaqflGlnlpPYLsgdflrGANFAsagAtIlgtslipflni
Sriml  82 NFTACSGAR---------------------------------------------------------- 90
Scoel  48 TNLAVRGRL---------------------------------------------------------- 56
Scoe2  51 ANLAVRGKL---------------------------------------------------------- 59
Scoe3 284 VNEGISGNR---------------------------------------------------------- 292
Scoe4 114 GSVAQPGAC---------------------------------------------------------- 122
Scoe5 117 WNYAVTGAR---------------------------------------------------------- 125
Ahyd1   92 YNKISWNPK--------------------------------------------------------- 100
Asal1   80 ANEAEGGAT--------------------------------------------------------- 88
Ahyd2 104 YNKISWNPK---------------------------------------------------------- 112

                                                          ▼
Pfam       QvqFkdfkskvlelrga......lgllqellrlvpvldakspdlvtimiGtNDl...itvakfgpks
Sriml  91 ------------------.......---TGDVLAKQLTPVNSGTDLVSITIGGNDAgfaDTMTTCNLQG 131
Scoel  57 ------------------.......--LDQIVAEQVPRVVGLAPDLVSFAAGGNDI...-----I---- 86
Scoe2  60 ------------------.......--IGQIVDEQVDVAAAMGADVITLVGGLNDT...---------- 88
Scoe3 293 -------LLTSRPGRPA......DNPSGLSRPQRDVLERTNVKAVVVVLGVNDV...----------- 333
Scoe4 123 ------------------......SDDLDRQVALVLAEPDRVPDICVIMVGANDV...----------- 153
Scoe5 126 ------------------.......---MADLTAQVTRAAQREPELVAVMAGANDA...---------CR 155
Ahyd1 101 -------------YQVI......NNLDYEVTQFLQKDSFKPDDLVILWVGANDY...---------LA 137
Asal1   89 -------AVAYNKISWNpkyqvyNNLDYEVTQFLQKDSFKPDDLVILWVGANDY...---------LA 137
Ahyd2 113 -------------YQVI......NNLDYEVTQFLQKDSFKPDDLVILWVGANDY...---------LA 149

Pfam       ......tksdrnvsvpefrdnlrklikrLrsangariiilitlvllnlpl...........plGCl
Sriml 132 esaclarIAKARAYIQQTLPAQLDQVYDAIDSRAPAA-----QVVVLGYP-.............----- 176
Scoel  87 .......---RPGTDPDEVAERFELAVAALT-AAAGTVLVTTGFDTRGVP-.............----- 125
Scoe2  89 .......---------LRPKCDMARVRDLLTQAVERLAPHCEQLVLMRSP-.............----- 122
Scoe3 334 .......LNSPELADRDAILTGLRTLVDRAHARGLRVVGATITPFGGYGG-.............----- 376
Scoe4 154 .......---THRMPATRSVRHLSSAVRRLR-TAGAEVVVGTCPDLGTIE-.............----- 192
Scoe5 156 .......STTSAMTPVADFRAQFEEAMATLR-KKLPKAQVYVSSIPDLKRLwsqgrtnplgkQVWKL 214
Ahyd1 138 .......YGWNTEQDAKRVRDAISDAANRMV-LNGAK-----EILLFNLP-.............----- 174
Asal1 138 .......YGWNTEQDAKRVRDAISDAANRMV-LNGAK-----QILLFNLP-.............----- 174
Ahyd2 150 .......YGWNTEQDAKRVRDAISDAANRMV-LNGAK-----QILLFNLP-.............----- 186

Pfam       pq.klalalassknvdatgclerlneavadynealrelaei.ek.l.q.aqlrkdglpdlkeanvpy
Sriml 177 --.RFYKLGGSCAVGLSEKSRAAINAAADDINAVTAKRA--.--.-.-.-----------ADHGFAF 219
Scoel 126 --.-----------------VLKHLRGKIATYNGHVRAIA--.--.-.-.-----------DRYGCPV 152
Scoe2 123 --.------------GRQGPVLERFRPRMEALFAVIDDLA--.--.-.-.-----------GRHGAVV 154
Scoe3 377 --.YTEARETMRQEVNEEIRSGRVFDTVVDFDKALRDPY--.--.-.-.------------------ 412
Scoe4 193 --.----------------------------RVRQPLRWLaRRaSrQlAAAQTIGAVEQGGRTVSL 227
Scoe5 215 GLcPSMLGDADSLDSAATLRRNTVRDRVADYNEVLREVC--.--.-.AkDRRCRSDDGAVHEFRFGT 273
Ahyd1 175 --.----DLGQNPSARSQKVVEAASHVSAYHNQLLLNLA--.--.-.-.RQLAPTGMVKLFEIDKQF 224
Asal1 175 --.----DLGQNPSARSQKVVEAVSHVSAYHNKLLLNLA--.--.-.-.RQLAPTGMVKLFEIDKQF 224
Ahyd2 187 --.----DLGQNPSARSQKVVEAVSHVSAYHNQLLLNLA--.--.-.-.RQLAPTGMVKLFEIDKQF 236

Pfam       VDlysifqdldgiqnpsayv.y....GFeet.kaCCGyGgr.yNyn.rv.CGnag.l.ck.vtakaC
Sriml 220 GDVNT---------------.-....-----.---------.-TFAgHElCSGAPwL.HS.VT---- 242
Scoel 153 LDLWSLRSVQDRRA------.-....-----.---------.----.--.-----.-.--.------ 166
Scoe2 155 VDLYGAQSLADPRM------.-....-----.---------.----.--.-----.-.--.------ 168
Scoe3 413 --------------------.-....-----.---------.----.--.-----.-.--.------ 413
Scoe4 228 GDLLGPEFAQNPREL-----.-....-----.---------.----.--.-----.-.--.------ 242
```

```
Scoe5  274 DQL-----------------.-....-----.---------.----.--.-----.-.--.------ 276
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303
```

```
                              ▼
Pfam       .dassyll.atlfwDgf.HpsekGykavAeal<-*
Sriml 243 .-------.--LPVENSyHPTANGQSKGYLPV    263
Scoe1 167 .-------.--WDADRL.HLSPEGHTRVALRA    186
Scoe2 169 .-------.--WDVDRL.HLTAEGHRRVAEAV    188
Scoe3 413 .-DPRRMRsDYDSGDHL.HPGDKGYARMGAVI    441
Scoe4 243 .-------.--FGPDNY.HPSAEGYATAAMAV    262
Scoe5 277 .-------.--SHWDWF.HPSVDGQARLAEIA    296
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

# Figure 34

```
                            ▼
Pfam         *->ivafGDSltdggg...............ayygdsdgggwgagladrltsla..rlrargrgvdv
Sriml  38       YVALGDSYSSGVG.............agSYDSSSGSCKRSTKSYPALWAAS..-----HTGTRF 81
Scoe1   5       YVAVGDSFTEG--...............--VGDPGPDGAFVGWADRLAVLL..ADRRPEGDFTY 47
Scoe2  10       LVAVGDSFTEG--...............--MSDLLPDGSYRGWADLLATRM..--AARSPGFRY 50
Ahyd1  28       IVMFGDSLSDTGKmyskmrgylpssppyYEGRFSNGPVWLEQLTNEFPGLTiaNEAEGGPTAVA 91
Asal1  28       IVMFGDSLSDTGKmyskmrgylpssppyYEGRFSNGPVWLEQLTKQF----.------PGLTI 79
Ahyd2  40       IVMFGDSLSDTGKmyskmrgylpssppyYEGRFSNGPVWLEQLTKQFPGLTiaNEAEGGATAVA 103

Pfam         fnrgisGrtsdGrlvvDarlvatllFlaqflGlnlpPYLsgdflrGANFAsagAtIlgtslipflni
Sriml  82 NFTACSGAR---------------------------------------------------------- 90
Scoe1  48 TNLAVRGRL---------------------------------------------------------- 56
Scoe2  51 ANLAVRGKL---------------------------------------------------------- 59
Ahyd1   92 YNKISWNPK---------------------------------------------------------- 100
Asal1   80 ANEAEGGAT---------------------------------------------------------- 88
Ahyd2 104 YNKISWNPK---------------------------------------------------------- 112

                                                                ▼
Pfam         QvqFkdfkskvlelrqa......lgllqellrlvpvldakspdlvtimiGtNDl...itvakfgpks
Sriml  91 -----------------......---TGDVLAKQLTPVNSGTDLVSITIGGNDAgfaDTMTTCNLQG 131
Scoe1  57 -----------------......--LDQIVAEQVPRVVGLAPDLVSFAAGGNDI...-----I---- 86
Scoe2  60 -----------------......--IGQIVDEQVDVAAAMGADVITLVGGLNDT...---------- 88
Ahyd1 101 -------------YQVI......NNLDYEVTQFLQKDSFKPDDLVILWVGANDY...--------LA 137
Asal1   89 -------AVAYNKISWNpkyqvyNNLDYEVTQFLQKDSFKPDDLVILWVGANDY...--------LA 137
Ahyd2 113 -------------YQVI......NNLDYEVTQFLQKDSFKPDDLVILWVGANDY...--------LA 149

Pfam         .......tksdrmvsvpefrdnlrklikrLrsangariiilitlvllnlplplGCl
Sriml 132 esaclarIAKARAYIQQTLPAQLDQVYDAIDSRAPAA-----QVVVLGYP------ 176
Scoe1  87 .......---RPGTDPDEVAERFELAVAALT-AAAGTVLVTTGPDTRGVP------ 125
Scoe2  89 .......---------LRPKCDMARVRDLLTQAVERLAPHCEQLVLMRSP------ 122
Ahyd1 138 .......YGWNTEQDAKRVRDAISDAANRMV-LNGAK-----EILLFNLP------ 174
Asal1 138 .......YGWNTEQDAKRVRDAISDAANRMV-LNGAK-----QILLFNLP------ 174
Ahyd2 150 .......YGWNTEQDAKRVRDAISDAANRMV-LNGAK-----QILLFNLP------ 186

Pfam         pqklalalasskmvdatgclerlneavadynealrelaeieklqaqlrkdglpdlkeanvpy
Sriml 177 --RFYKLGGSCAVGLSEKSRAAINAAADDINAVTAKRA-----------------ADHGFAF 219
Scoe1 126 -----------------VLKHLRGKIATYNGHVRAIA-----------------DRYGCPV 152
Scoe2 123 -------------GRQGPVLERFRPRMEALFAVIDDLA-----------------GRHGAVV 154
Ahyd1 175 ------DLGQNPSARSQKVVEAASHVSAYHNQLLLNLA------RQLAPTGMVKLFEIDKQF 224
Asal1 175 ------DLGQNPSARSQKVVEAVSHVSAYHNKLLLNLA------RQLAPTGMVKLFEIDKQF 224
Ahyd2 187 ------DLGQNPSARSQKVVEAVSHVSAYHNQLLLNLA------RQLAPTGMVKLFEIDKQF 236

Pfam         VDlysifqdldgiqnpsayv.y....GFeet.kaCCGyGgr.yNyn.rv.CGnag.l.ck.vtakaC
Sriml 220 GDVNT--------------.-....-----.---------.-TFAgHElCSGAPwL.HS.VT---- 242
Scoe1 153 LDLWSLRSVQDRRA------.-....-----.---------.----.--.-----.-.--.------ 166
Scoe2 155 VDLYGAQSLADPRM------.-....-----.---------.----.--.-----.-.--.------ 168
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                            ▼
Pfam         .dassyll.atlfwDgf.HpsekGykavAeal<-*
Sriml 243 .--------.--LPVENSyHPTANGQSKGYLPV    263
Scoe1 167 .--------.--WDADRL.HLSPEGHTRVALRA    186
Scoe2 169 .--------.--WDVDRL.HLTAEGHRRVAEAV    188
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

Figure 35

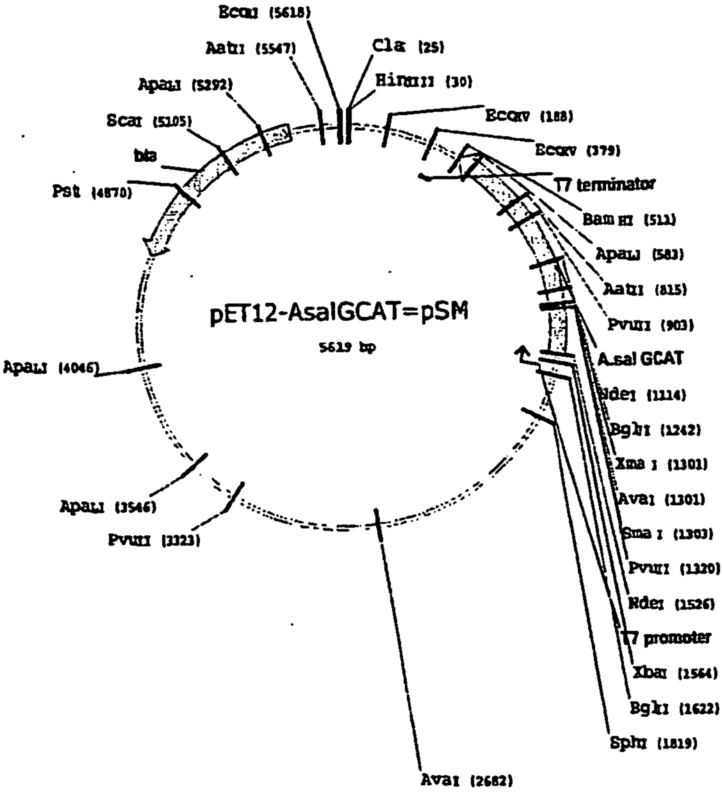
EcoRI (5618)
AatII (5547)
ClaI (25)
HindIII (30)
ApaLI (5292)
ScaI (5105)
bla
PstI (4870)
EcoRV (188)
EcoRV (379)
T7 terminator
BamHI (511)
ApaLI (583)
AatII (815)
PvuII (903)
A.sal GCAT
NdeI (1114)
BglII (1242)
XmaI (1301)
AvaI (1301)
SmaI (1303)
PvuII (1320)
NdeI (1526)
T7 promoter
XbaI (1564)
BglII (1622)
SphI (1819)
pET12-AsalGCAT=pSM
5619 bp
ApaLI (4046)
ApaLI (3546)
PvuII (3323)
AvaI (2682)

Figure 36

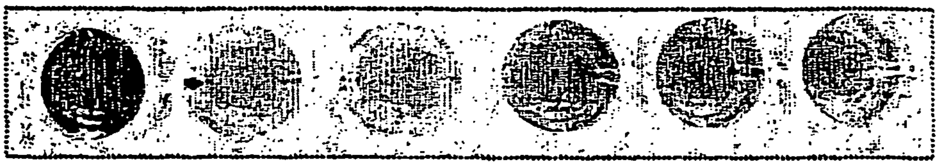

Figure 37

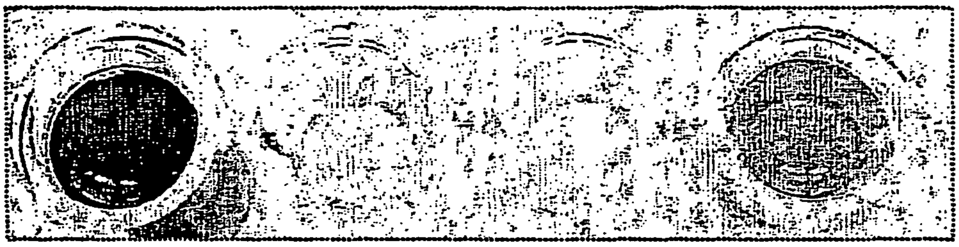

Figure 38

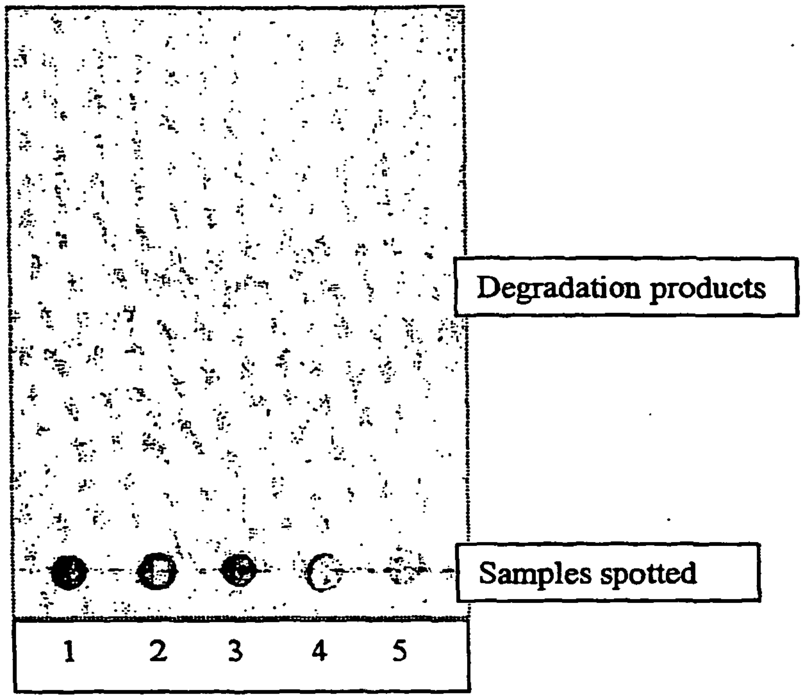

Figure 39

Controls

Pos Neg SE His SE His SE His SE His

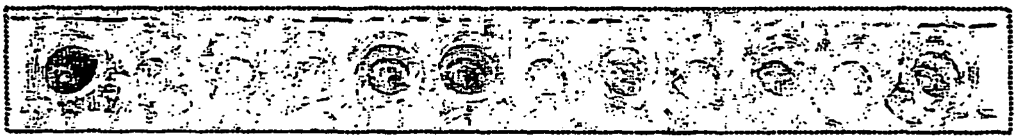

A. hydrophila enzyme

A. salmonicida enzyme

Figure 40.

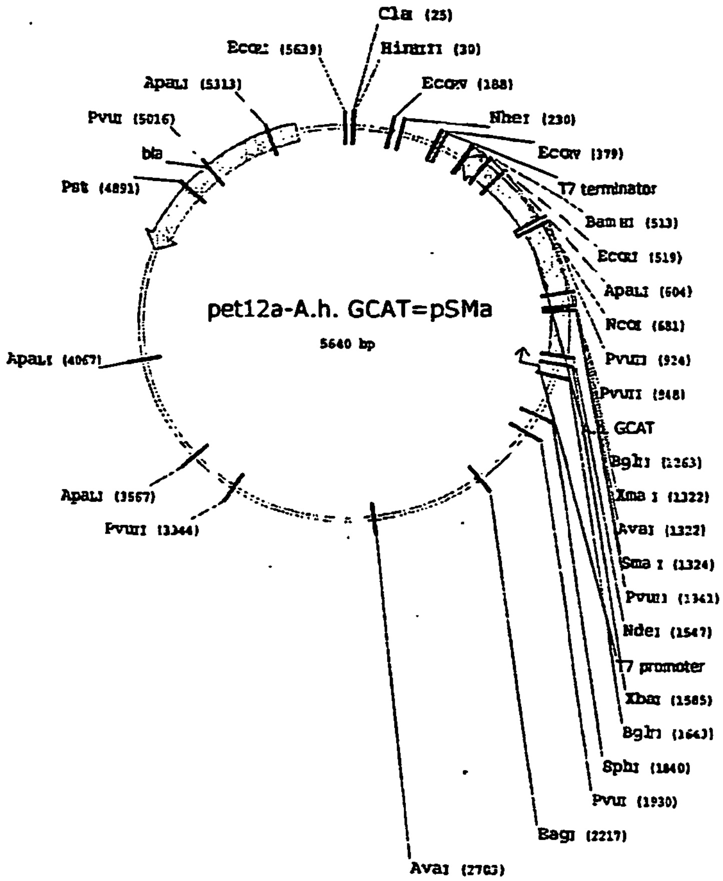
pet12a-A.h. GCAT=pSMa
5640 bp
Cla (25)
HindIII (30)
EcoRV (188)
NheI (230)
EcoRV (379)
T7 terminator
BamHI (513)
EcoRI (519)
ApaLI (604)
NcoI (681)
PvuII (924)
PvuII (948)
GCAT
BglI (1263)
Xma I (1322)
AvaI (1322)
Sma I (1324)
PvuII (1343)
NdeI (1547)
T7 promoter
XbaI (1585)
BglII (1643)
SphI (1840)
PvuII (1930)
EagI (2217)
AvaI (2703)
PvuII (3344)
ApaLI (3567)
ApaLI (4067)
Pst (4891)
bla
PvuI (5016)
ApaLI (5313)
EcoRI (5639)

Figure 41:

CONTROL 5 ul 10 ul

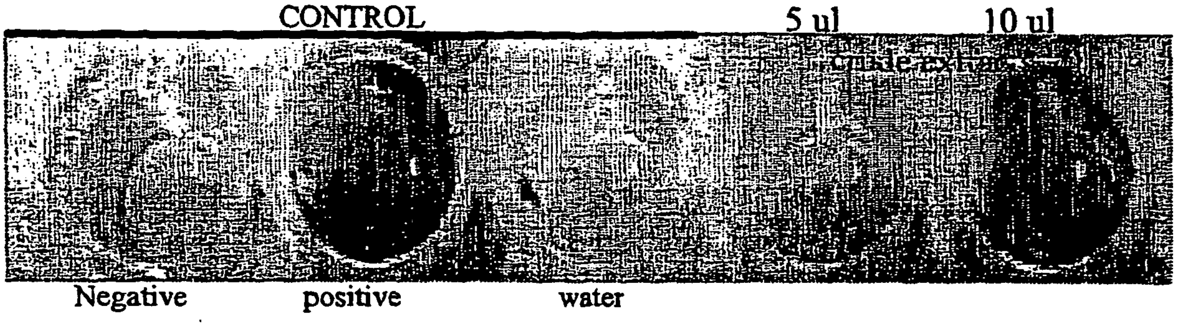

Negative positive water

Figure 42

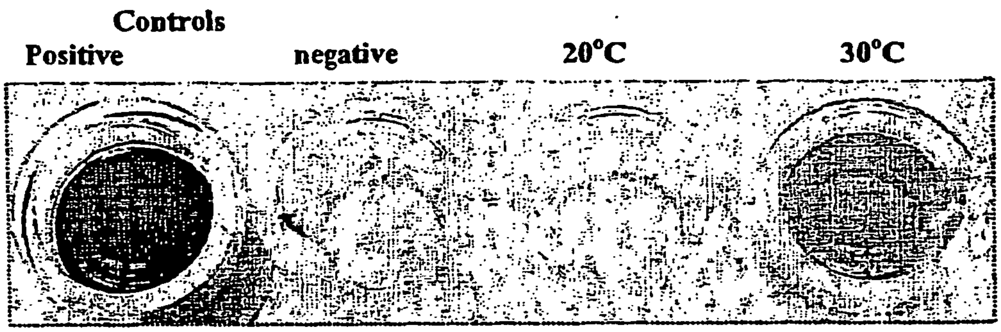

Figure 43

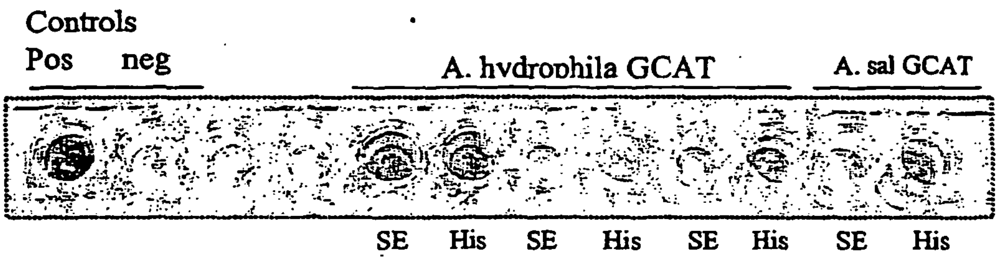
Controls
Pos neg
A. hydrophila GCAT
A. sal GCAT
SE His SE His SE His SE His

Figure 44

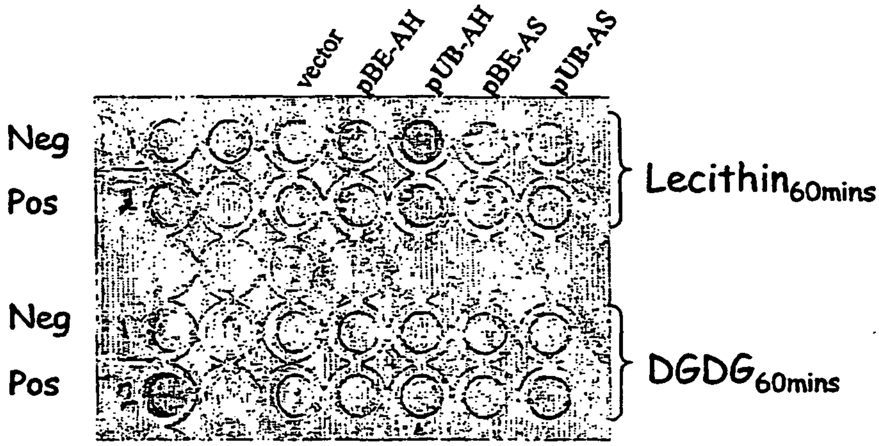
vector
pBE-AH
pUB-AH
pBE-AS
pUB-AS
Neg
Pos
Neg
Pos
Lecithin60mins
DGDG60mins

Figure 45

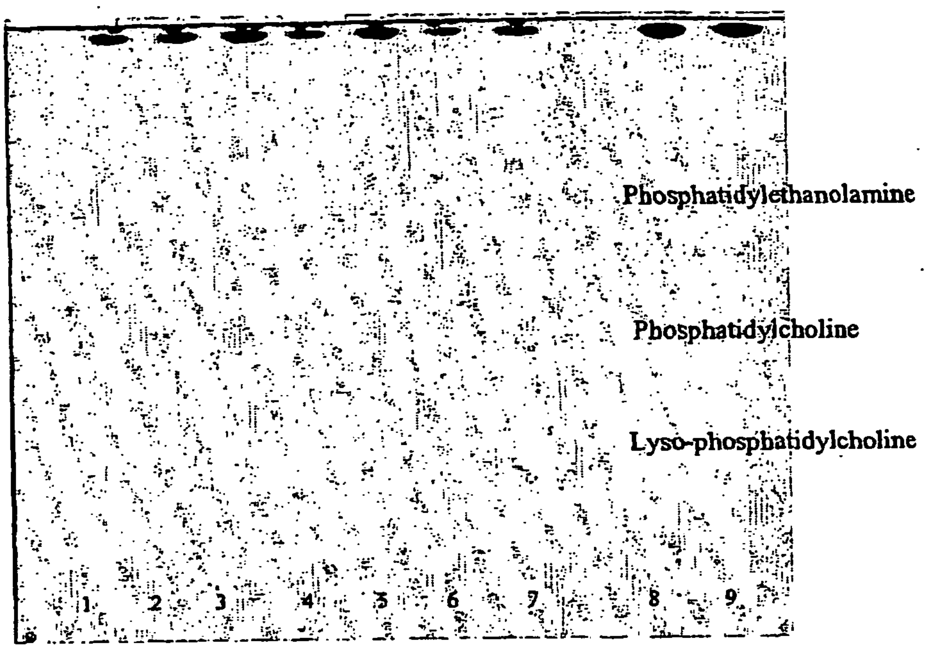
Phosphatidylethanolamine
Phosphatidylcholine
Lyso-phosphatidylcholine
1
2
3
4
5
6
7
8
9

Figure 46

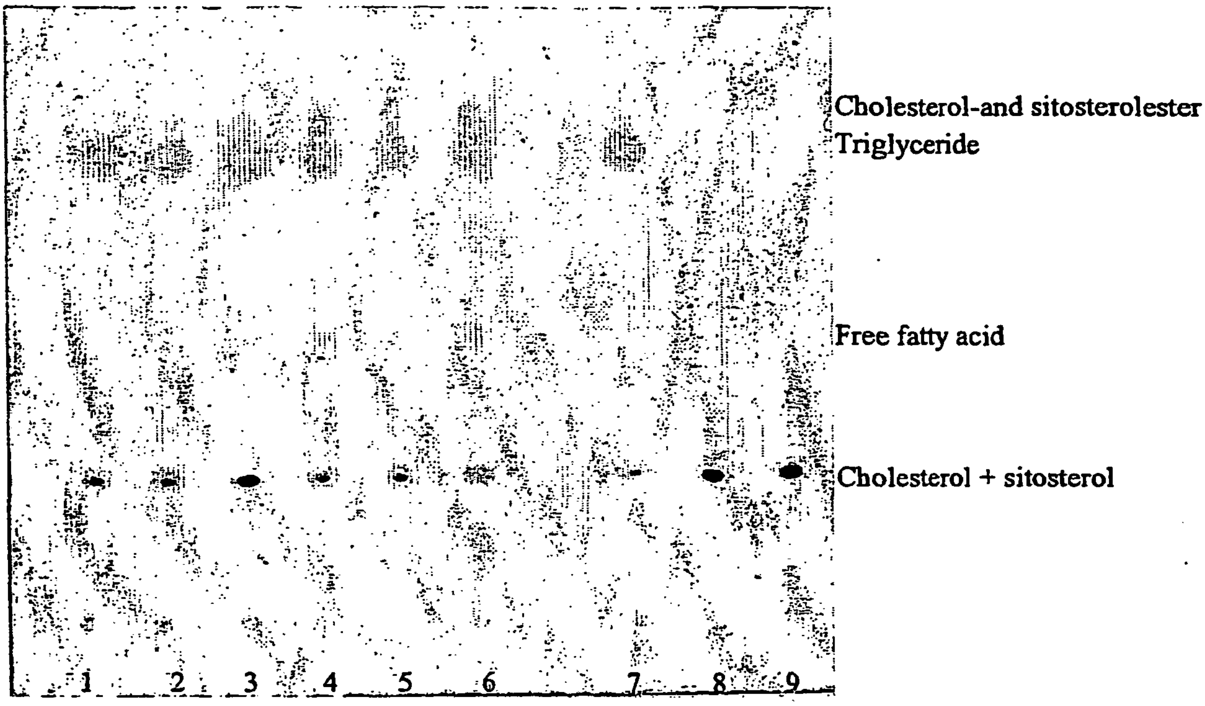
Cholesterol-and sitosterolester
Triglyceride
Free fatty acid
Cholesterol + sitosterol
1
2
3
4
5
6
7
8
9

Figure 47

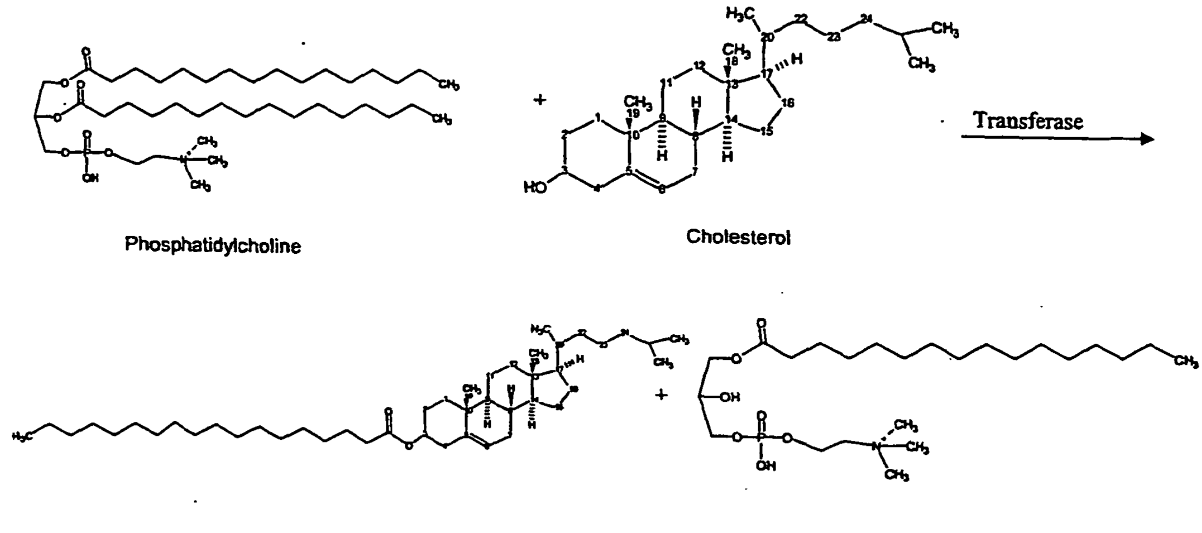


Phosphatidylcholine

Cholesterol

Cholesterolester

Lyso-phosphatidylcholine

Figure 48

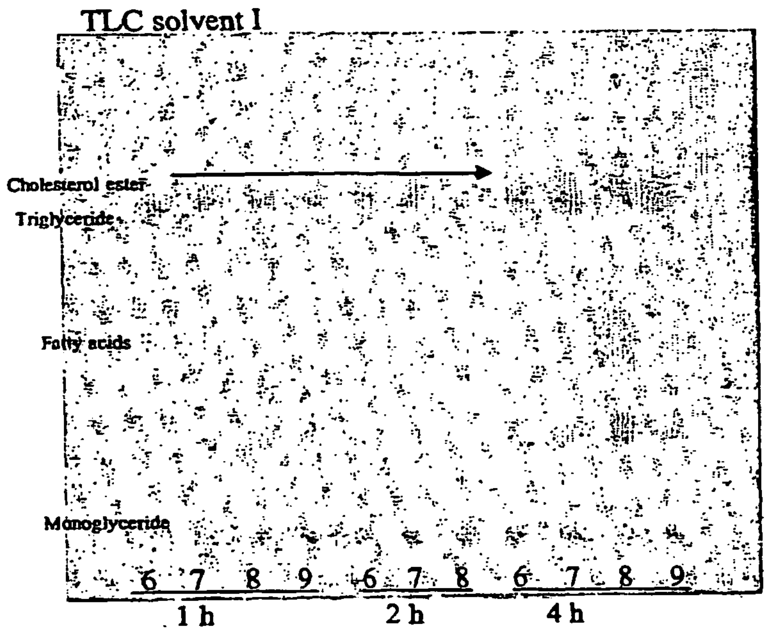
TLC solvent I
Cholesterol ester
Triglyceride
Fatty acids
Monoglyceride
6
7
8
9
6
7
8
6
7
8
9
1 h
2 h
4 h

Figure 49

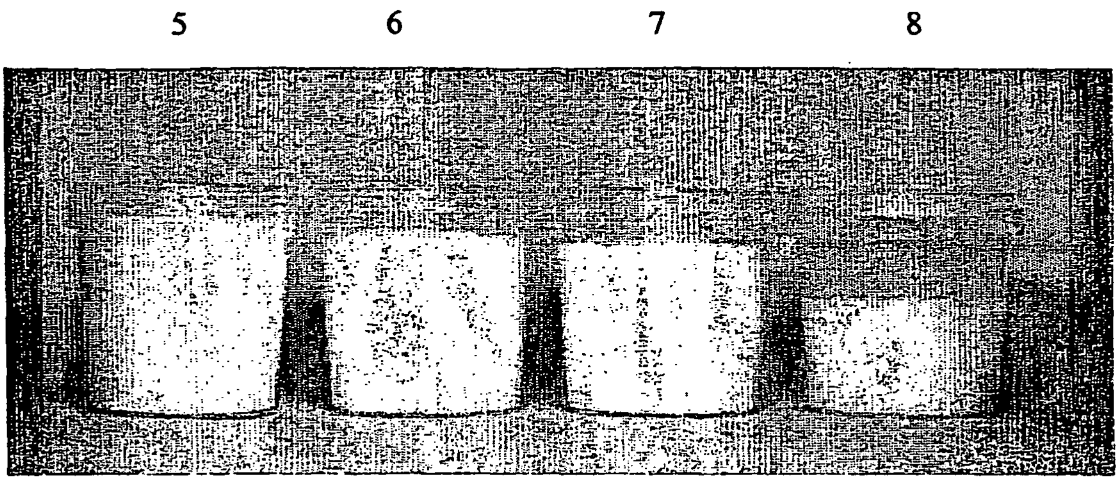

Figure 50

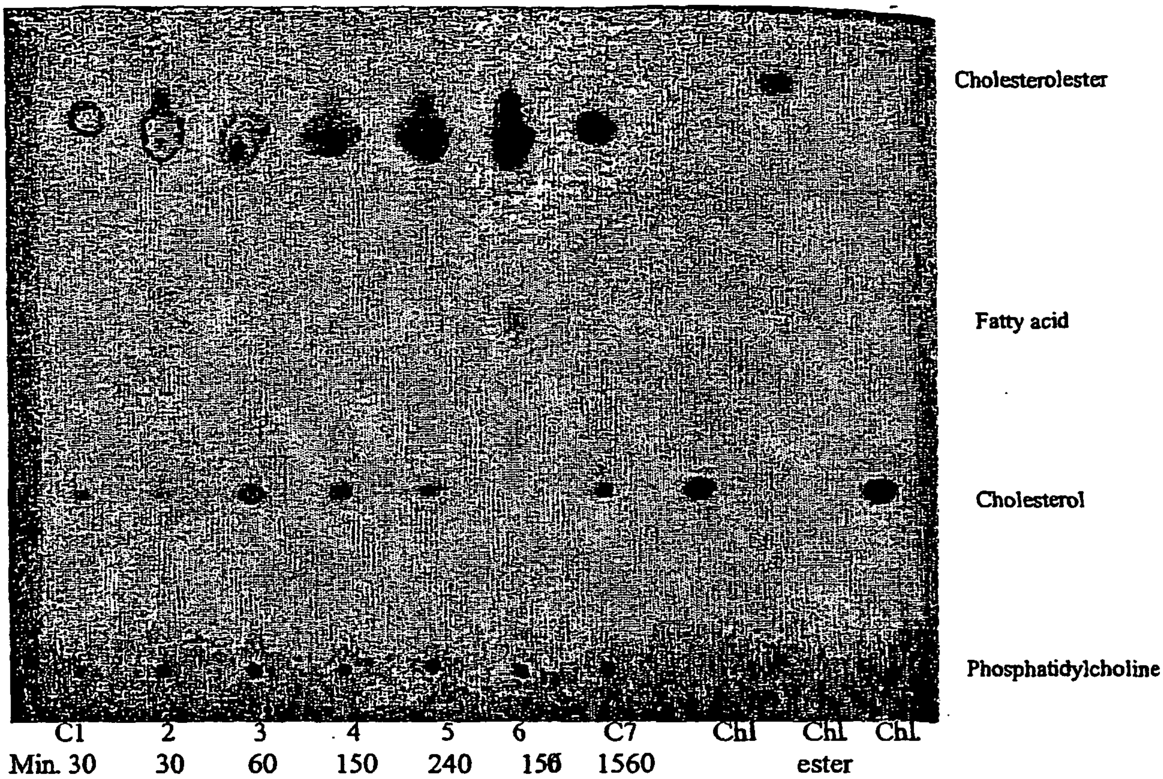
Cholesterolester
Fatty acid
Cholesterol
Phosphatidylcholine
C1 2 3 4 5 6 C7 Chl Chl Chl
Min. 30 30 60 150 240 156 1560 ester

Figure 51

2254-82-5. TLC of egg yolk lipid treated with transferase #135

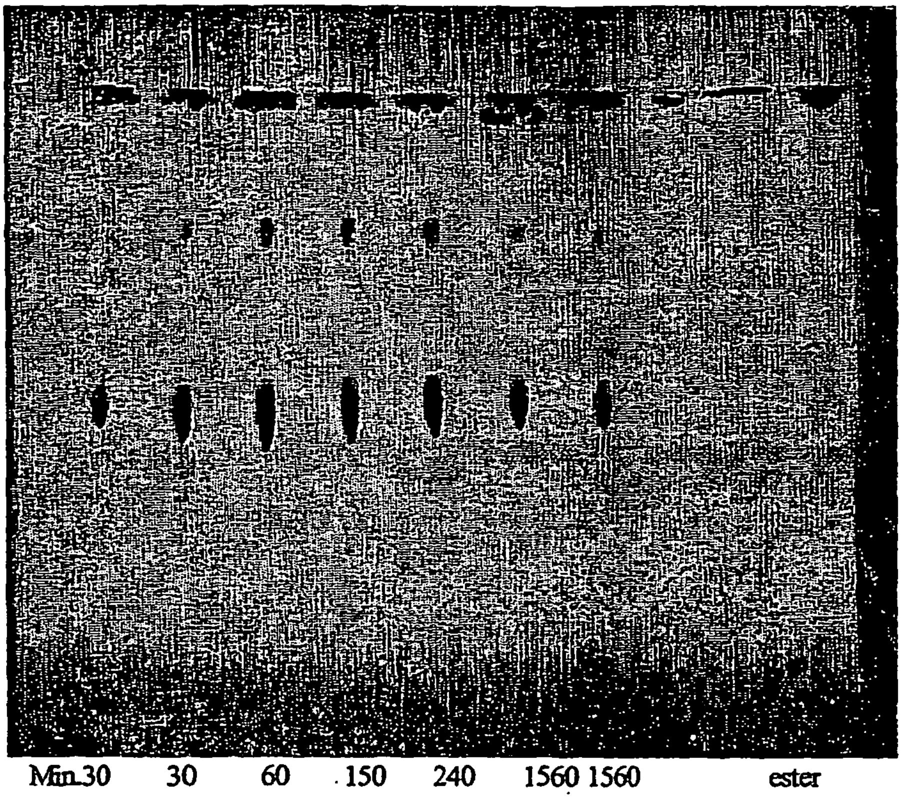

Figure 52

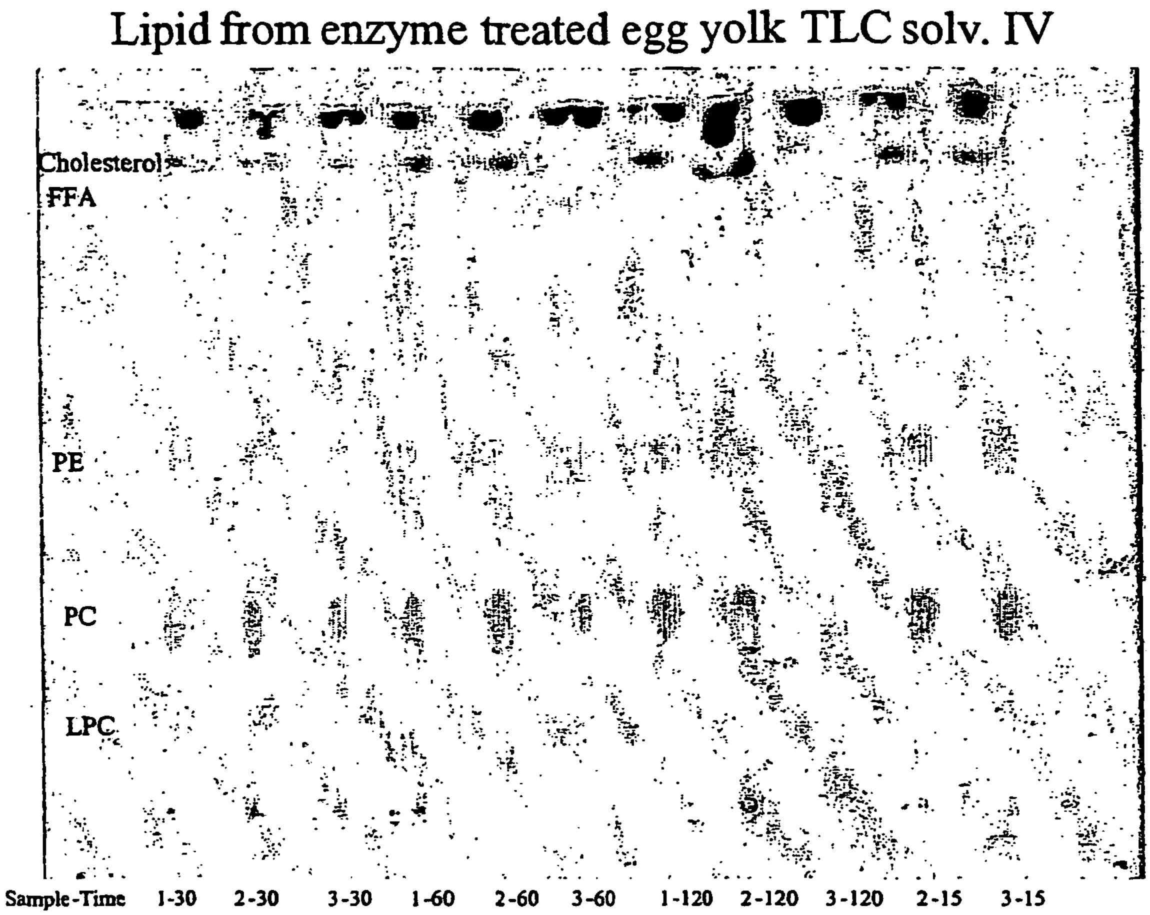
Lipid from enzyme treated egg yolk TLC solv. IV
Cholesterol
FFA
PE
PC
LPC
Sample-Time
1-30
2-30
3-30
1-60
2-60
3-60
1-120
2-120
3-120
2-15
3-15

Figure 53

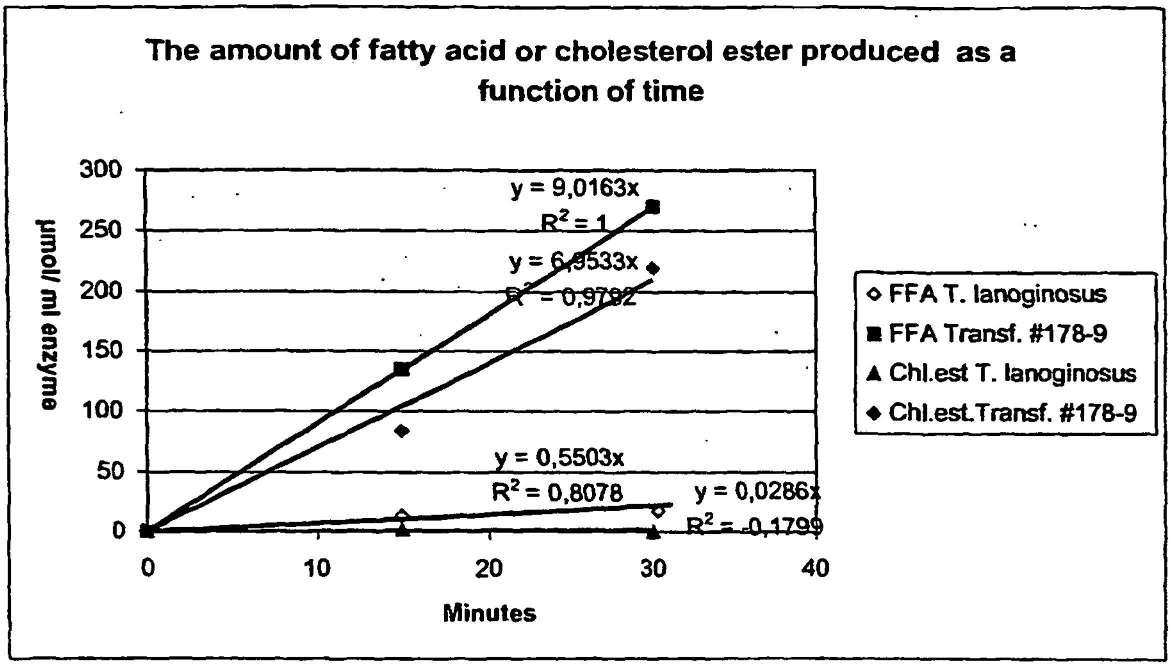
The amount of fatty acid or cholesterol ester produced as a function of time
300
250
200
150
100
50
0
µmol/ ml enzyme
0
10
20
30
40
Minutes
y = 9,0163x
R² = 1
y = 6,9533x
R² = 0,9792
y = 0,5503x
R² = 0,8078
y = 0,0286x
R² = -0,1799
FFA T. lanoginosus
FFA Transf. #178-9
Chl.est T. lanoginosus
Chl.est.Transf. #178-9

Figure 54

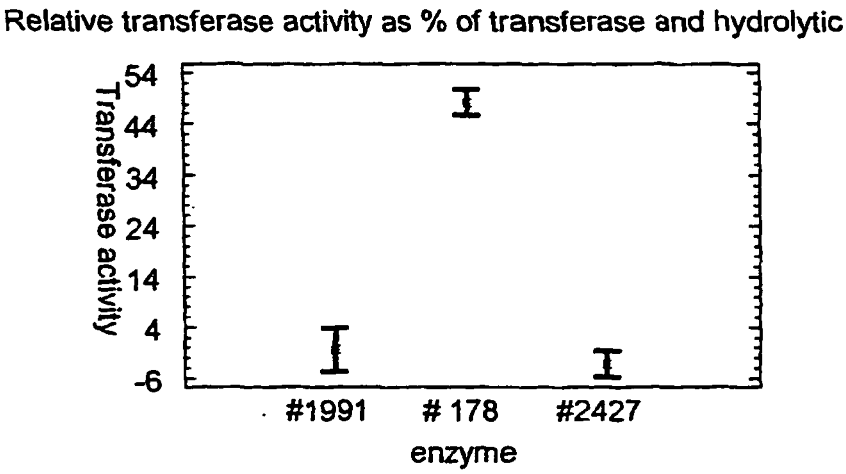
Relative transferase activity as % of transferase and hydrolytic
Transferase activity
54
44
34
24
14
4
-6
#1991
# 178
#2427
enzyme

Figure 55

Transferase activity for #178 as a function of % water in the assay.

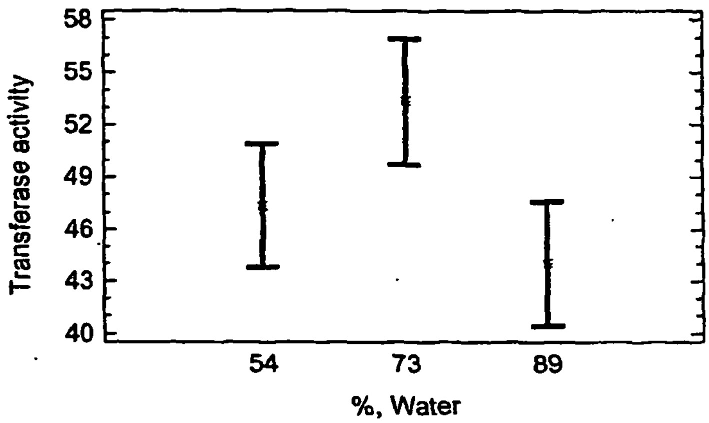

Figure 56

Transferase activity for #178 as a function of reaction time the assay.

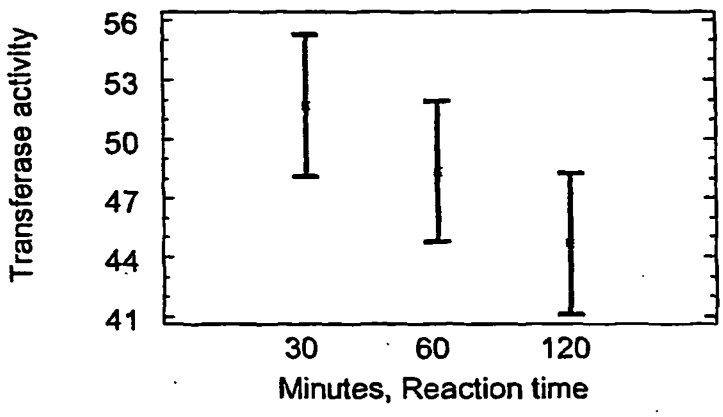

Figure 57

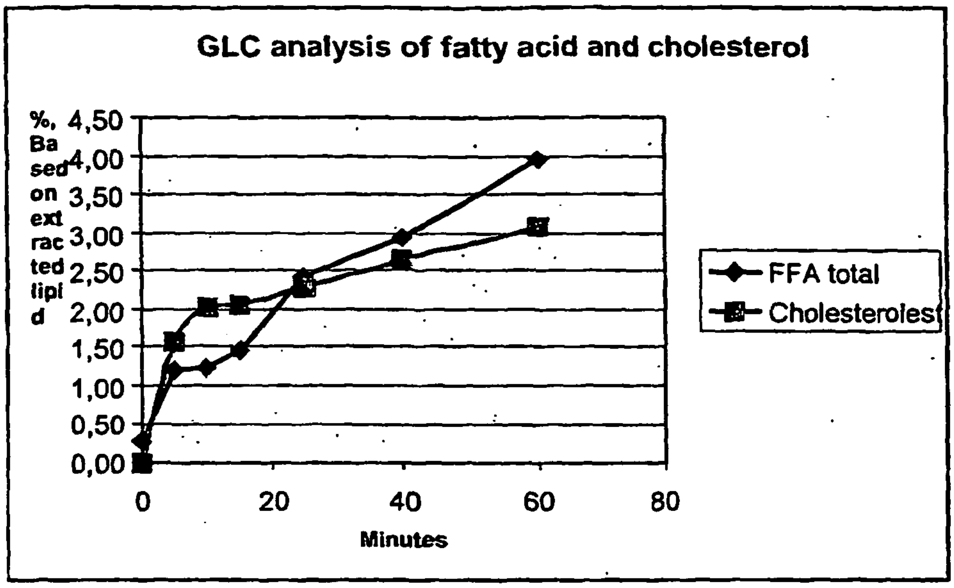
GLC analysis of fatty acid and cholesterol
%, Based on extracted lipid
4,50
4,00
3,50
3,00
2,50
2,00
1,50
1,00
0,50
0,00
0
20
40
60
80
Minutes
FFA total
Cholesterolest

Figure 58

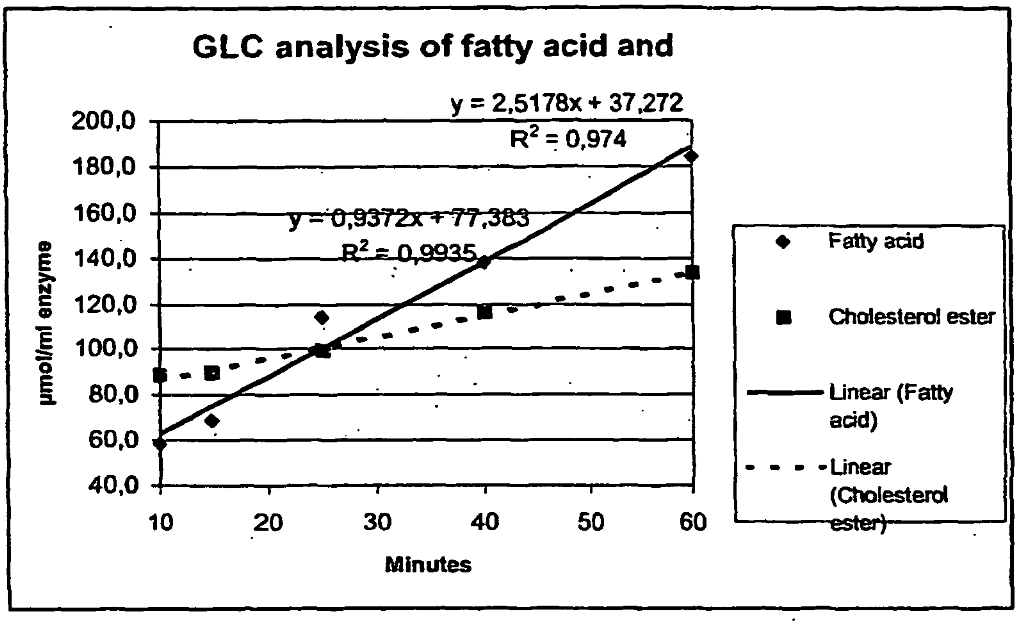
GLC analysis of fatty acid and
y = 2,5178x + 37,272
$R^2$ = 0,974
y = 0,9372x + 77,383
$R^2$ = 0,9935
200,0
180,0
160,0
140,0
120,0
100,0
80,0
60,0
40,0
µmol/ml enzyme
10
20
30
40
50
60
Minutes
Fatty acid
Cholesterol ester
Linear (Fatty acid)
Linear (Cholesterol ester)

Figure 59

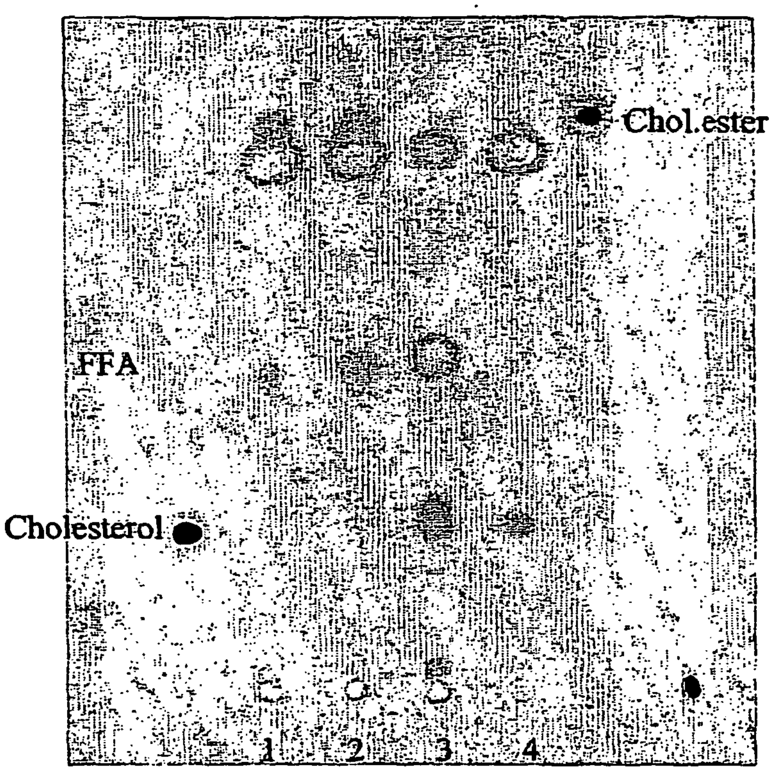

Figure 60

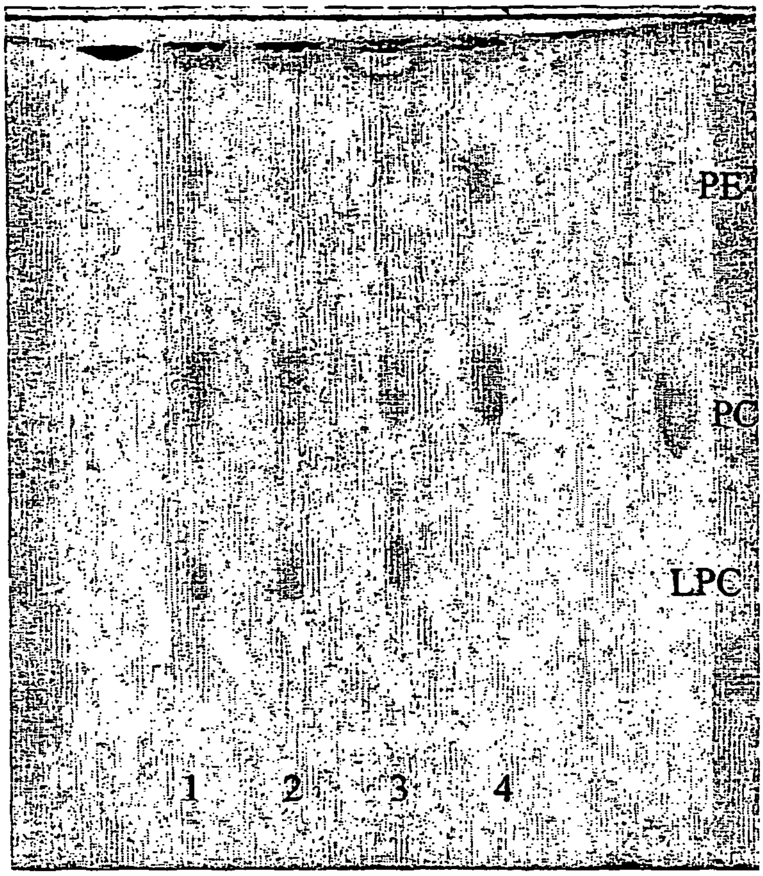

Figure 61

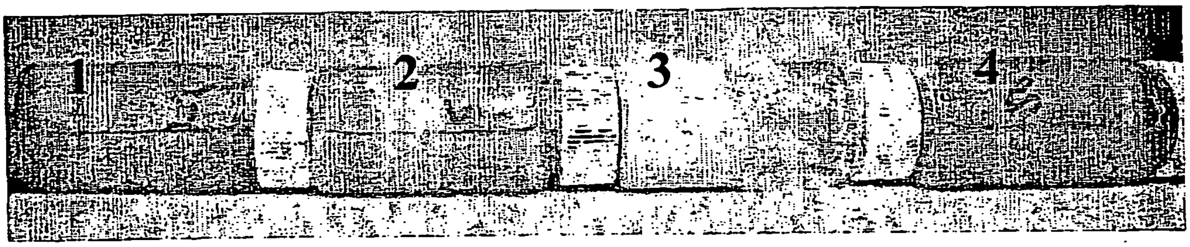

Figure 62

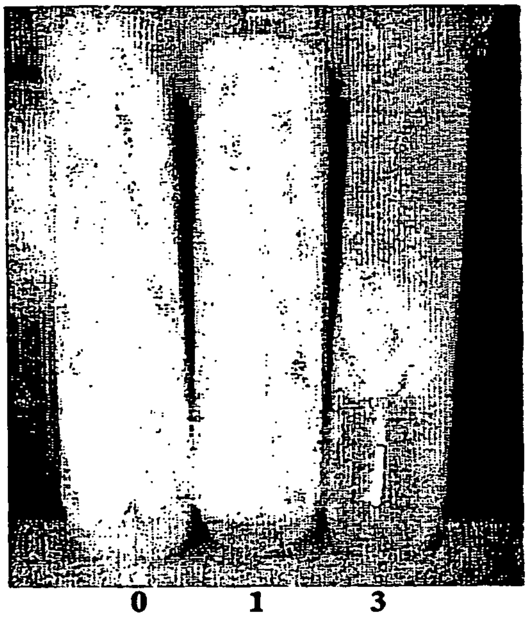

Figure 63

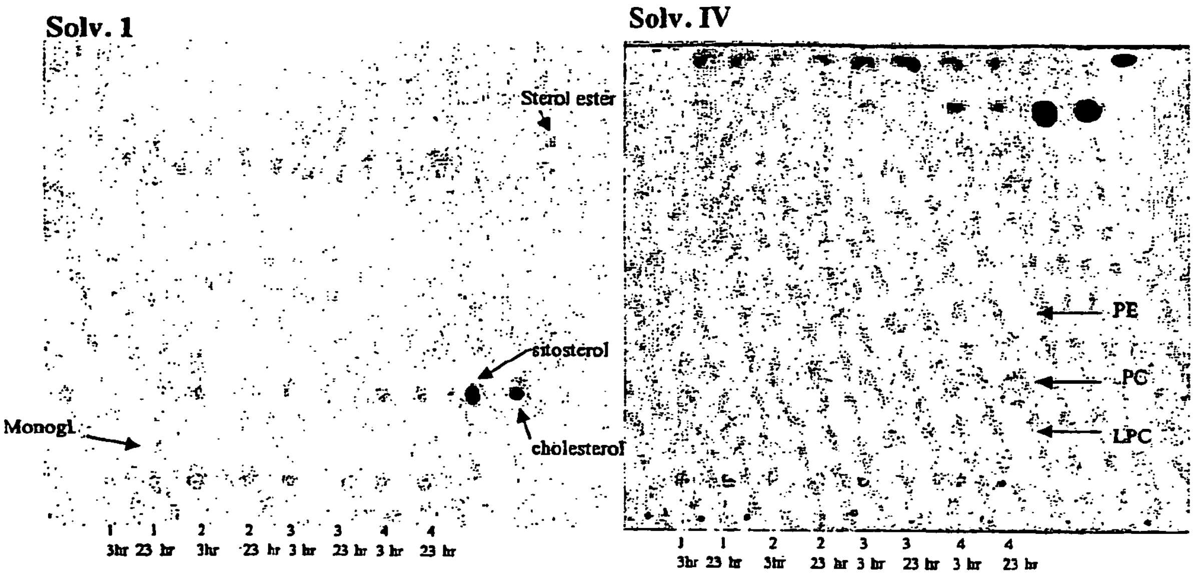
Solv. 1
Sterol ester
sitosterol
Monogl.
cholesterol
1 1 2 2 3 3 4 4
3hr 23 hr 3hr 23 hr 3 hr 23 hr 3 hr 23 hr
Solv. IV
PE
PC
LPC
1 1 2 2 3 3 4 4
3hr 23 hr 3hr 23 hr 3 hr 23 hr 3 hr 23 hr

Figure 64

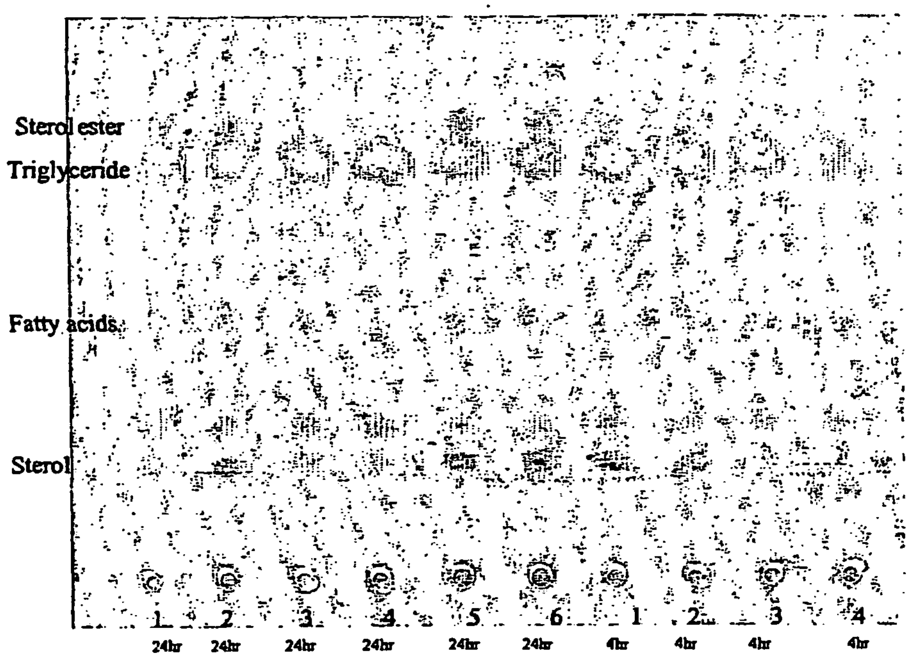
Sterol ester
Triglyceride
Fatty acids
Sterol
1
2
3
4
5
6
1
2
3
4
24hr
24hr
24hr
24hr
24hr
24hr
4hr
4hr
4hr
4hr

Figure 65

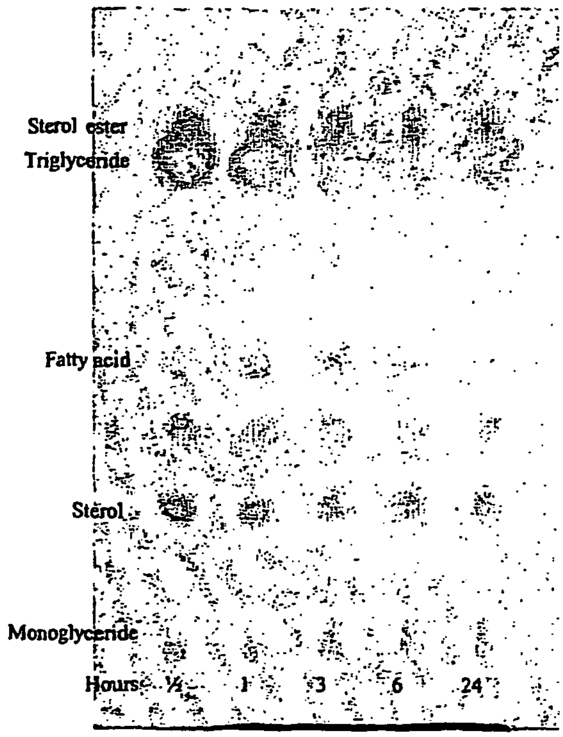
Sterol ester
Triglyceride
Fatty acid
Sterol
Monoglyceride
Hours ½ 1 3 6 24

Figure 66

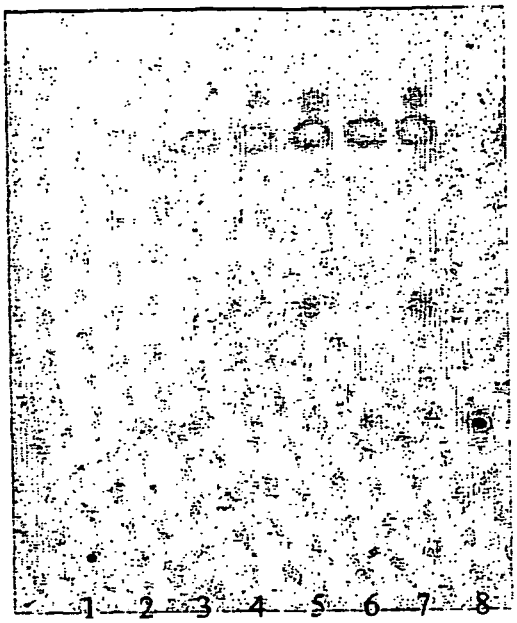

Figure 67

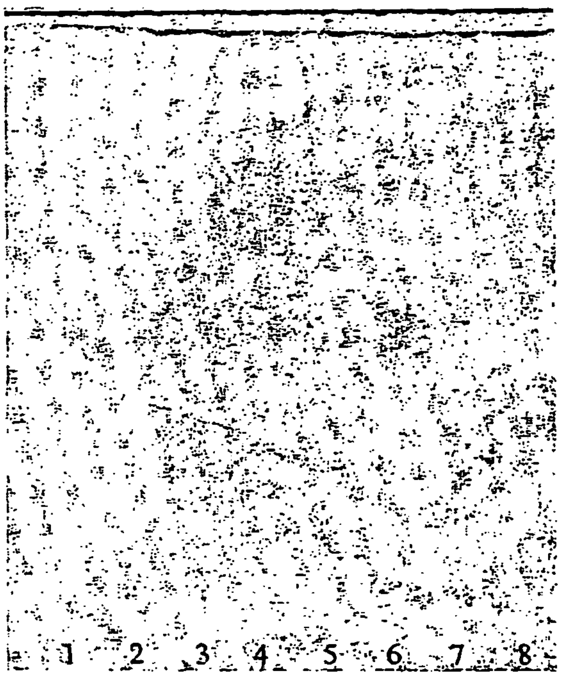

Figure 68

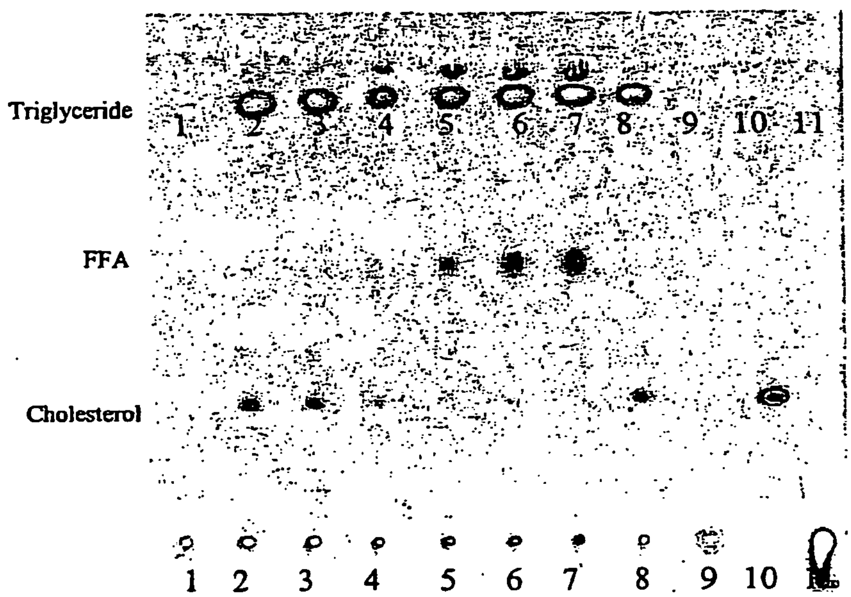
Triglyceride
FFA
Cholesterol
1 2 3 4 5 6 7 8 9 10 11
1 2 3 4 5 6 7 8 9 10

Figure 69

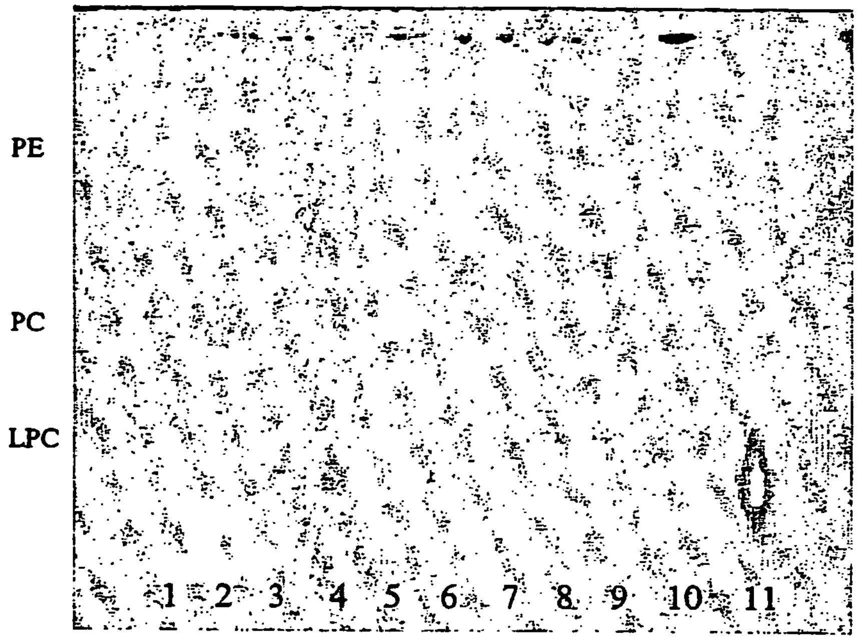

Figure 70

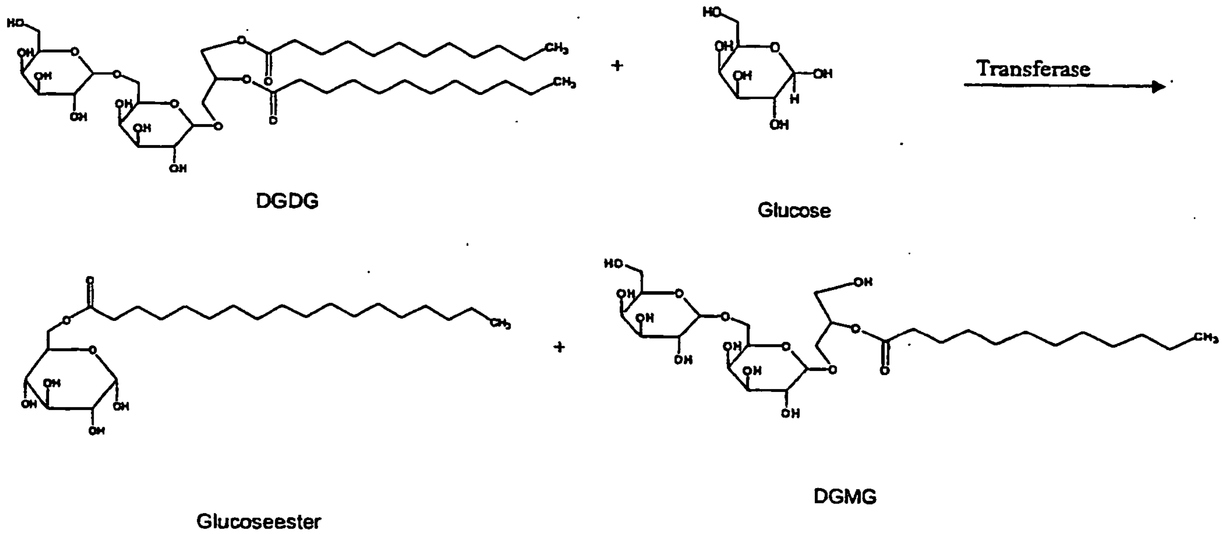


DGDG + Glucose → Glucoseester + DGMG

Figure 71

(SEQ ID No. 36)

```
  1  MFKPKKNFLV GLSAALMSIS LPSATASAAS ADSRPAFSRI VMFGDSLSDT
 51  GKMYSKMRGY LPSSPPYYEG RFSNGPVWLE QLTKQFPGLT IANEAEGGAT
101  AVAYNKISWN PKYQVINNLD YEVTQFLQKD SFKPDDLVIL WVGANDYLAY
151  GWNTEQDAKR VRDAISDAAN RMVLNGAKQI LLFNLPDLGQ NPSARSQKVV
201  EAVSHVSAYH NQLLLNLARQ LAPTGMVKLF EIDKQFAEML RDPQNFGLSD
251  VENPCYDGGY VWKPFATRSV STDRQLSAFS PQERLAIAGN PLLAQAVASP
301  MARRSASPLN CEGKMFWDQV HPTTVVHAAL SERAATFIAN QYEFLAH**
```

Figure 72 (SEQ ID No. 54)

```
   1  ATGTTTAAGT TTAAAAAGAA TTTCTTAGTT GGATTATCGG CAGCTTTAAT
      TACAAATTCA AATTTTTCTT AAAGAATCAA CCTAATAGCC GTCGAAATTA

  51  GAGTATTAGC TTGTTTTCGG CAACCGCCTC TGCAGCTAGC GCCGACAGCC
      CTCATAATCG AACAAAAGCC GTTGGCGGAG ACGTCGATCG CGGCTGTCGG

 101  GTCCCGCCTT TTCCCGGATC GTGATGTTCG GCGACAGCCT CTCCGATACC
      CAGGGCGGAA AAGGGCCTAG CACTACAAGC CGCTGTCGGA GAGGCTATGG

 151  GGCAAAATGT ACAGCAAGAT GCGCGGTTAC CTCCCCTCCA GCCCGCCCTA
      CCGTTTTACA TGTCGTTCTA CGCGCCAATG GAGGGGAGGT CGGGCGGGAT

 201  CTATGAGGGC CGTTTCTCCA ACGGACCCGT CTGGCTGGAG CAGCTGACCA
      GATACTCCCG GCAAAGAGGT TGCCTGGGCA GACCGACCTC GTCGACTGGT

 251  AACAGTTCCC GGGTCTGACC ATCGCCAACG AAGCGGAAGG CGGTGCCACT
      TTGTCAAGGG CCCAGACTGG TAGCGGTTGC TTCGCCTTCC GCCACGGTGA

 301  GCCGTGGCTT ACAACAAGAT CTCCTGGAAT CCCAAGTATC AGGTCATCAA
      CGGCACCGAA TGTTGTTCTA GAGGACCTTA GGGTTCATAG TCCAGTAGTT

 351  CAACCTGGAC TACGAGGTCA CCCAGTTCTT GCAGAAAGAC AGCTTCAAGC
      GTTGGACCTG ATGCTCCAGT GGGTCAAGAA CGTCTTTCTG TCGAAGTTCG

 401  CGGACGATCT GGTGATCCTC TGGGTCGGTG CCAATGACTA TCTGGCCTAT
      GCCTGCTAGA CCACTAGGAG ACCCAGCCAC GGTTACTGAT AGACCGGATA

 451  GGCTGGAACA CGGAGCAGGA TGCCAAGCGG GTTCGCGATG CCATCAGCGA
      CCGACCTTGT GCCTCGTCCT ACGGTTCGCC CAAGCGCTAC GGTAGTCGCT

 501  TGCGGCCAAC CGCATGGTAC TGAACGGTGC CAAGCAGATA CTGCTGTTCA
      ACGCCGGTTG GCGTACCATG ACTTGCCACG GTTCGTCTAT GACGACAAGT

 551  ACCTGCCGGA TCTGGGCCAG AACCCGTCAG CTCGCAGTCA GAAGGTGGTC
      TGGACGGCCT AGACCCGGTC TTGGGCAGTC GAGCGTCAGT CTTCCACCAG

 601  GAGGCGGTCA GCCATGTCTC CGCCTATCAC AACCAGCTGC TGCTGAACCT
      CTCCGCCAGT CGGTACAGAG GCGGATAGTG TTGGTCGACG ACGACTTGGA

 651  GGCACGCCAG CTGGCCCCCA CCGGCATGGT AAAGCTGTTC GAGATCGACA
      CCGTGCGGTC GACCGGGGGT GGCCGTACCA TTTCGACAAG CTCTAGCTGT

 701  AGCAATTTGC CGAGATGCTG CGTGATCCGC AGAACTTCGG CCTGAGCGAC
      TCGTTAAACG GCTCTACGAC GCACTAGGCG TCTTGAAGCC GGACTCGCTG

 751  GTCGAGAACC CCTGCTACGA CGGCGGCTAT GTGTGGAAGC CGTTTGCCAC
      CAGCTCTTGG GGACGATGCT GCCGCCGATA CACACCTTCG GCAAACGGTG

 801  CCGCAGCGTC AGCACCGACC GCCAGCTCTC CGCCTTCAGT CCGCAGGAAC
      GGCGTCGCAG TCGTGGCTGG CGGTCGAGAG GCGGAAGTCA GGCGTCCTTG

 851  GCCTCGCCAT CGCCGGCAAC CCGCTGCTGG CACAGGCCGT TGCCAGTCCT
      CGGAGCGGTA GCGGCCGTTG GGCGACGACC GTGTCCGGCA ACGGTCAGGA

 901  ATGGCCCGCC GCAGCGCCAG CCCCCTCAAC TGTGAGGGCA AGATGTTCTG
      TACCGGGCGG CGTCGCGGTC GGGGGAGTTG ACACTCCCGT TCTACAAGAC

 951  GGATCAGGTA CACCCGACCA CTGTCGTGCA CGCAGCCCTG AGCGAGCGCG
      CCTAGTCCAT GTGGGCTGGT GACAGCACGT GCGTCGGGAC TCGCTCGCGC

1001  CCGCCACCTT CATCGCGAAC CAGTACGAGT TCCTCGCCCA CTGATGA
      GGCGGTGGAA GTAGCGCTTG GTCATGCTCA AGGAGCGGGT GACTACT
```

Figure 73

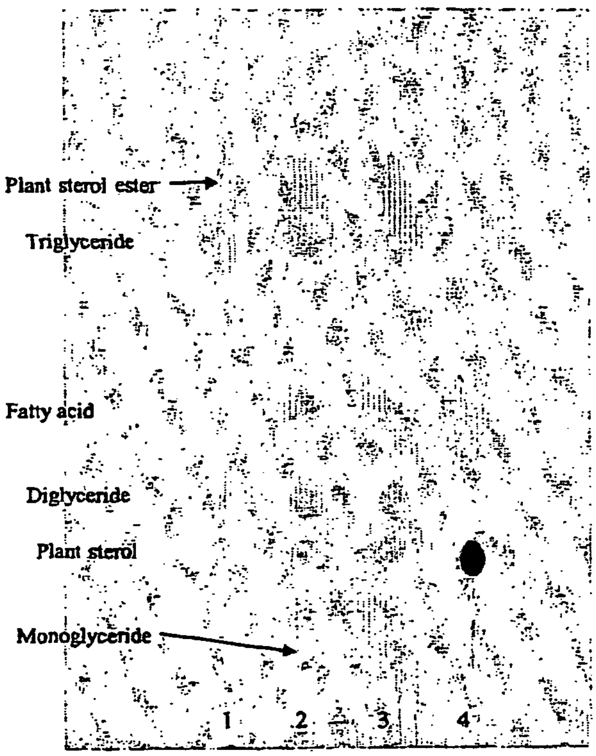
Plant sterol ester
Triglyceride
Fatty acid
Diglyceride
Plant sterol
Monoglyceride
1
2
3
4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0585988 A
- WO 9404035 A
- US 4034124 A
- EP 1193314 A
- EP 0977869 A
- WO 0139602 A
- WO 0032758 A
- WO 02094123 A
- WO 03060112 A
- WO 0005396 A
- WO 03100044 A
- US 5523237 A
- WO 00061771 A
- WO 9299640 A
- EP 0770037 A
- EP 0702712 A
- EP 0746608 A
- WO 0397835 A
- WO 9714713 A
- US 4683202 A
- EP 0583265 A
- EP 0866796 A
- WO 0206457 A
- EP 0752008 A
- EP 1138763 A
- EP 1103606 A
- US 6180406 B
- WO 0134835 A
- WO 0058517 A
- US 6344328 B
- US 6361974 B
- WO 9117243 A
- EP 0238023 A
- EP 0449375 A
- US 5741665 A
- US 5674707 A
- WO 0139544 A
- WO OI16308 A
- US 3817837 A
- US 3850752 A
- US 3939350 A
- US 3996345 A
- US 4277437 A
- US 4275149 A
- US 4366241 A
- US 4816567 A
- US P4959229 A
- US P4581240 A


### Non-patent literature cited in the description


- **Dutihl ; Groger.** *J. Sci. Food Agric.,* 1981, vol. 32, 451-458
- **P.J. Barnes.** Lipid in Cereal Technology. Academic Press, 1983
- **Madsen,J.S. ; Qvist,K.B.** Hydrolysis of milk protein by a Bacillus licheniformis protease specific for acidic amino acid residues. *J. Food Sci.,* 1997, vol. 62, 579-582
- **Buckley.** *Biochemistry,* 1983, vol. 22, 5490-5493
- **Upton ; Buckley.** *TIBS,* 20 May 1995, 178-179
- **Brumlik ; Buckley.** *J. of Bacteriology,* April 1996, 2060-2064
- **Milton et al.** *Biochemistry,* 1990, vol. 29, 9072-9078
- **Brumlik ; Buckley.** *Journal of Bacteriology,* April 1996, vol. 178 (7), 2060-2064
- **Bateman A et al.** *Nucleic Acids Res.,* 2002, vol. 30, 276-280
- **Bateman A ; Haft DH.** *Brief Bioinform,* 2002, vol. 3, 236-245
- **Durbin R ; Eddy S ; Krogh A.** Biological sequence analysis; probabilistic models of proteins and nucleic acids. Cambridge University Press, 1998
- Program Manual for the Wisconsin Package. Genetics Computer Group, 575 Science Drive, Madison, August 1994
- **Needleman ; Wunsch.** *J. of Molecular Biology,* 1970, vol. 48, 443-45
- Separation and Purification Processes for Recovery of Industrial Enzymes. **T. Becker.** Bioseparation Processes in Foods. Marcel Dekker, 427-445
- **Balcao VM ; Paiva AL ; Malcata FX.** *Enzyme Microb Technol.,* 01 May 1996, vol. 18 (6), 392-416
- **Reetz MT ; Jaeger KE.** *Chem Phys Lipids.,* June 1998, vol. 93 (1-2), 3-14

- **Bornscheuer UT ; Bessler C ; Srinivas R ; Krishna SH.** *Trends Biotechnol.,* October 2002, vol. 20 (10), 433-7
- **Andreas Sander ; Eberhard Eilers ; Andrea Heilemann.** Herstellung und Anvendungmöglichkeiten von Eiweiss-Fettsäurekondensaten. 1997, vol. 99, 115-120
- **Beucage S.L. et al.** *Tetrahedron Letters,* 1981, vol. 22, 1859-1869
- **Matthes et al.** *EMBO J.,* 1984, vol. 3, 801-805
- **Saiki R K et al.** *Science,* 1988, vol. 239, 487-491
- **Caruthers MH et al.** *Nuc Acids Res Symp Ser,* 1980, 215-23
- **Hom T et al.** *Nuc Acids Res Symp Ser,* 1980, 225-232
- **Morinaga et al.** *Biotechnology,* 1984, vol. 2, 646-649
- **Nelson ; Long.** *Analytical Biochemistry,* 1989, vol. 180, 147-151
- **Hilton ; Buckley.** *J Biol. Chem.,* 15 January 1991, vol. 266 (2), 997-1000
- **Robertson et al.** *J. Biol. Chem.,* 21 January 1994, vol. 269 (3), 2146-50
- **Brumlik et al.** *J. Bacteriol,* April 1996, vol. 178 (7), 2060-4
- **Peelman et al.** *Protein Sci.,* March 1998, vol. 7 (3), 587-99
- **Devereux et al.** *Nuc. Acids Research,* 1984, vol. 12, 387
- **Ausubel et al.** Short Protocols in Molecular Biology. 1999
- **Altschul et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8, tatiana@ncbi.nlm.nih.gov
- **Higgins DG ; Sharp PM.** *Gene,* 1988, vol. 73 (1), 237-244
- **Simon RJ et al.** *PNAS,* 1992, vol. 89 (20), 9367-9371
- **Horwell DC.** *Trends Biotechnol.,* 1995, vol. 13 (4), 132-134
- **Berger ; Kimmel.** Guide to Molecular Cloning Techniques, Methods in Enzymology. Academic Press, 1987, vol. 152
- **Sambrook et al.** Molecular Cloning: A Laboratory Manual. Cold Spring Harbor Laboratory Press, 1989
- **Potrykus.** *Annu Rev Plant Physiol Plant Mol Biol,* 1991, vol. 42, 205-225
- **Christou.** *Agro-Food-Industry Hi-Tech,* March 1994, 17-27
- **Davis ; de Serres.** *Methods Enzymol,* 1971, vol. 17A, 79-143
- Vectors for genetic manipulation. **Turner G.** Aspergillus: 50 years on. Progress in industrial microbiology. Elsevier, 1994, vol. 29, 641-666
- **Punt et al.** *Trends Biotechnol,* May 2002, vol. 20 (5), 200-6
- **Archer ; Peberdy.** *Crit Rev Biotechnol,* 1997, vol. 17 (4), 273-306
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E Hinchcliffe ; E Kenny.** Yeasts. Academic Press Ltd, 1993, vol. 5
- **Hinnen et al.** *Proceedings of the National Academy of Sciences of the USA,* 1978, vol. 75, 1929
- **Beggs, J D.** *Nature,* 1978, vol. 275, 104
- **Ito, H et al.** *J Bacteriology,* 1983, vol. 153, 163-168
- *Curr. Opin Biotechnol.,* 1995, vol. 6 (5), 501-6
- **Upton C ; Buckley JT.** *Trends Biochem Sci,* 1995, vol. 20, 179-179
- **Kirk-Othmer.** Encyclopedia of Chemical Technology. John Wiley & Sons, Inc,
- *Biochimica et Biophysica Acta,* 1992, vol. 1124, 205-222
- Complex milk lipids account for about 1 % of the total milk fat and are mainly composed of phospholipids. Ullmann's Encyclopedia of Industrial ChemistryCopyright ©. Wiley-VCH Verlag GmbH & Co. KGaA, 2003
- Ullmann's Encyclopedia of Industrial Chemistry. Wiley-VCH Verlag GmbH & Co, 2003